# EUROPEAN PATENT APPLICATION

(11) **EP 1 302 538 A2**
(43) Date of publication of application: **16.04.2003**
(21) Application number: 02022322.8
(22) Date of filing: 08.10.2002
(51) Int. Cl.: C12N 9/04, C30B 29/58, C12Q 1/32

(54) **Three dimensional crystal structure of human sorbitol dehydrogenase and uses thereof**

(30) Priority: 15.10.2001 US 329396 P
(71) Applicant: Pfizer Products Inc., Groton, Connecticut 06340 (US)
(72) Inventor: Mylari, Banavara Lakshman, Waterford, Connecticut 06385 (US); Rath, Virginia Leigh, Kensington, California 94708 (US); Pauly, Thomas Adelbert, Richmond, California 94804 (US)
(74) Representative: Tesch, Rudolf

(57) **Abstract**

The present invention solves the three dimensional structure of human sorbitol dehydrogenase (hSDH) complexed with a ligand by X-ray crystallography. Atomic coordinates of hSDH derived from the analysis of high resolution X-ray diffraction patterns of crystals of SDH of the enzyme are provided, as well as methods for rational drug design, based on the structural data for hSDH and the binding mode of the ligand provided on computer readable media, as analyzed on a computer system having suitable computer algorithms. The binding mode of the required cofactor NADH and the nature of its interactions with the ligand are also provided.

## Description

### FIELD OF INVENTION

The present invention relates to the three-dimensional X-ray crystal structure of human sorbitol dehydrogenase (hSDH), the modeling of new structures, the binding mode of a SDH ligand, and the binding mode of the cofactor NADH, as well as methods for rational drug design based on the use of such data.

### BACKGROUND

The development of various diabetic complications is believed to be the result of increases of glucose entry into cells due to chronic hyperglycemia, activation of glucose metabolism through the polyol pathway, and accumulation of polyols (such as sorbitol). Sorbitol dehydrogenase (SDH), the second enzyme in the polyol pathway, oxidizes sorbitol to fructose and reduces NAD⁺ to NADH. Excess flux through SDH creates an imbalance in the cytoplasmic NAD⁺/NADH ratio and leads to a "pseudohypoxic" state that may be the determinant of pathology in diabetic tissues, thus making SDH a therapeutic target for the treatment of diabetic complications.

The amino acid and protein sequence for the human sorbitol dehydrogenase (hSDH) enzyme is available from Entrez Protein or GenBank, National Center for Biotechnology Information, National Library of Medicine, Bethesda, MD (Accession No. Q00796 or Accession No. U07361). The sequence and the procedures used to determine the sequence for SDH are described in Lee, F.K., et al., "The Human Sorbitol Dehydrogenase Gene: cDNA Cloning, Sequence Determination, and Mapping by Fluorescence *in situ* Hybridization," Genomics, **21**(2), 354-358 (1994).

U.S. Patent No. 5,728,704 discloses certain pyrimidine compounds that act as SDH ligands resulting in lowering fructose levels in the tissues of mammals affected by diabetes (e.g., nerve, kidney and retina tissue) and are therefore useful in the treatment and/or prevention of diabetic complications such as diabetic neuropathy, diabetic retinopathy, diabetic nephropathy, diabetic microangiopathy and diabetic macroangiopathy in mammals. A molecular modeling investigation of human sorbitol dehydrogenase complexed with the substrate sorbitol and the SDH inhibitor, 4-[4-(N,N-dimethylsulfamoyl)-piperazino]-2-hydroxymethylprimidine, was reported by D. Darmanin and O. EI-Kabbani in Bioorg. Med. Chem. Lett., **10**, 1101-1104 (2000). The model was based on the structures of human β₃ alcohol dehydrogenase, human α alcohol dehydrogenase and horse liver alcohol dehydrogenase. The tertiary structure of human β₃ alcohol dehydrogenase was used as the template for the construction of the model.

The crystal structure of rat sorbitol dehydrogenase complexed with NAD⁺ has been determined to 3.1 angstroms resolution (Johansson, K. et al., (2001) Chemico-Biological Interactions 130-132 (1-2): 351-358). The coordinates are not publicly available and therefore no direct comparison between the rat and human enzyme is possible. Based on the images in the publication, rat SDH appears to have the same overall quaternary and tertiary structure as human SDH, although, without the three dimensional coordinates, a detailed analysis is not possible.

### SUMMARY

The present invention provides a crystal form of human sorbitol dehydrogenase, or a subunit thereof, wherein the crystal form is defined as a crystal of space group P6₂ and cell constants having the values a =134.814 Å ± 15%, b =134.814 Å ± 15%, c = 225.184 Å ± 15%, α = 90.0°, β = 90.0°, and γ =120.0°. The crystal form may optionally contain NADH, a zinc ion, or a ligand that binds to the hSDH or a subunit thereof.

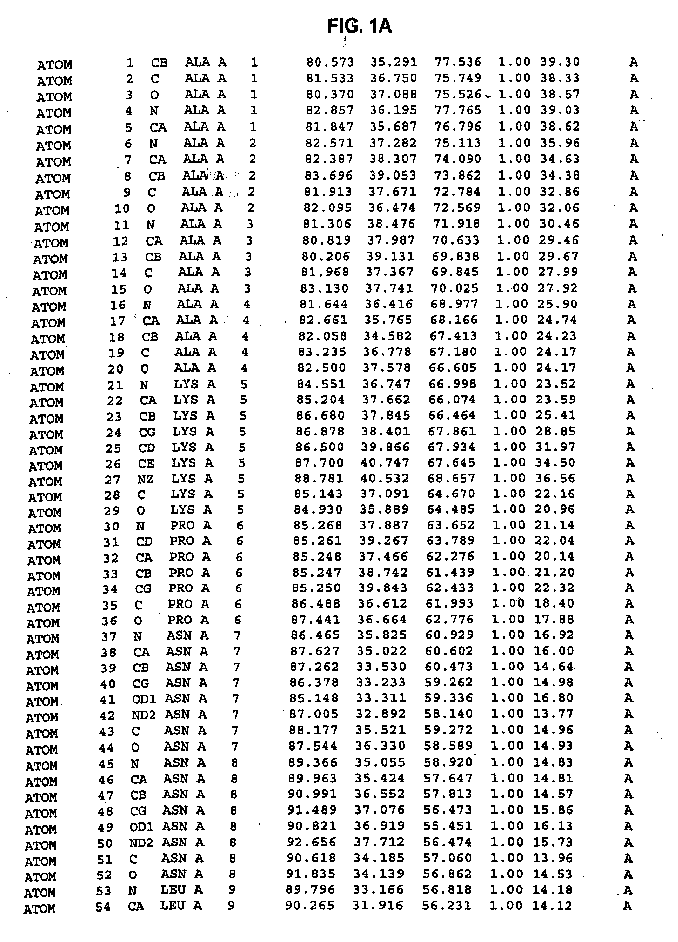

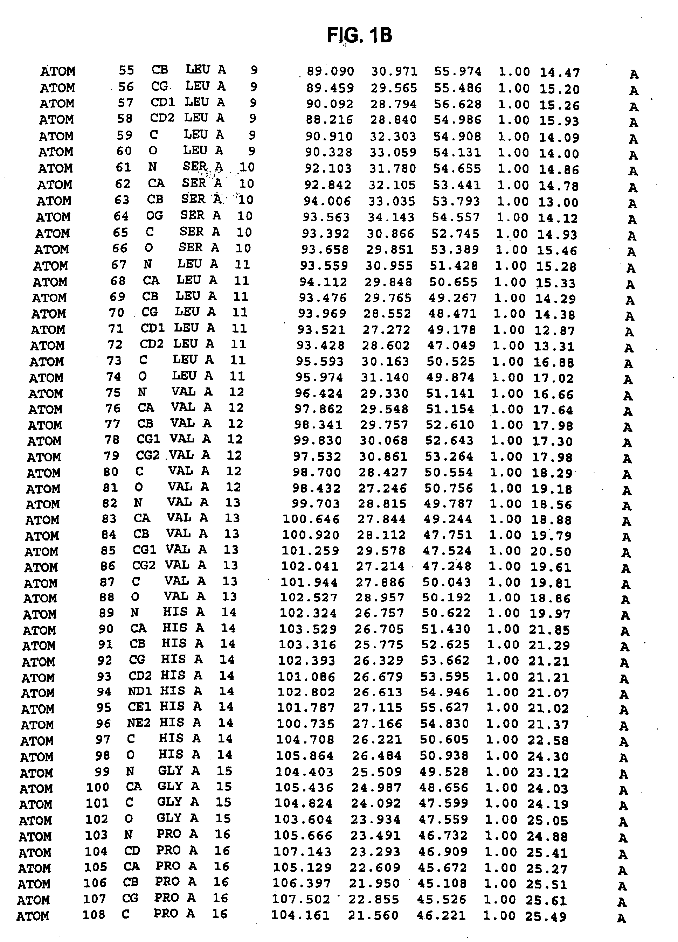

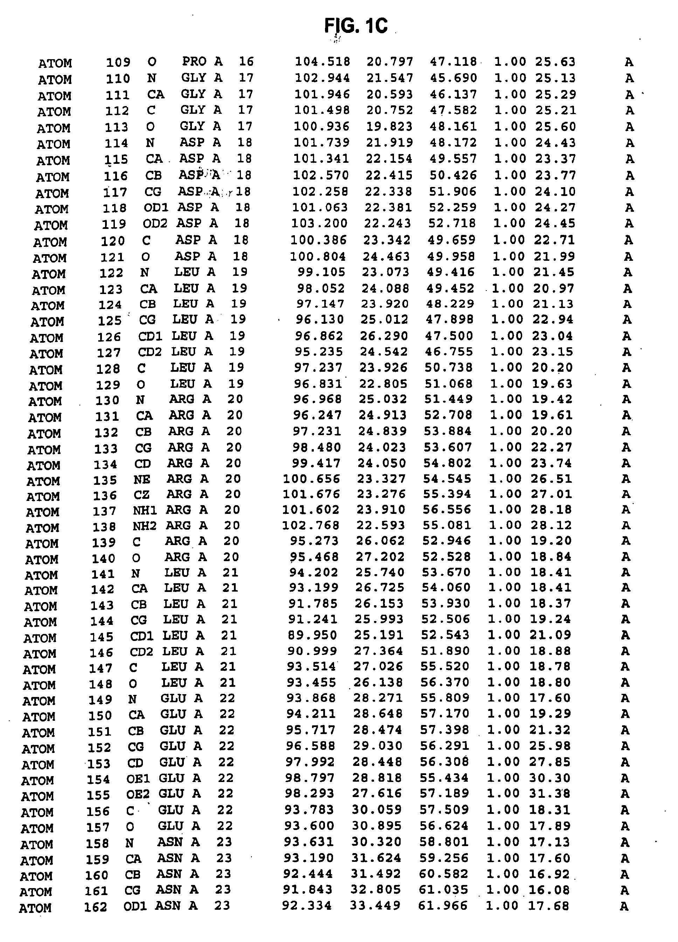

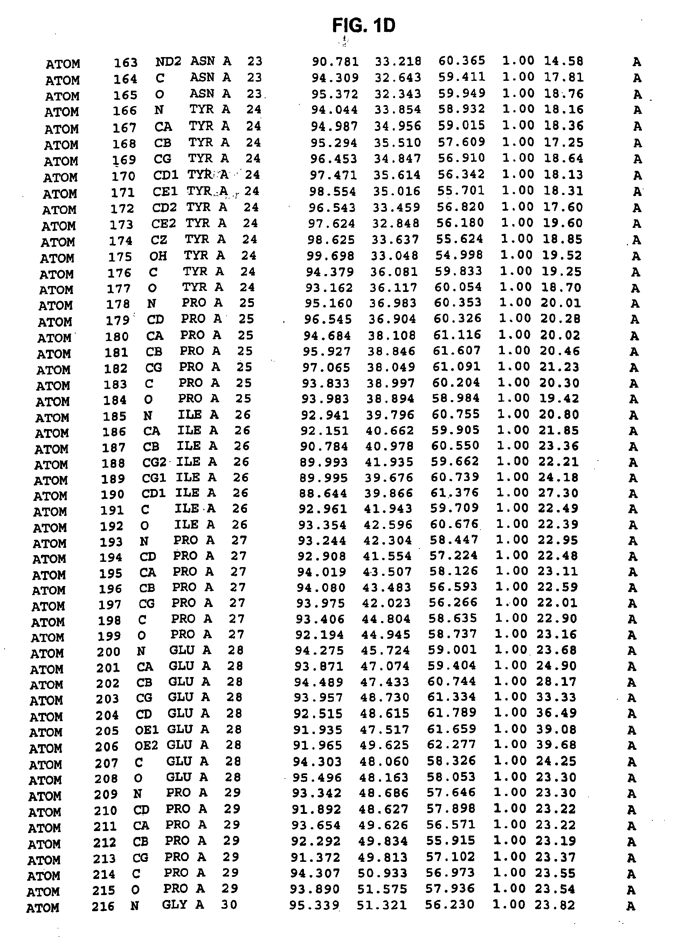

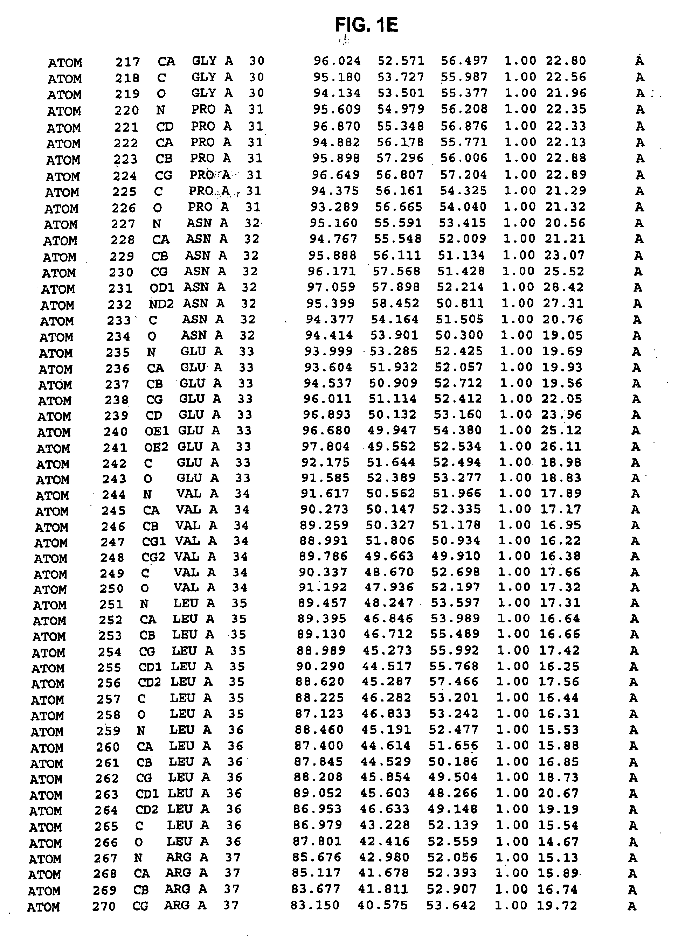

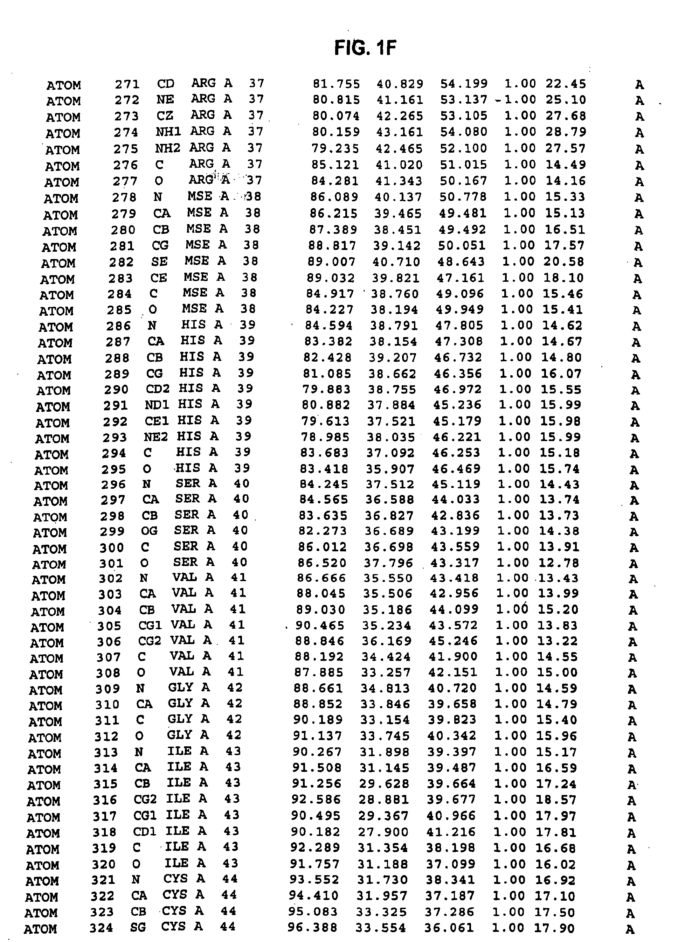

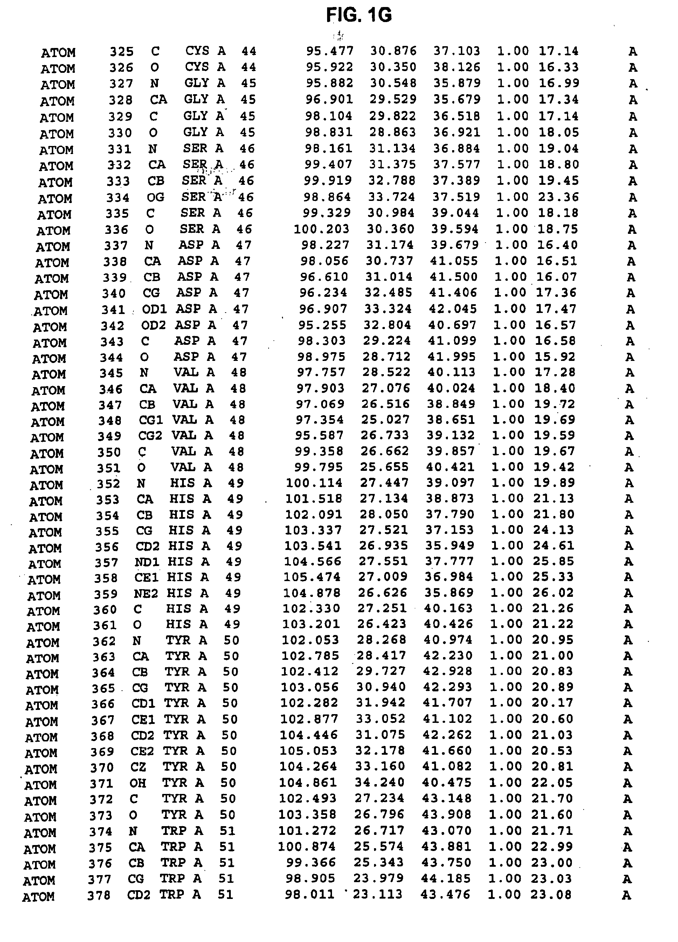

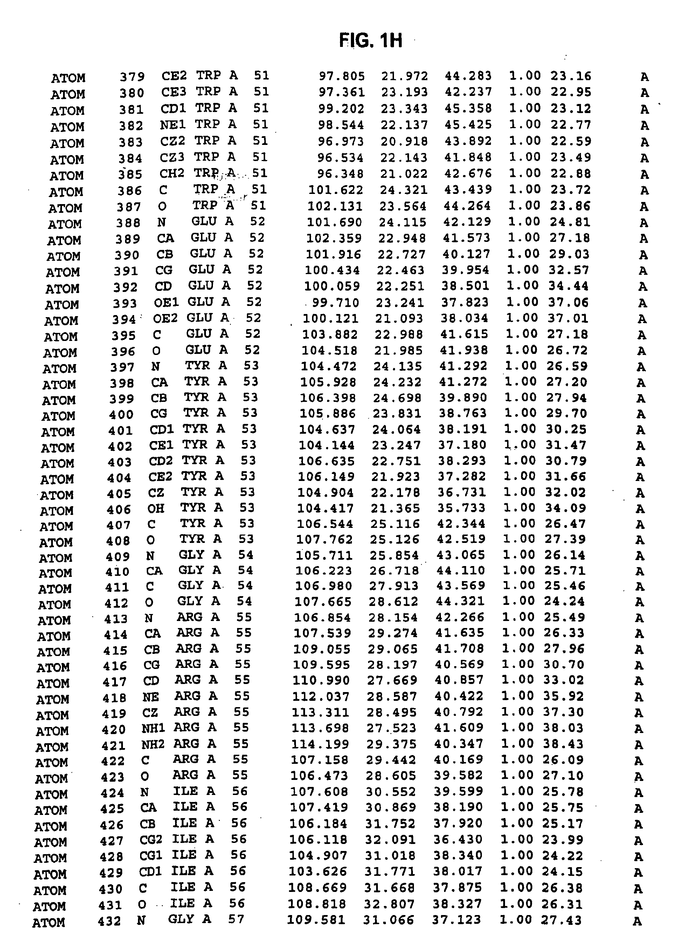

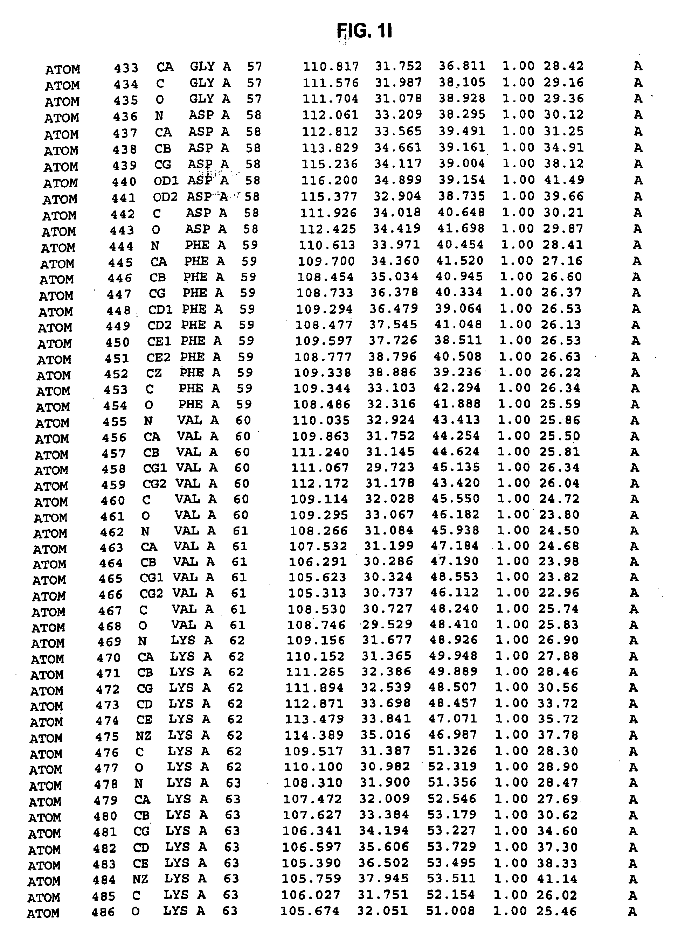

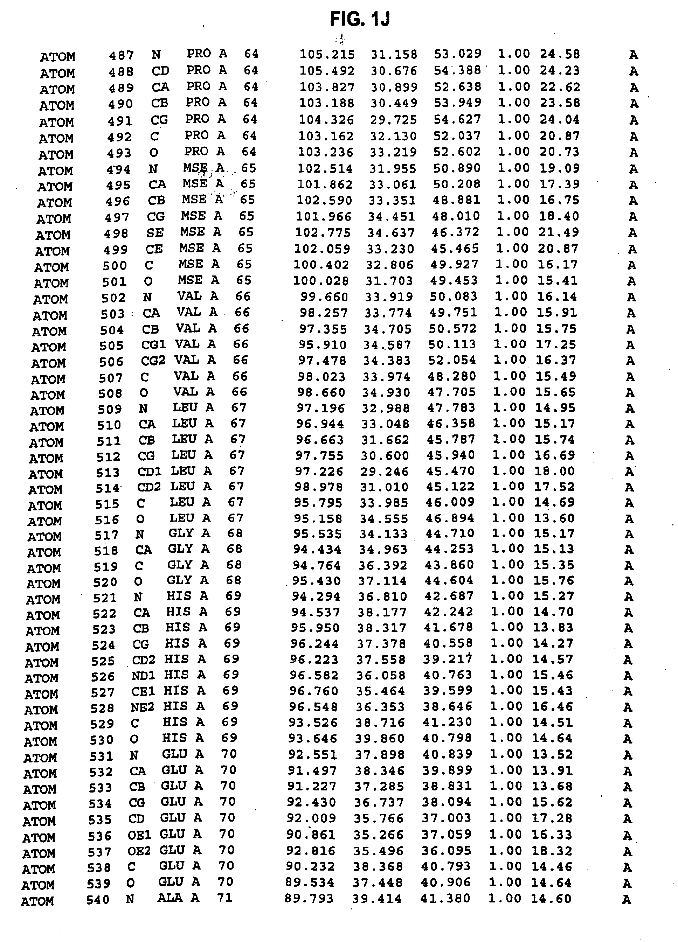

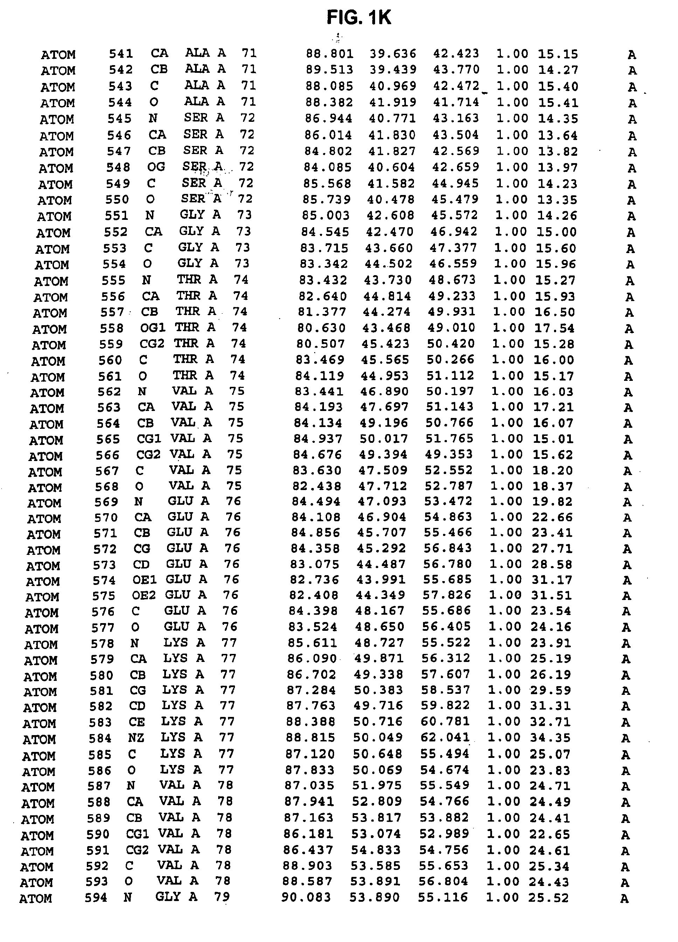

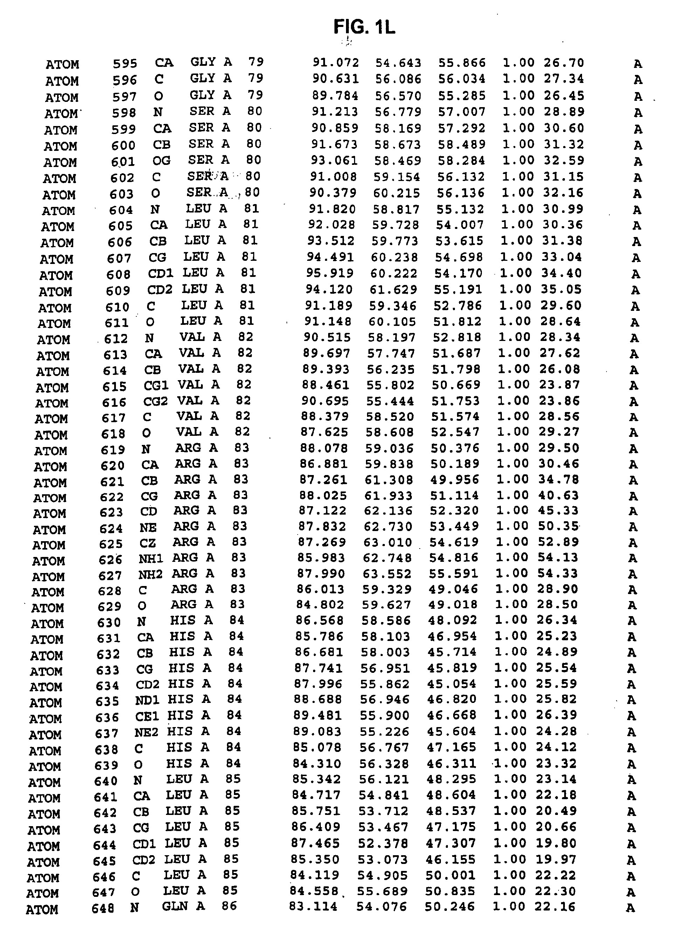

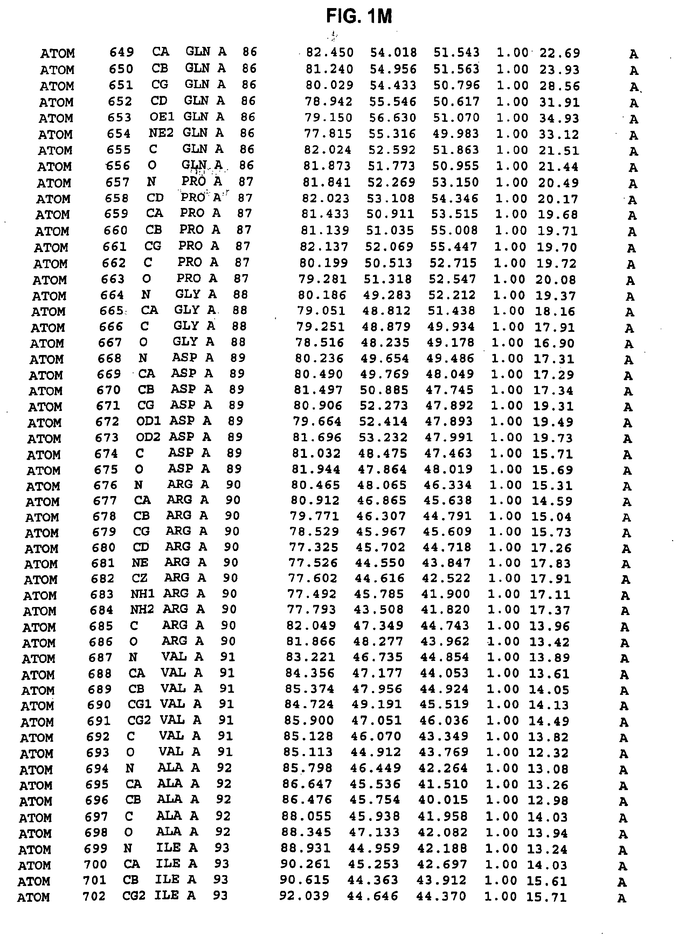

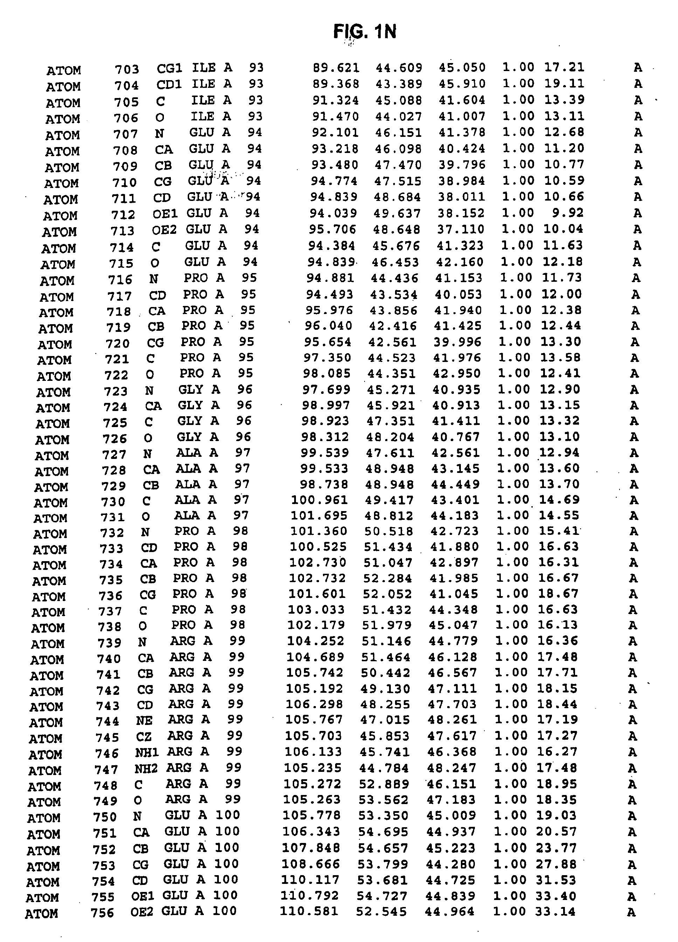

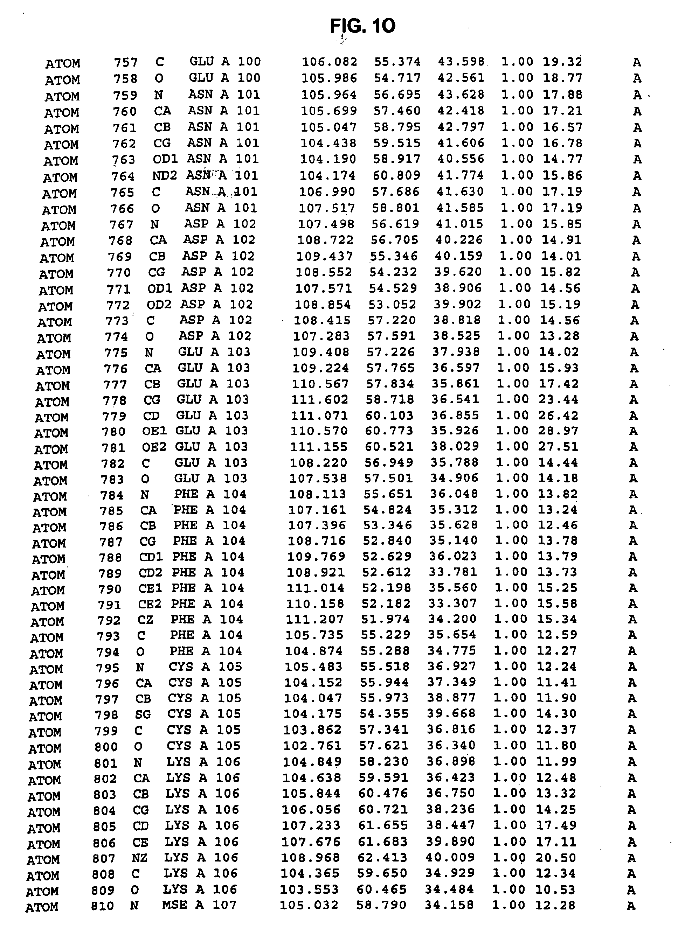

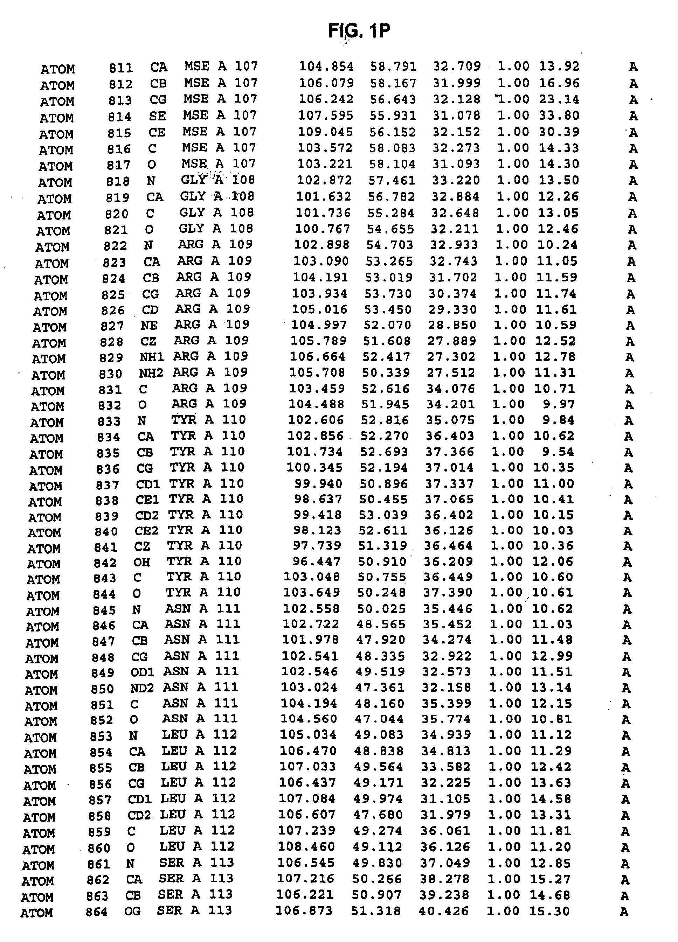

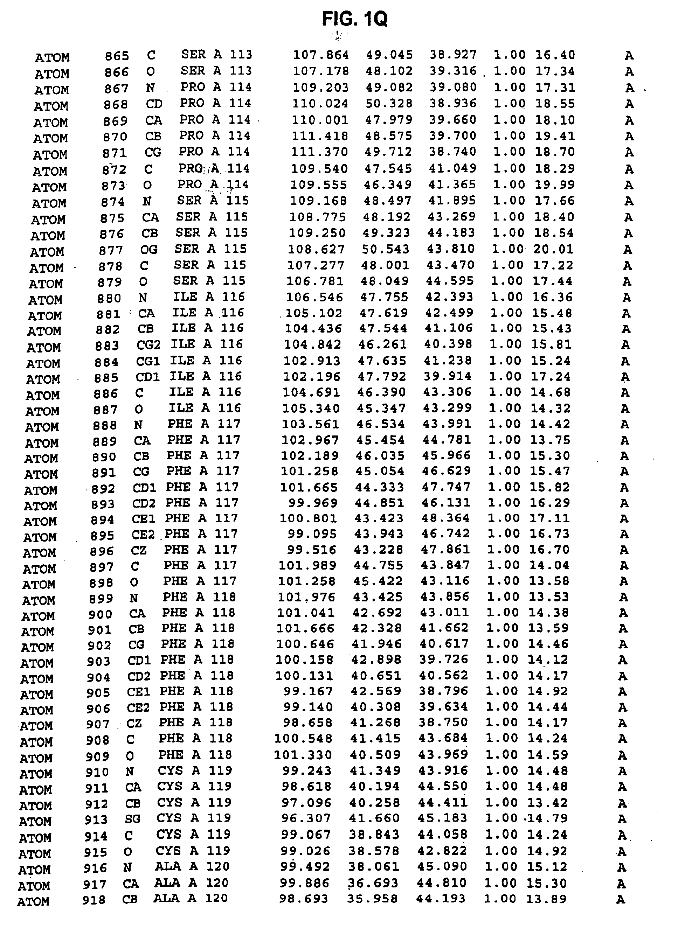

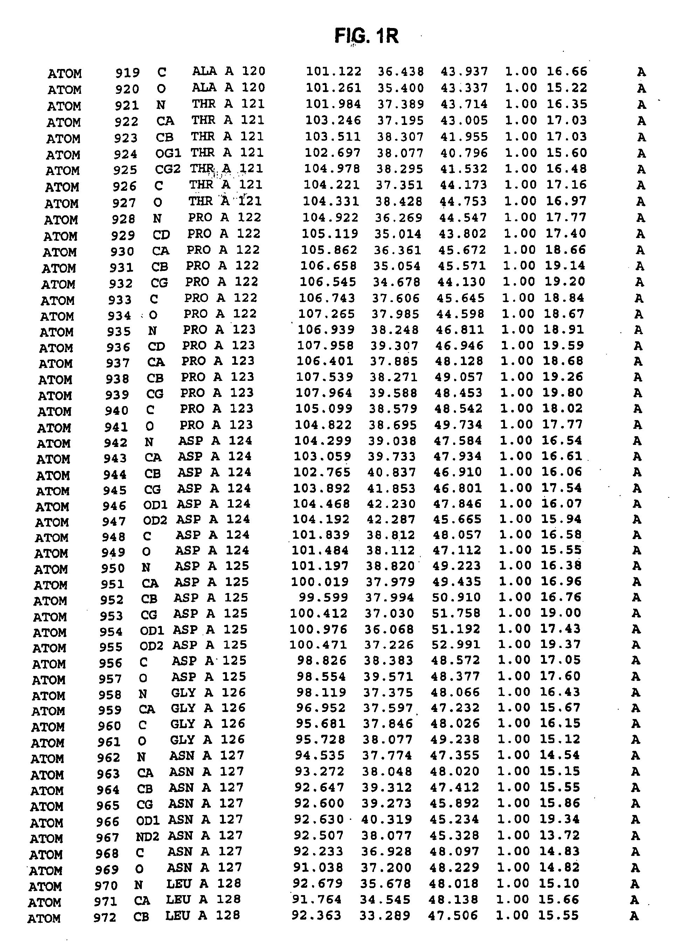

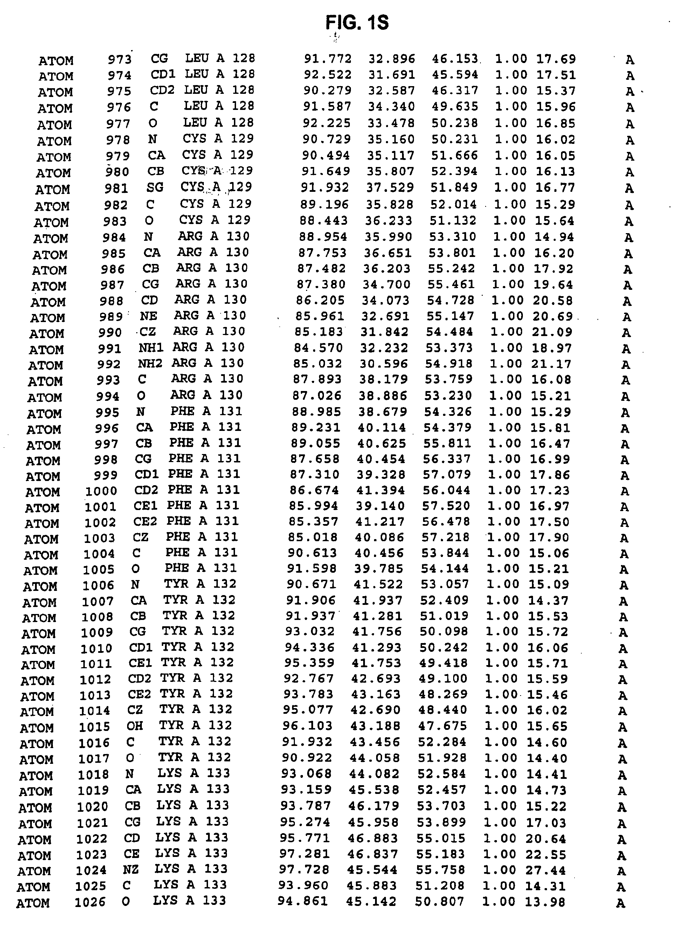

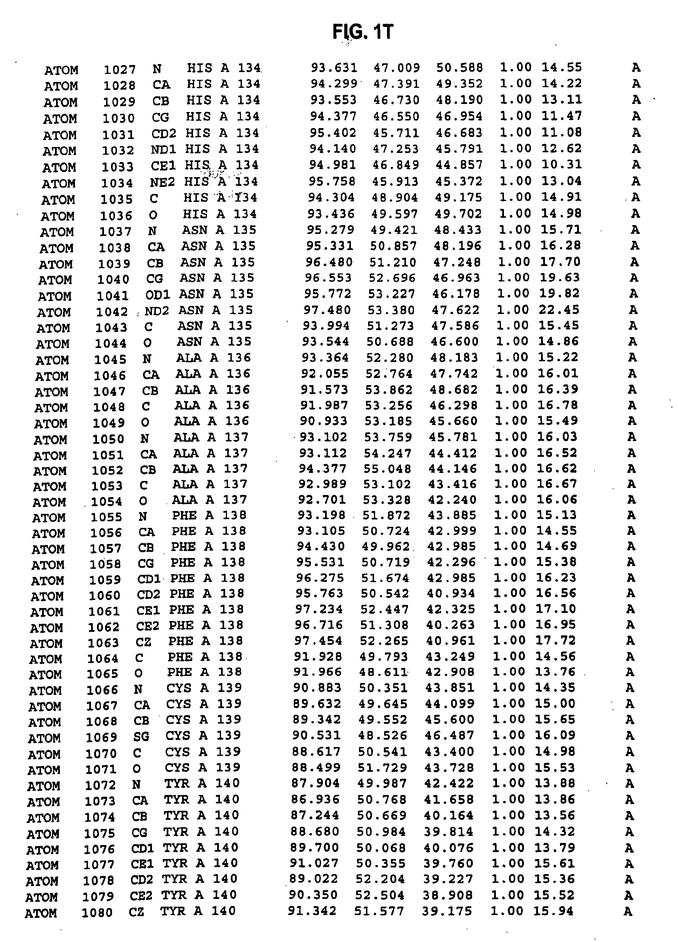

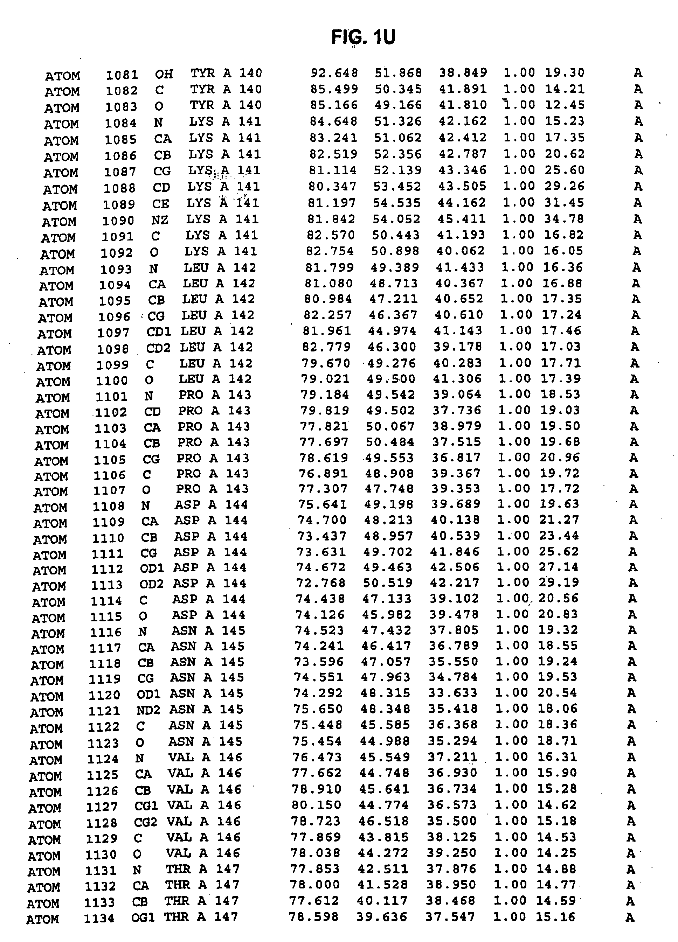

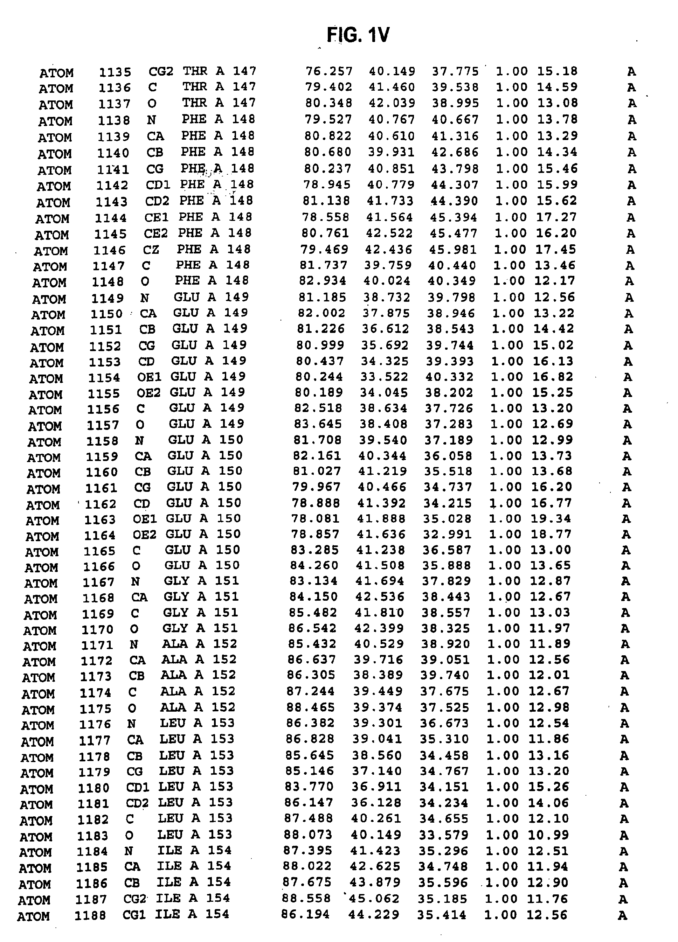

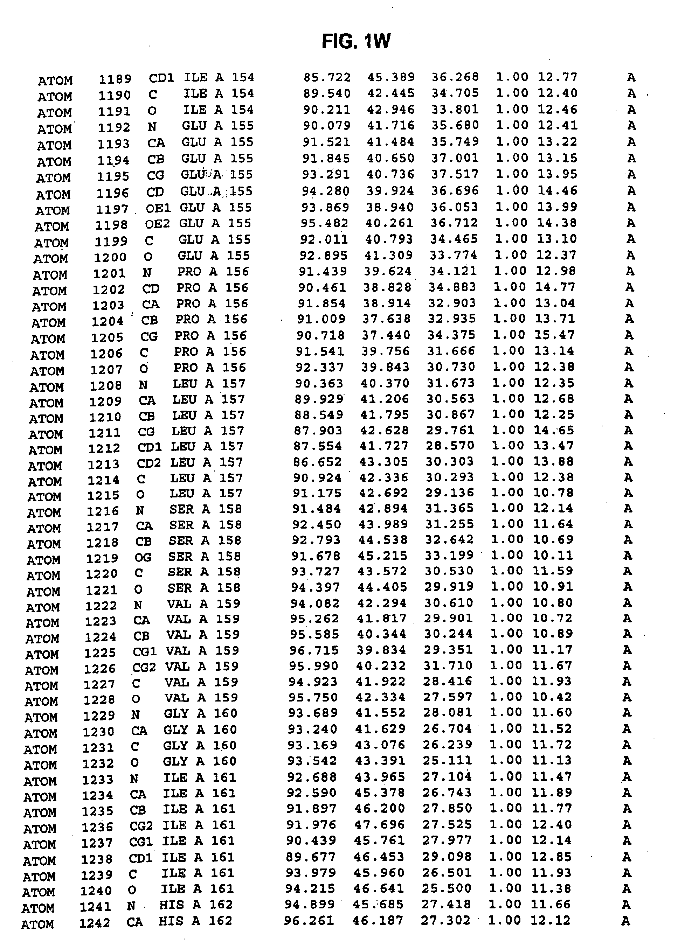

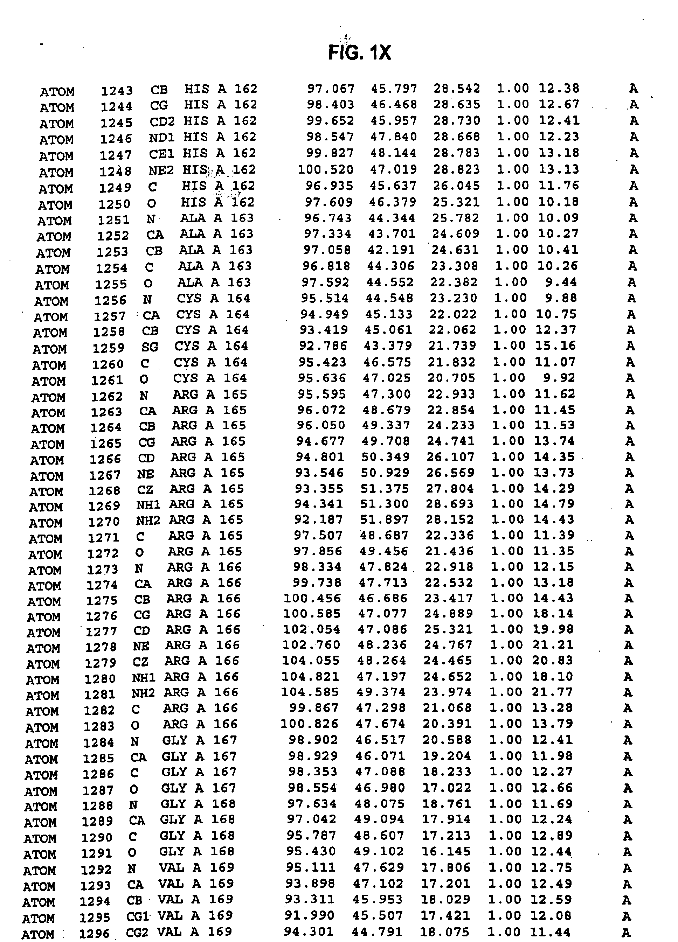

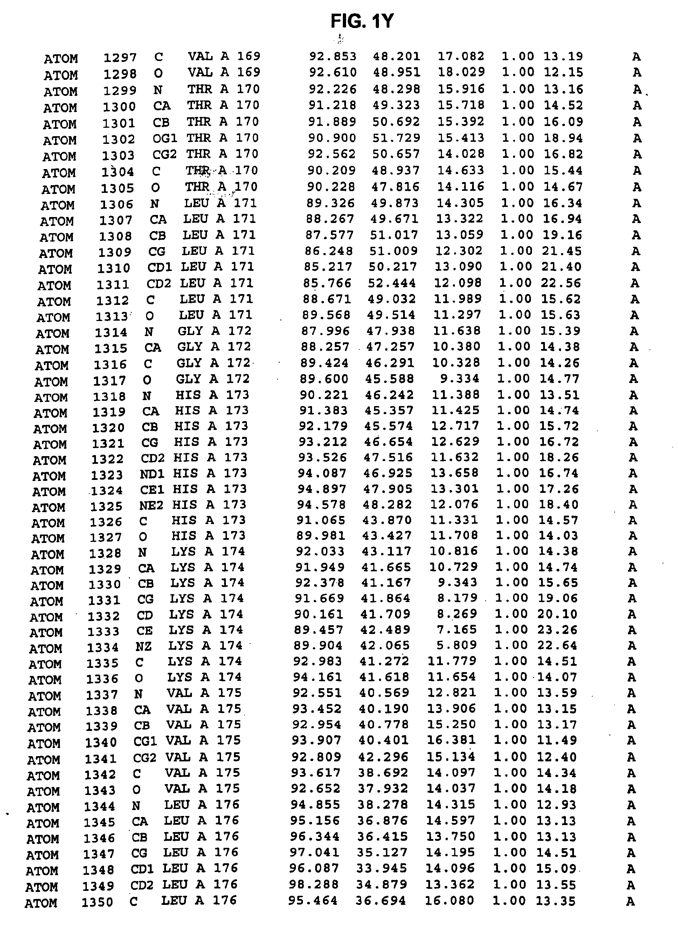

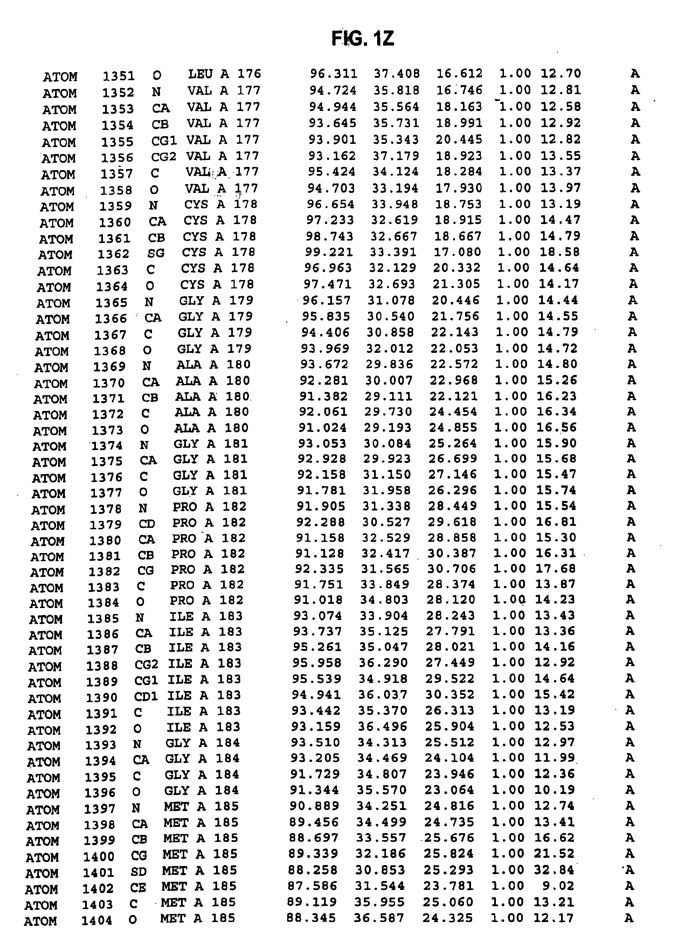

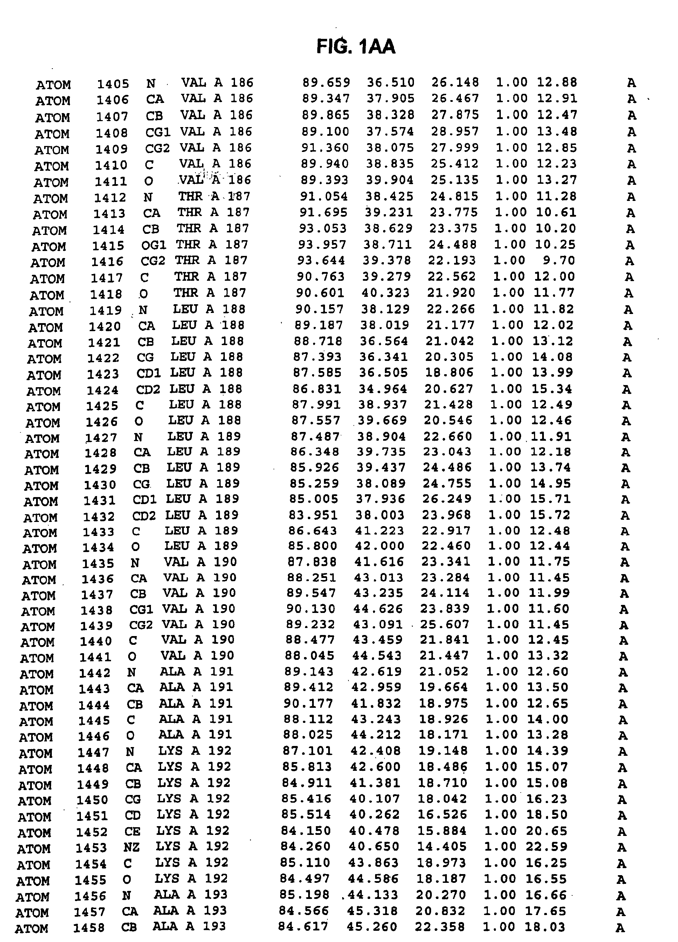

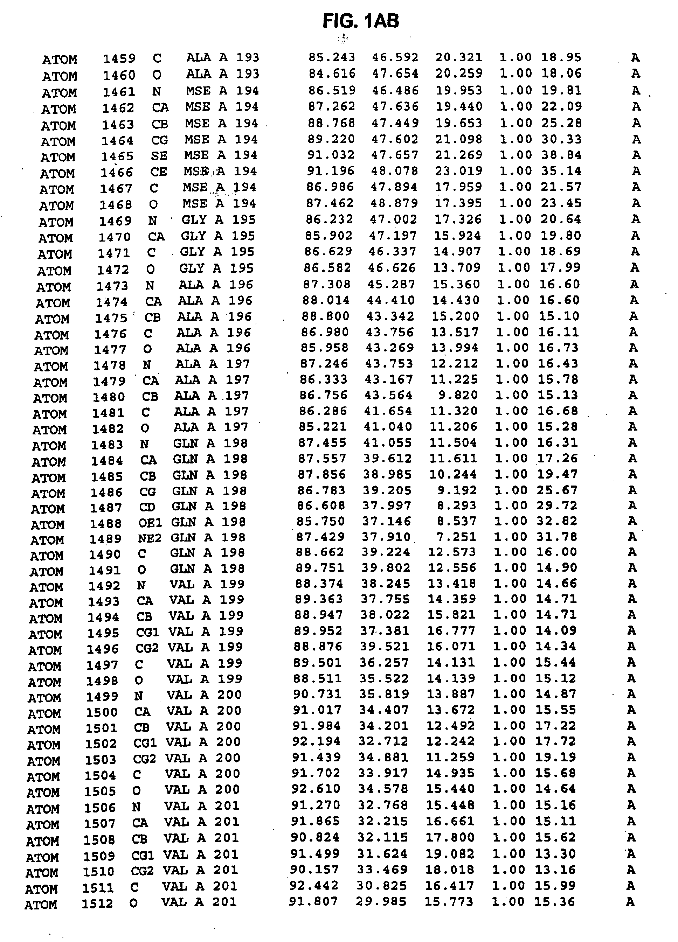

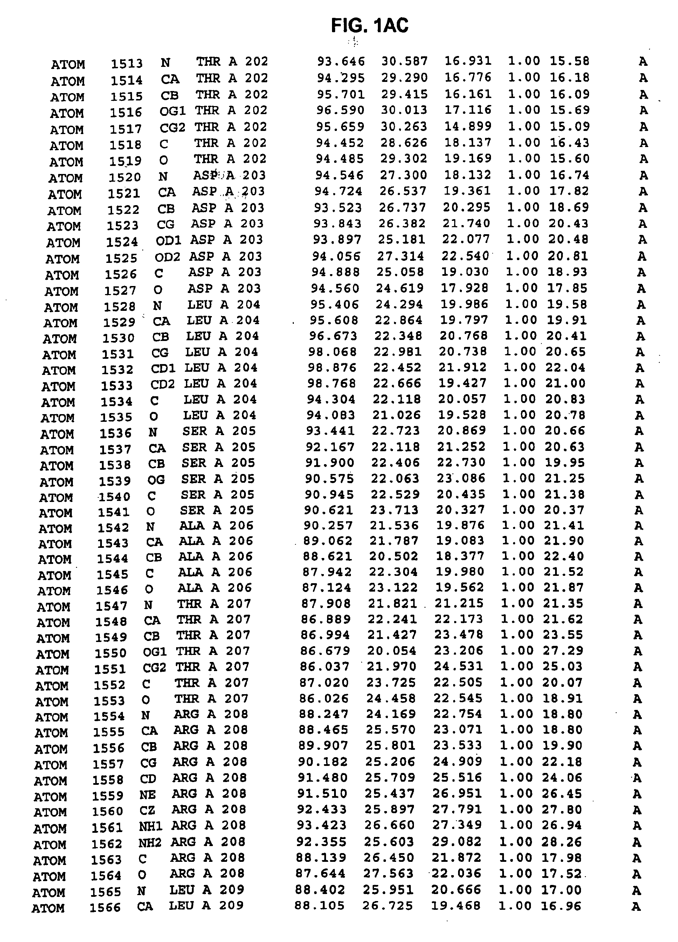

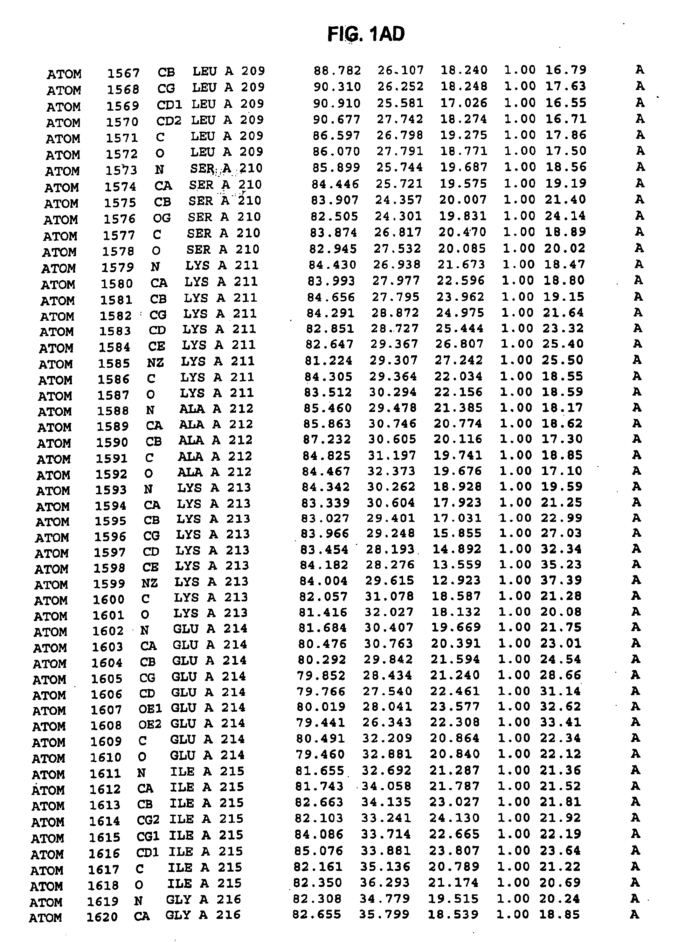

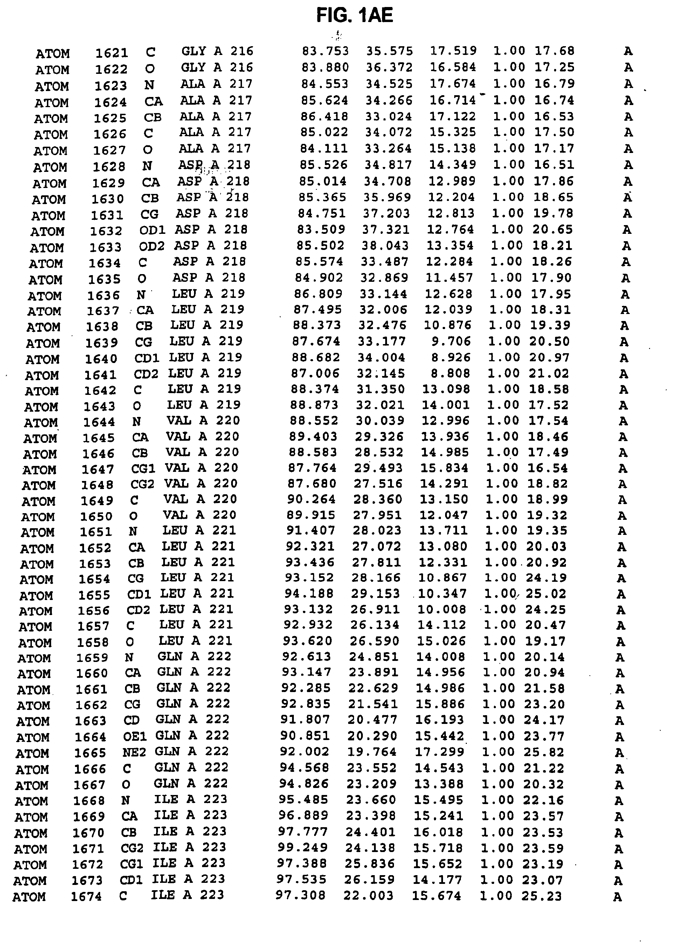

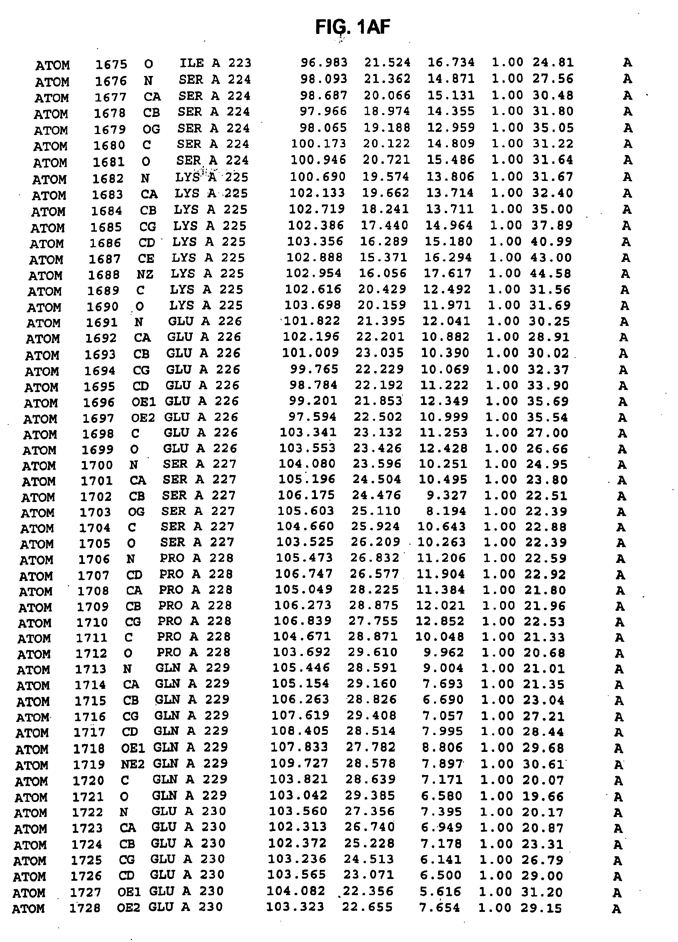

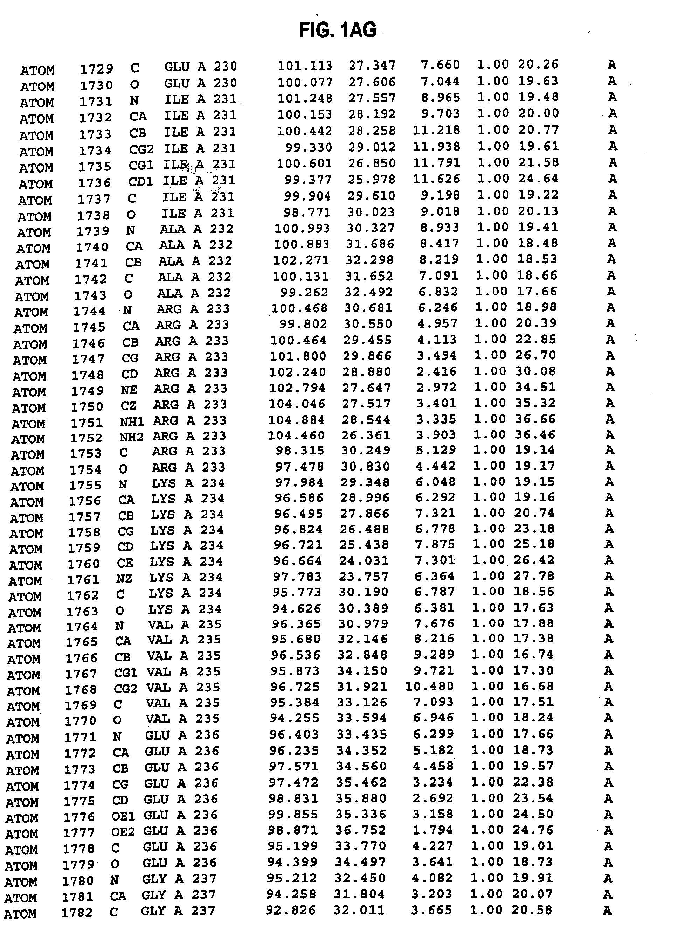

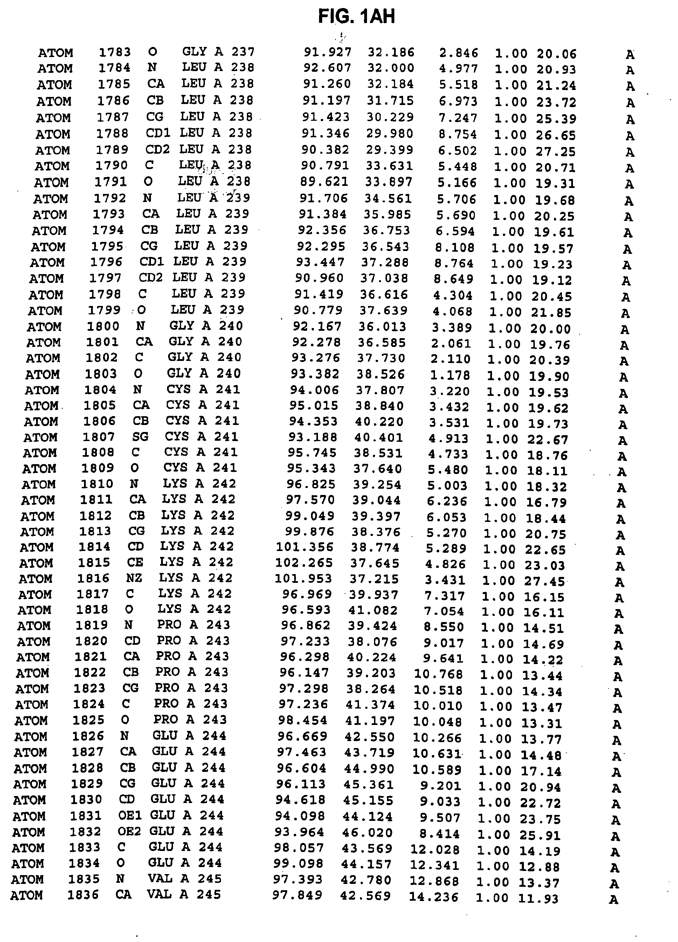

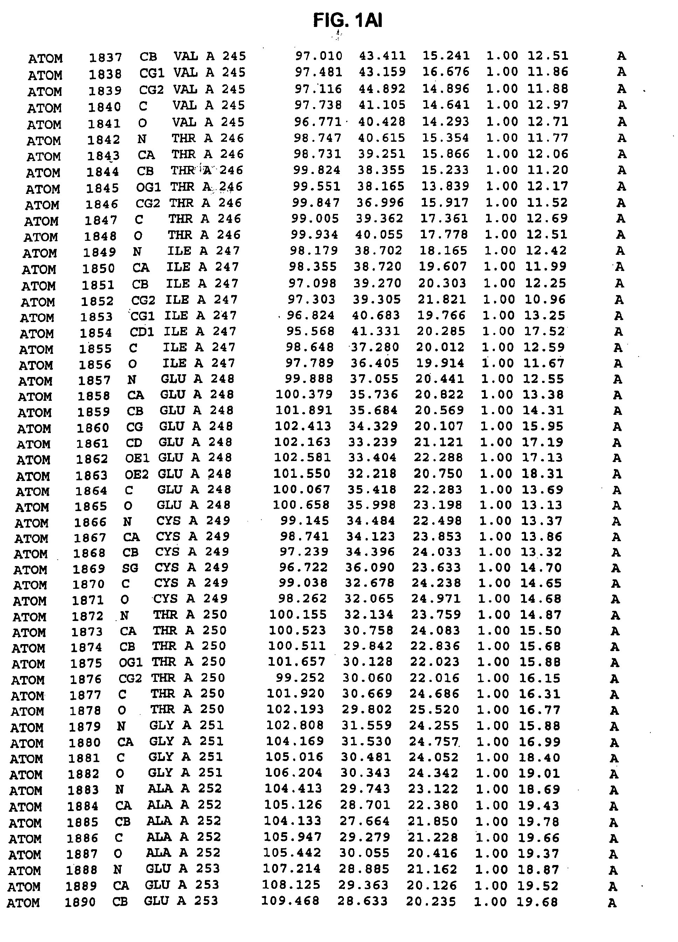

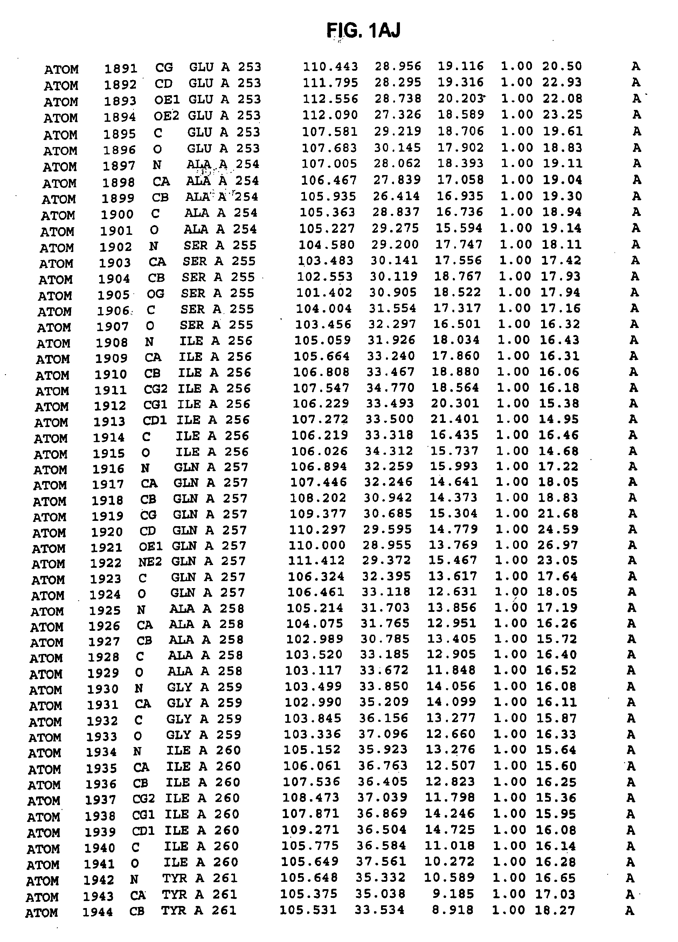

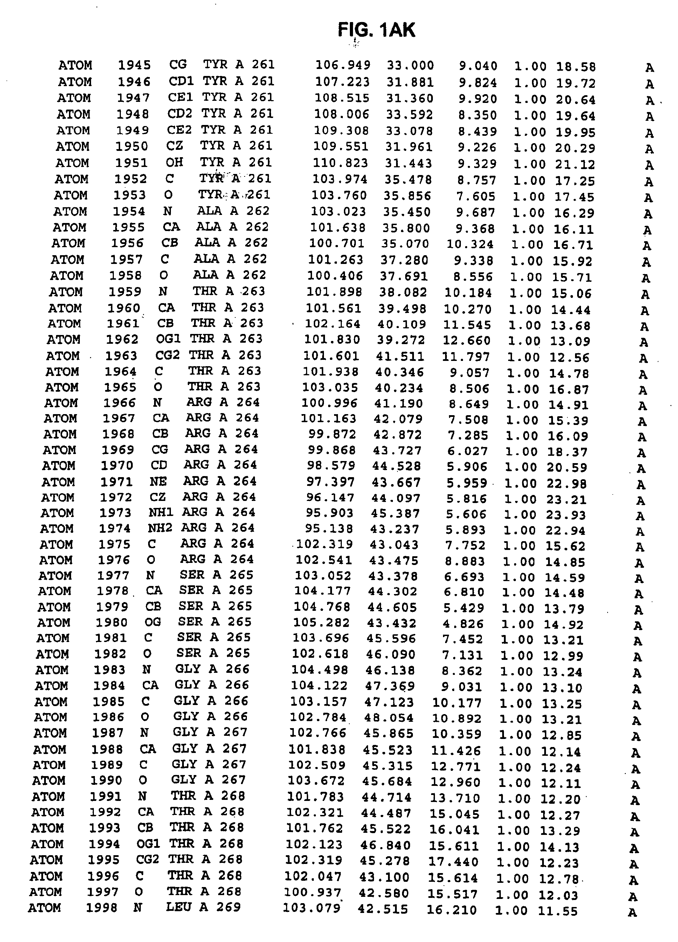

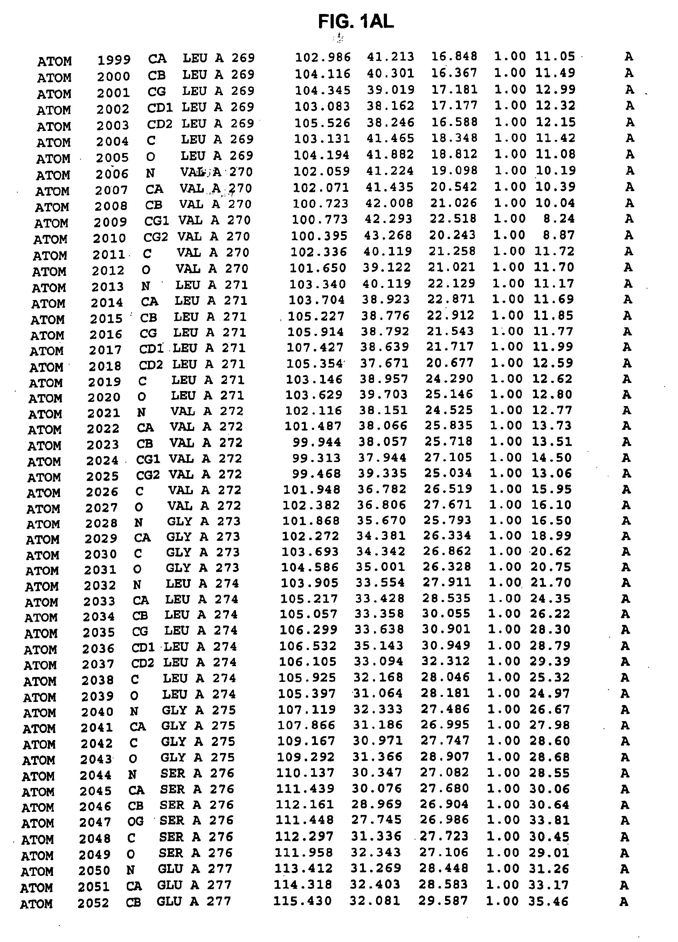

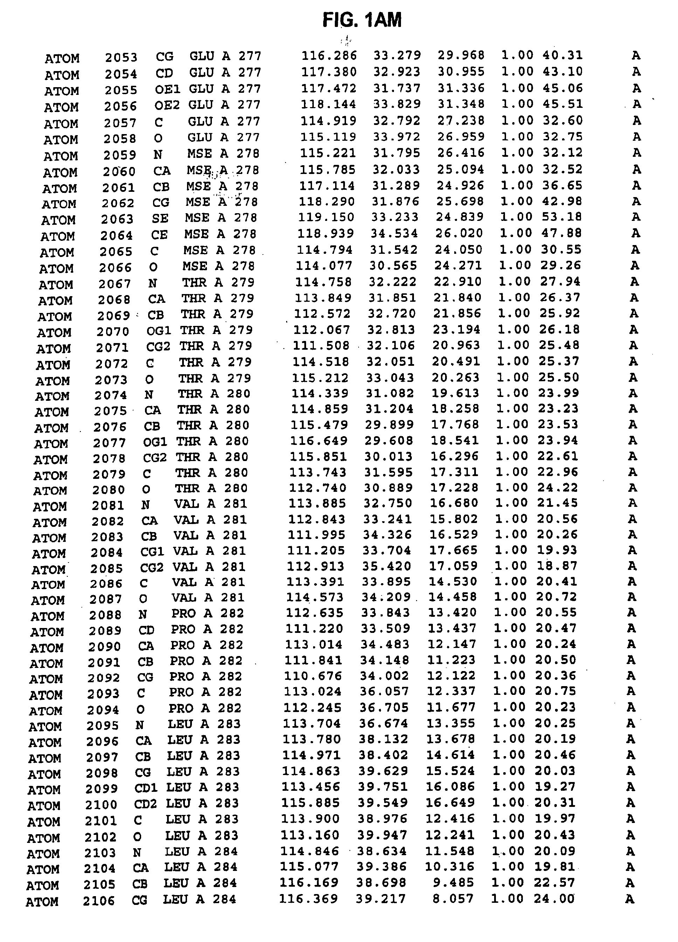

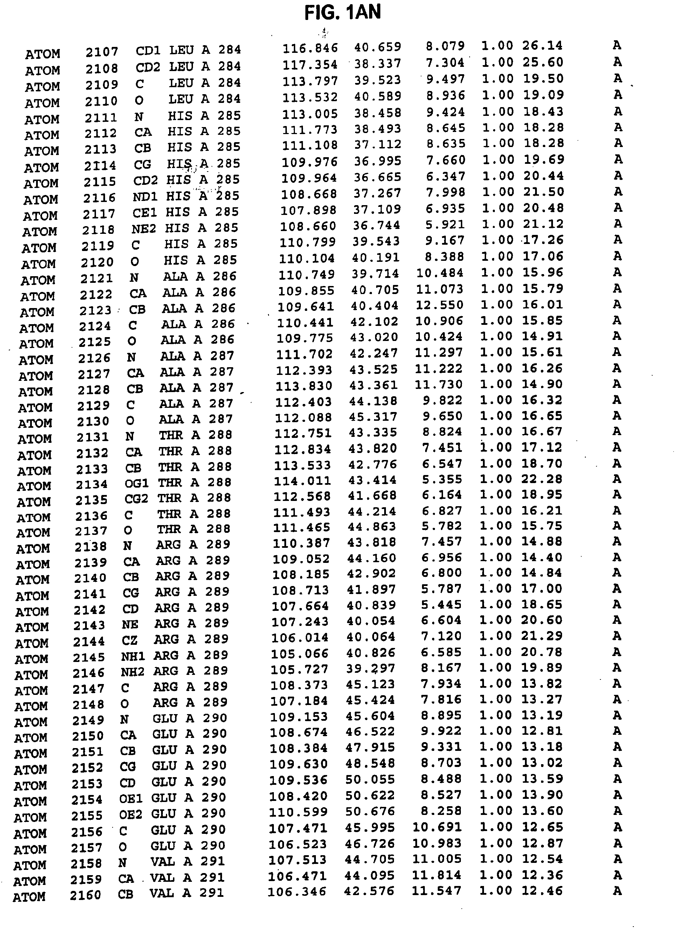

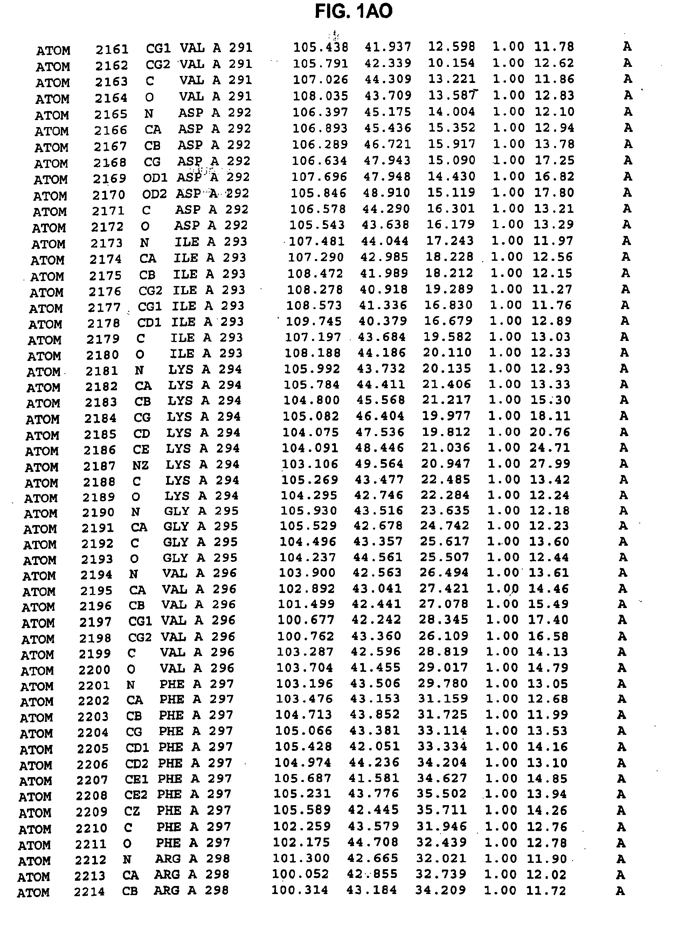

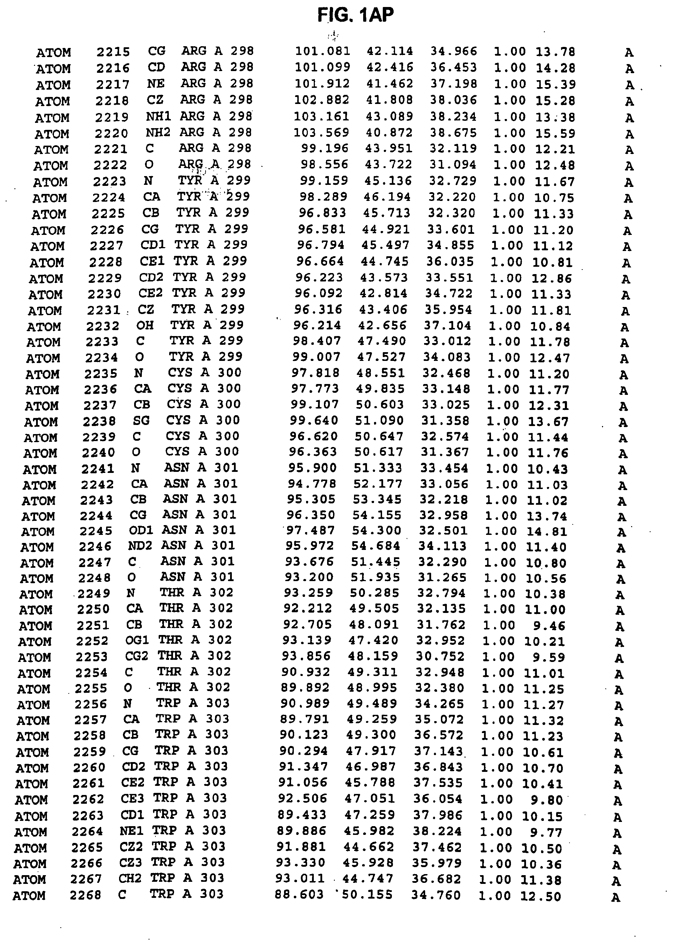

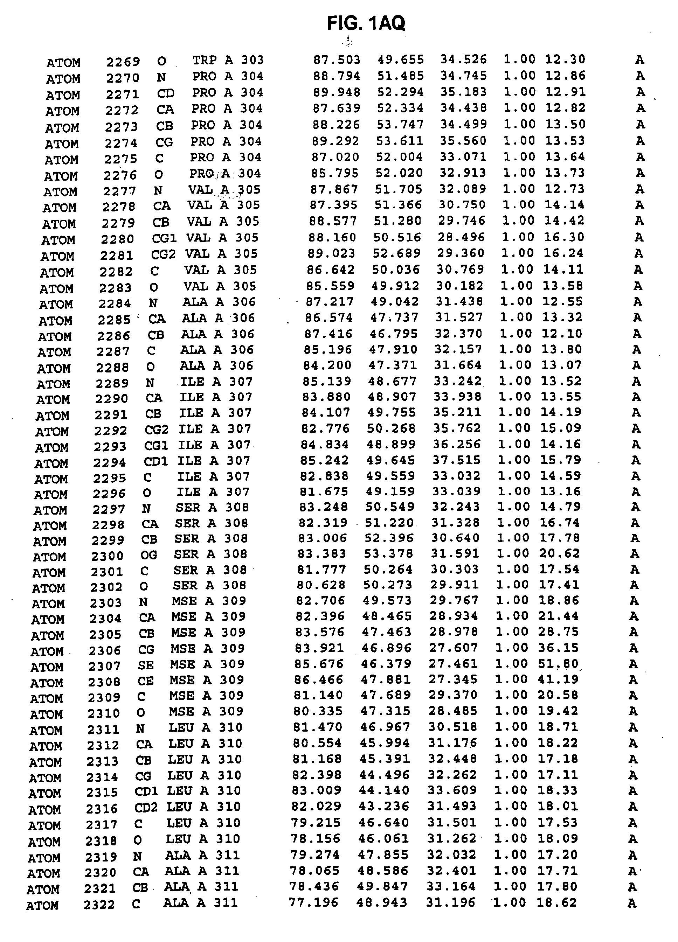

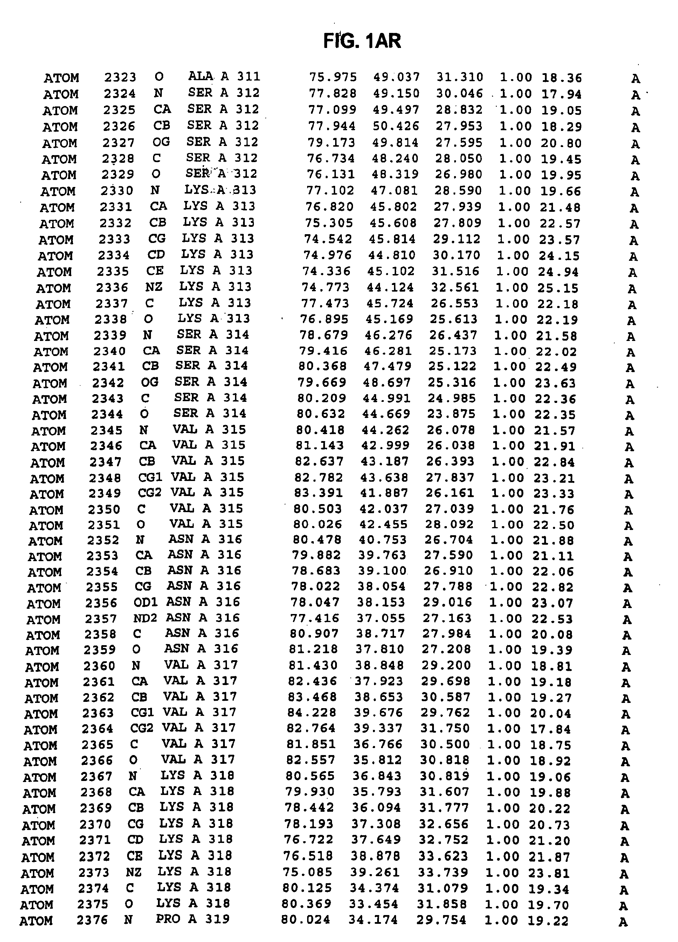

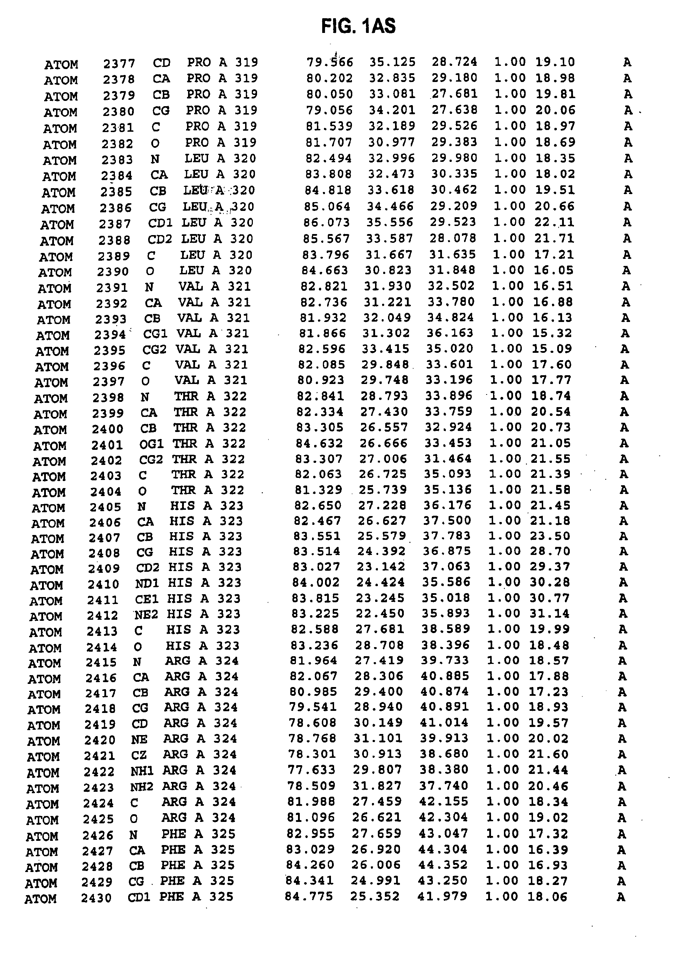

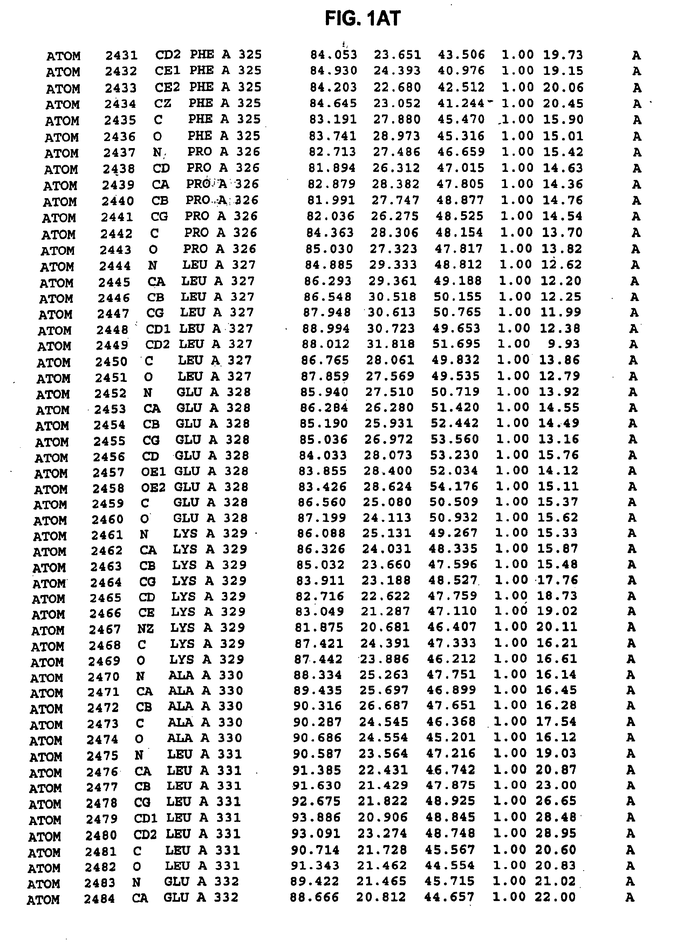

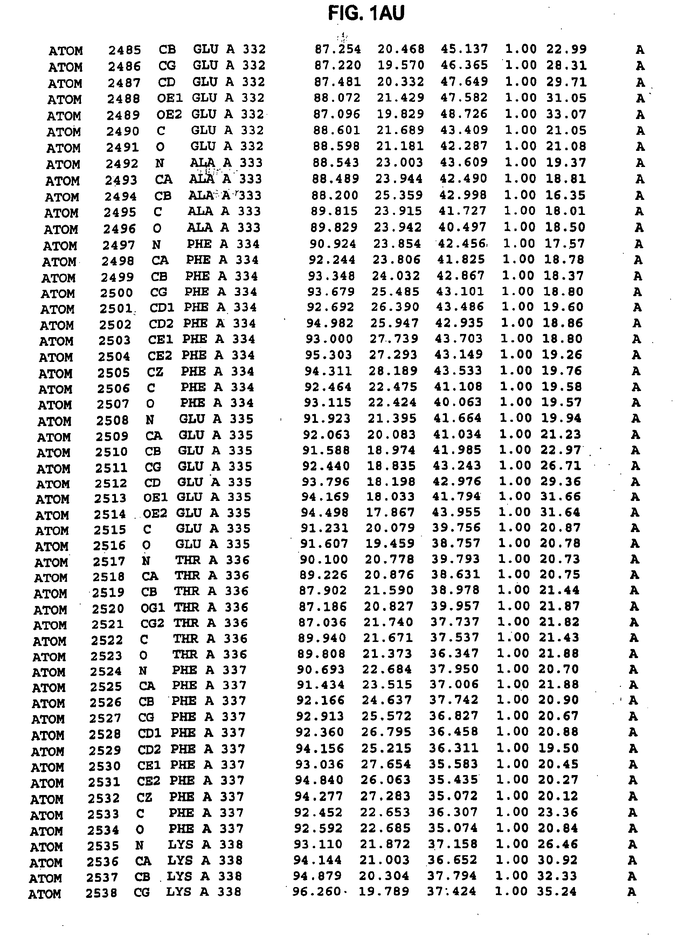

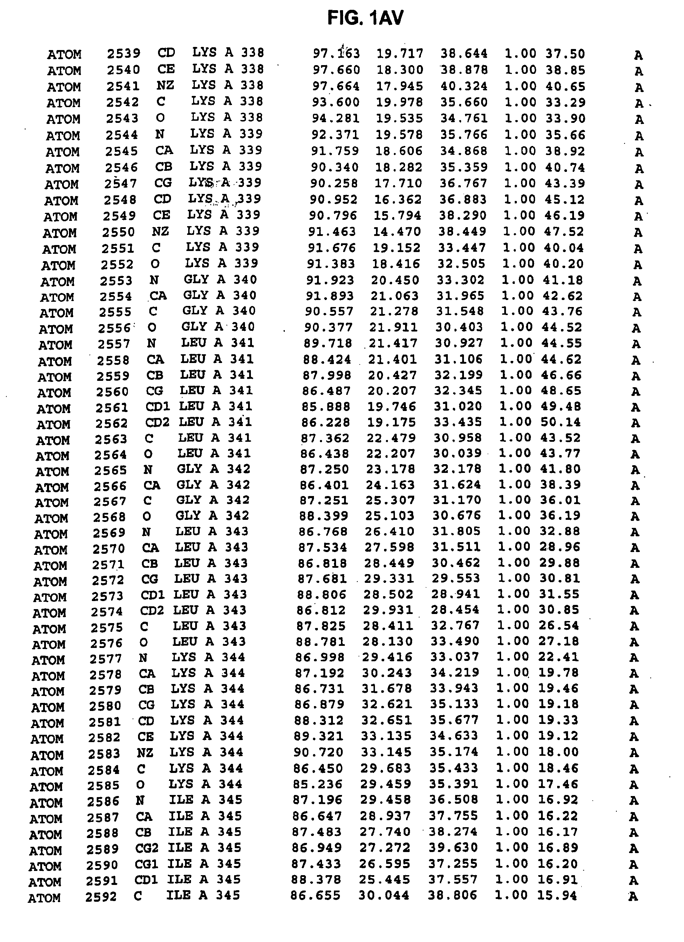

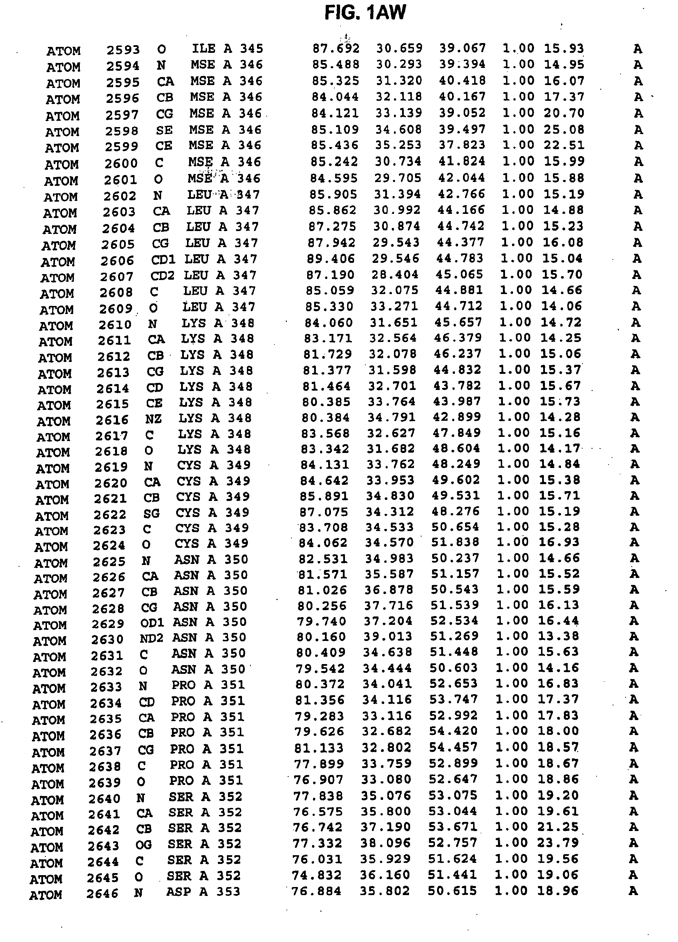

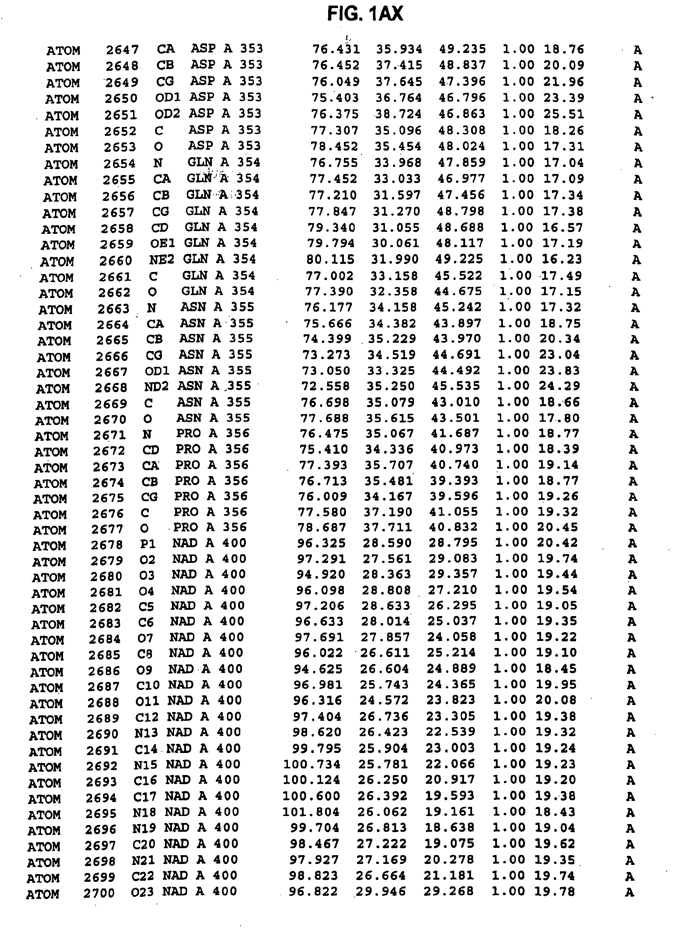

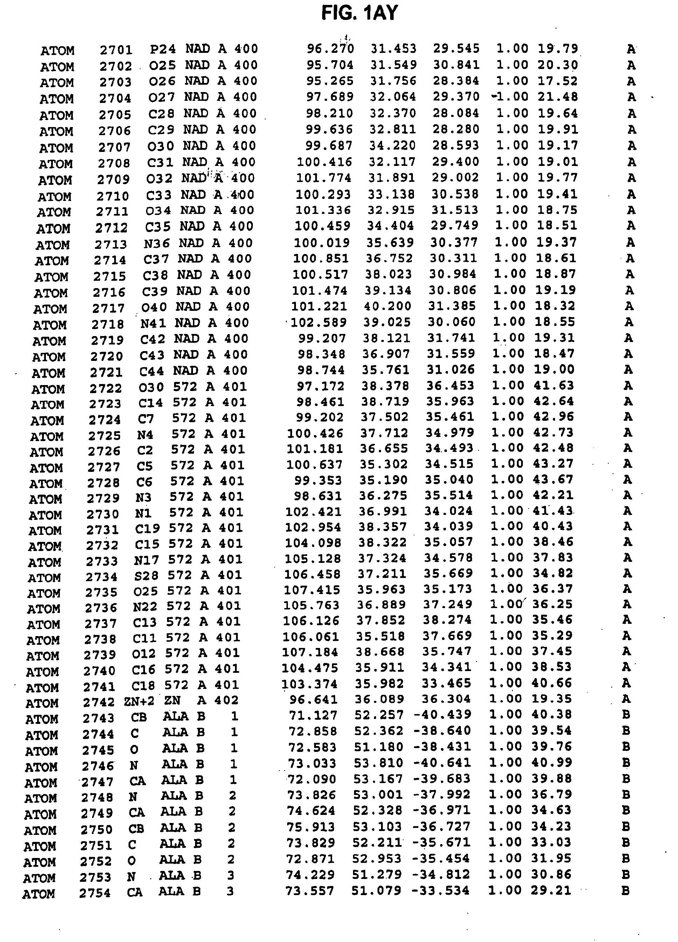

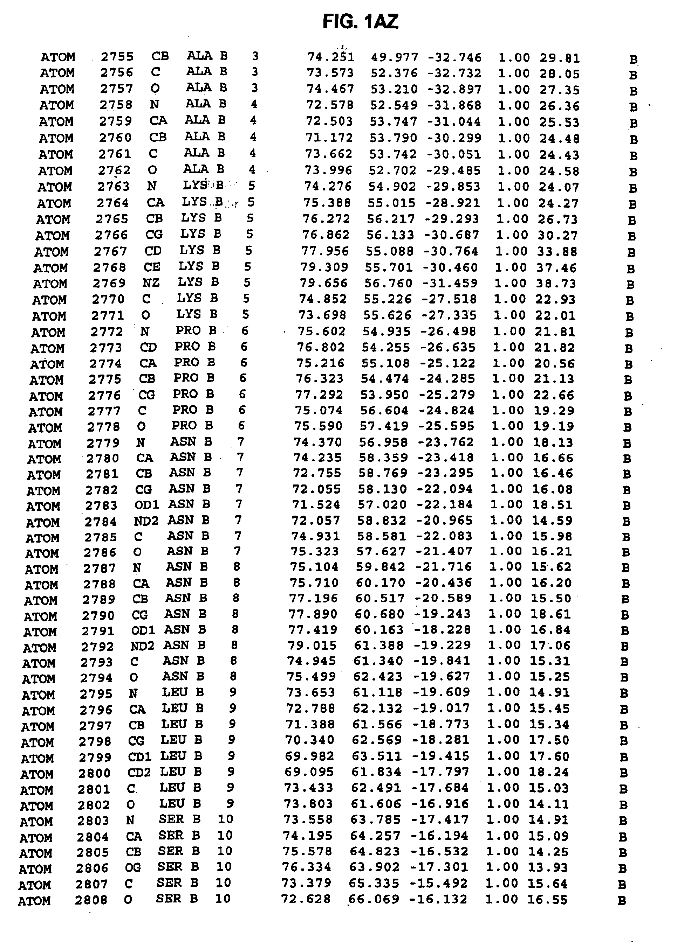

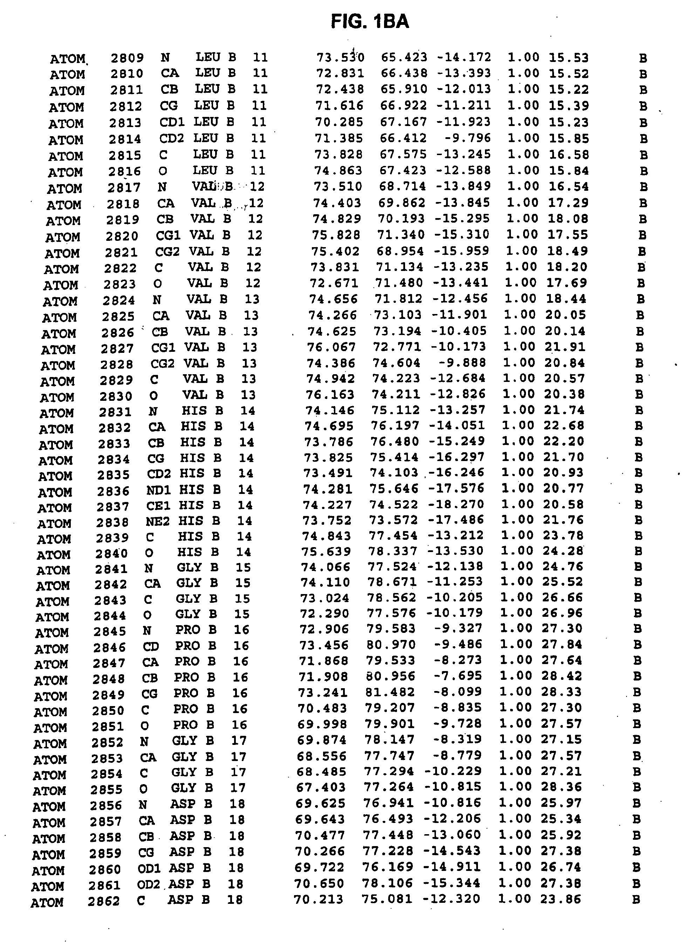

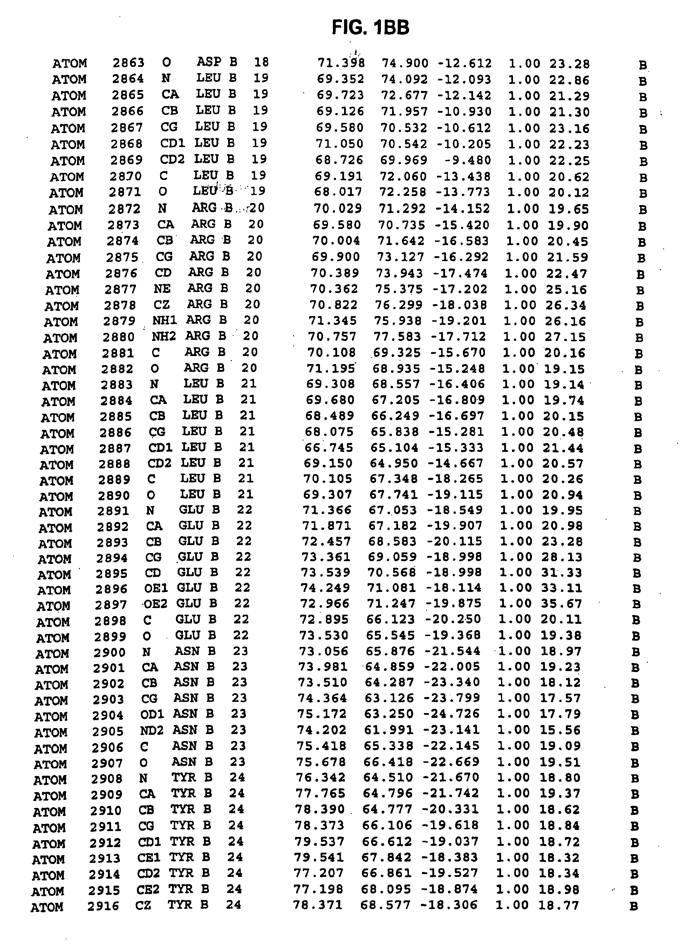

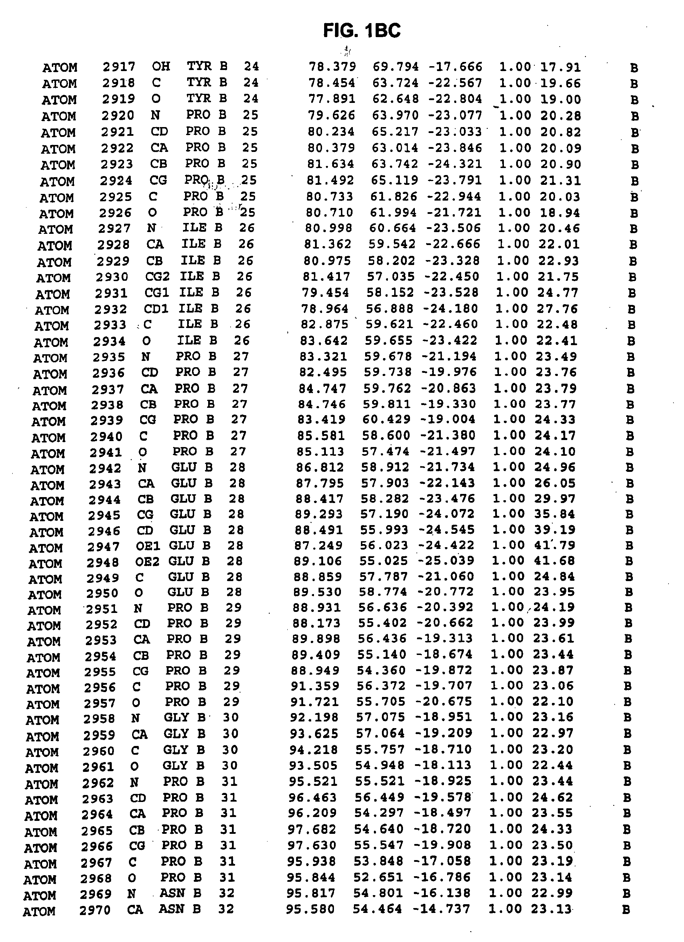

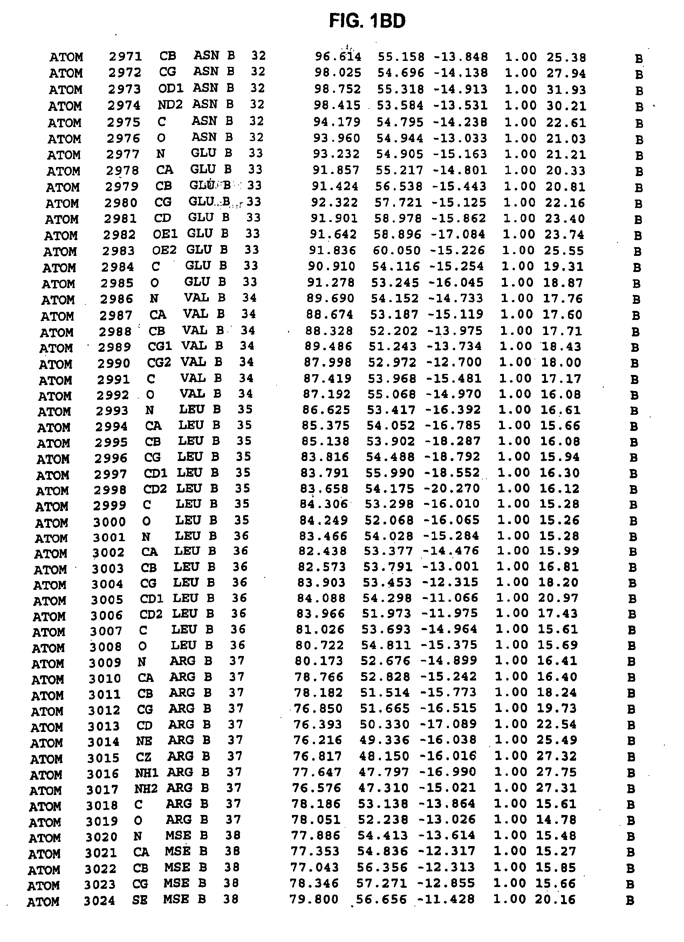

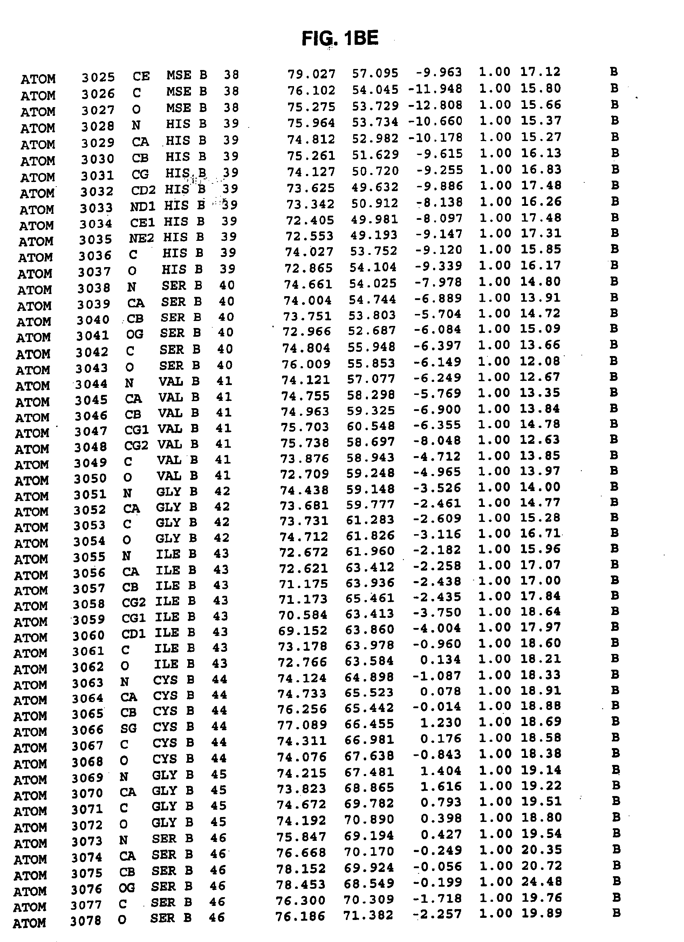

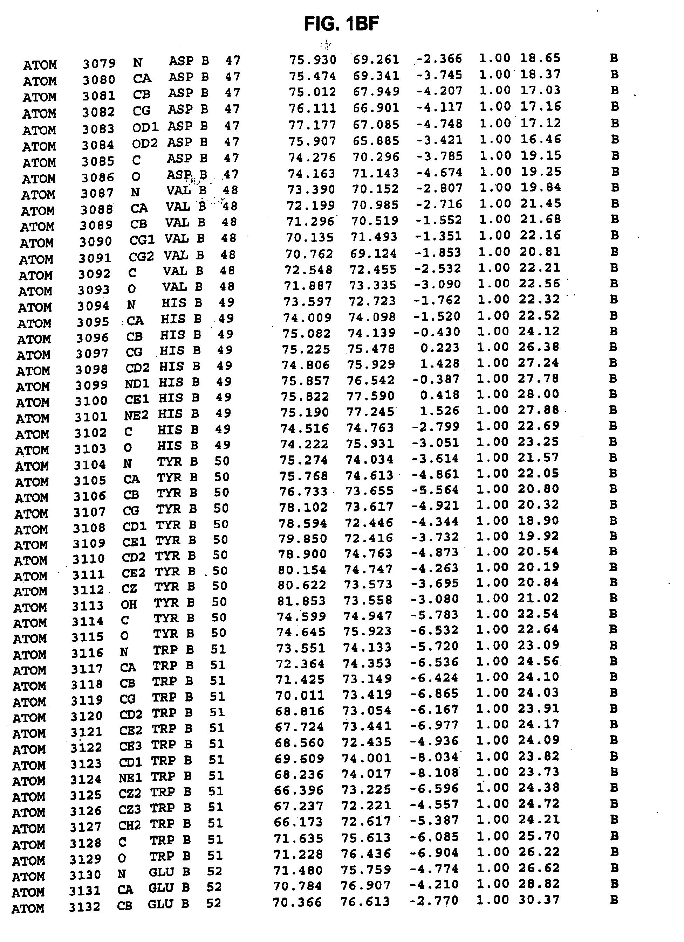

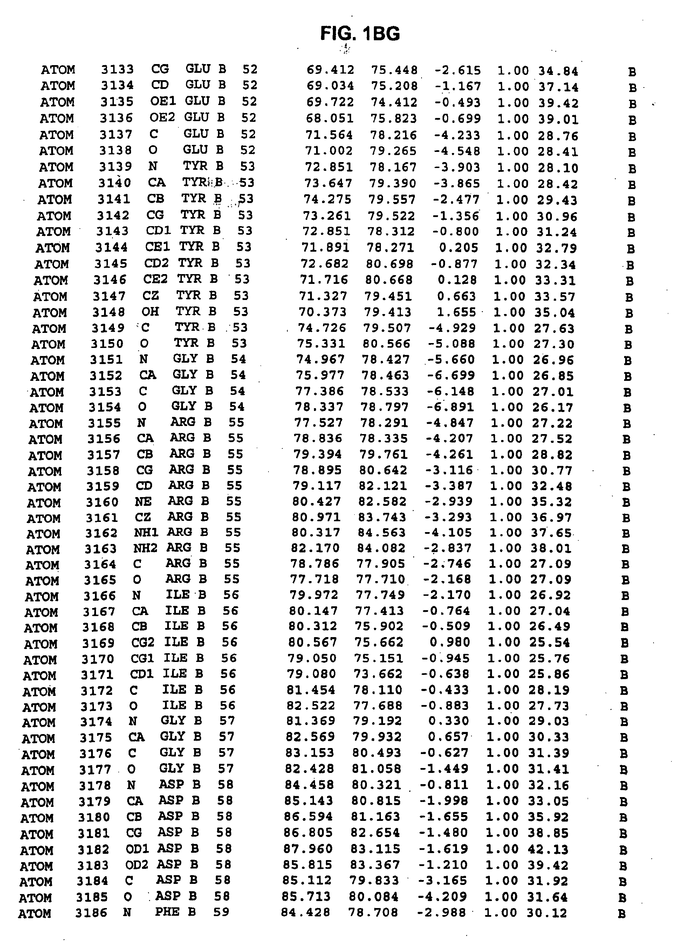

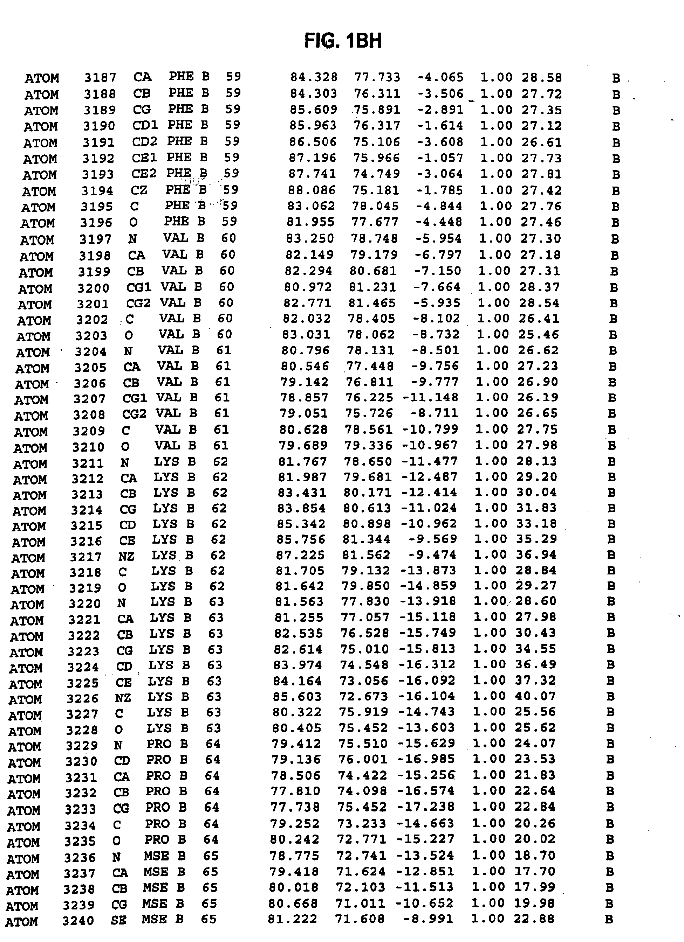

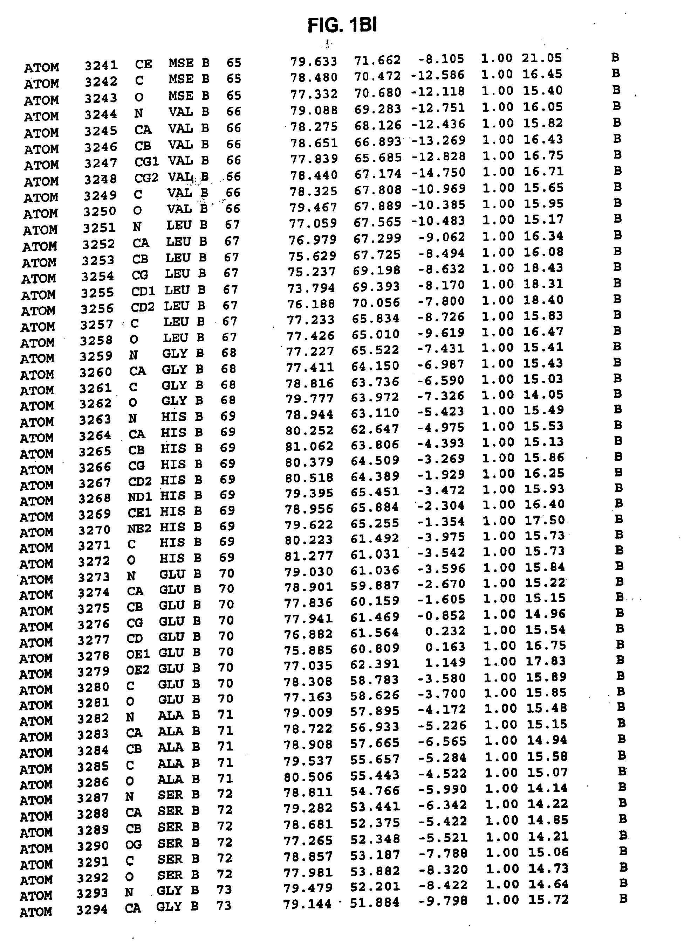

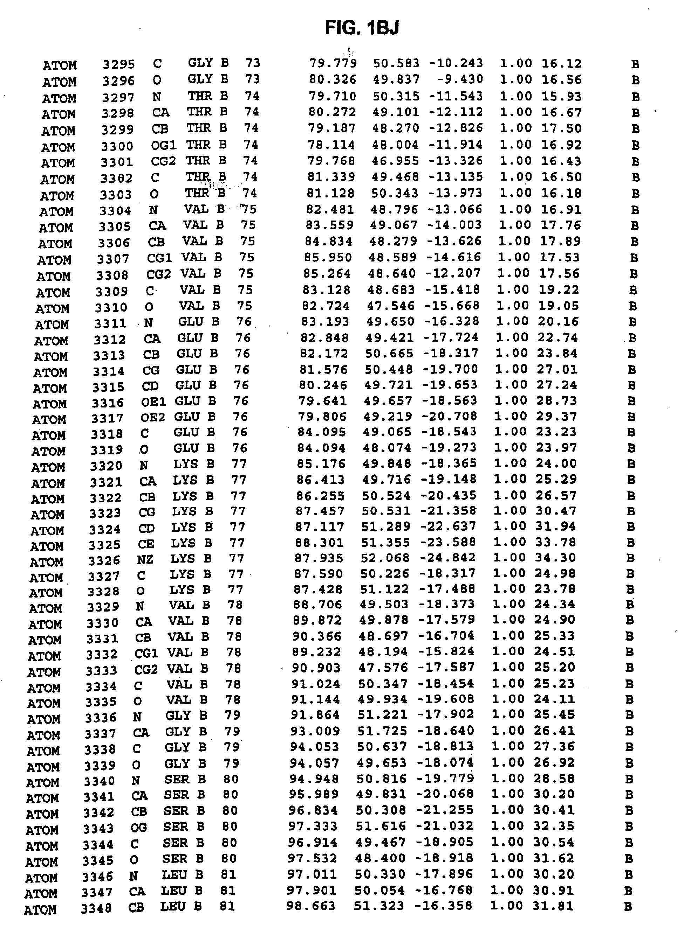

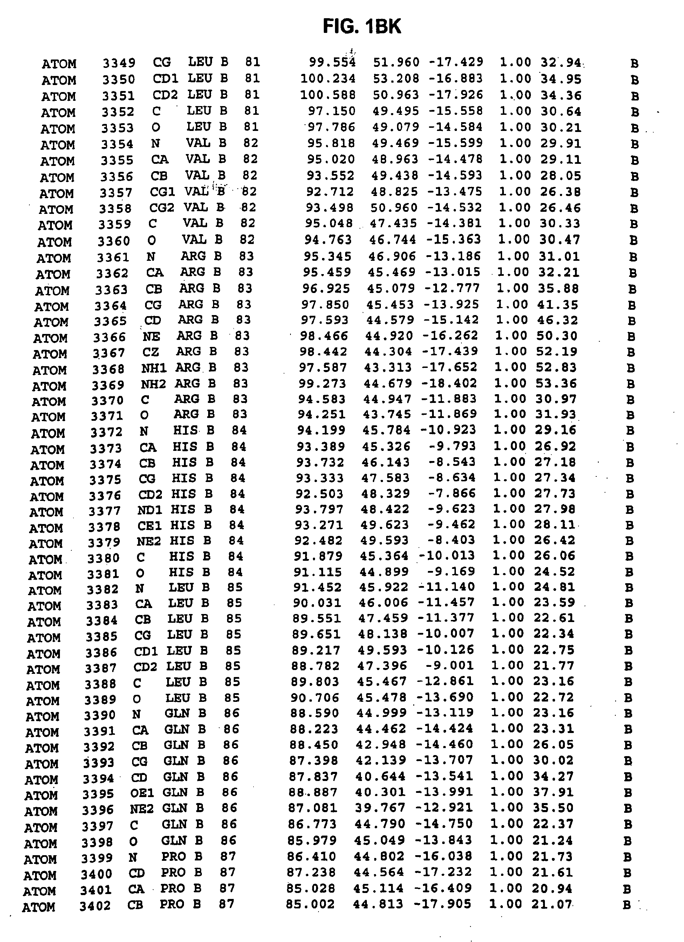

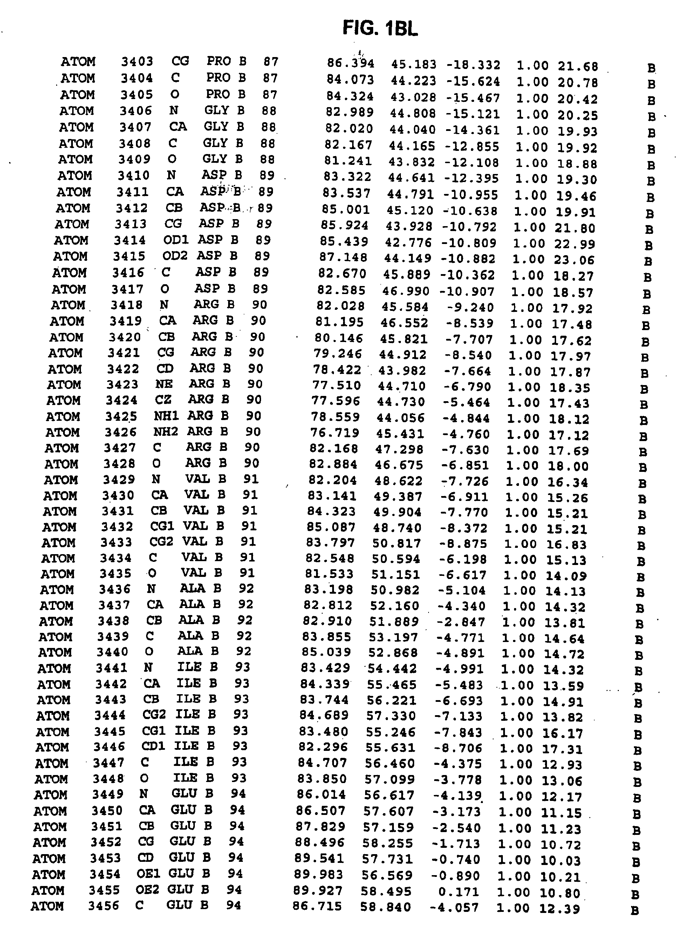

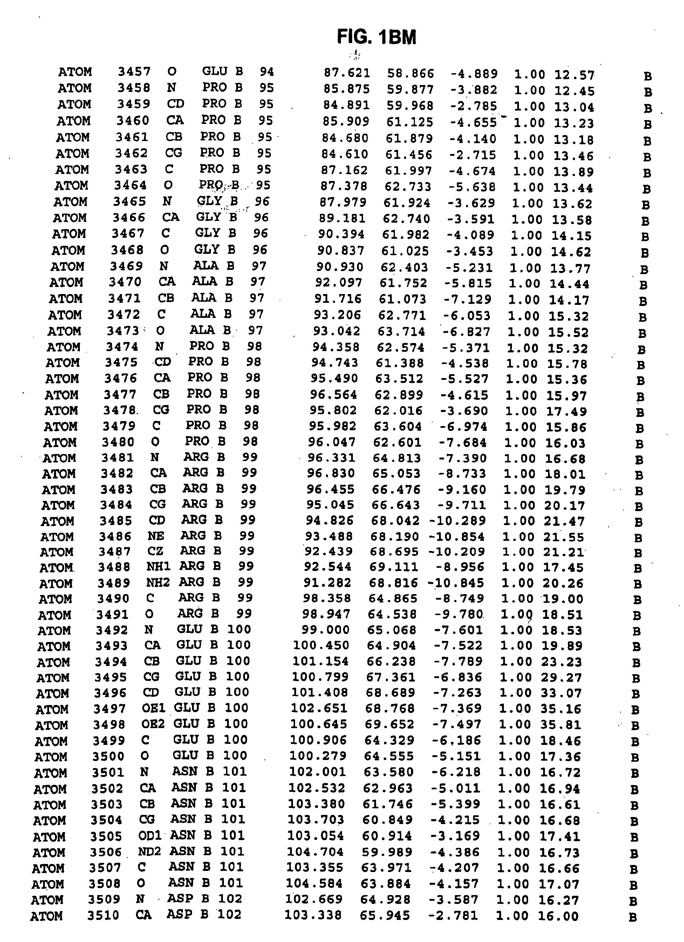

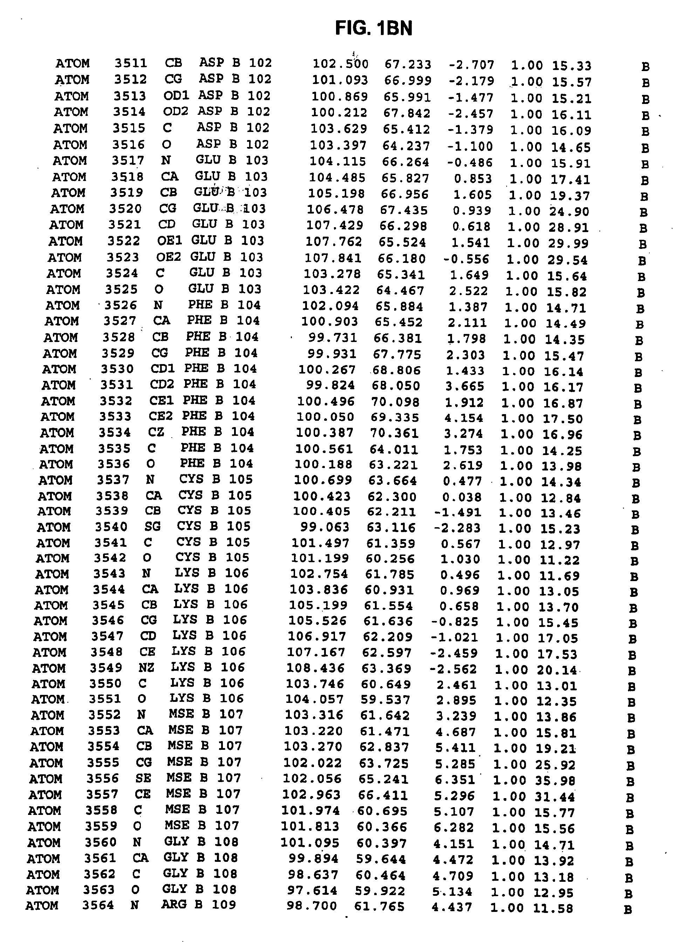

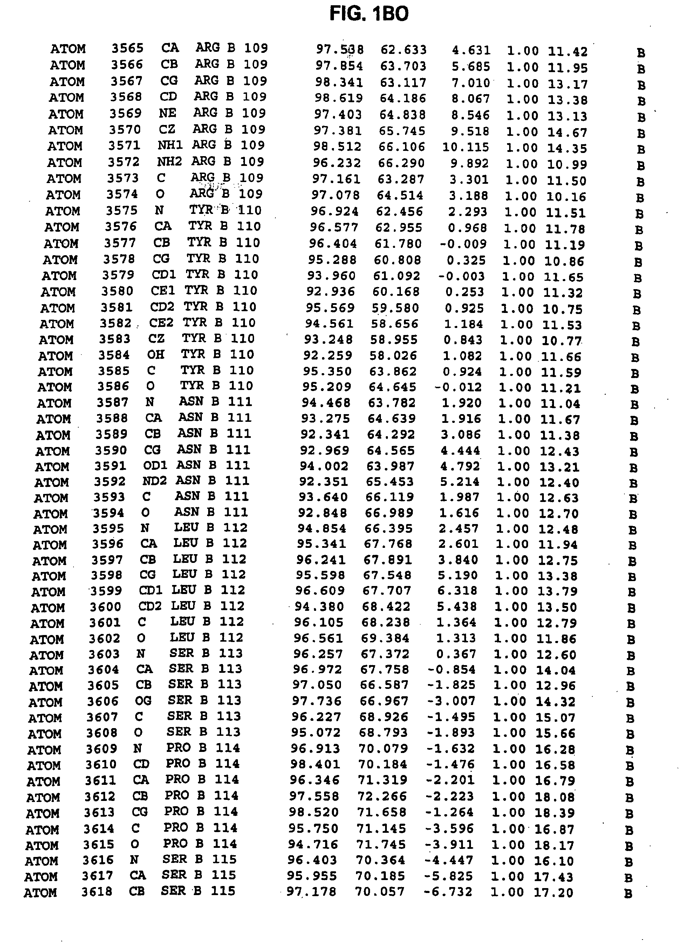

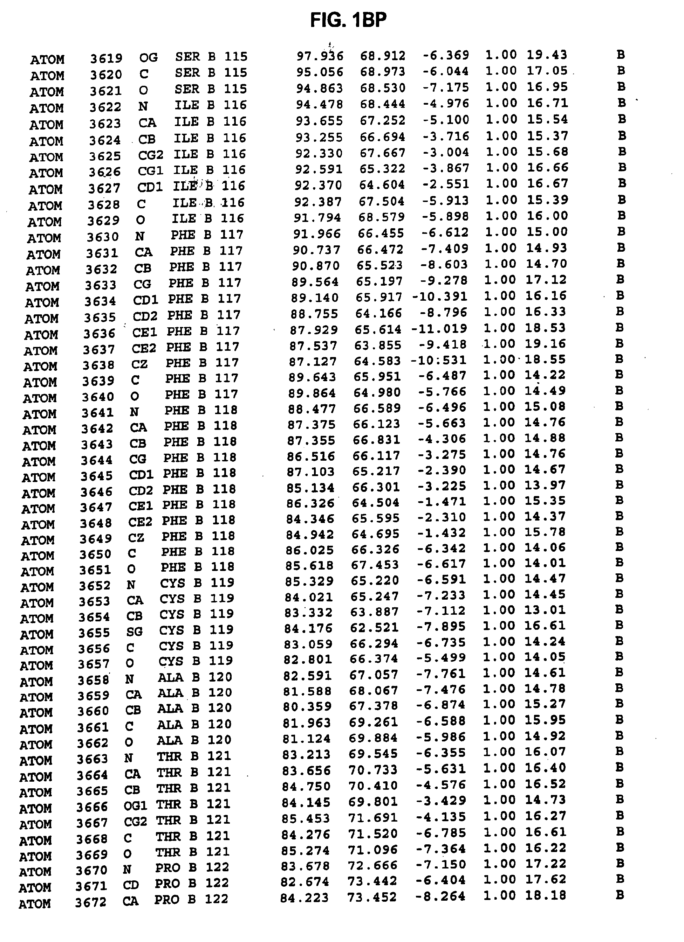

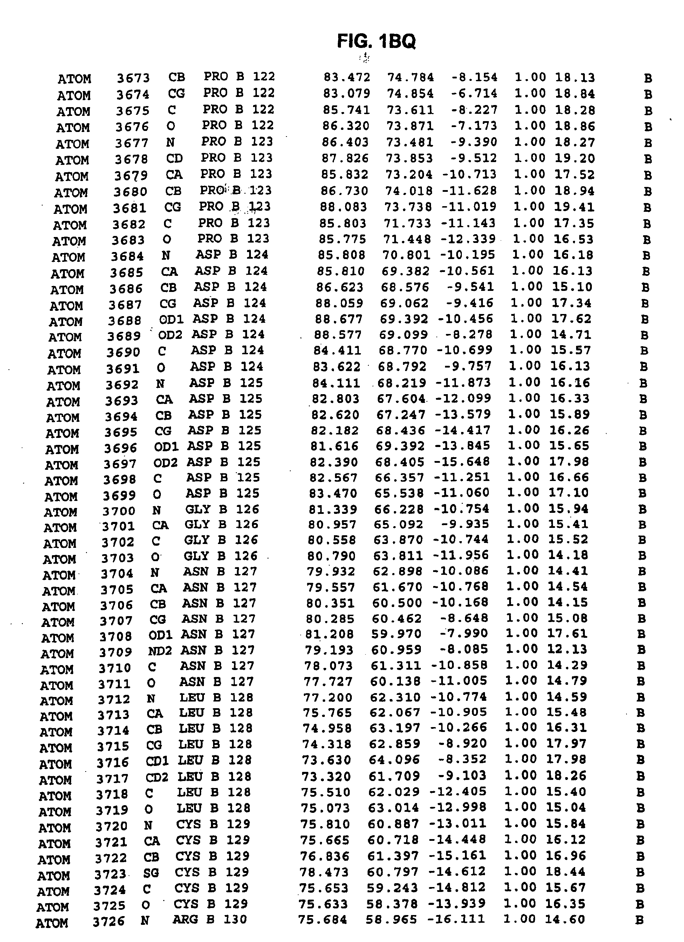

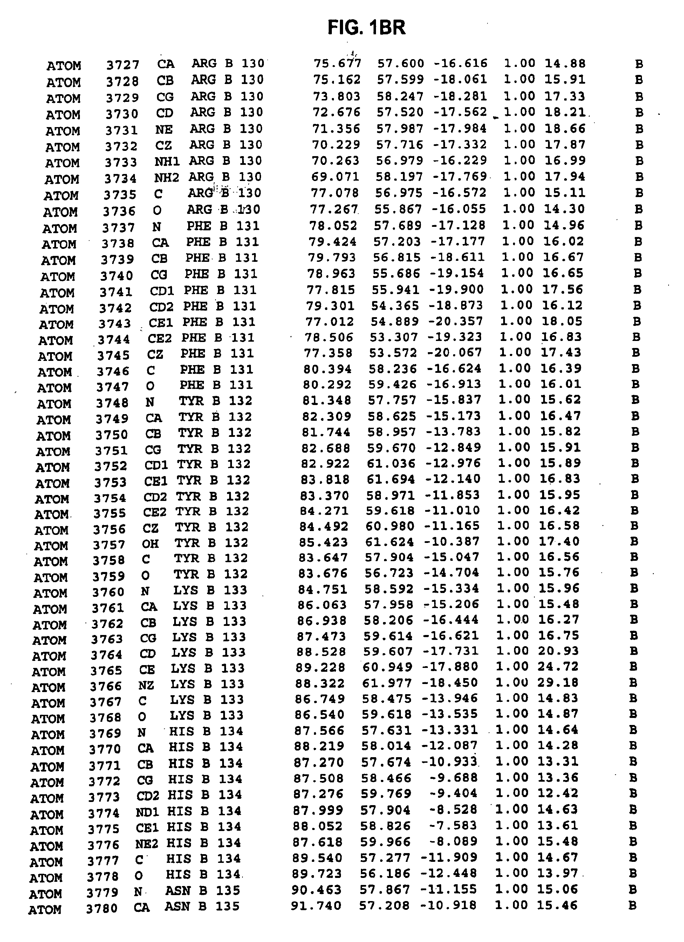

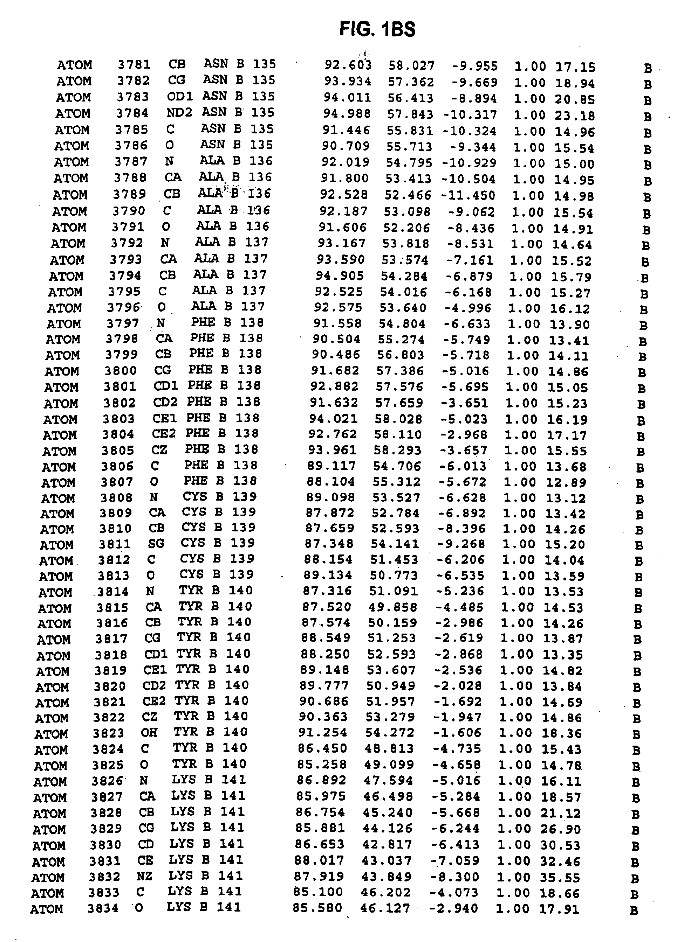

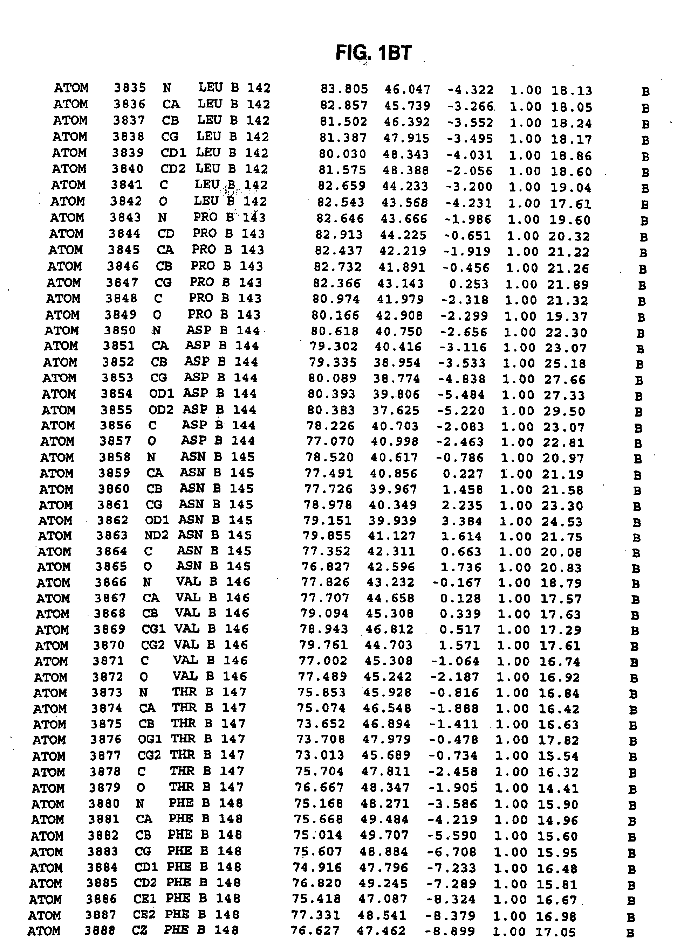

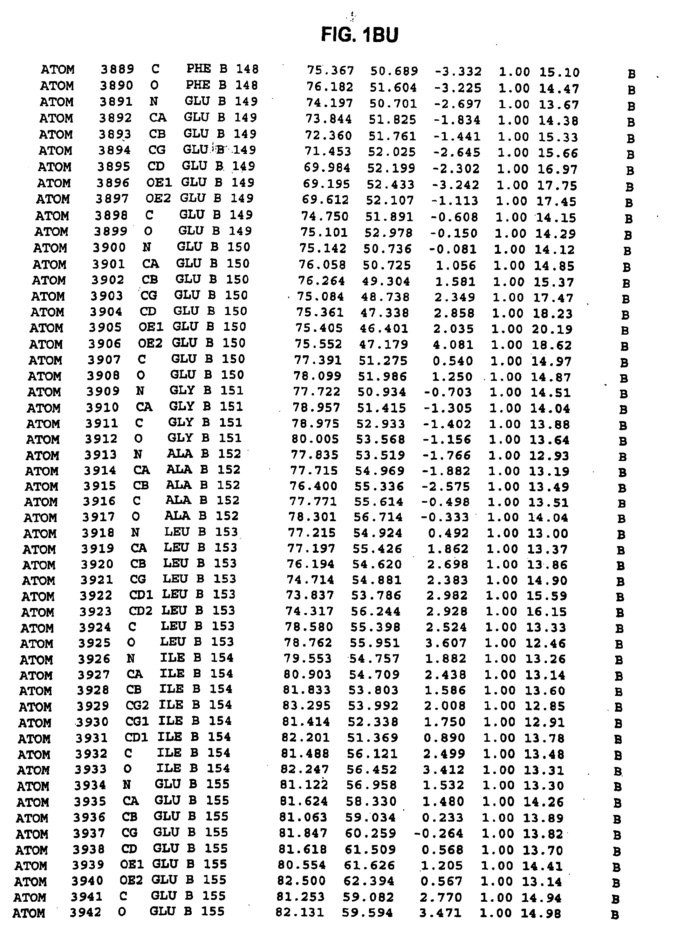

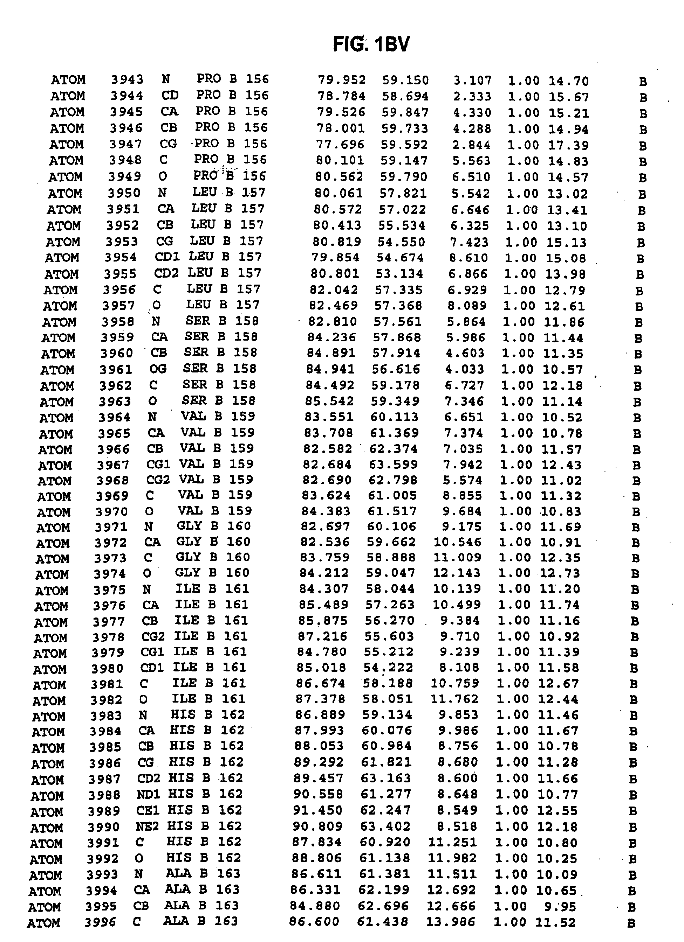

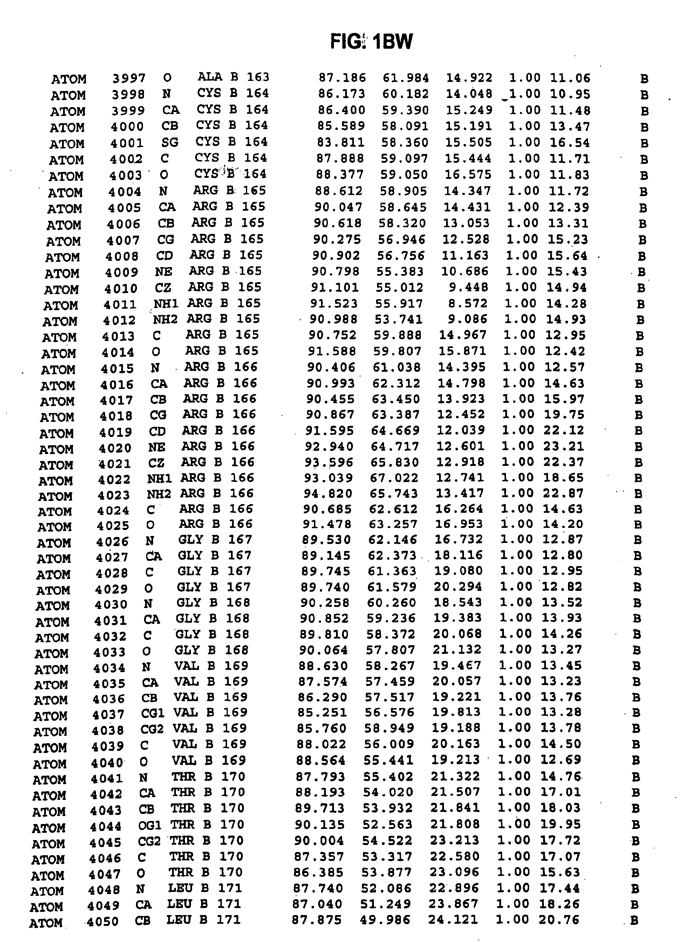

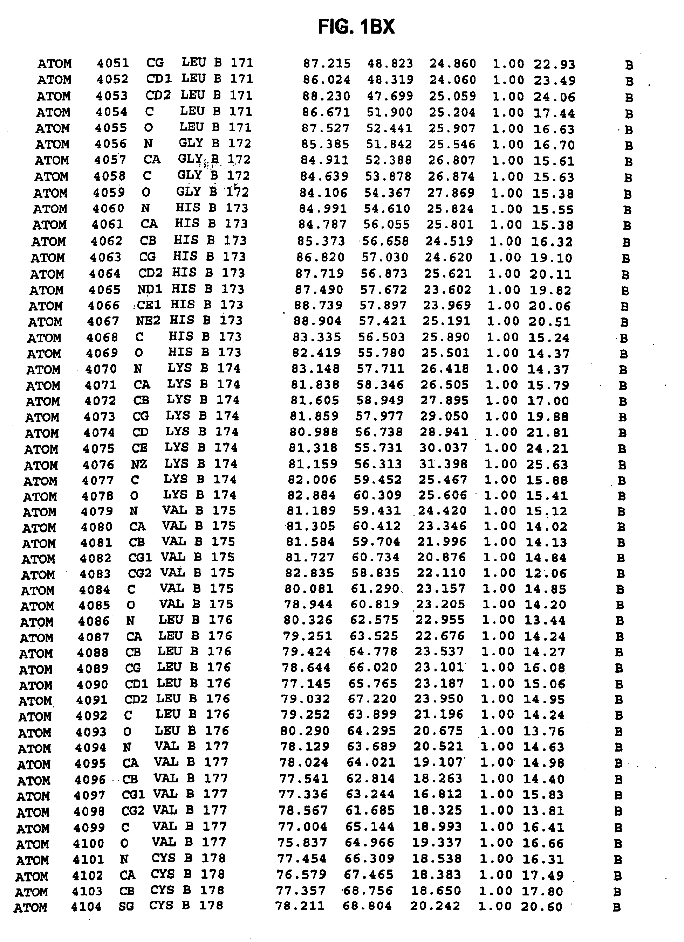

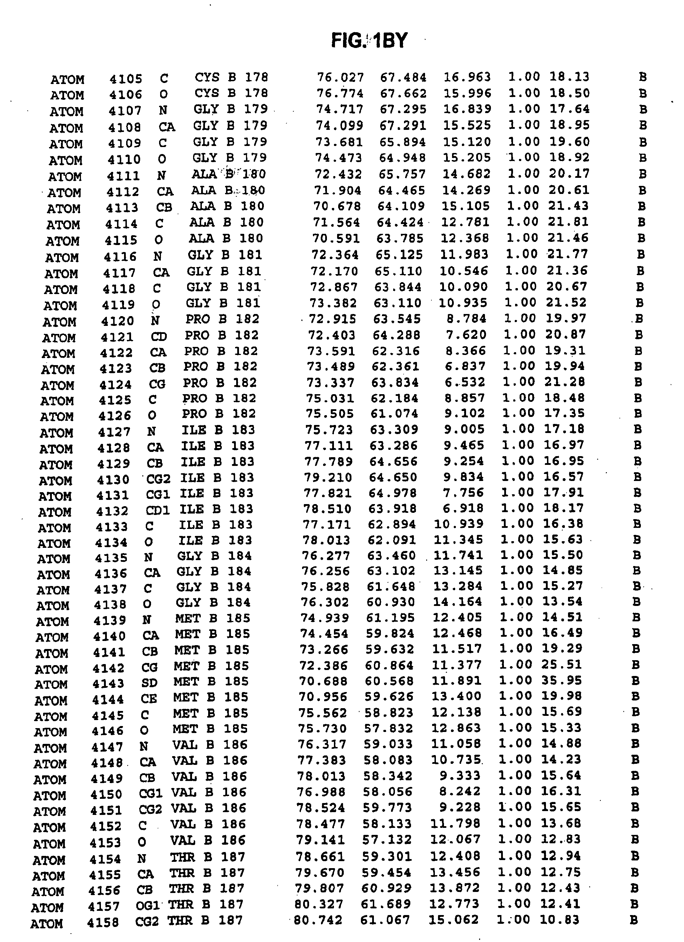

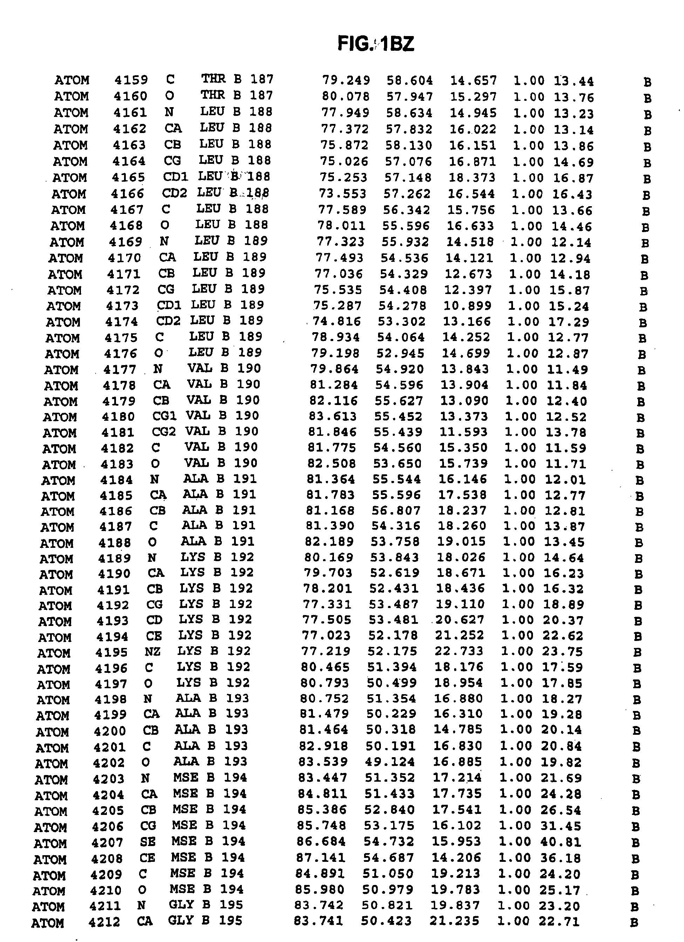

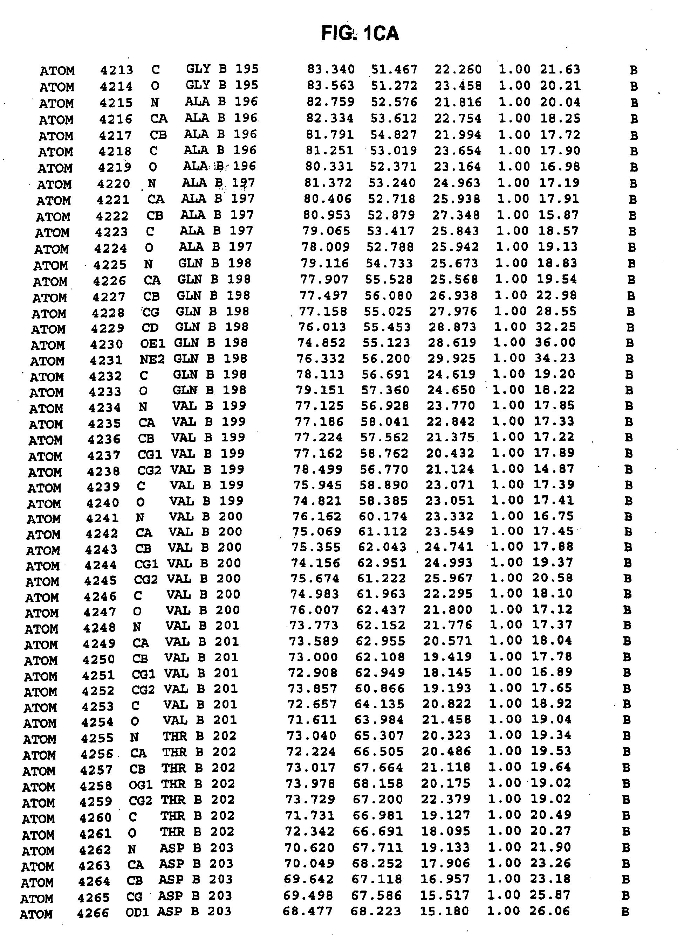

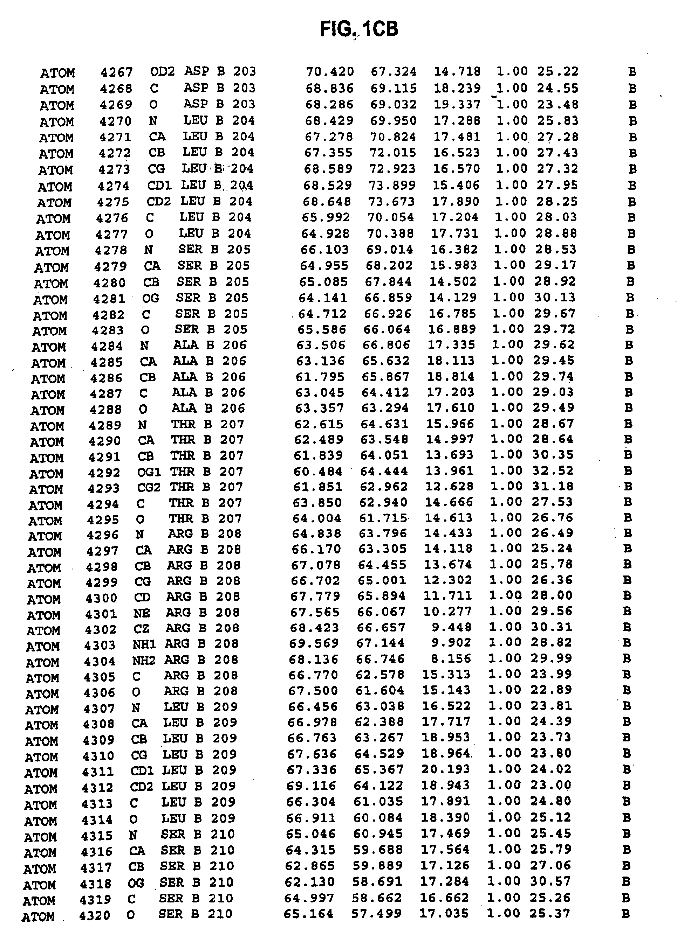

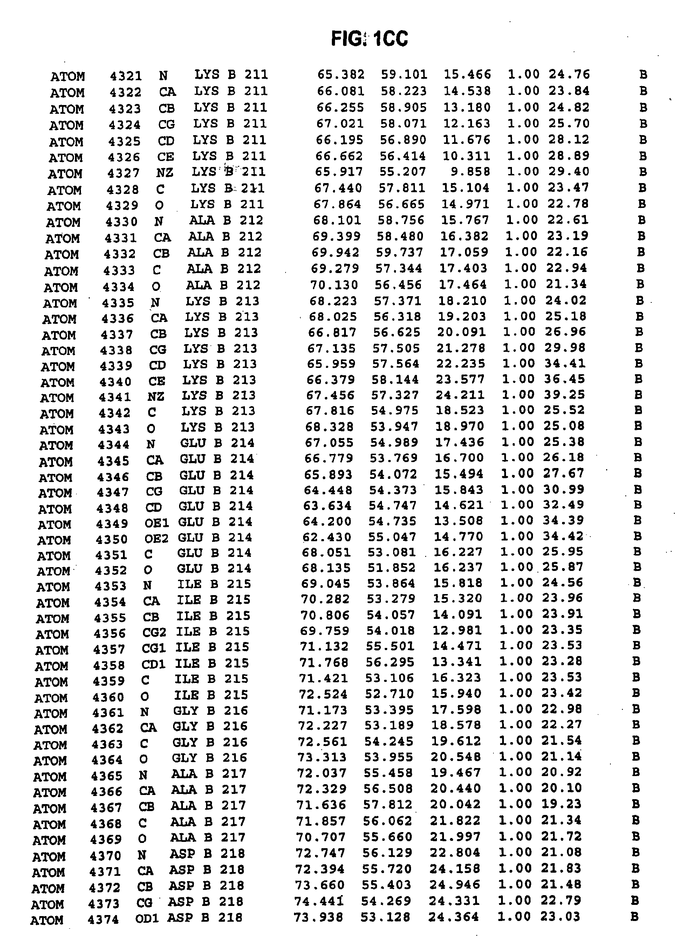

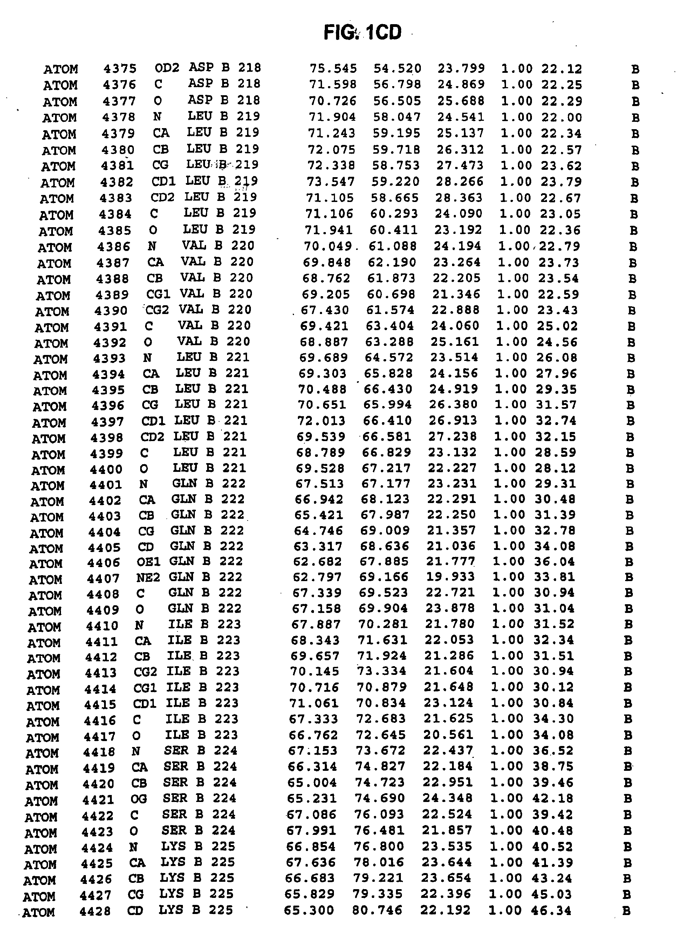

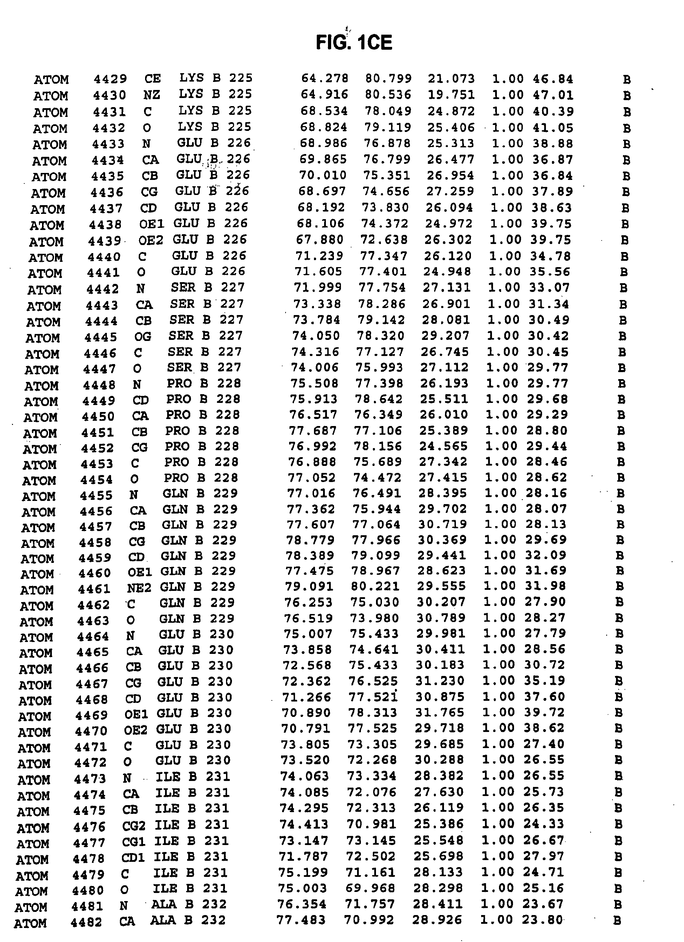

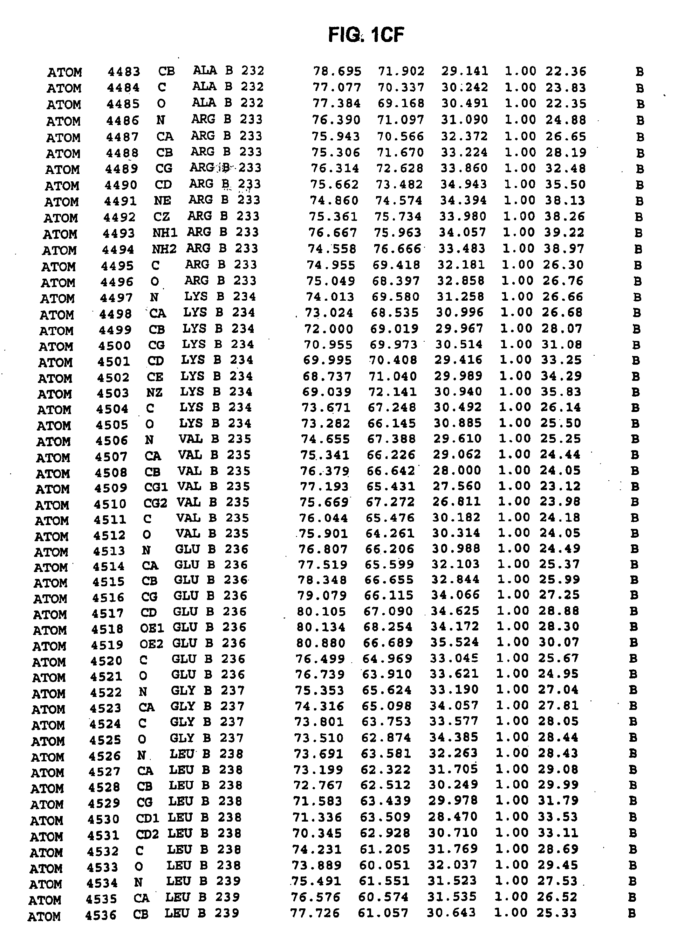

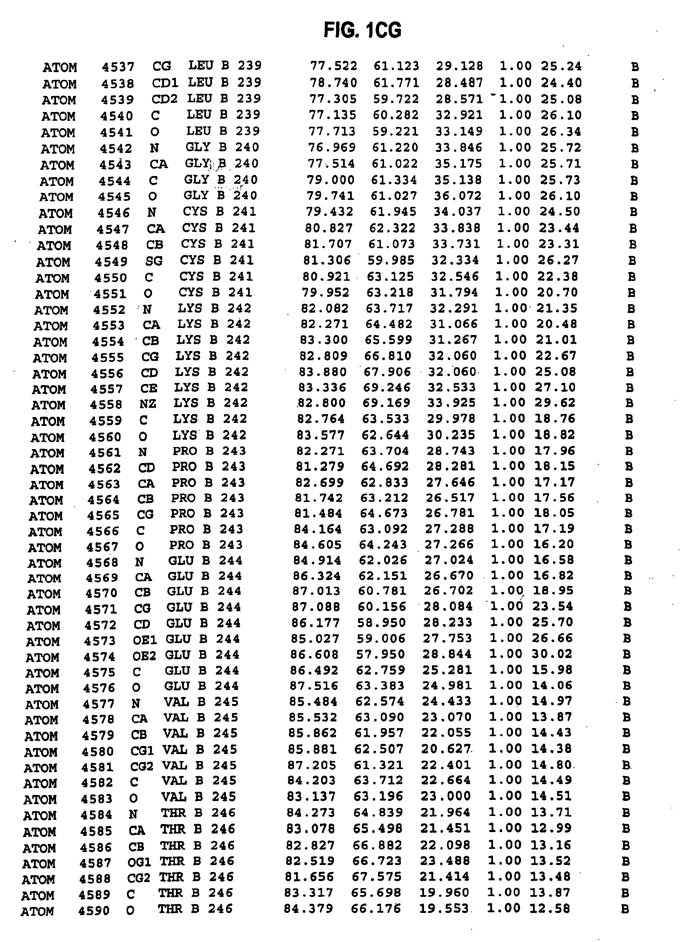

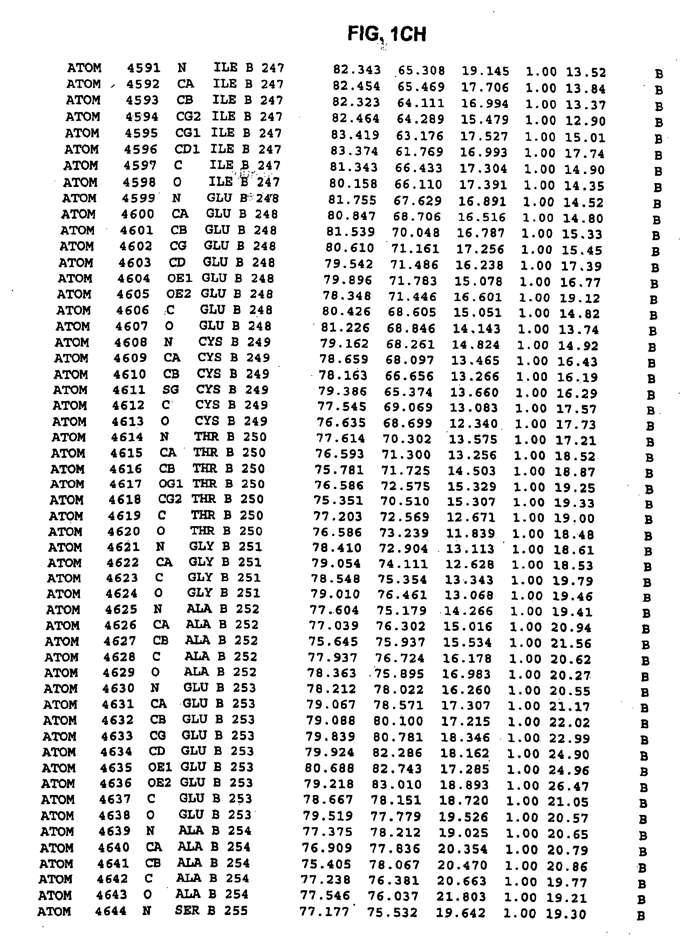

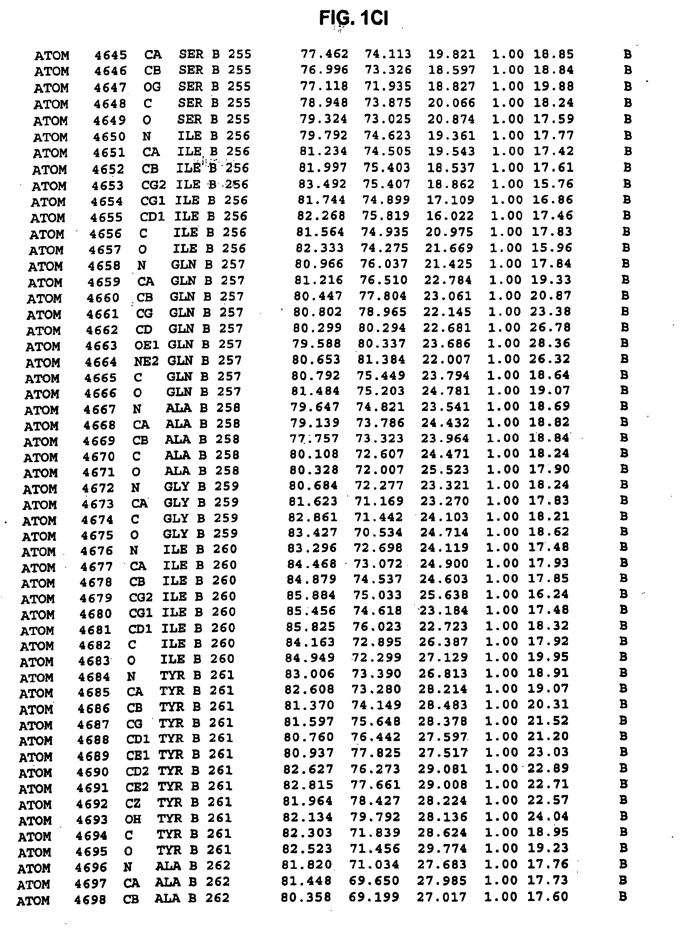

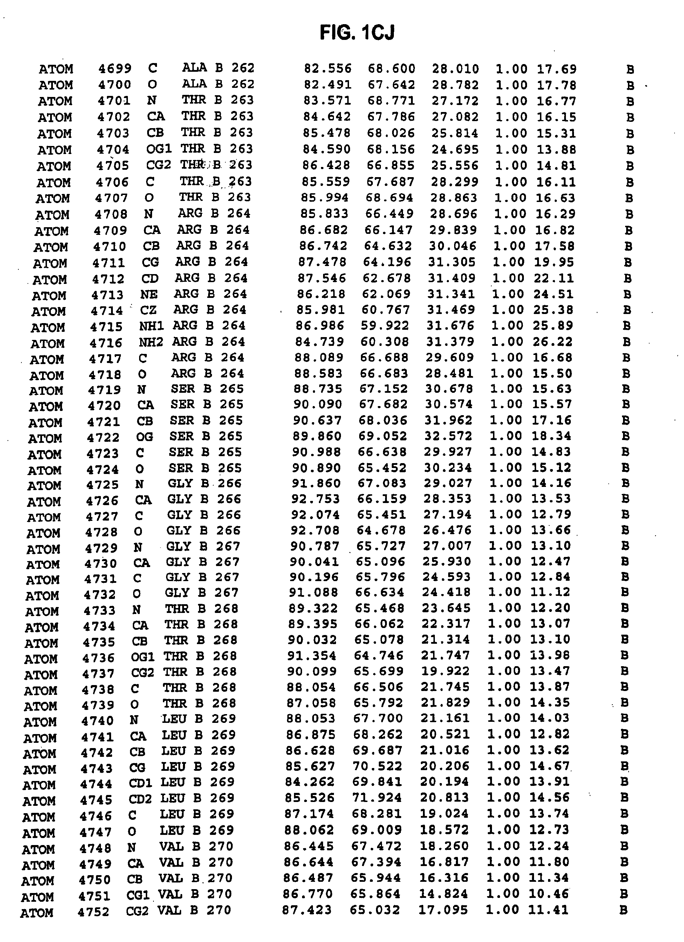

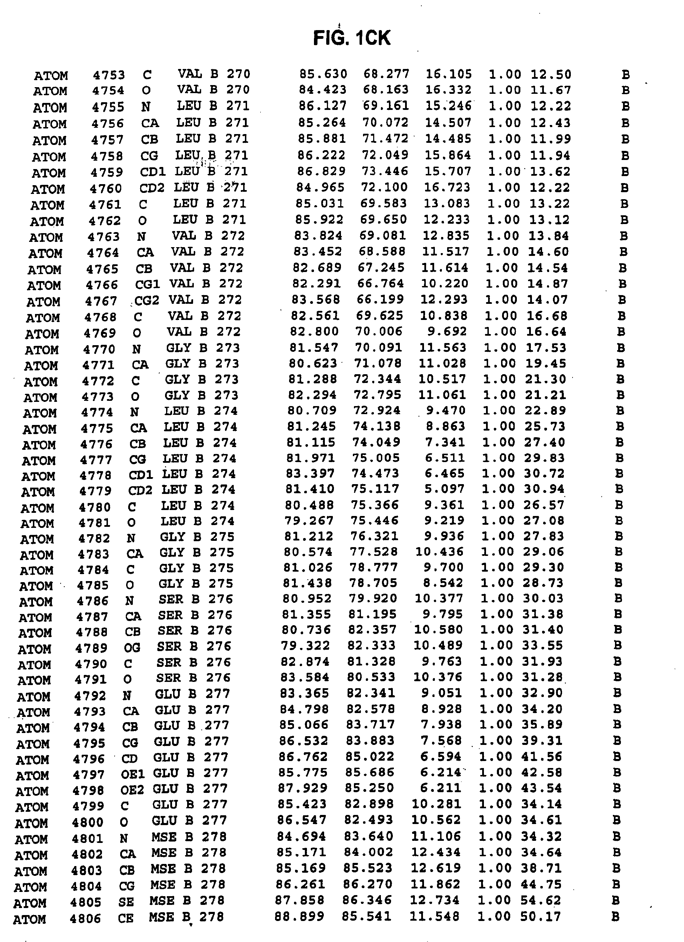

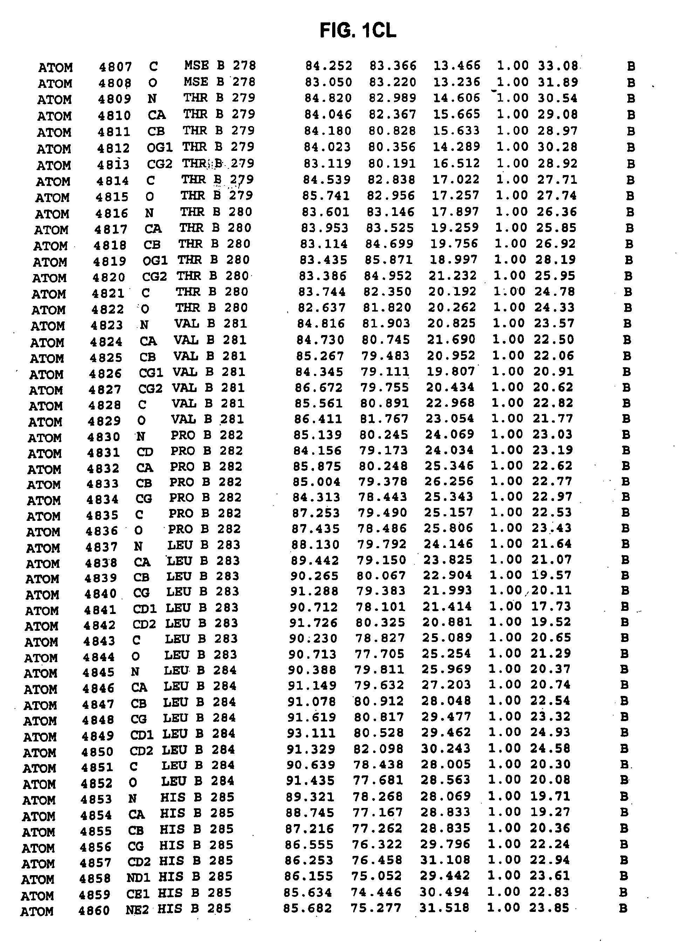

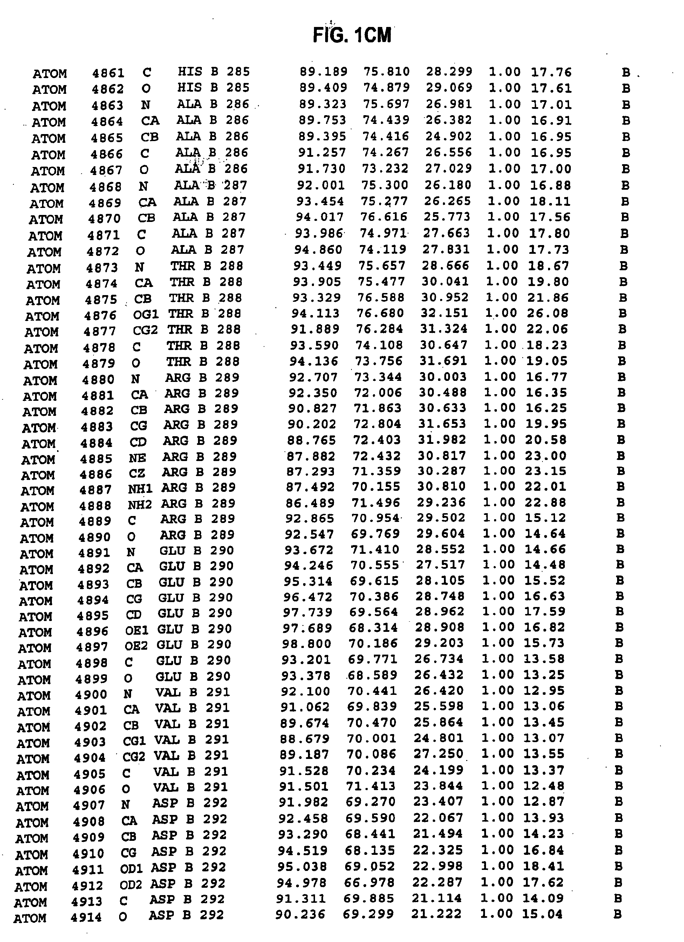

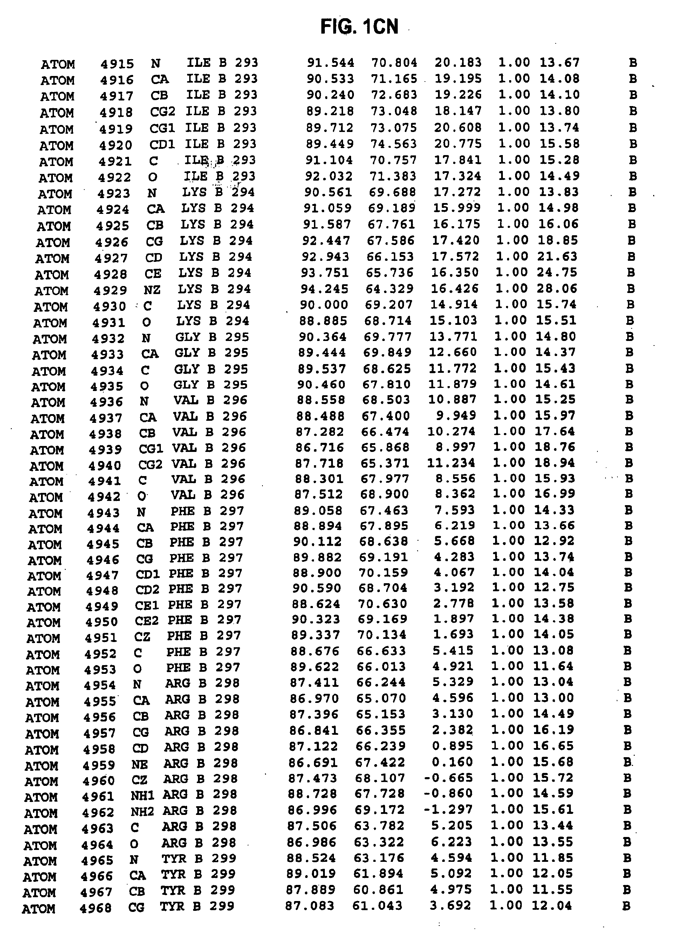

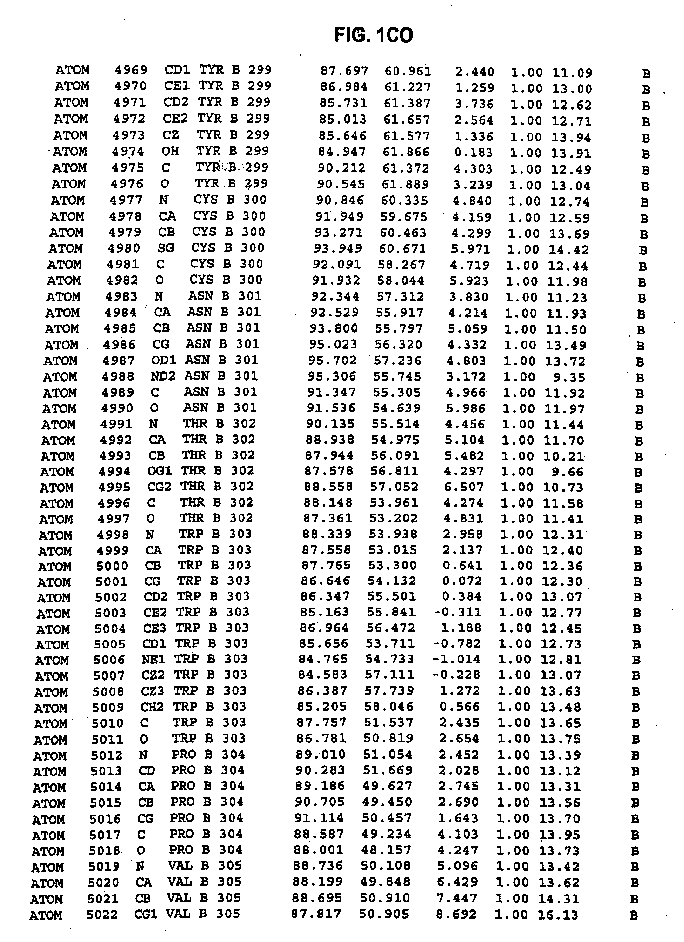

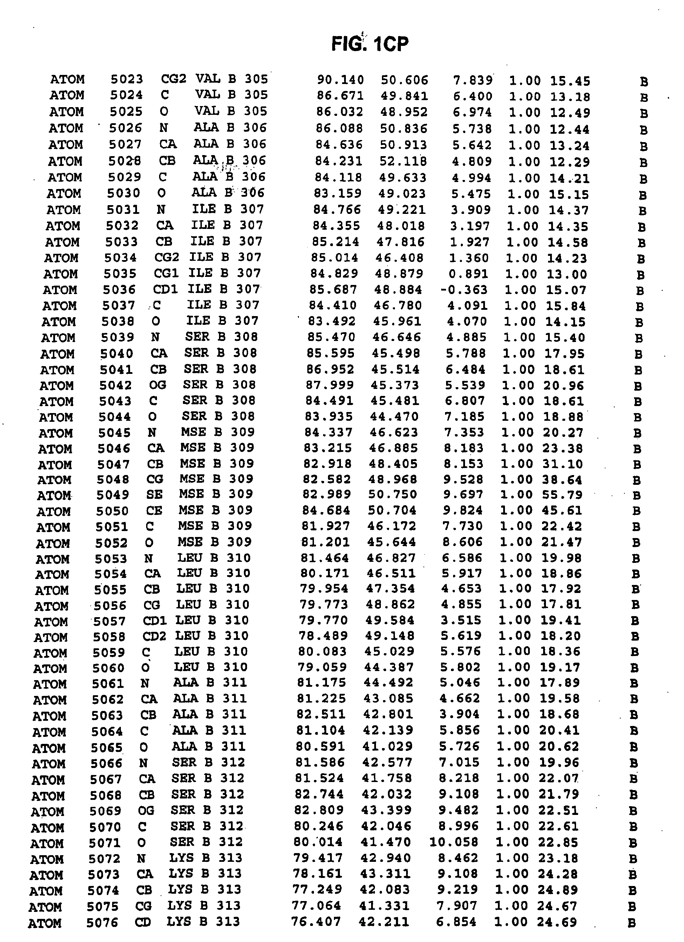

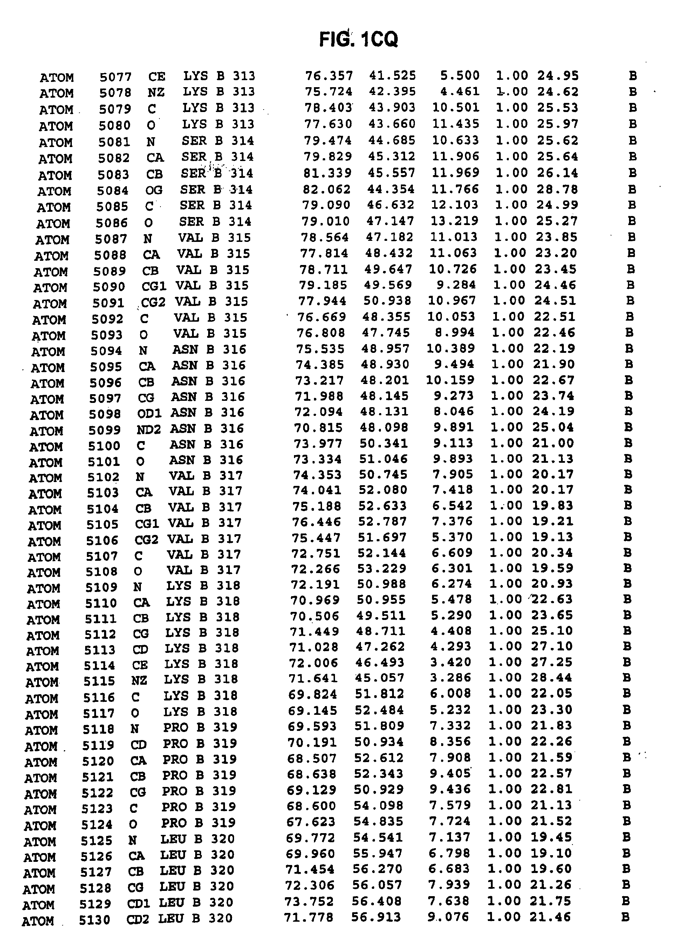

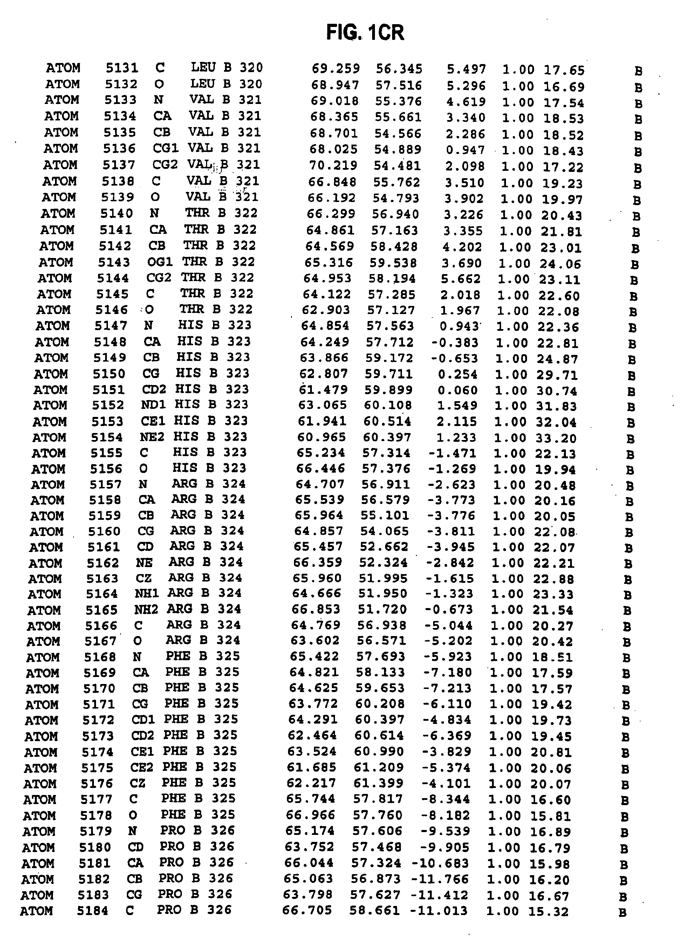

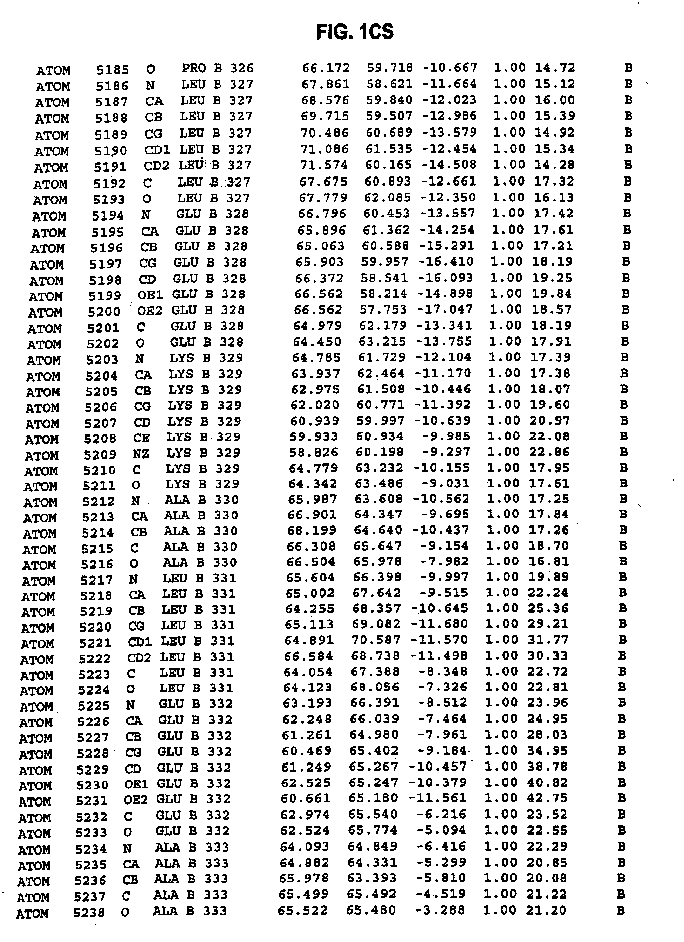

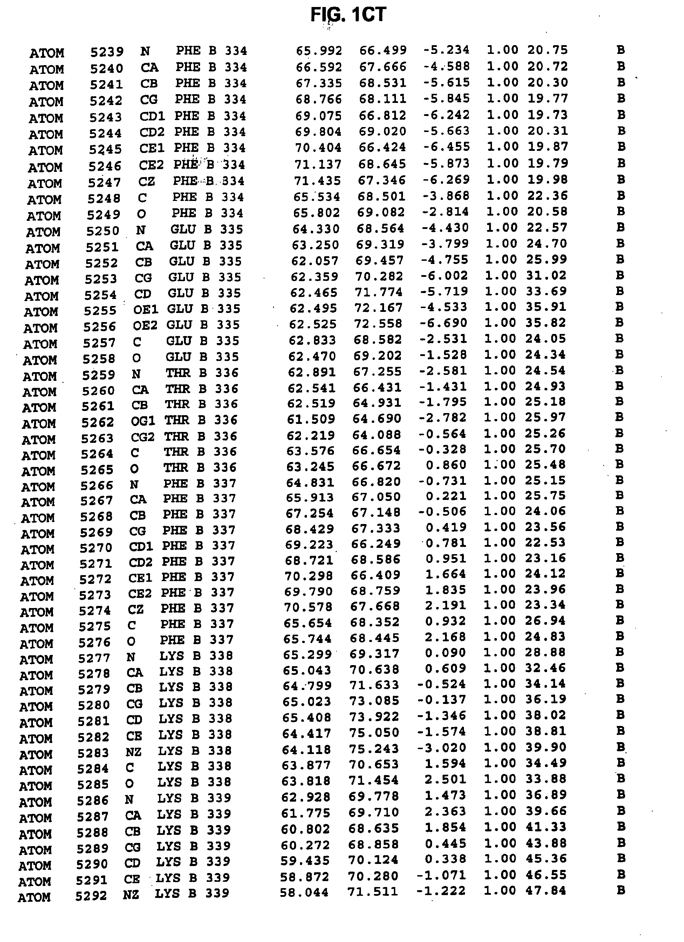

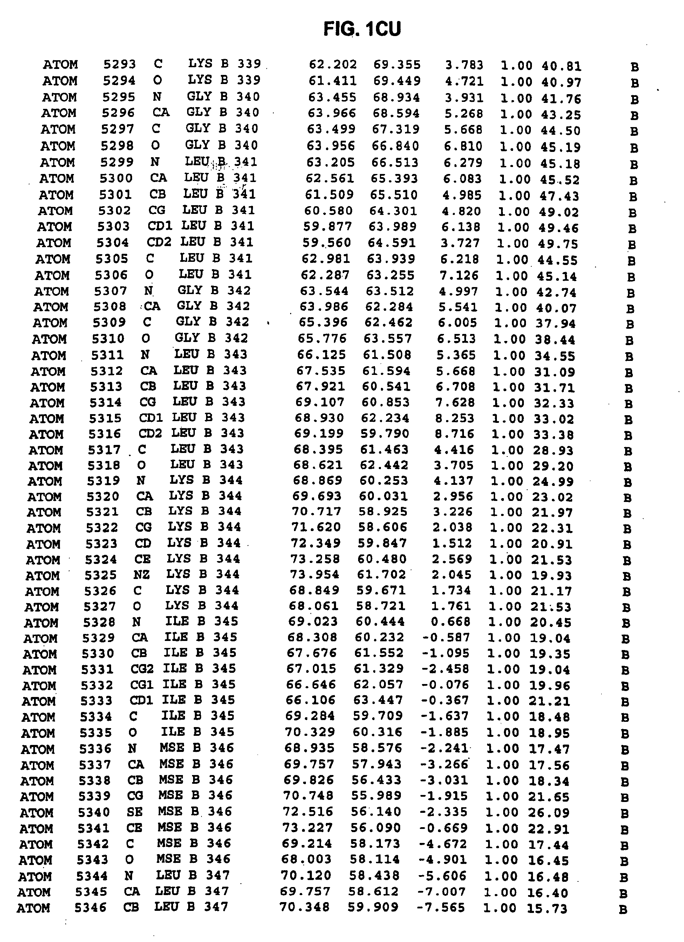

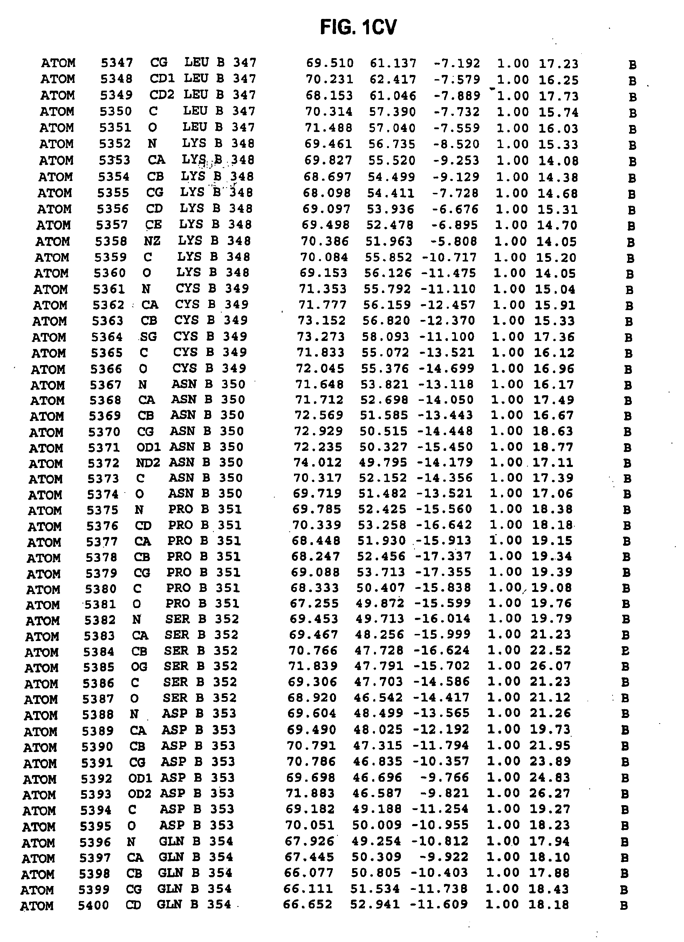

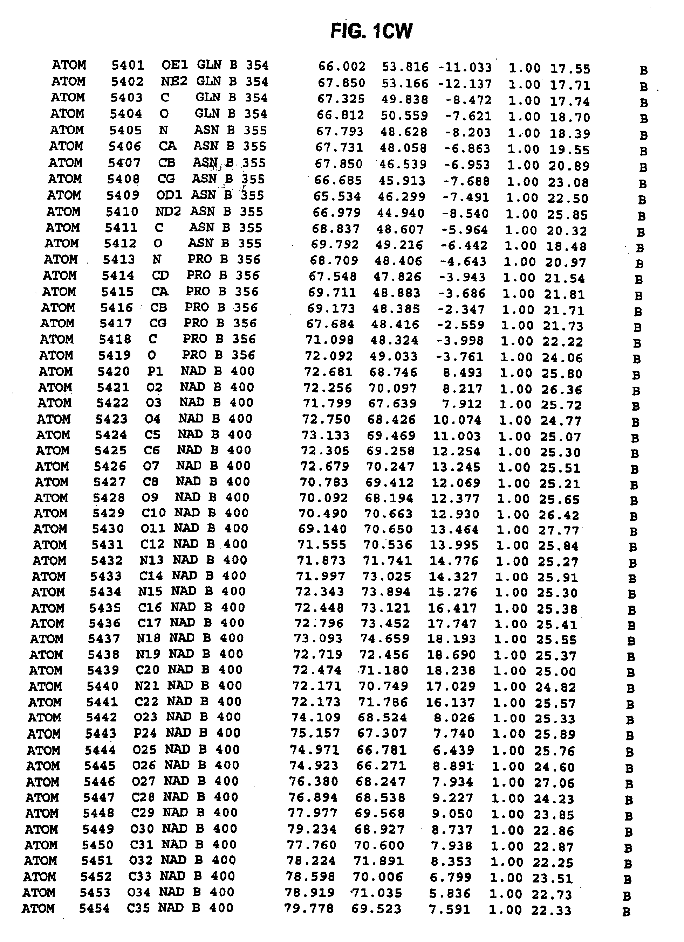

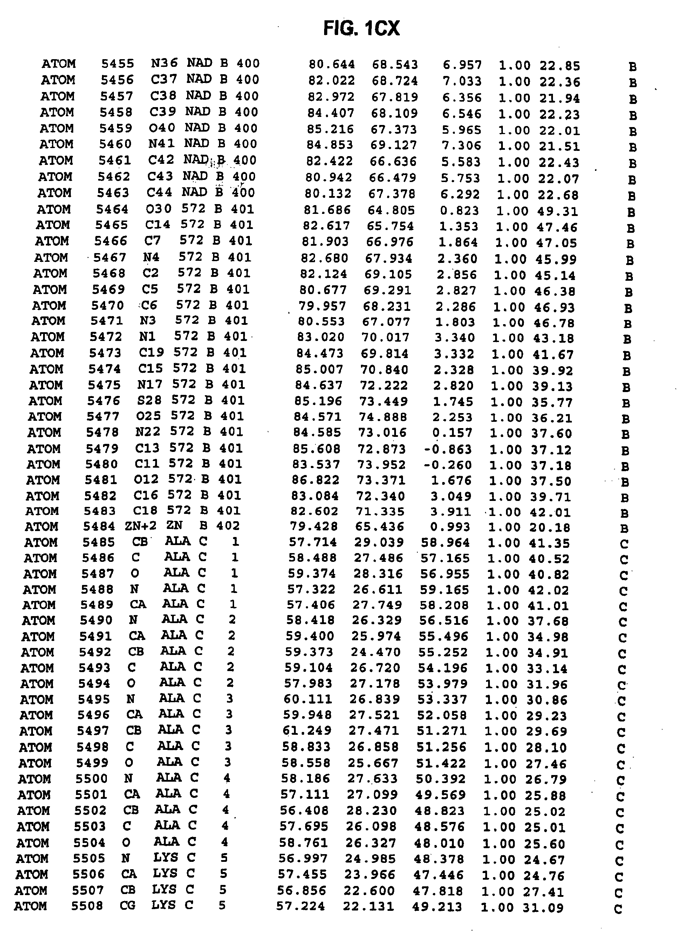

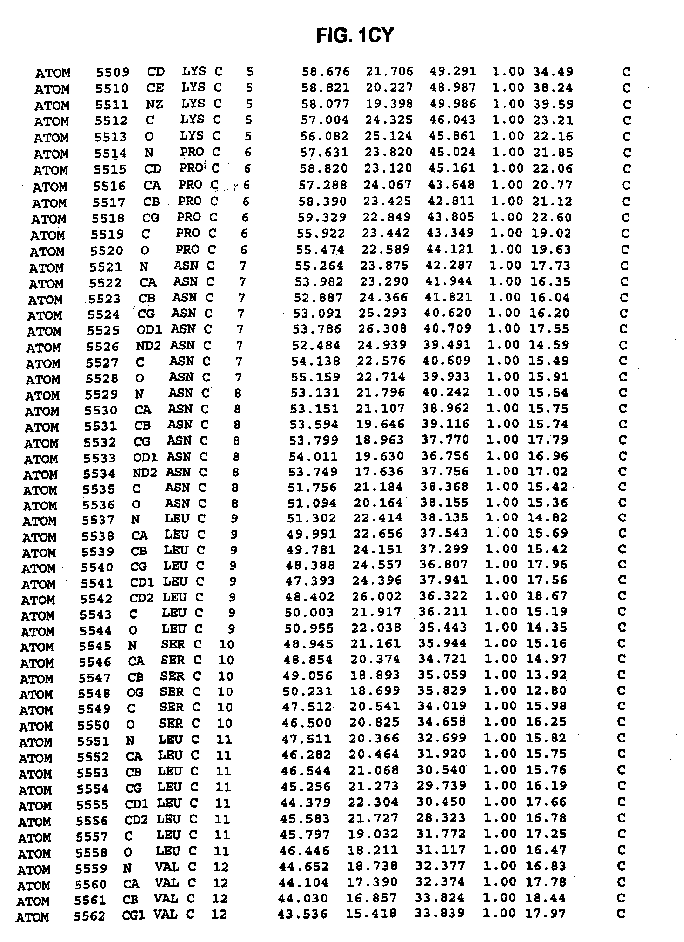

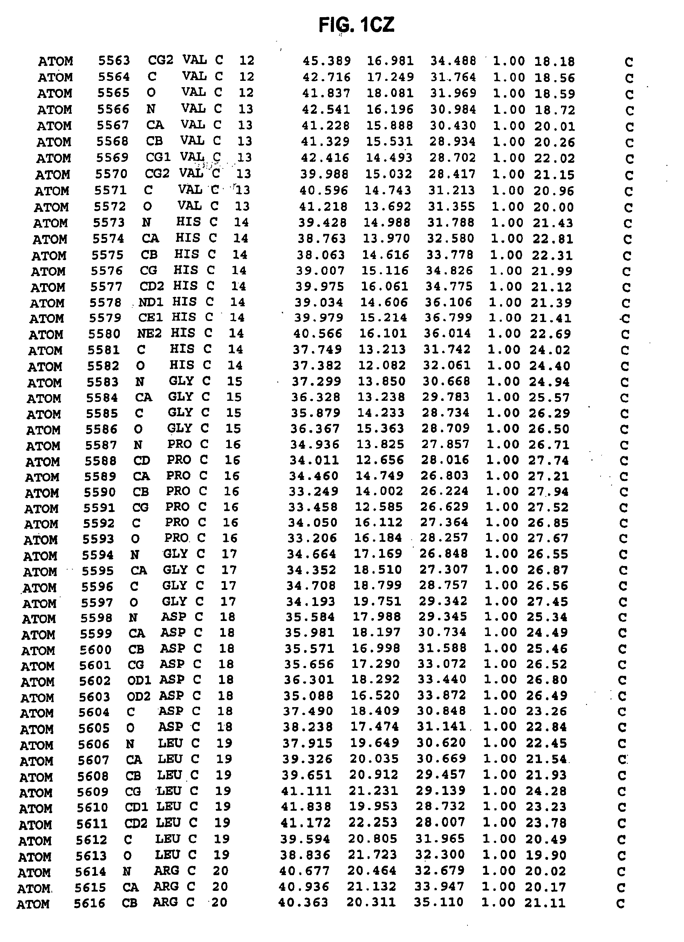

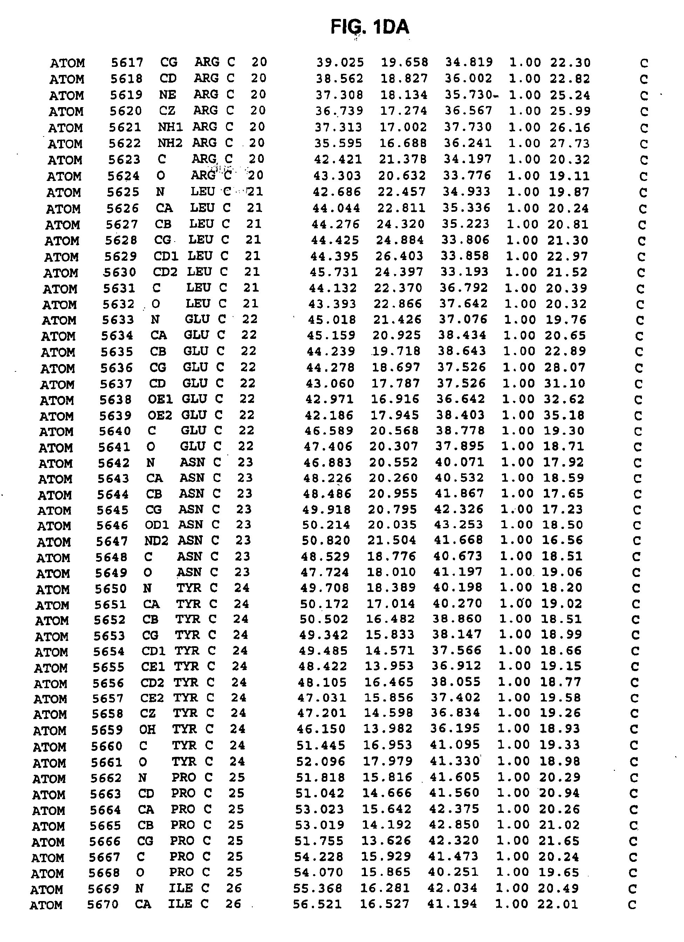

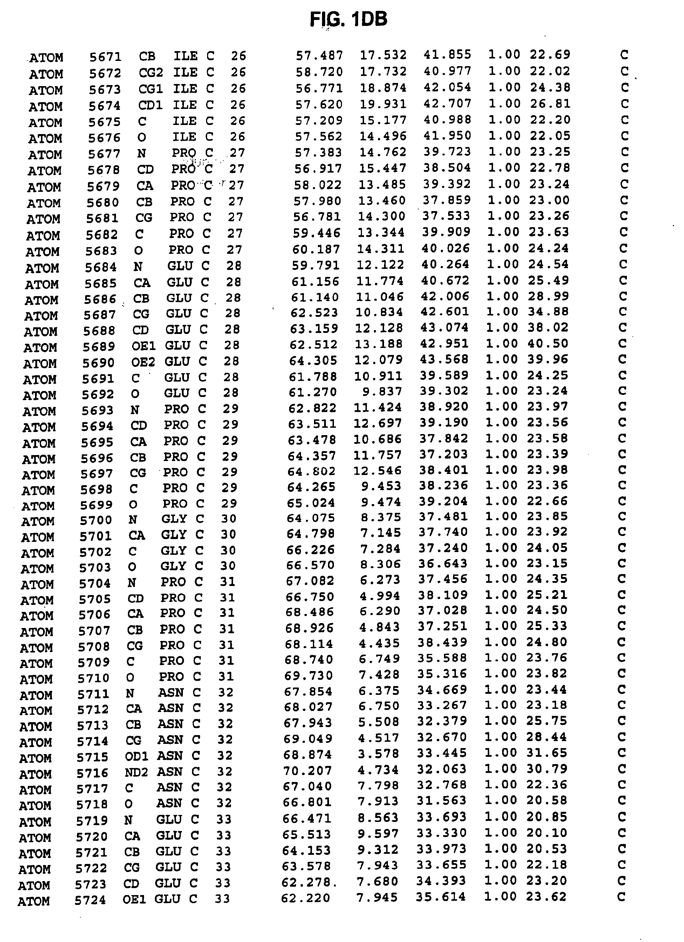

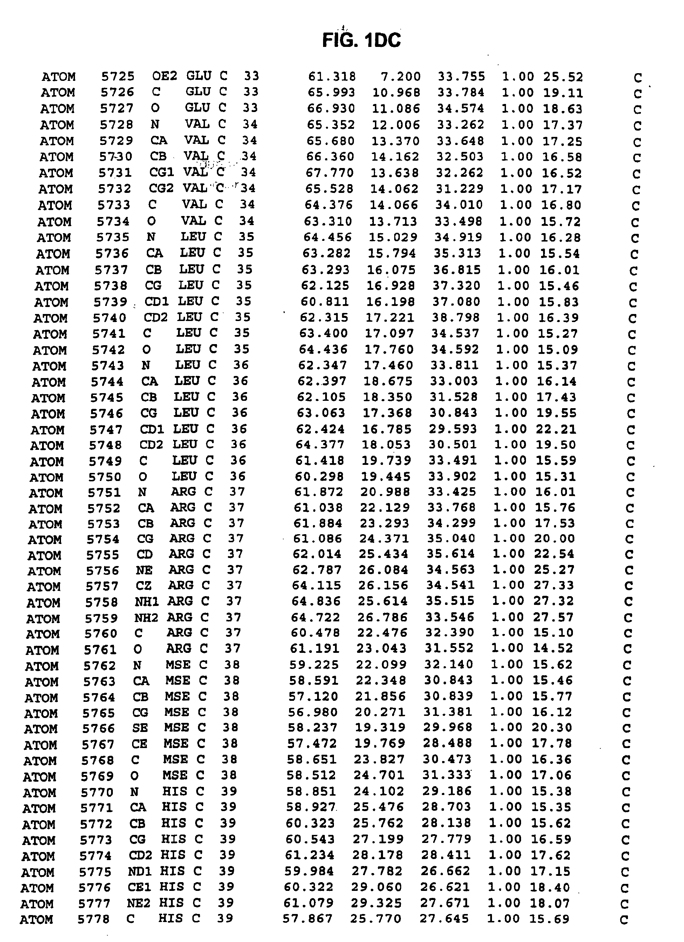

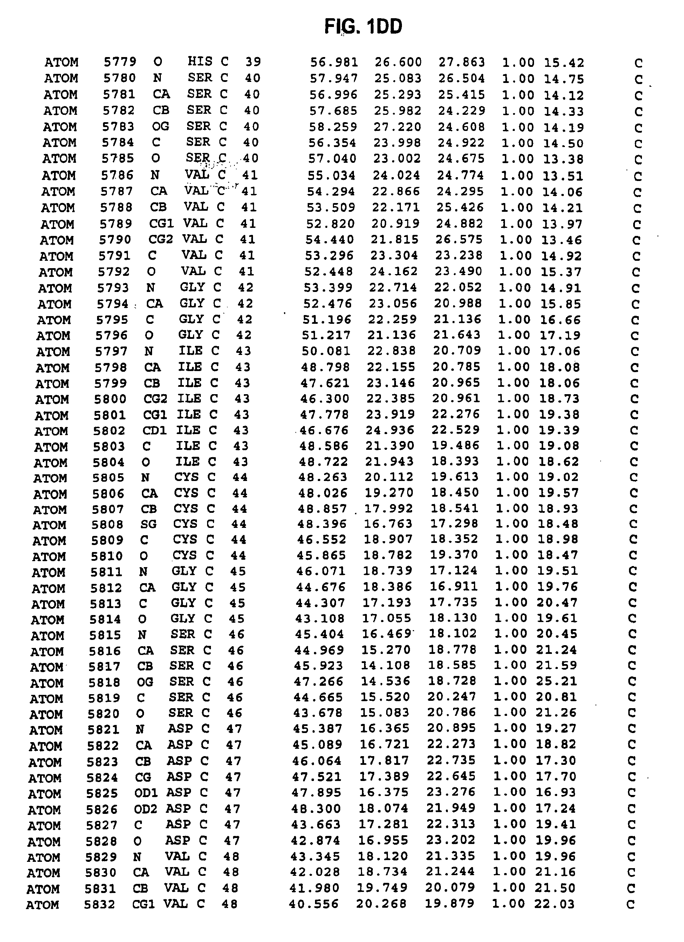

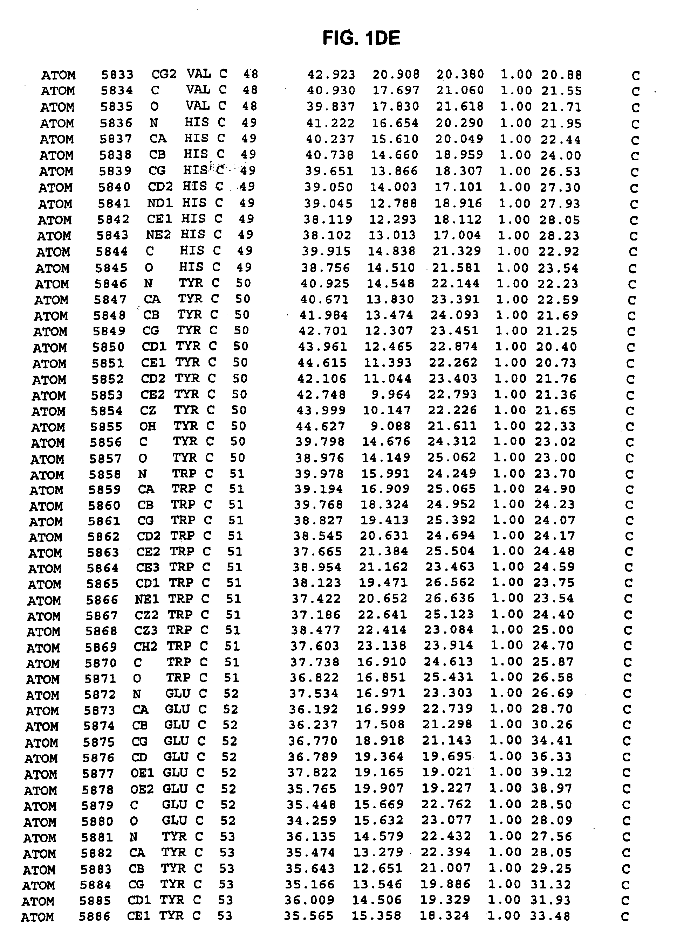

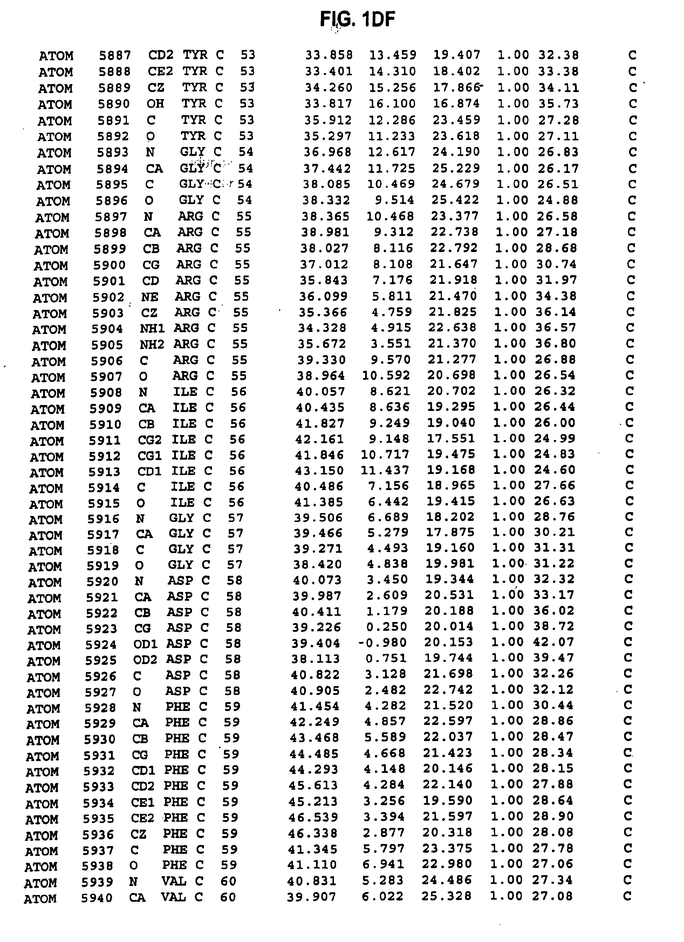

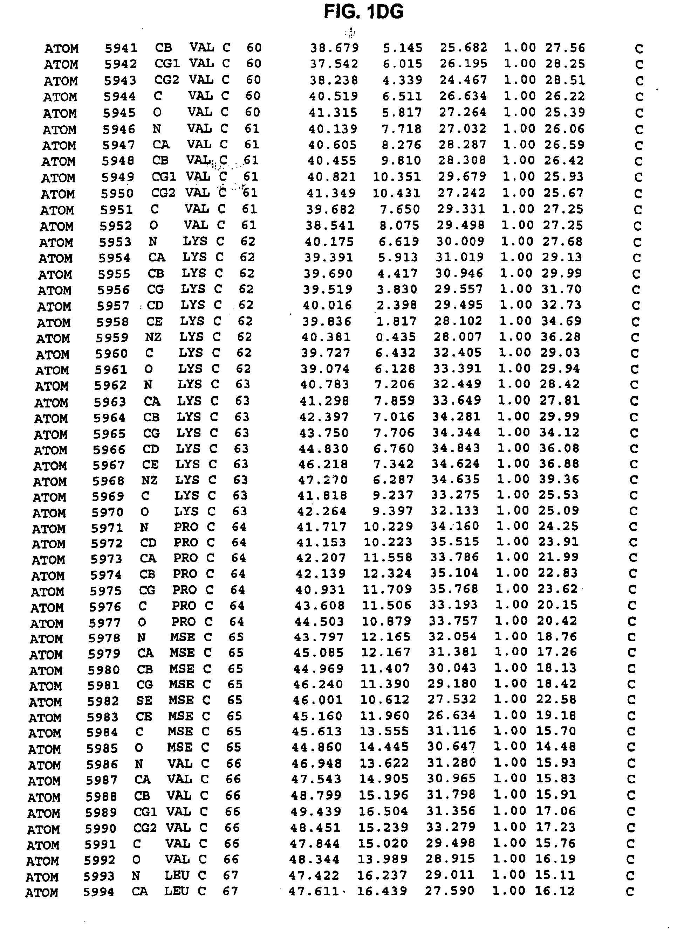

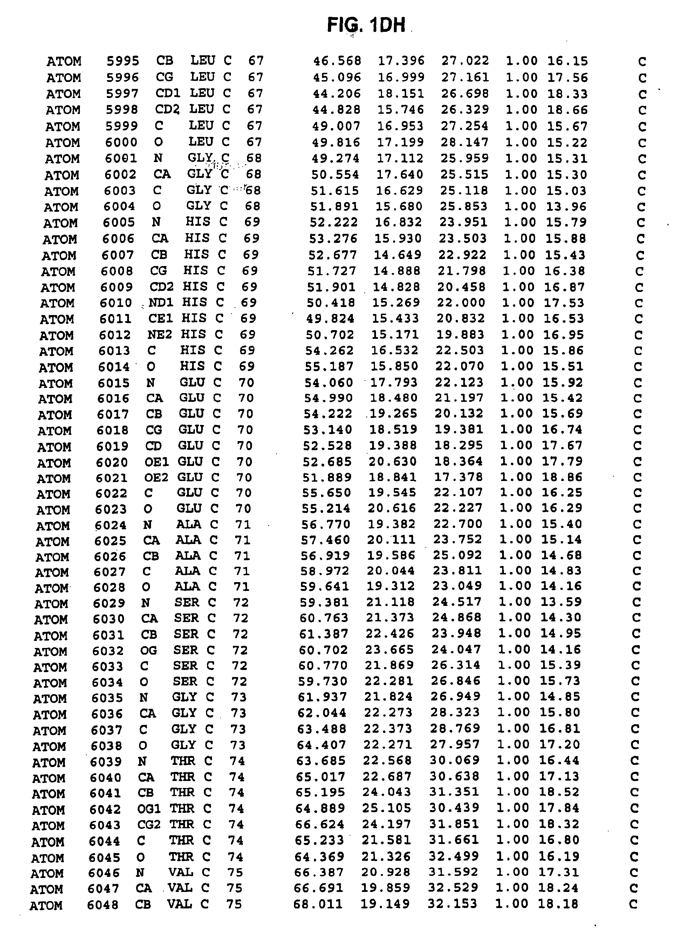

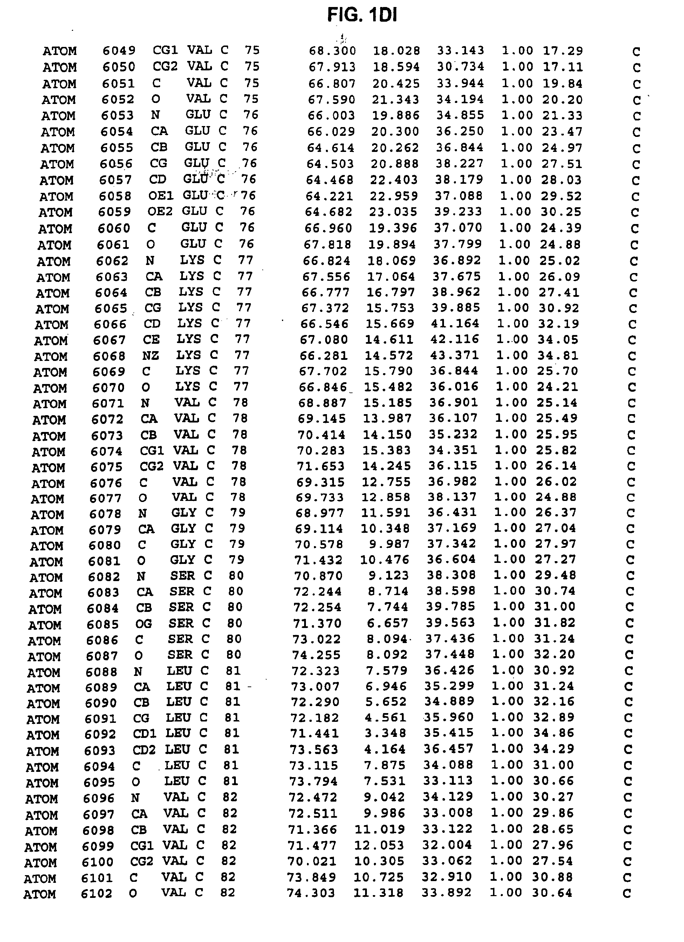

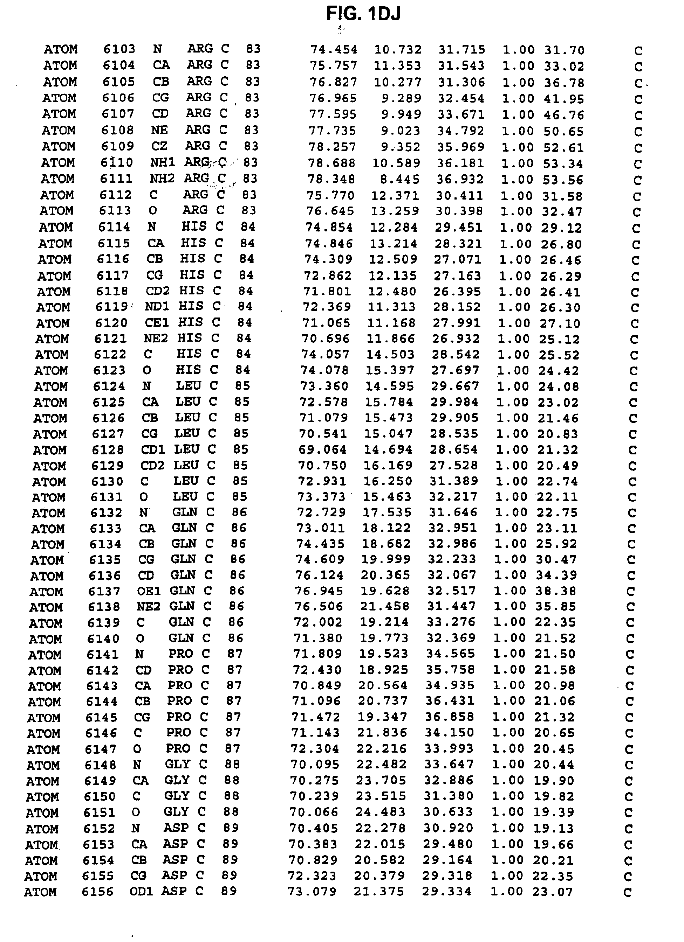

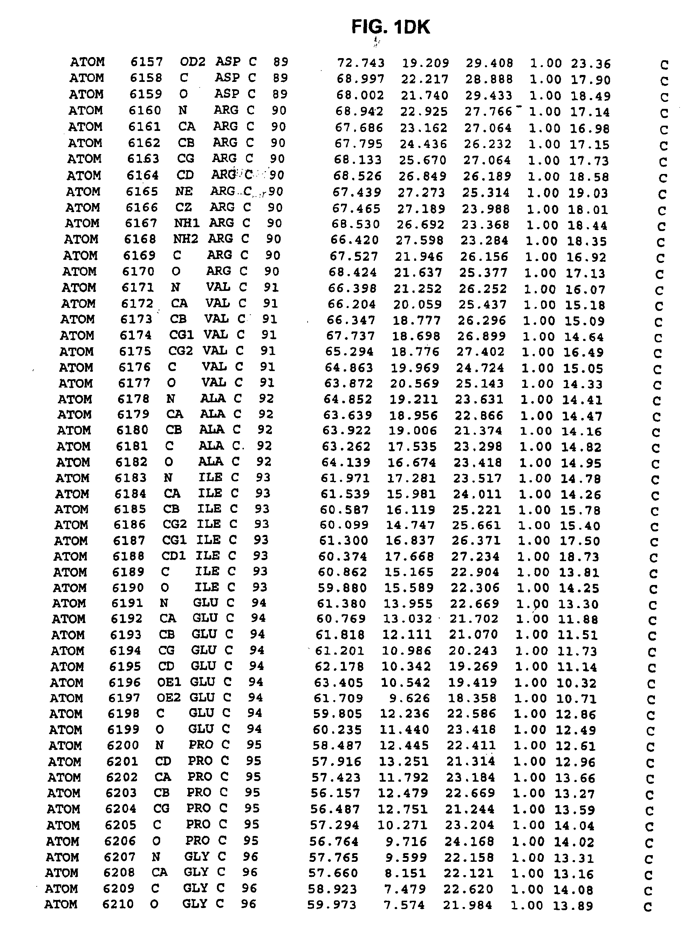

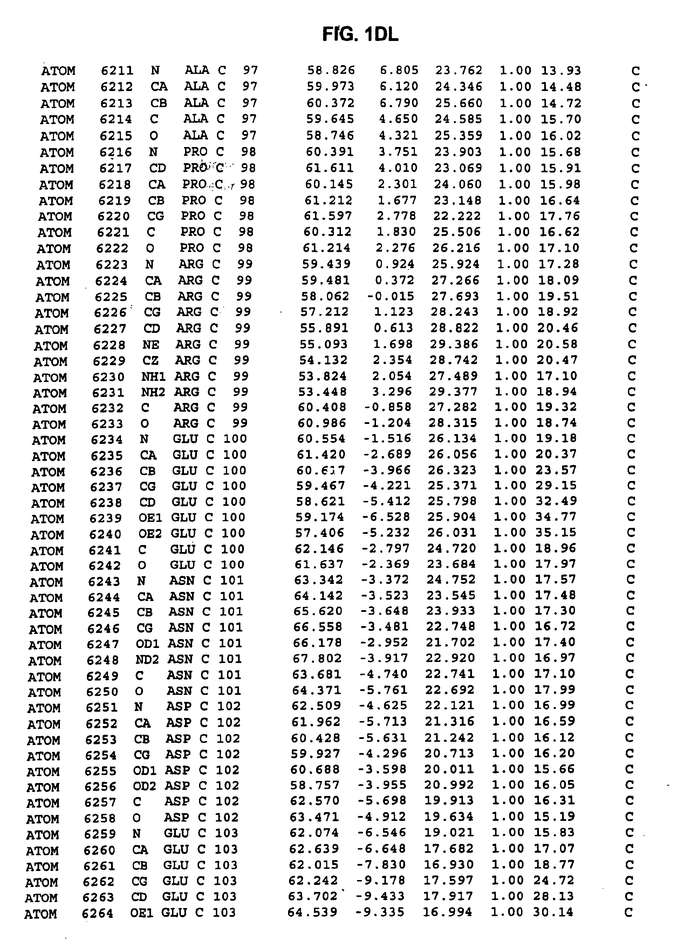

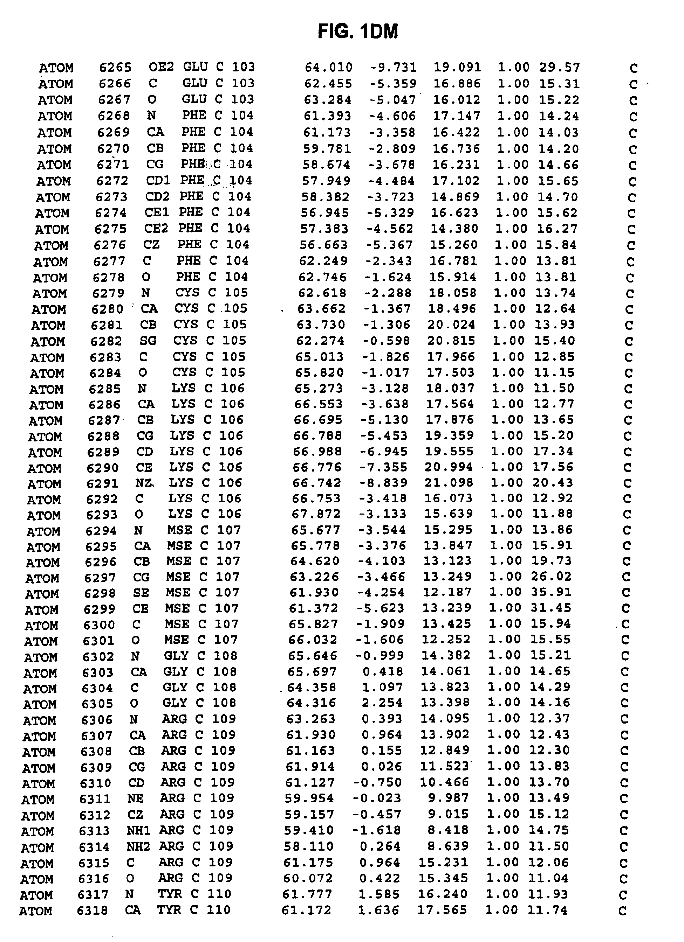

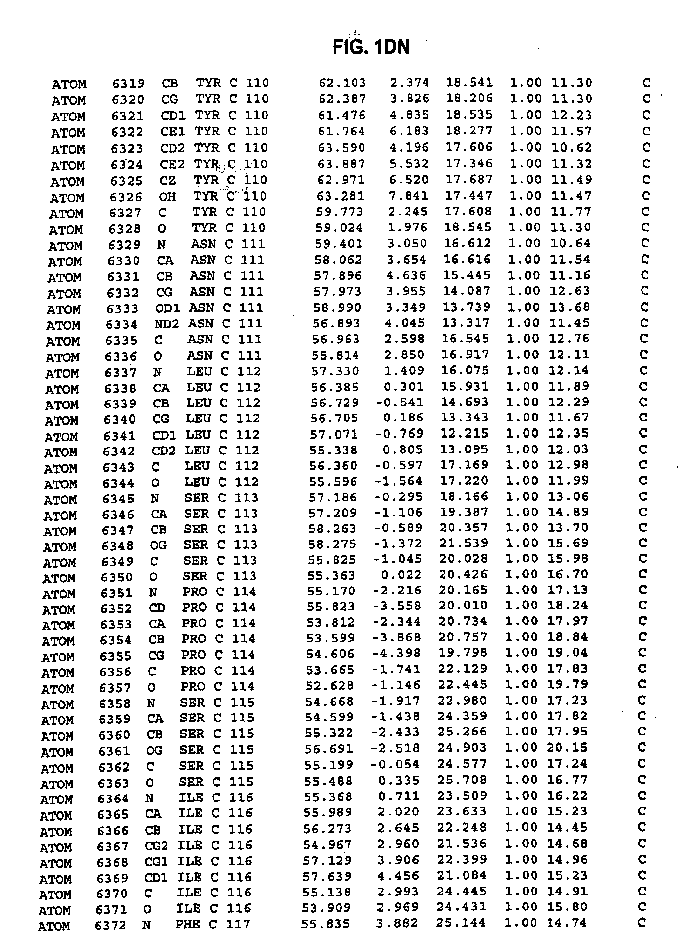

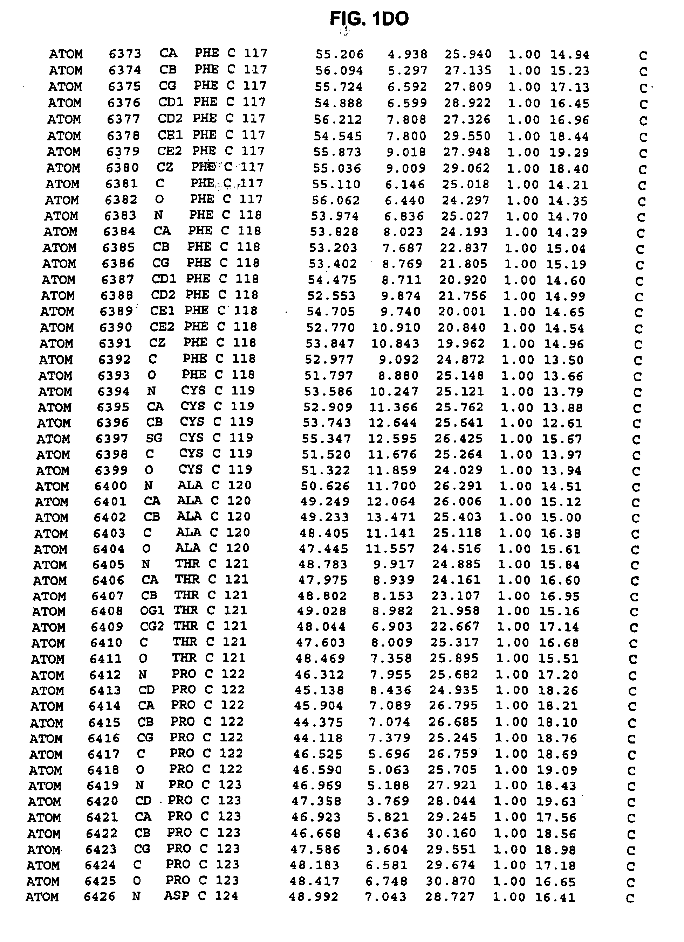

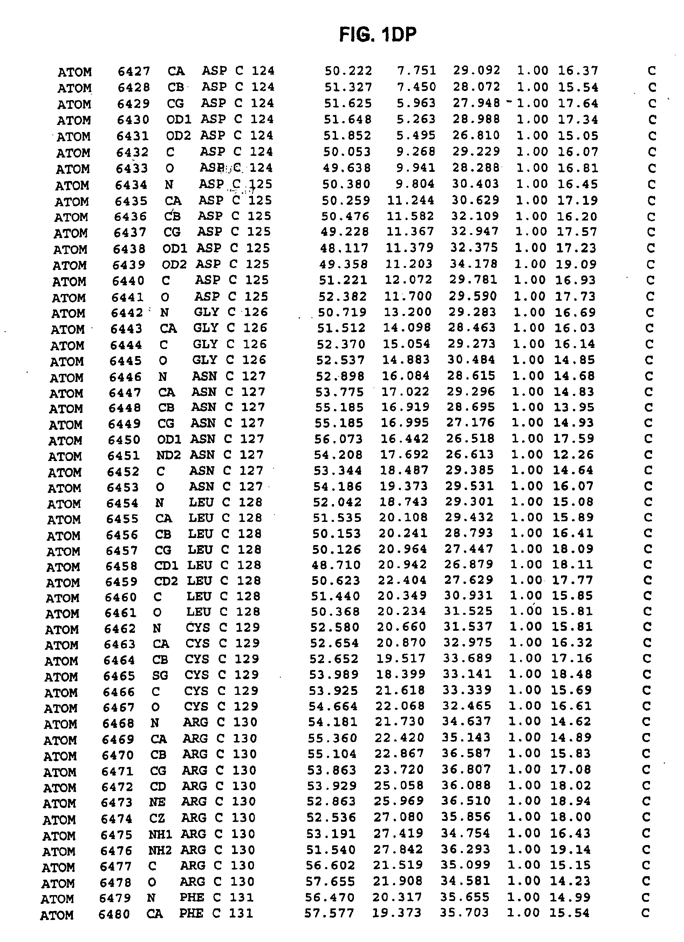

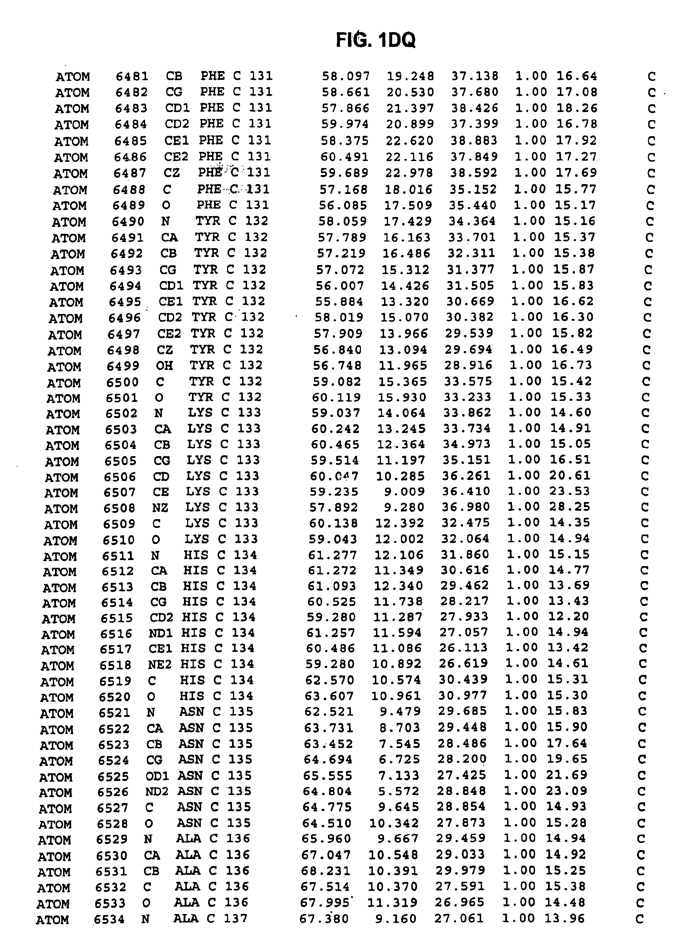

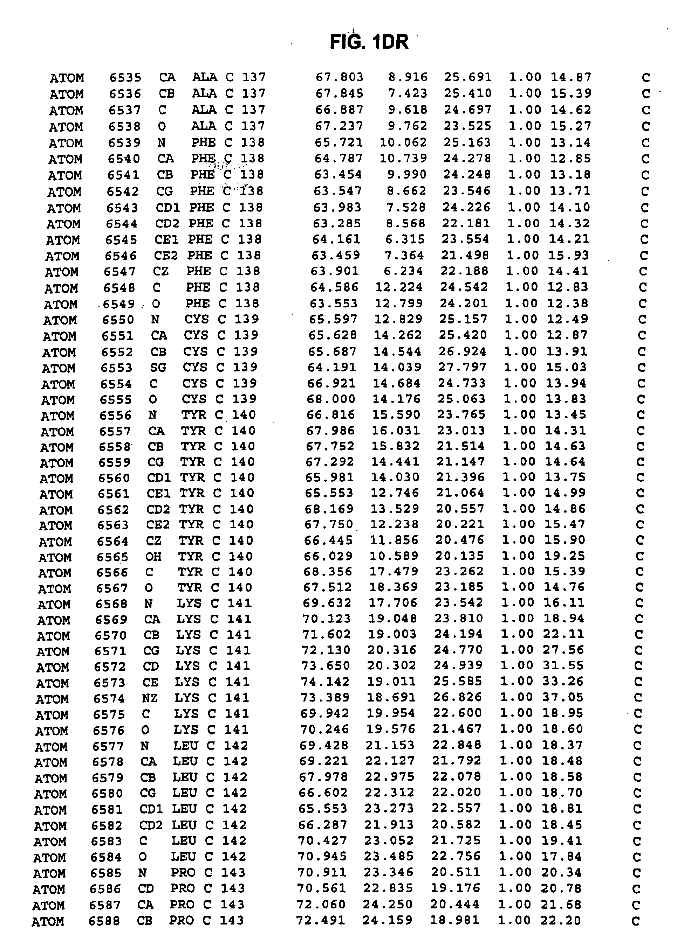

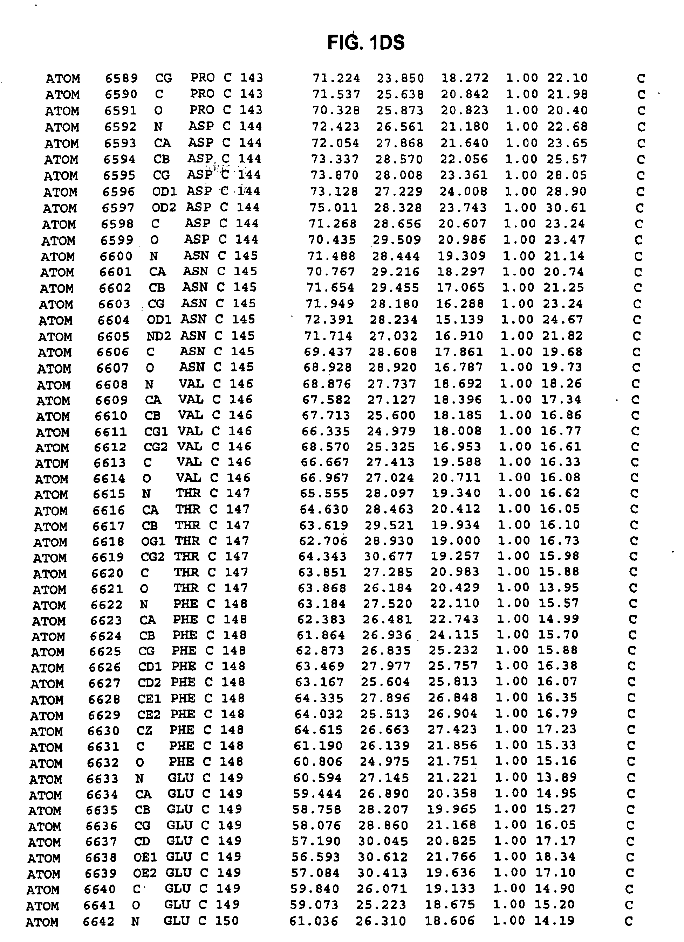

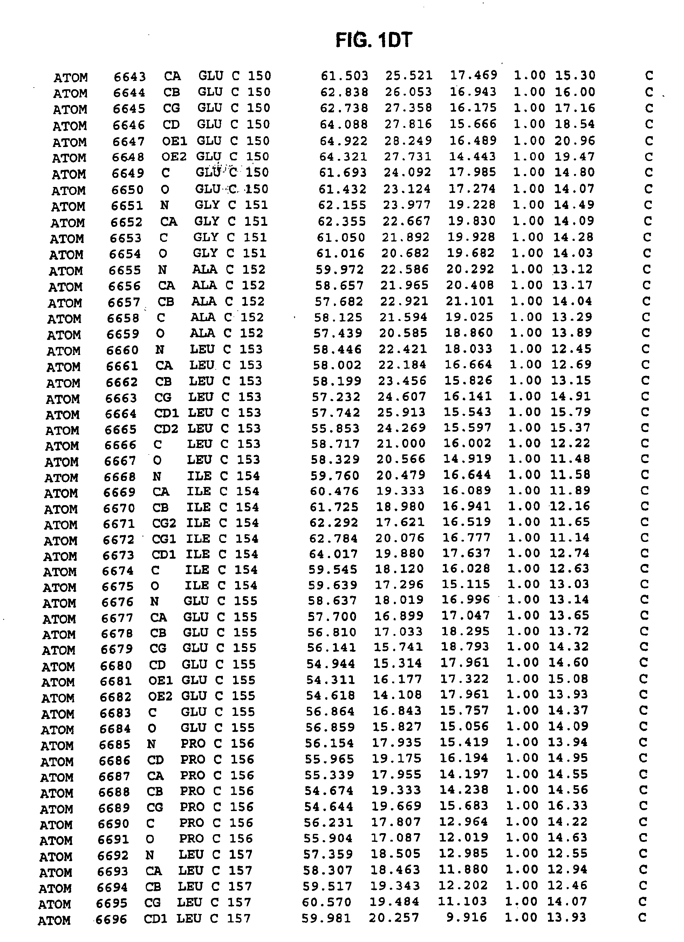

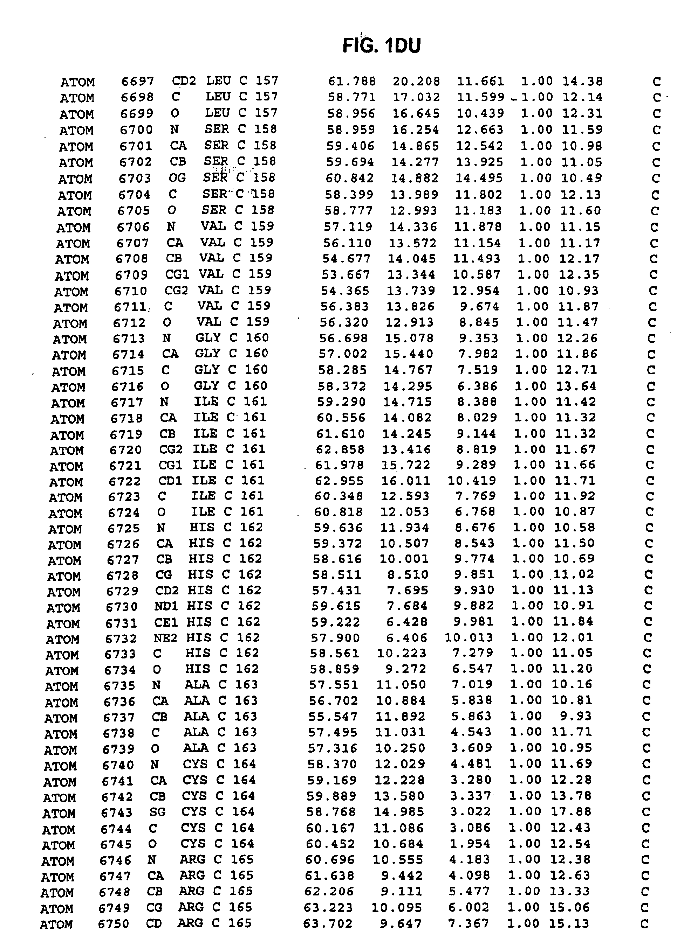

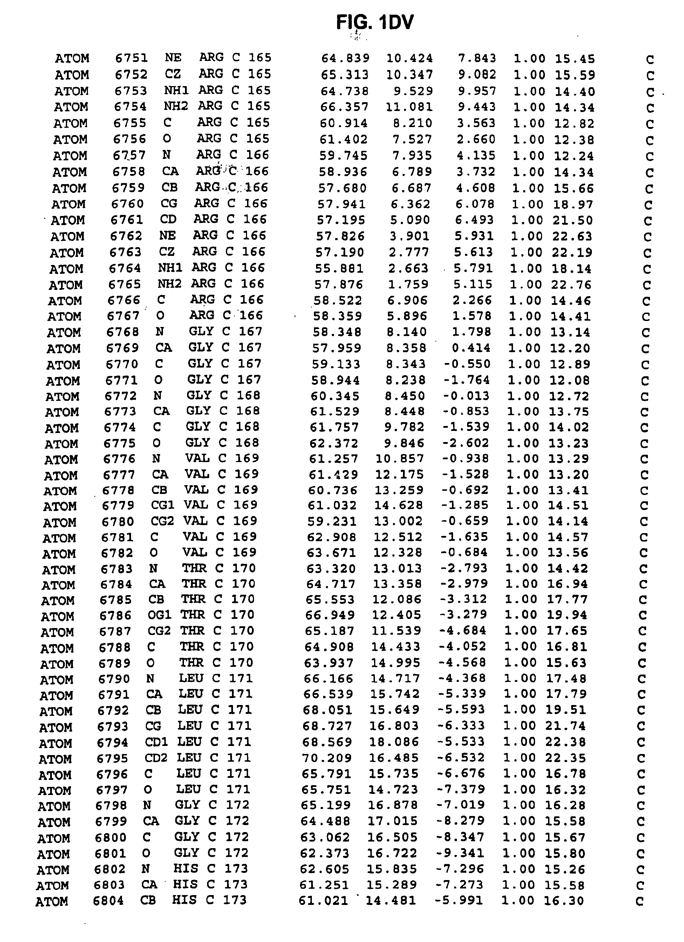

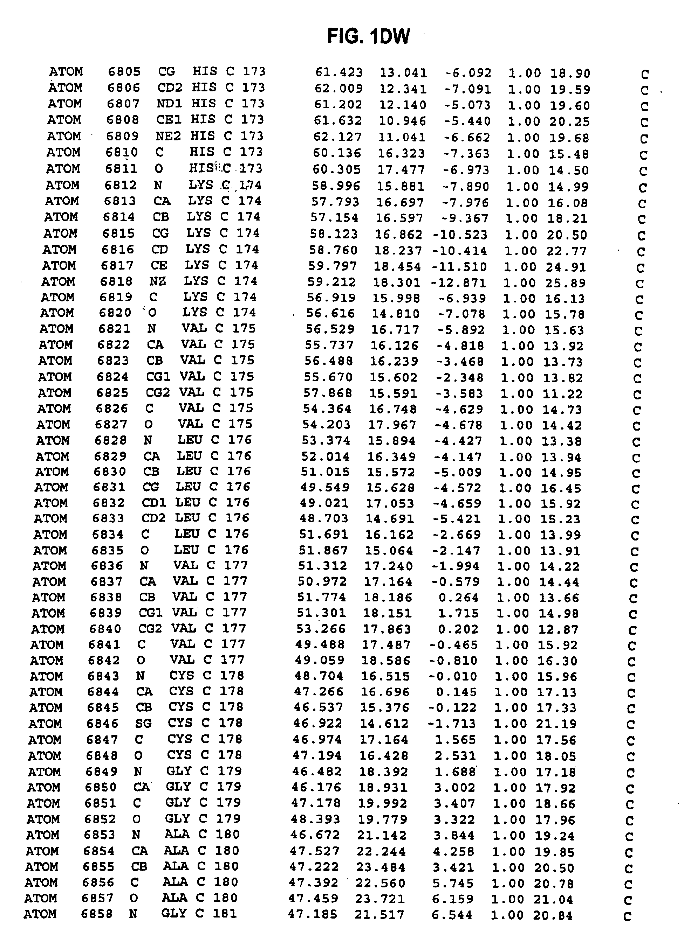

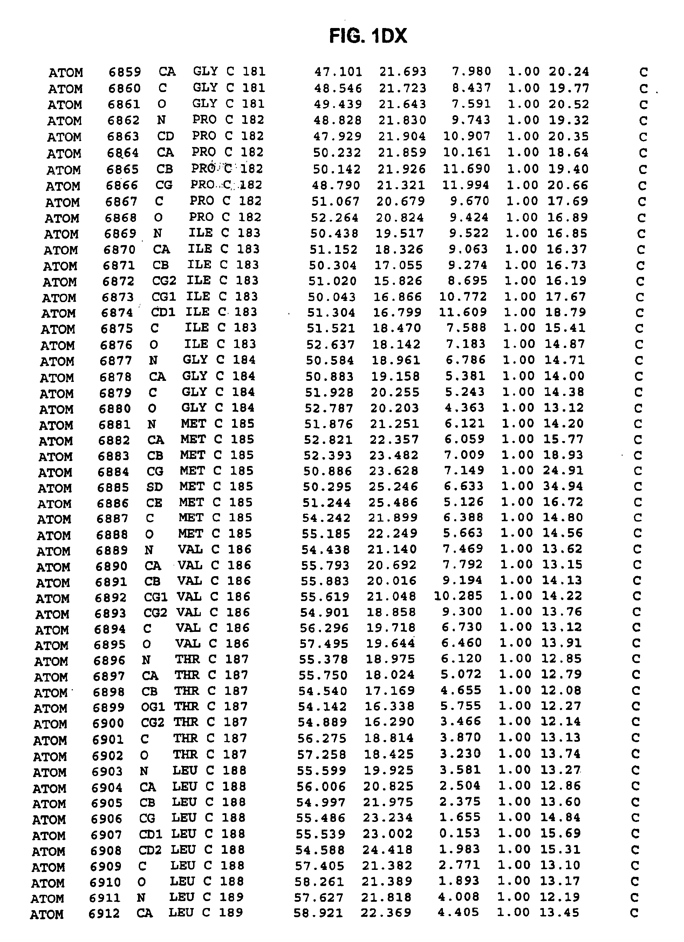

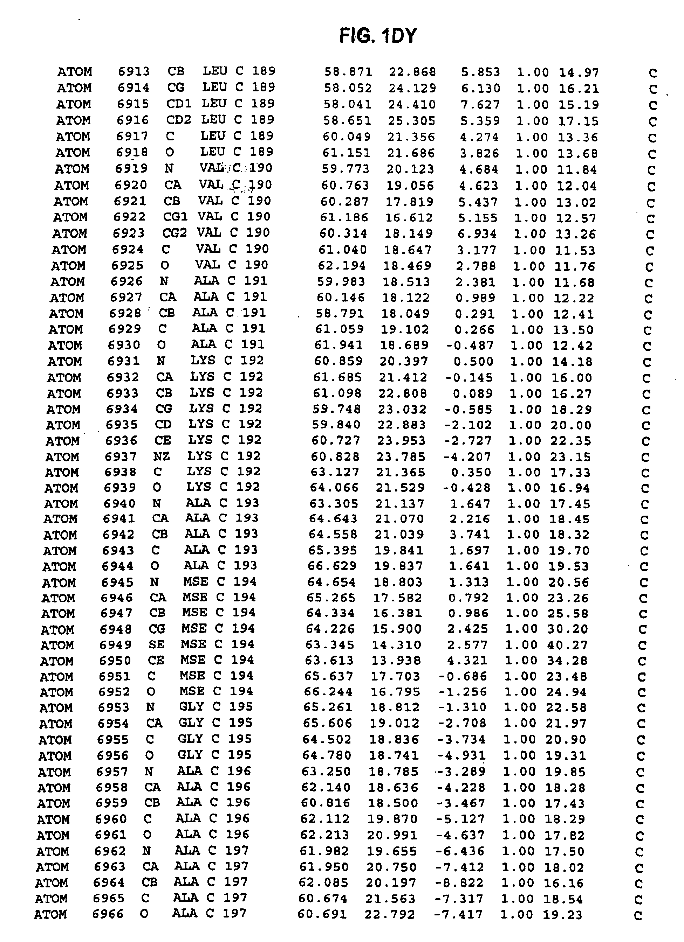

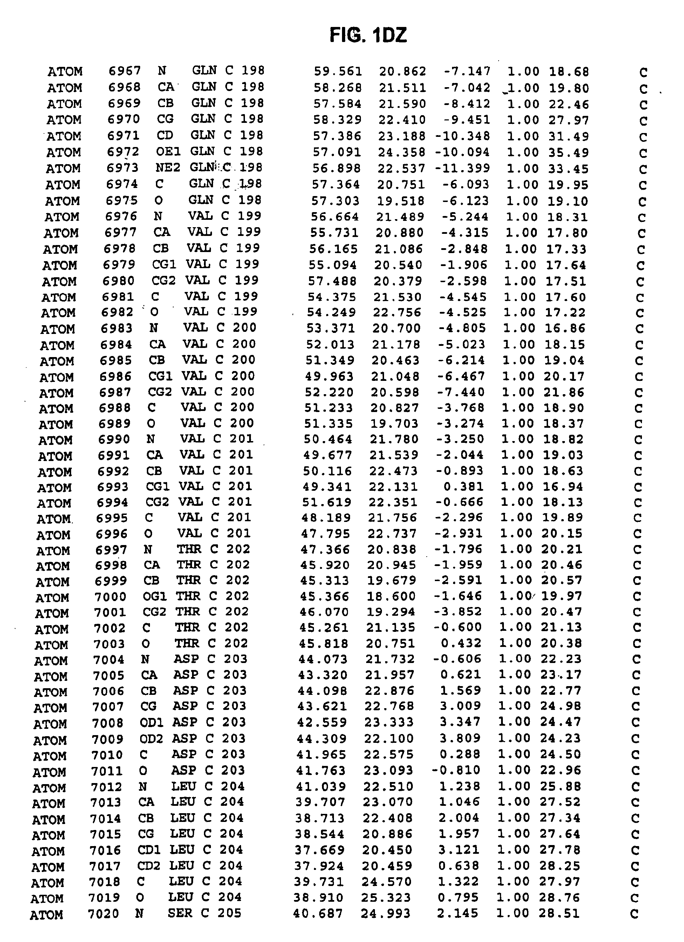

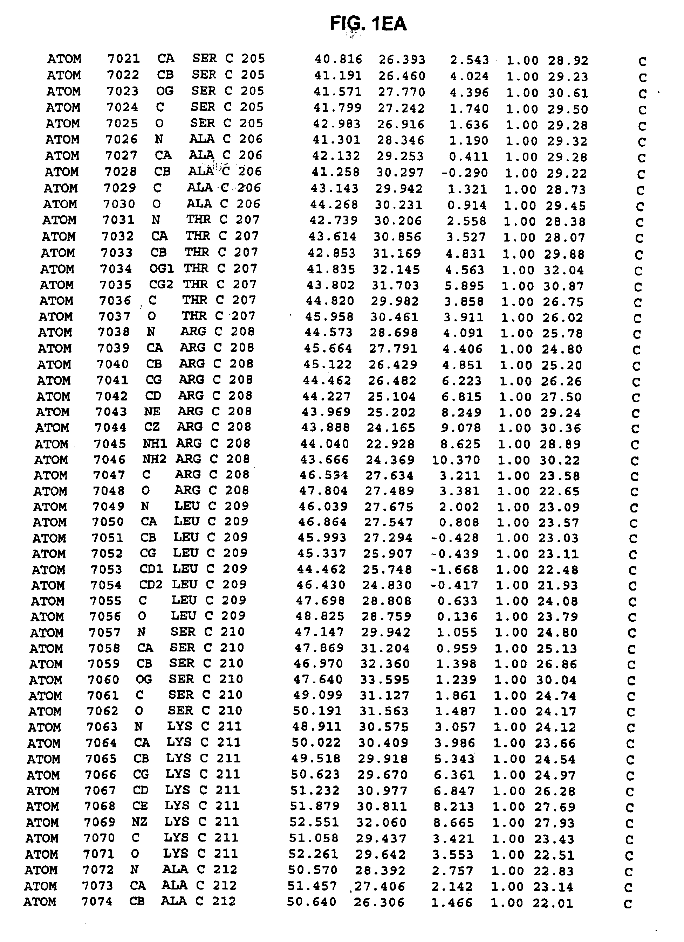

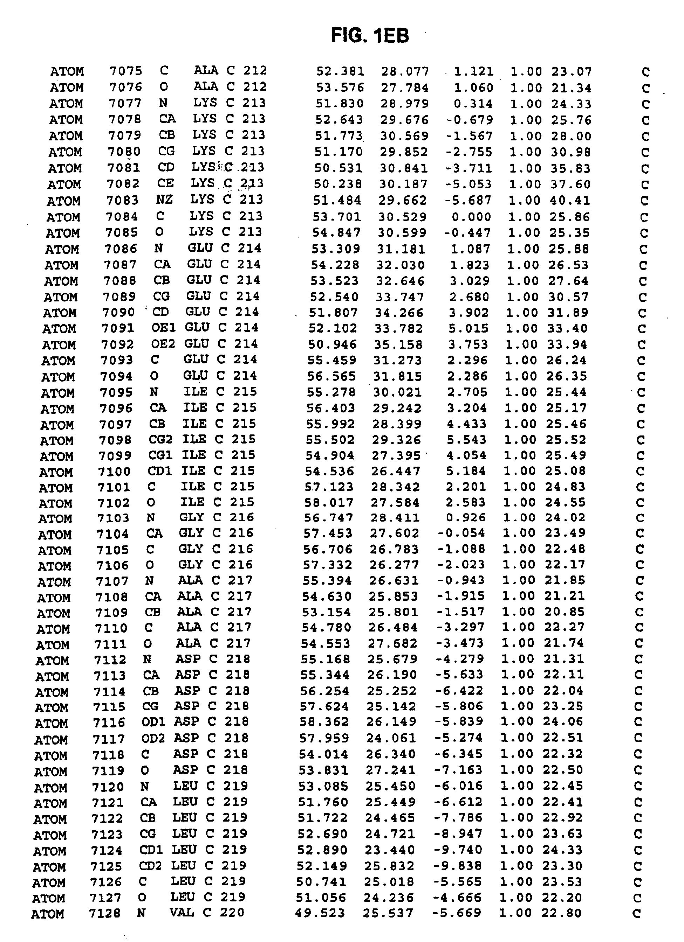

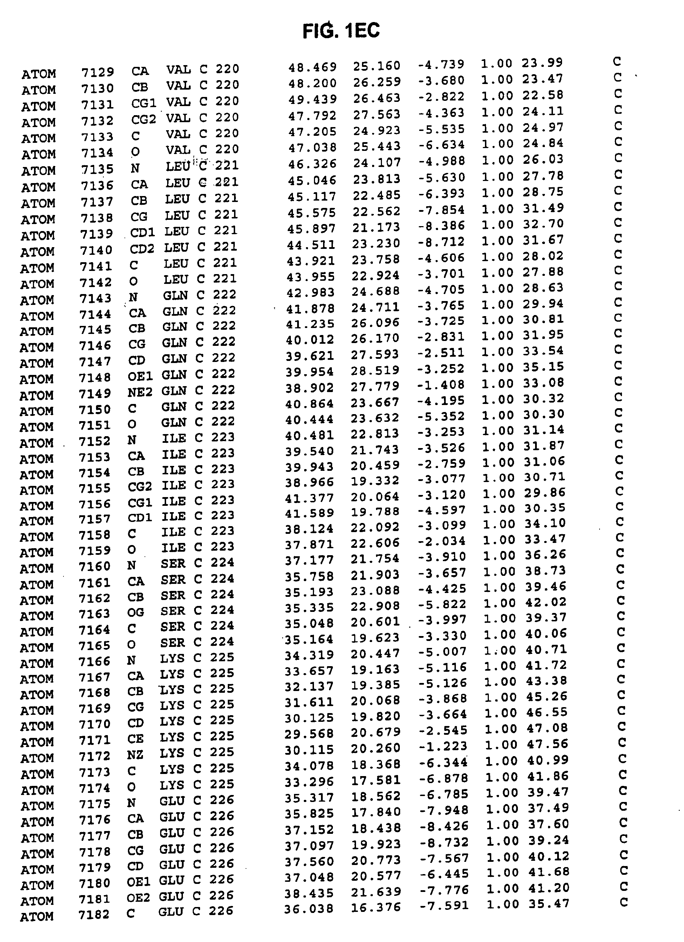

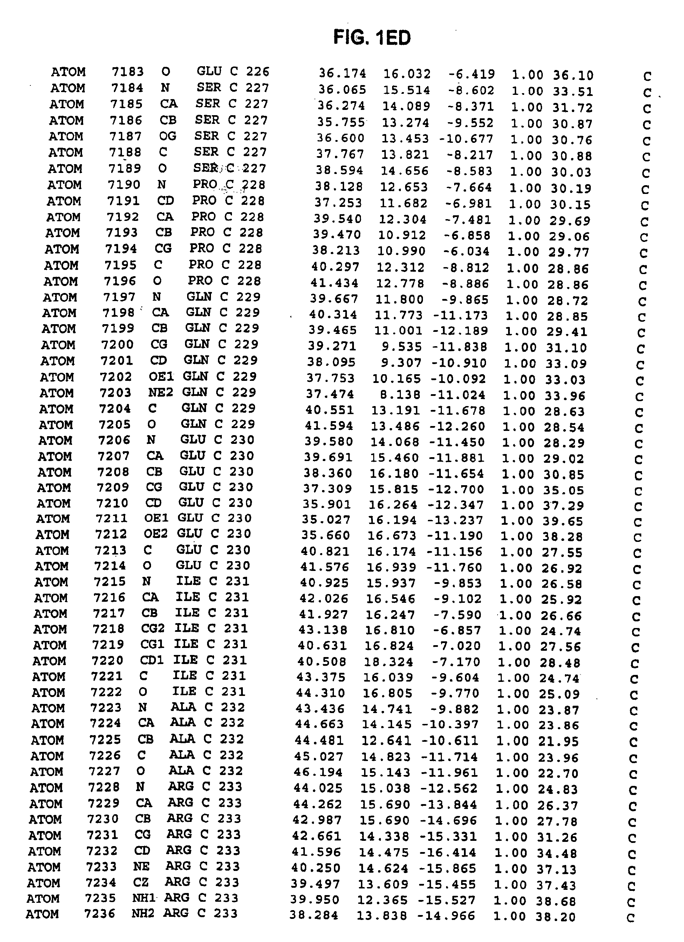

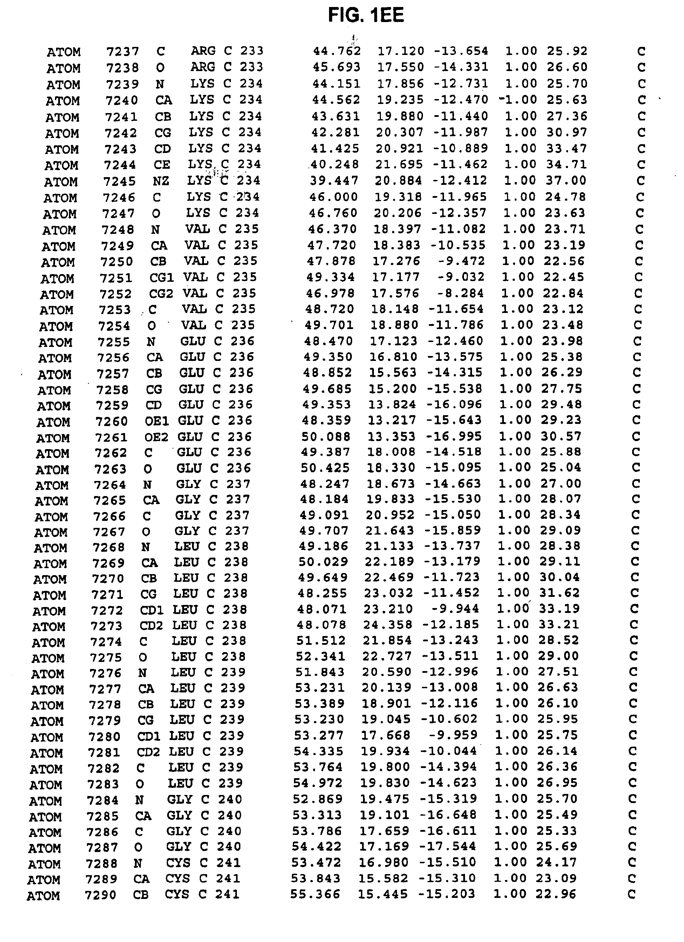

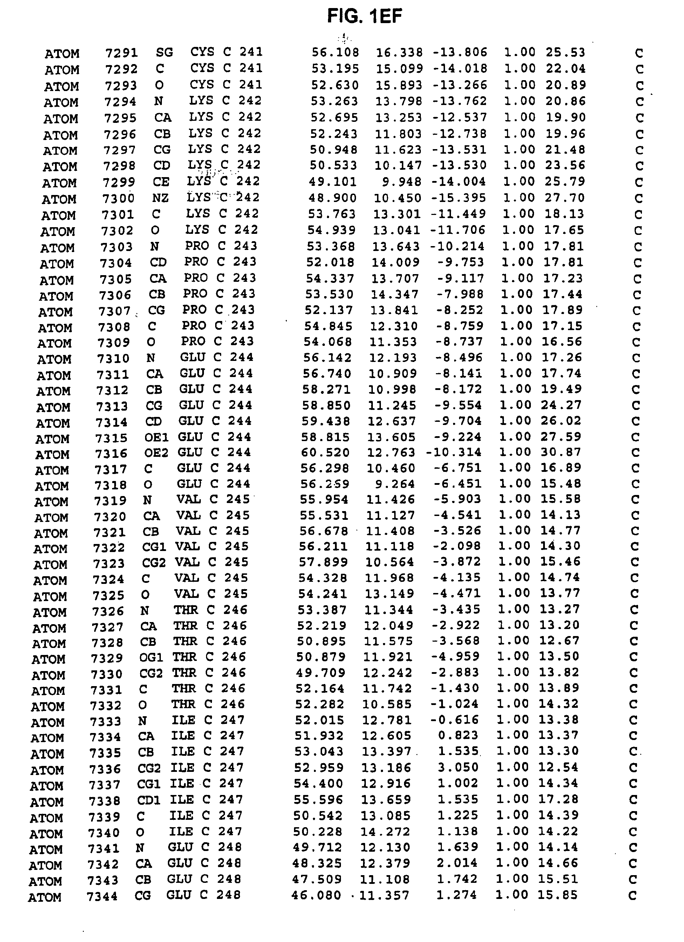

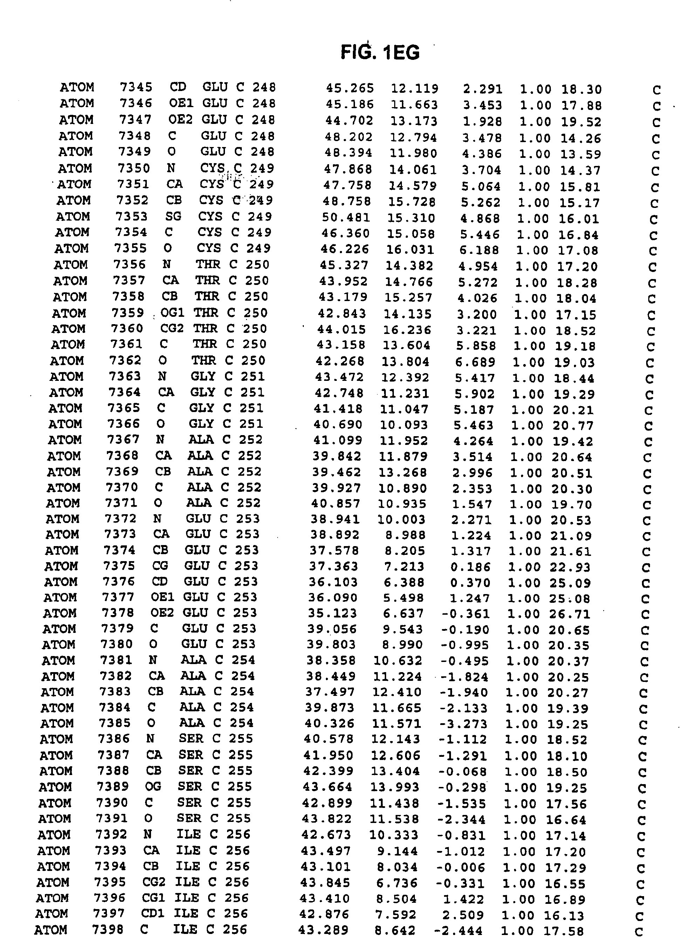

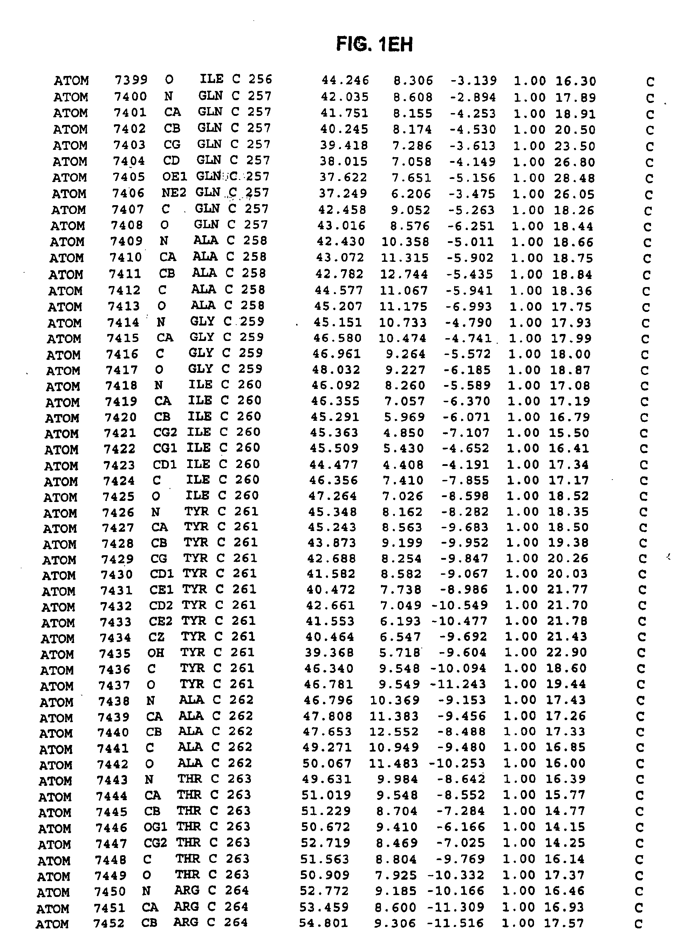

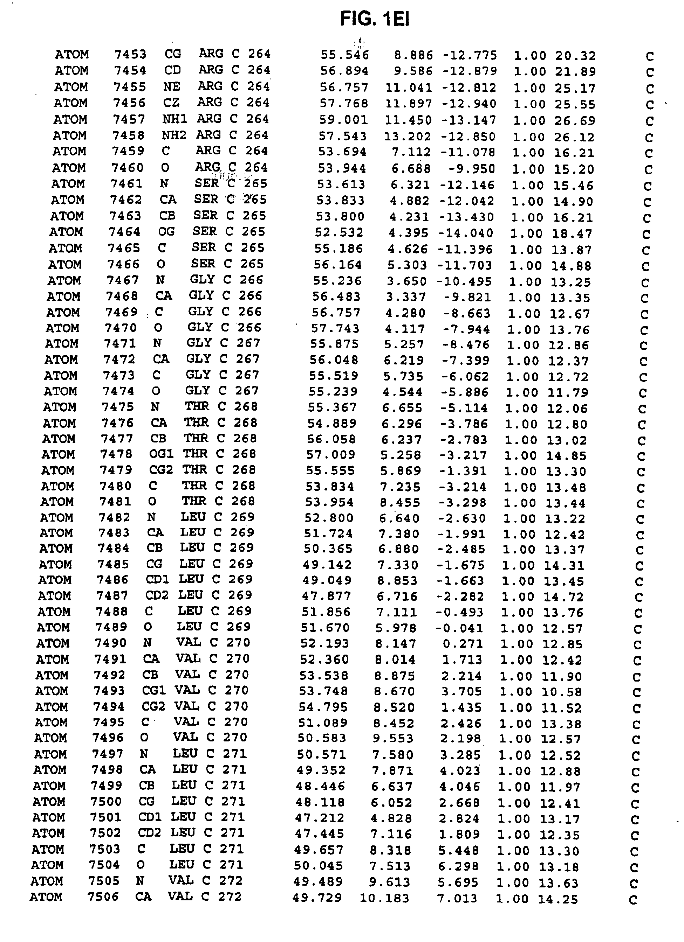

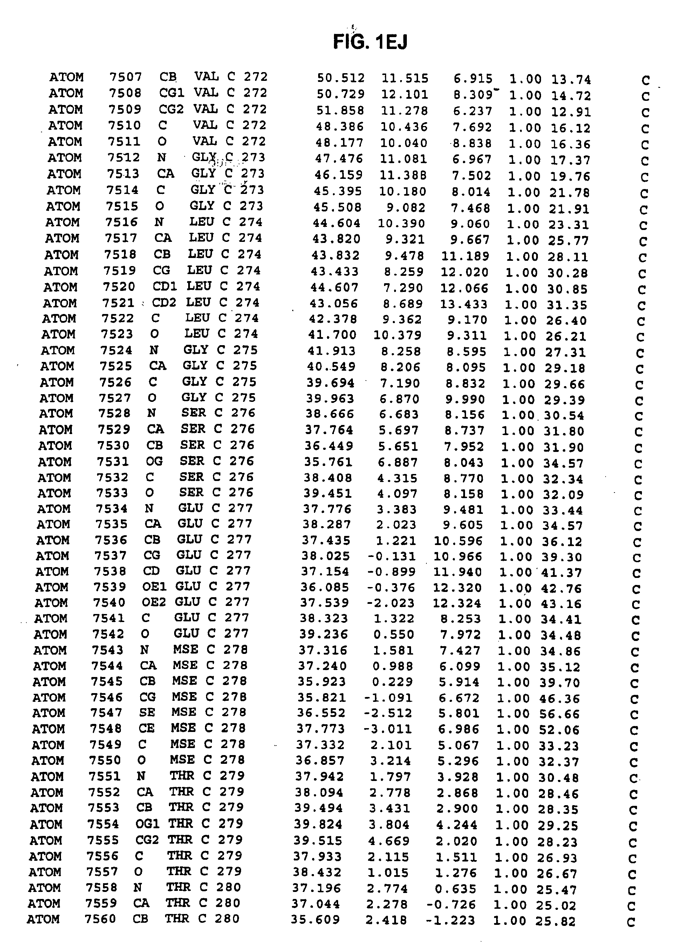

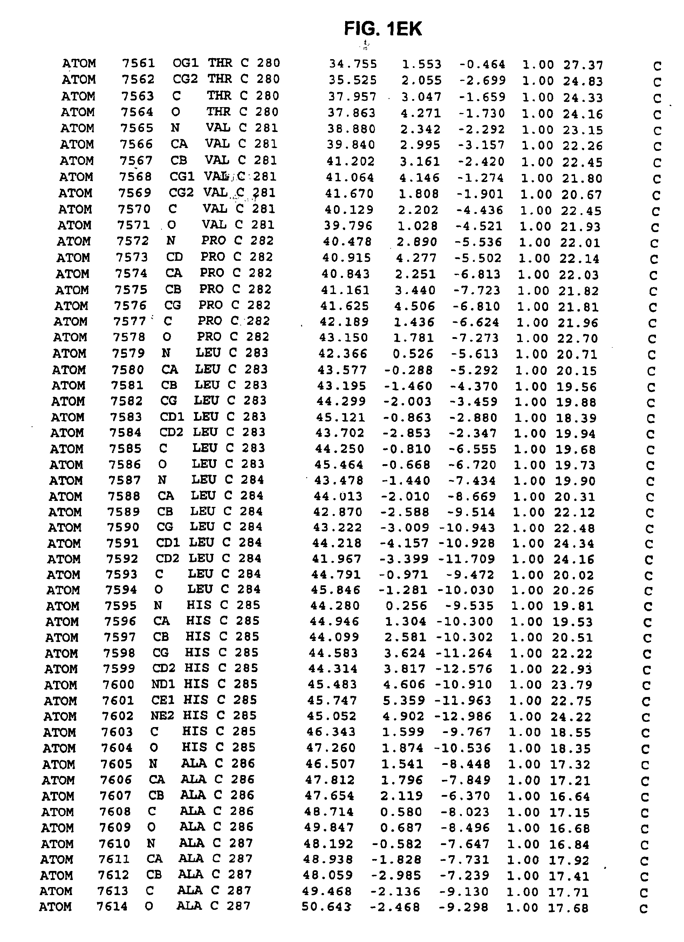

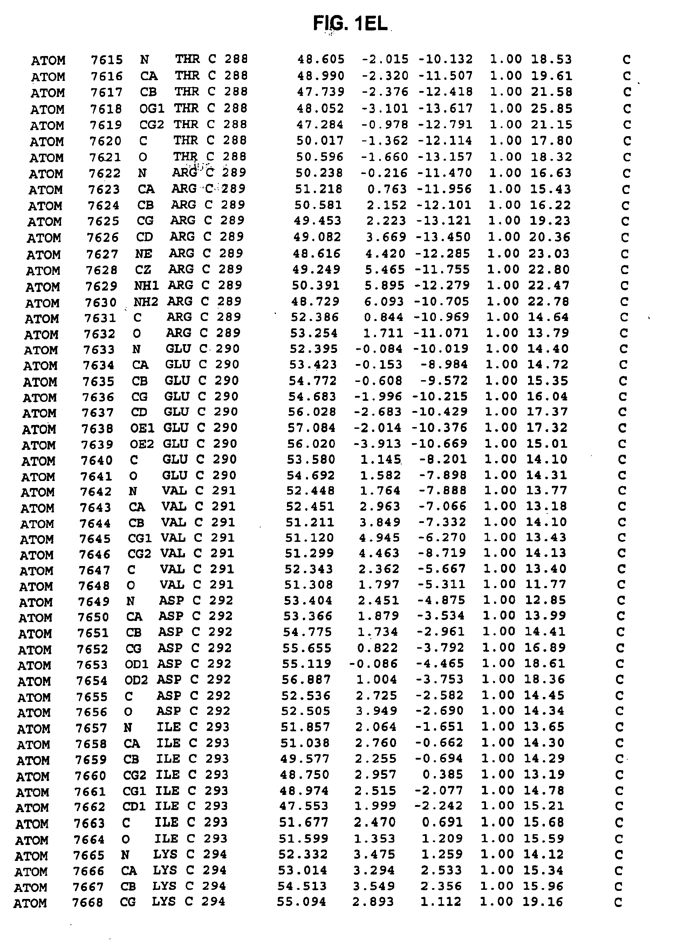

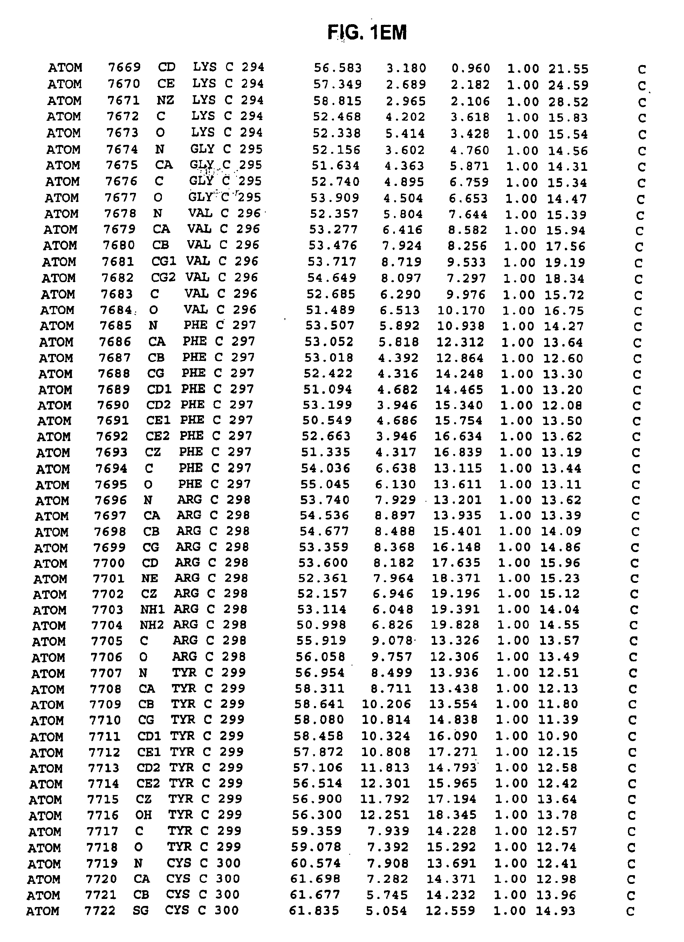

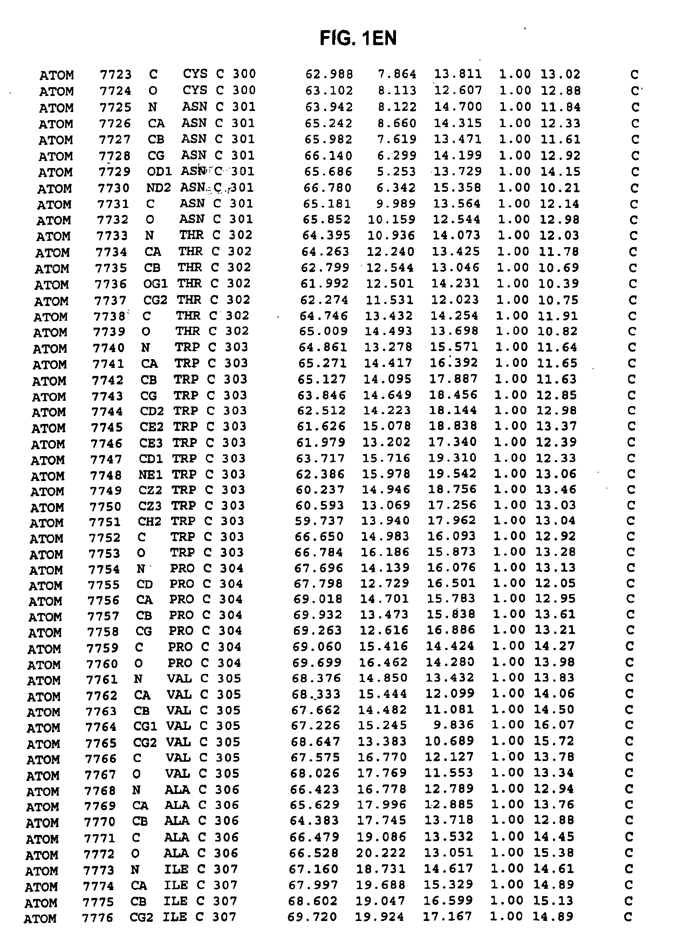

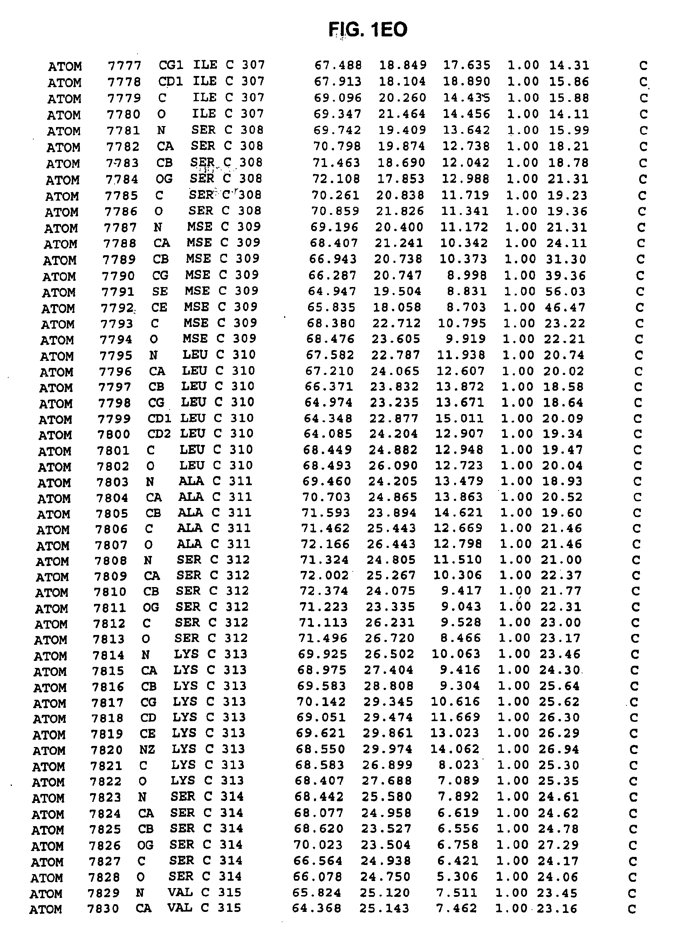

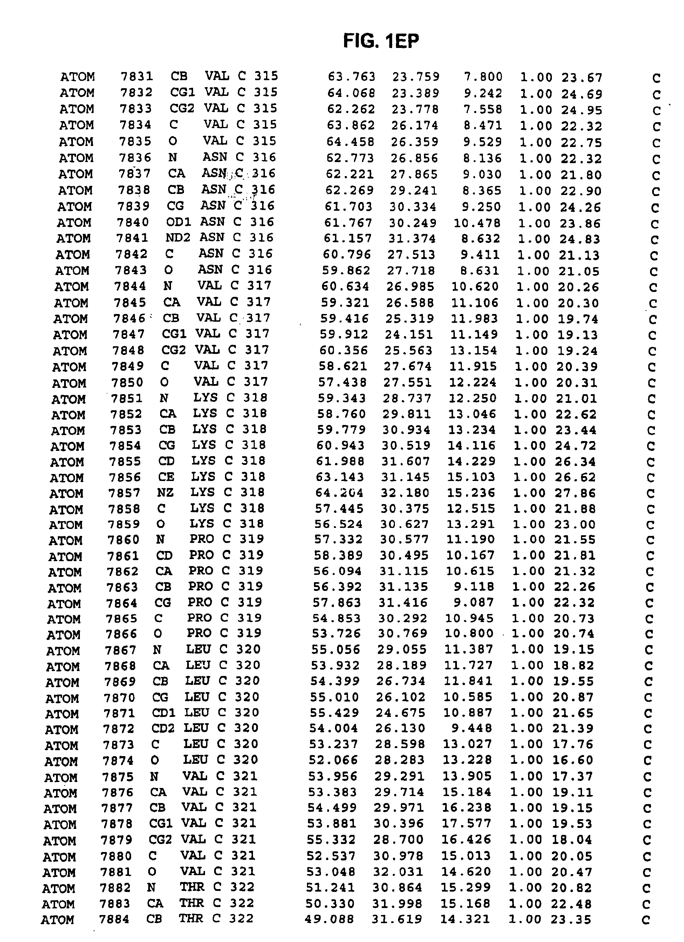

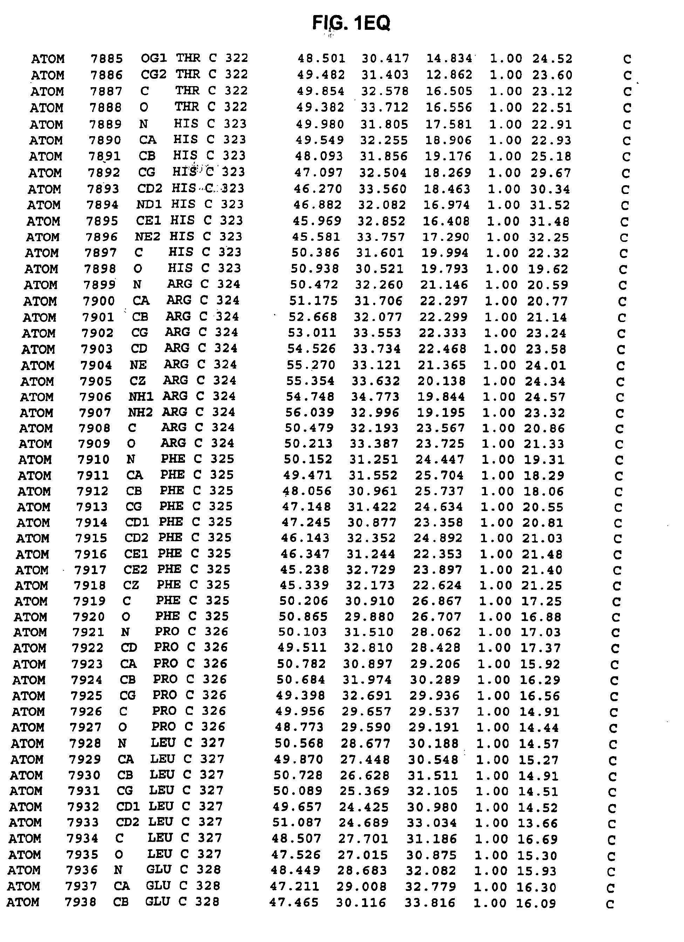

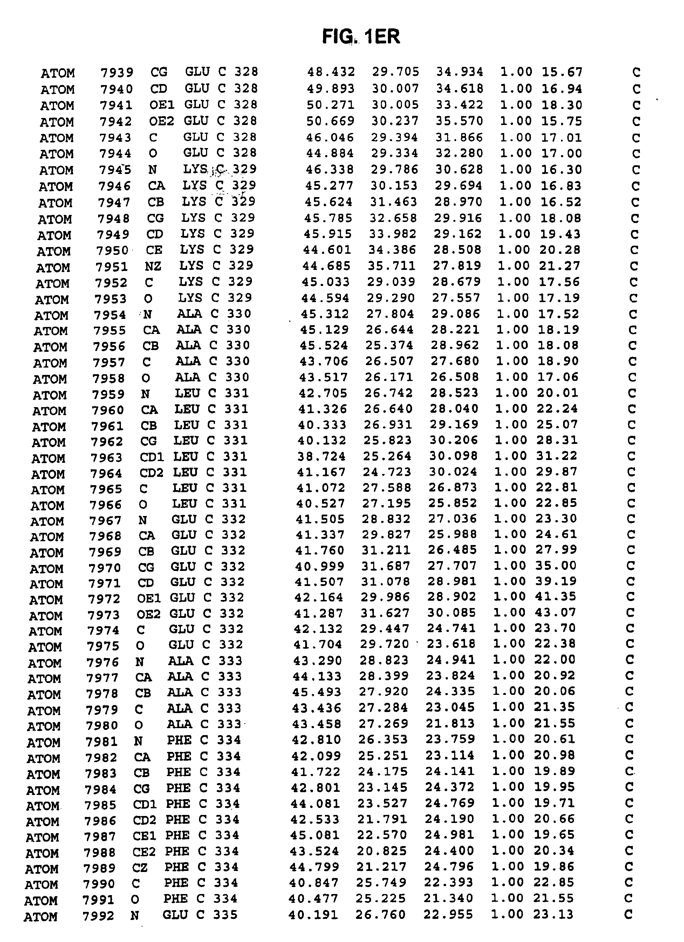

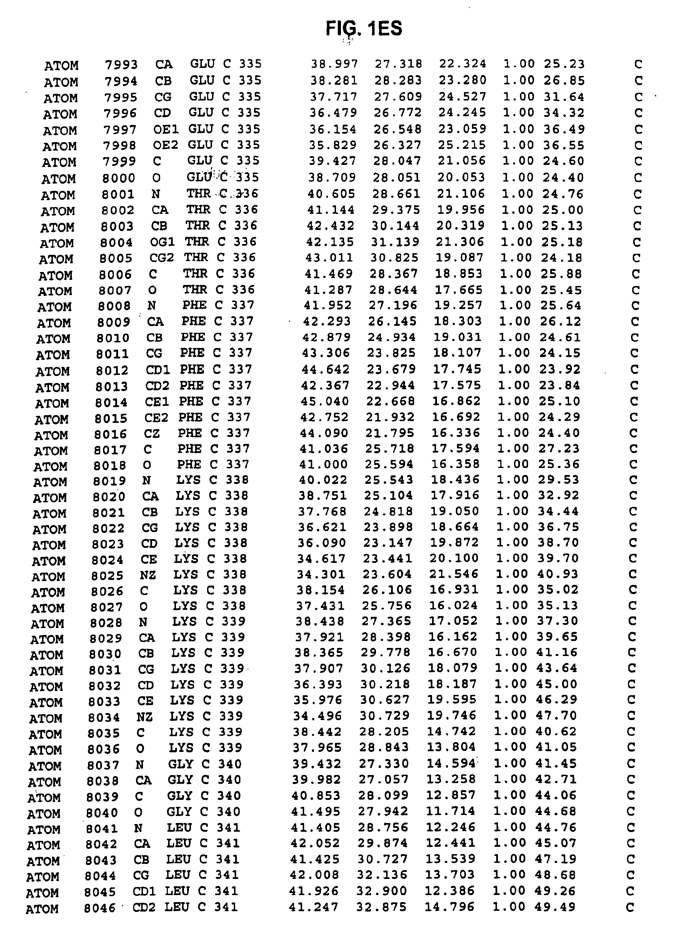

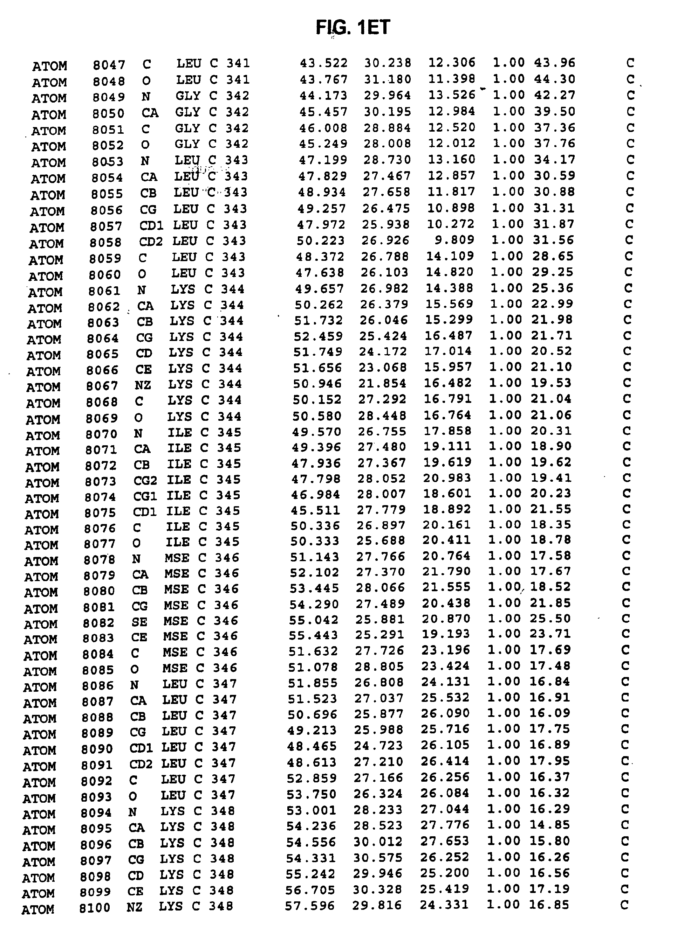

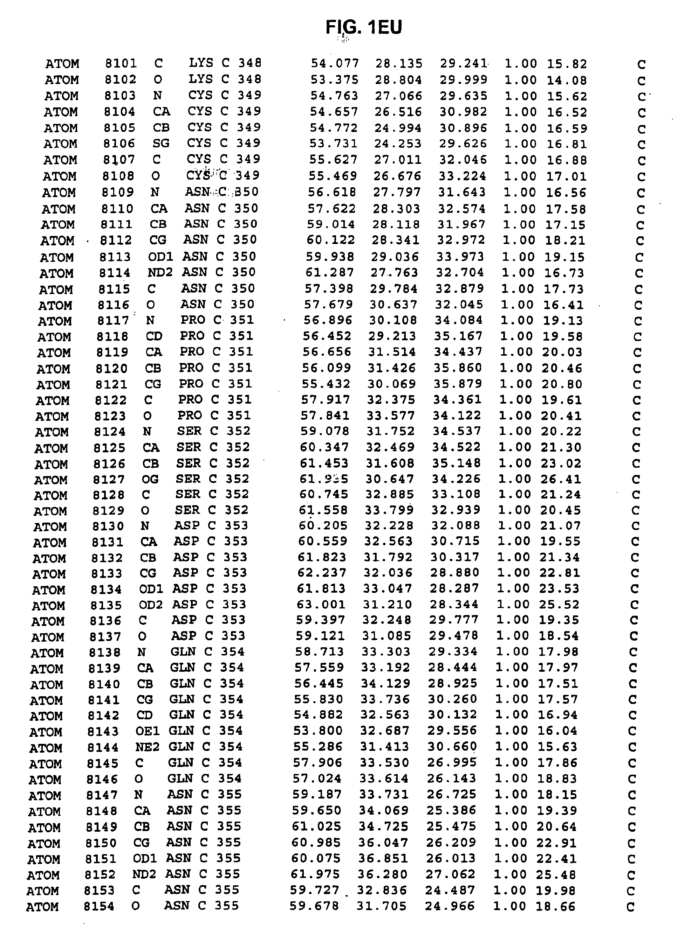

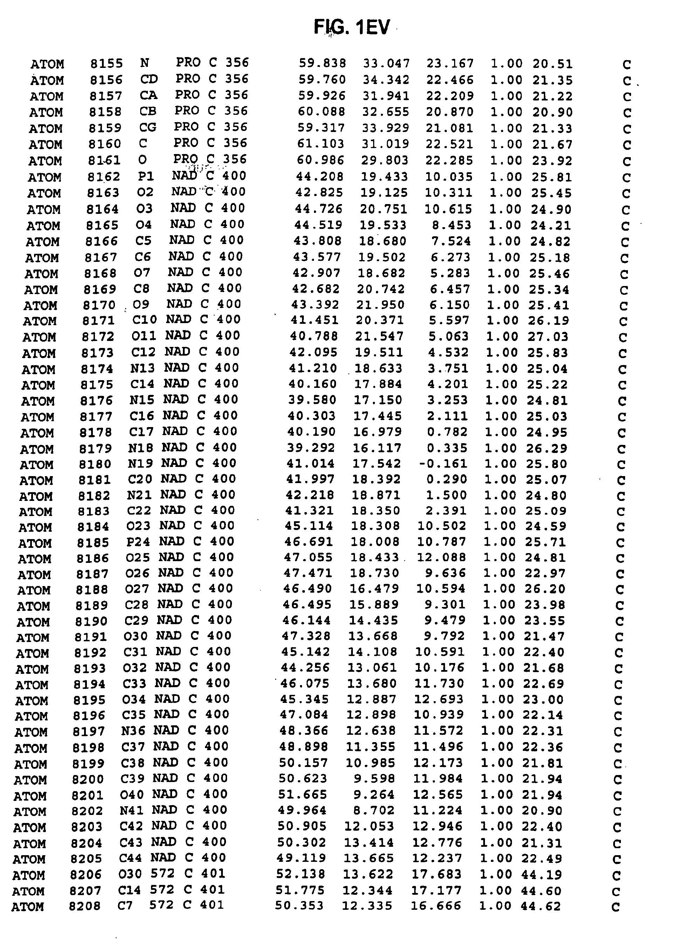

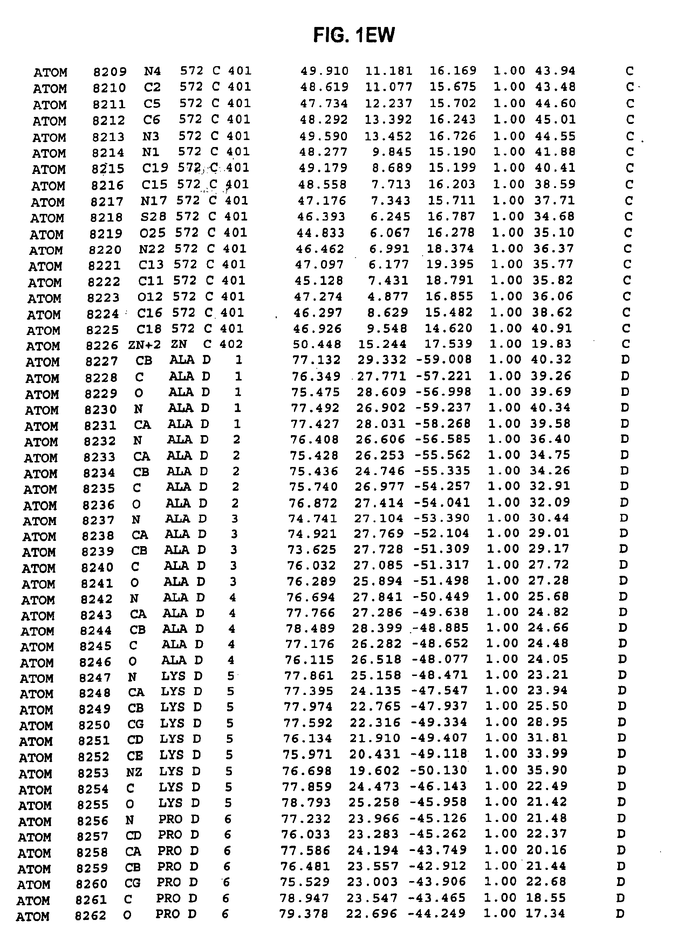

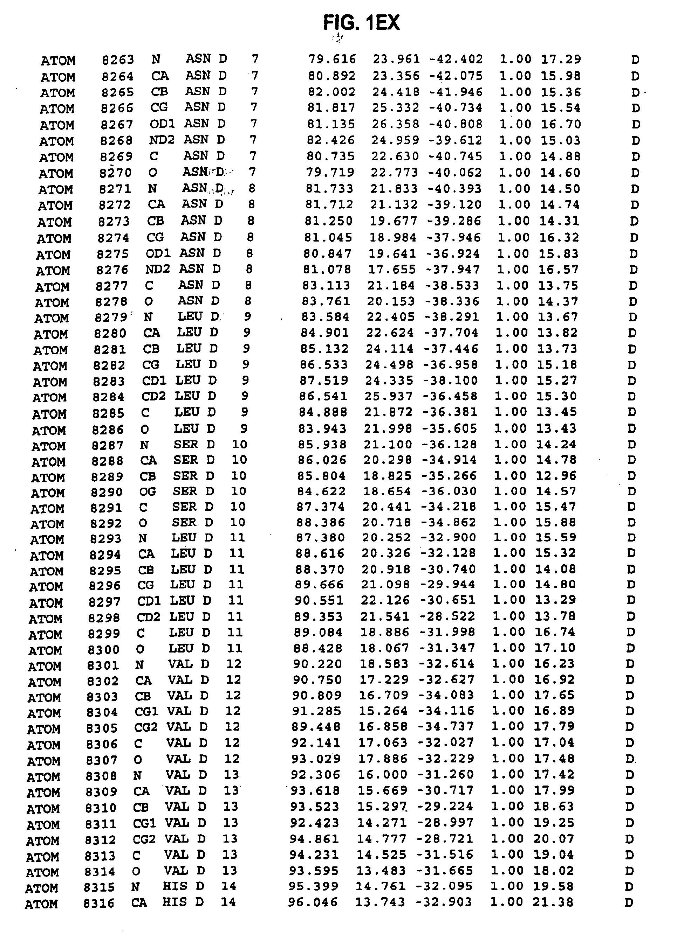

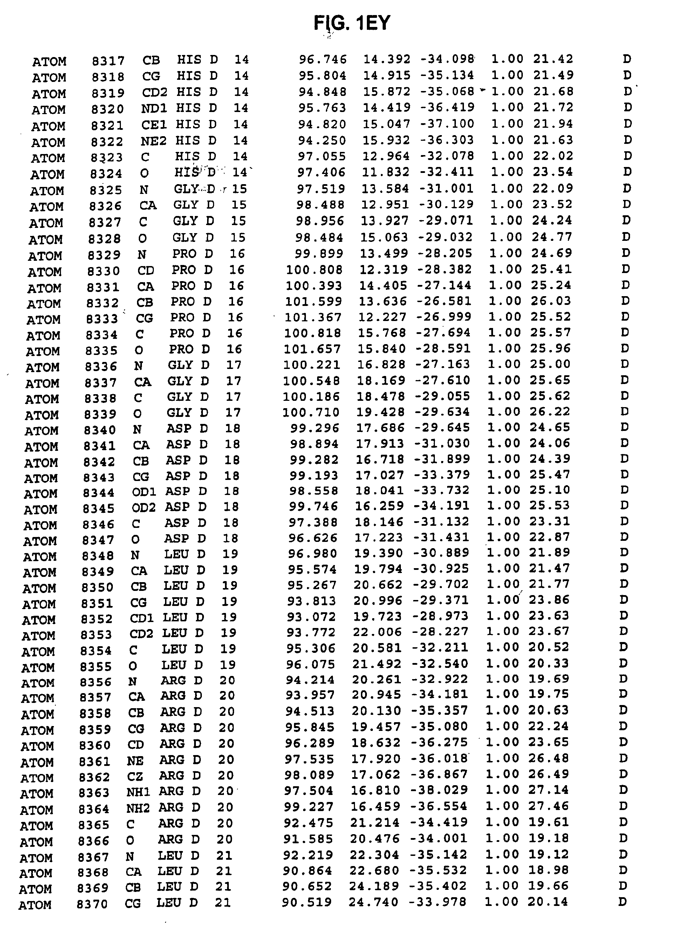

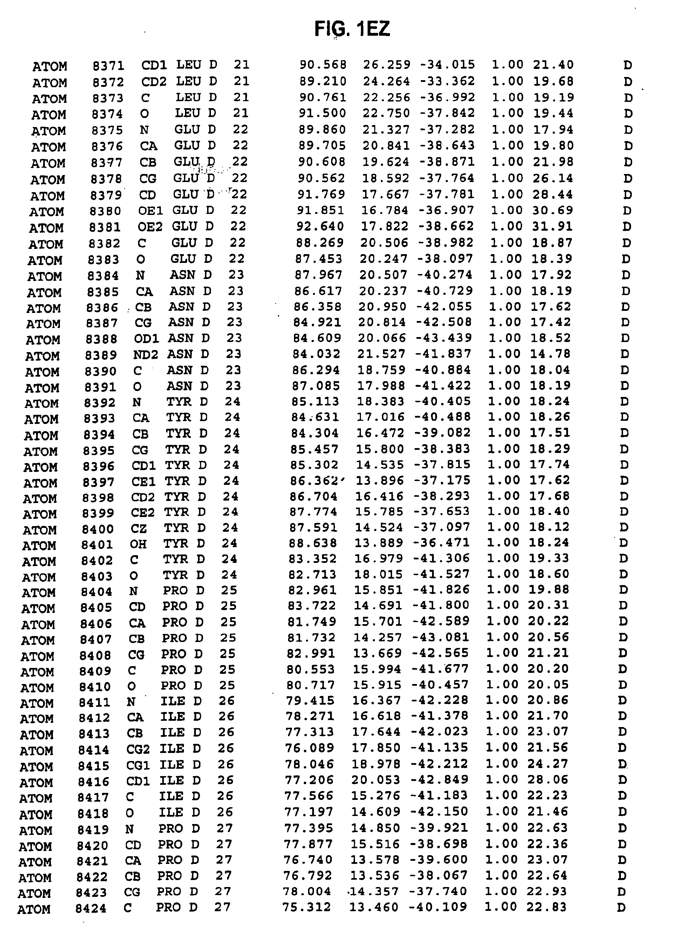

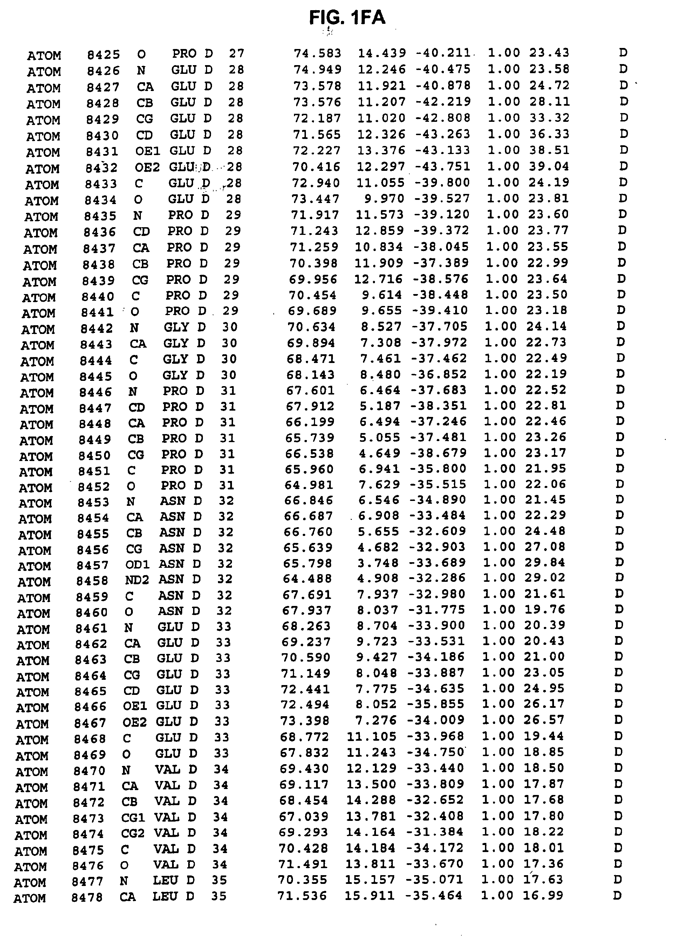

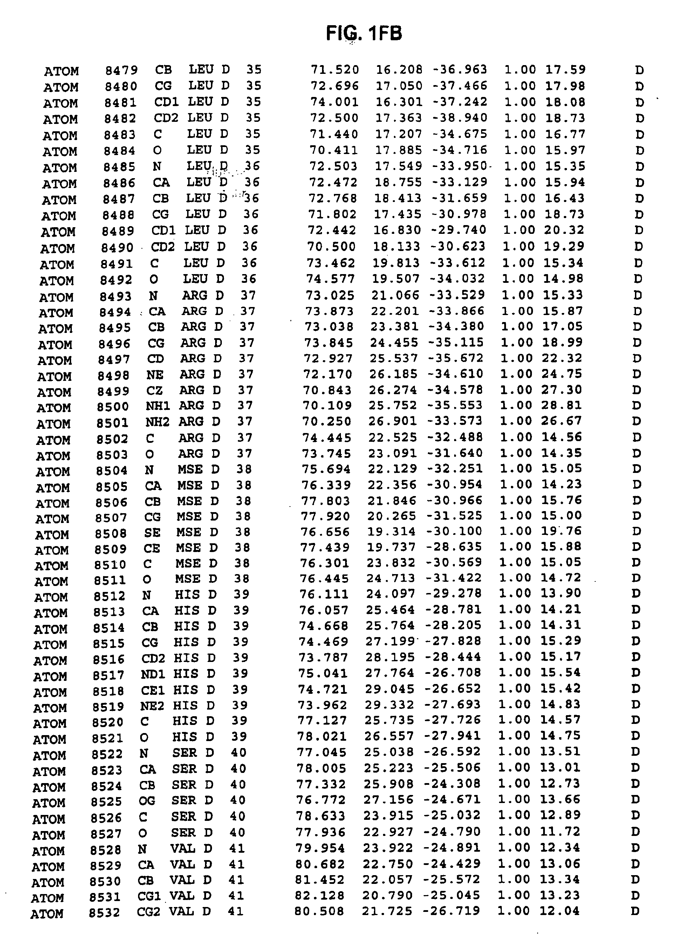

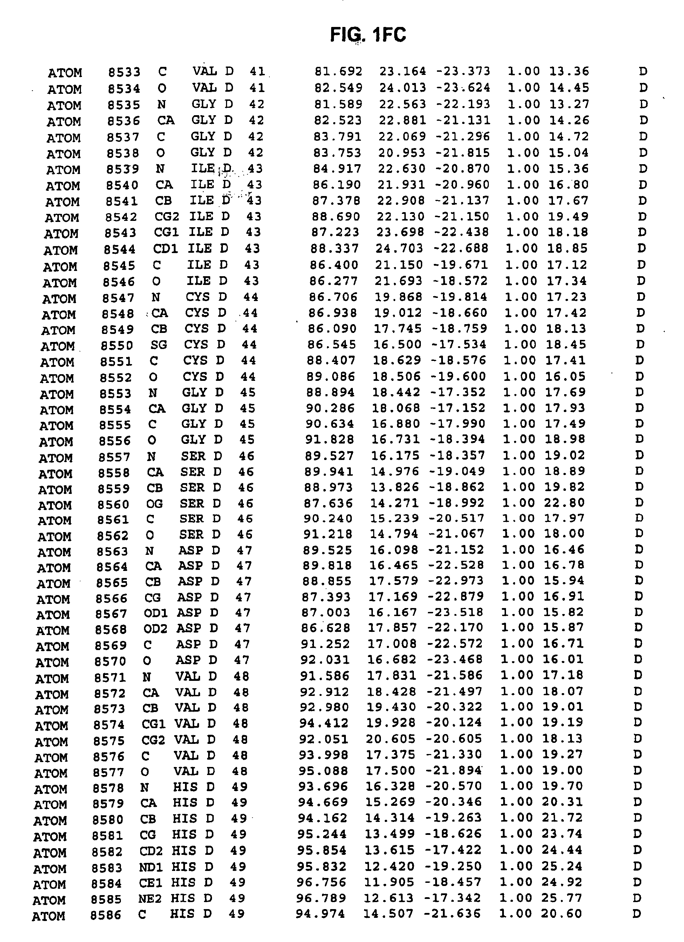

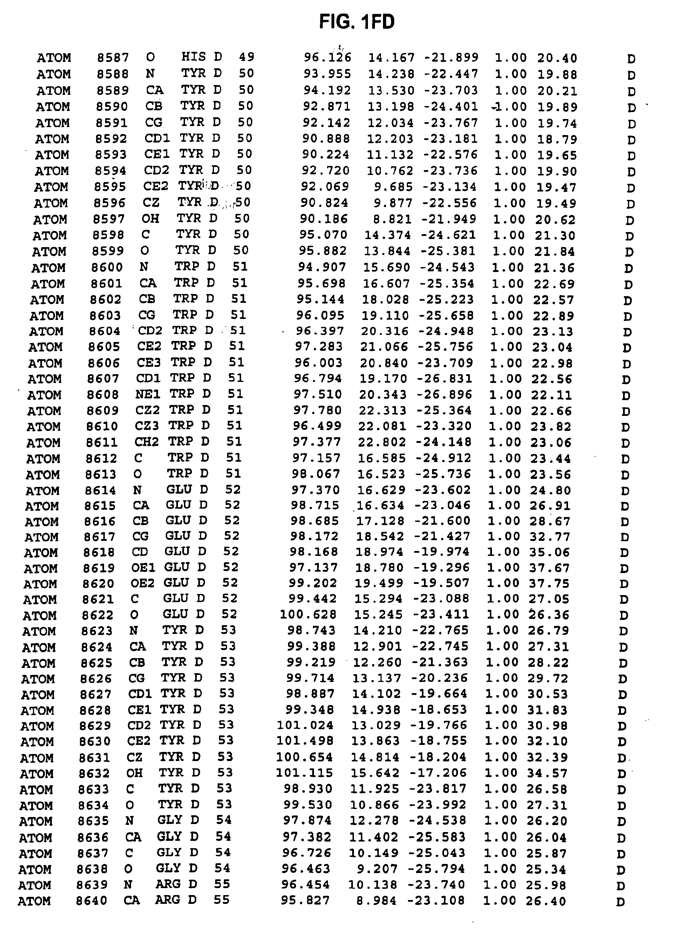

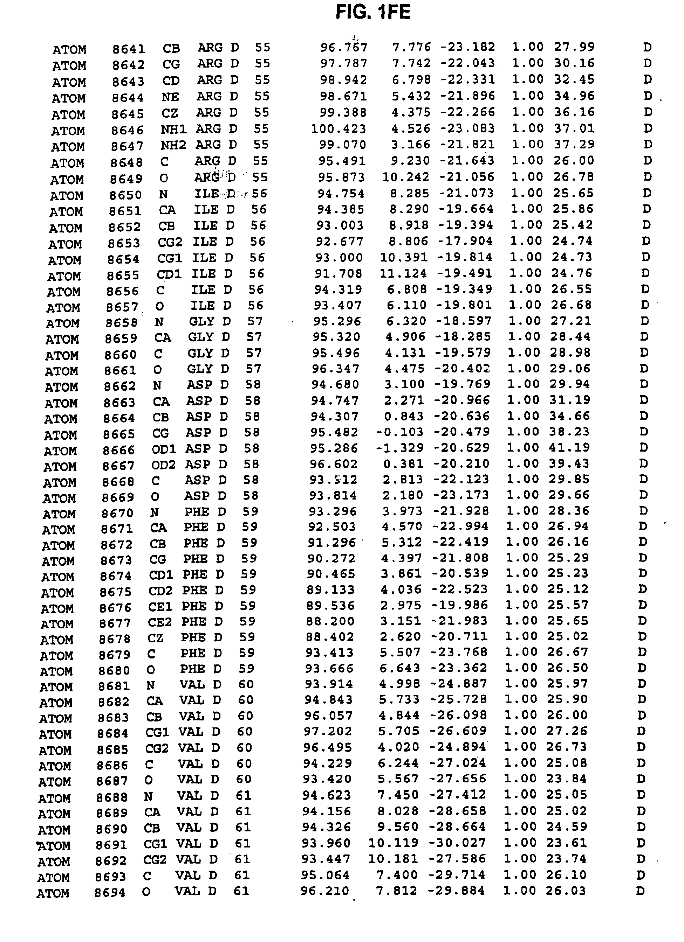

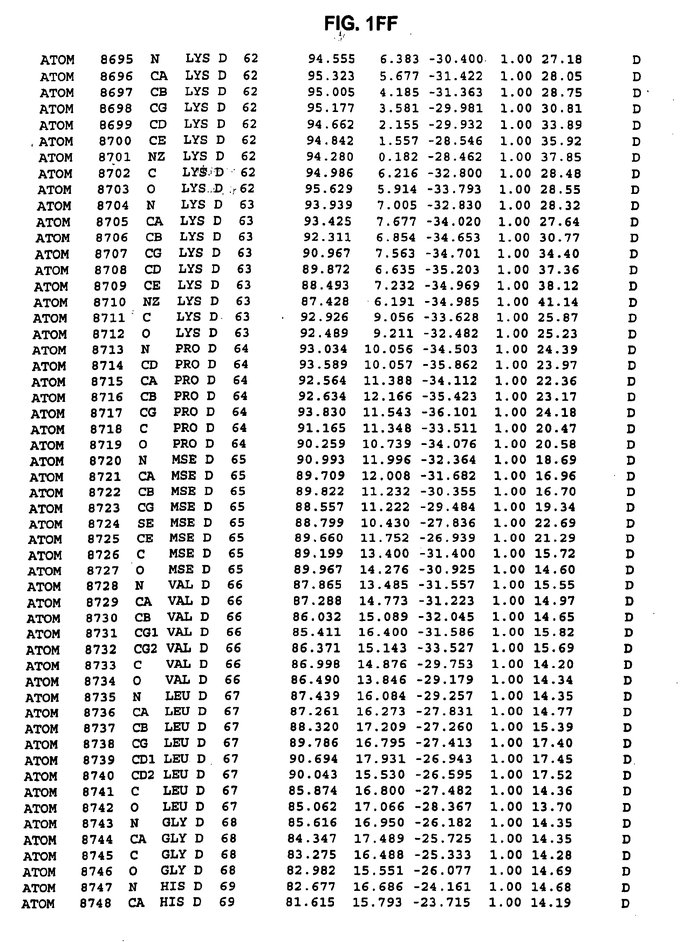

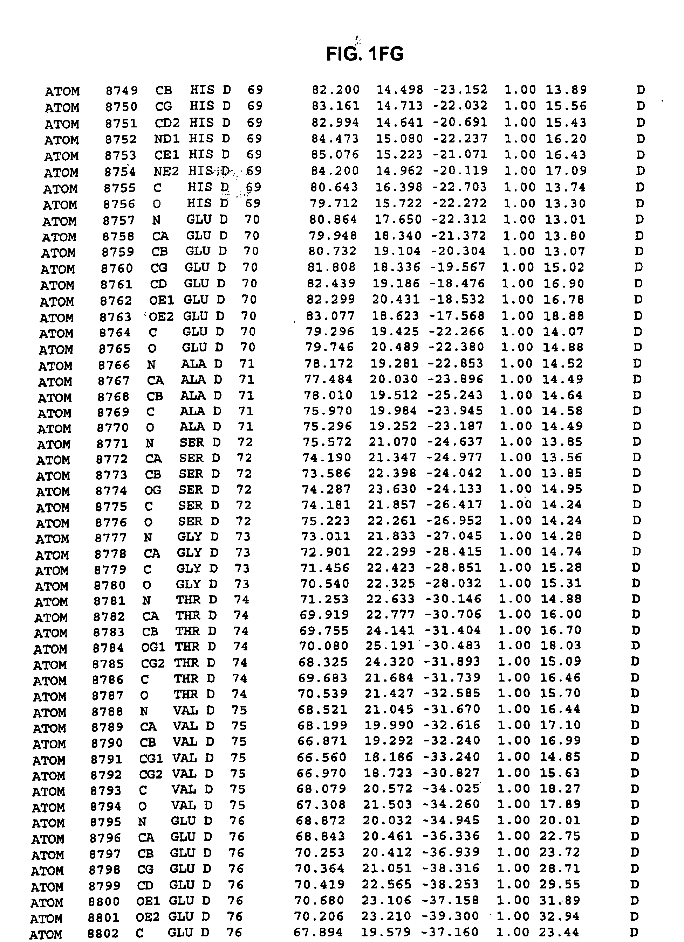

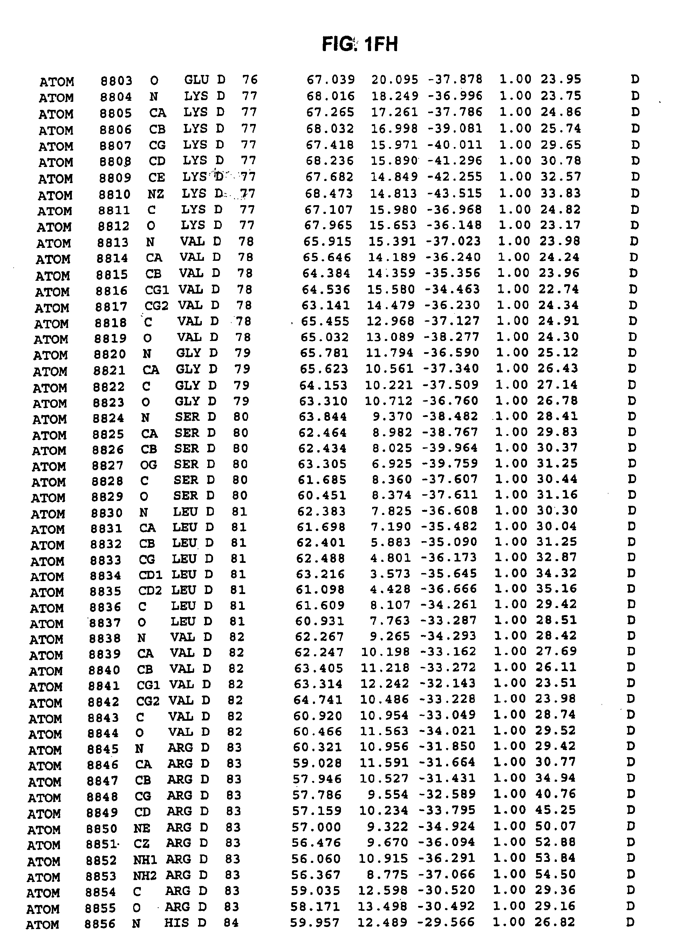

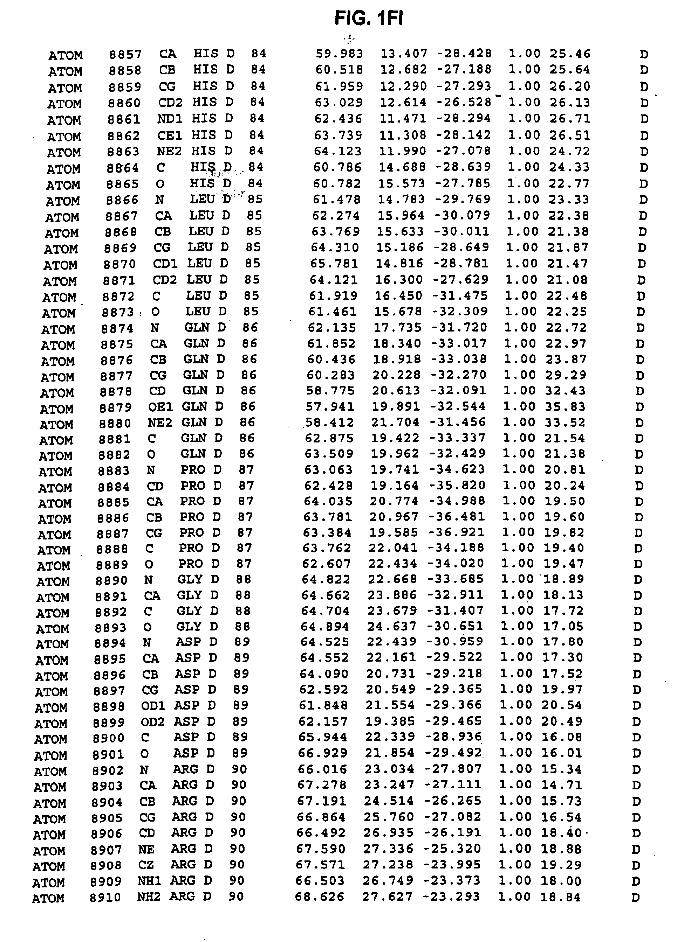

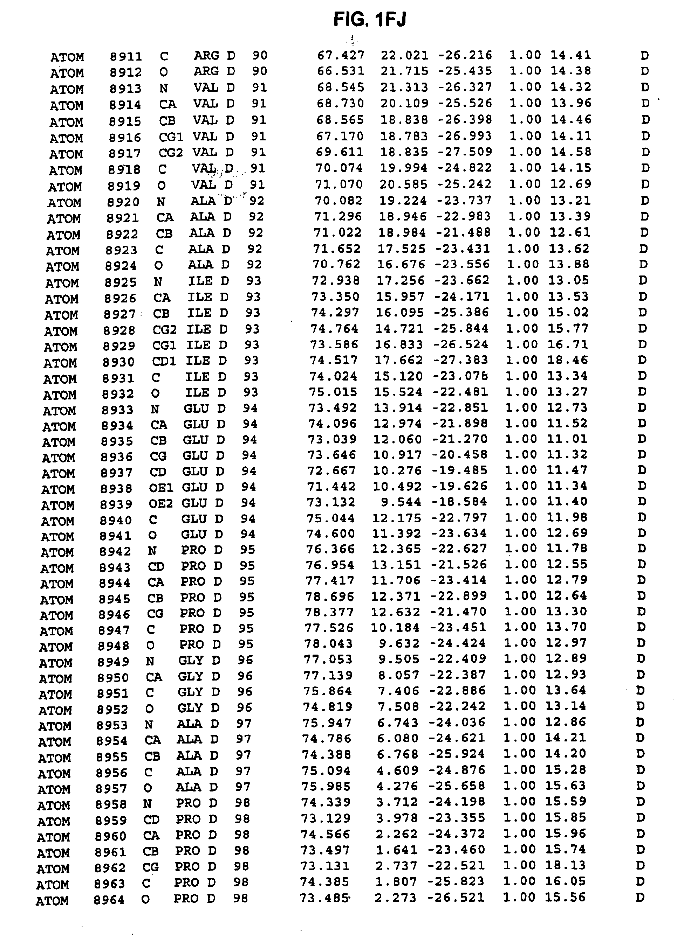

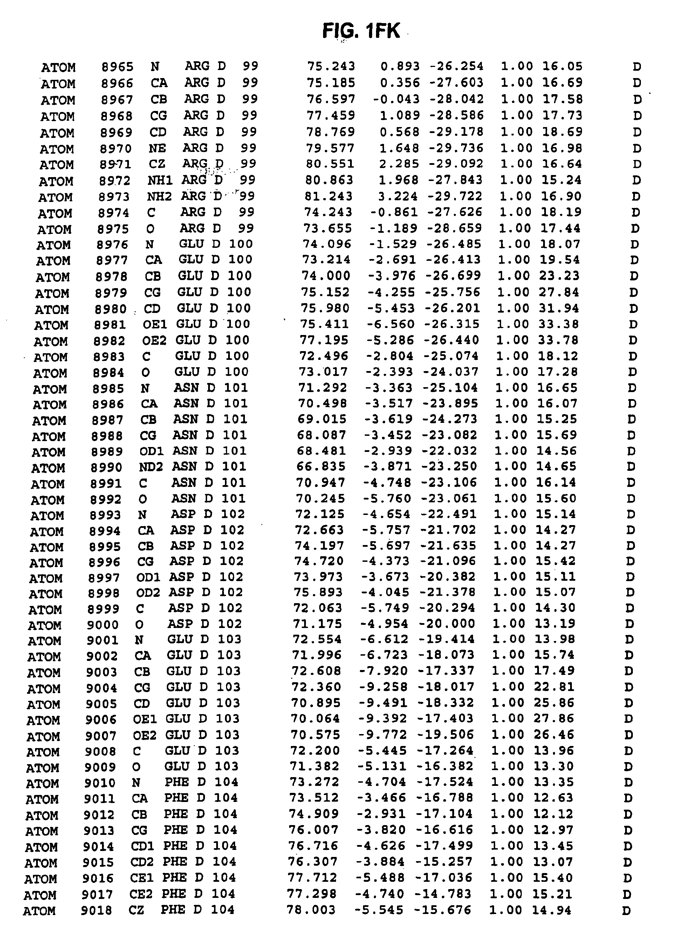

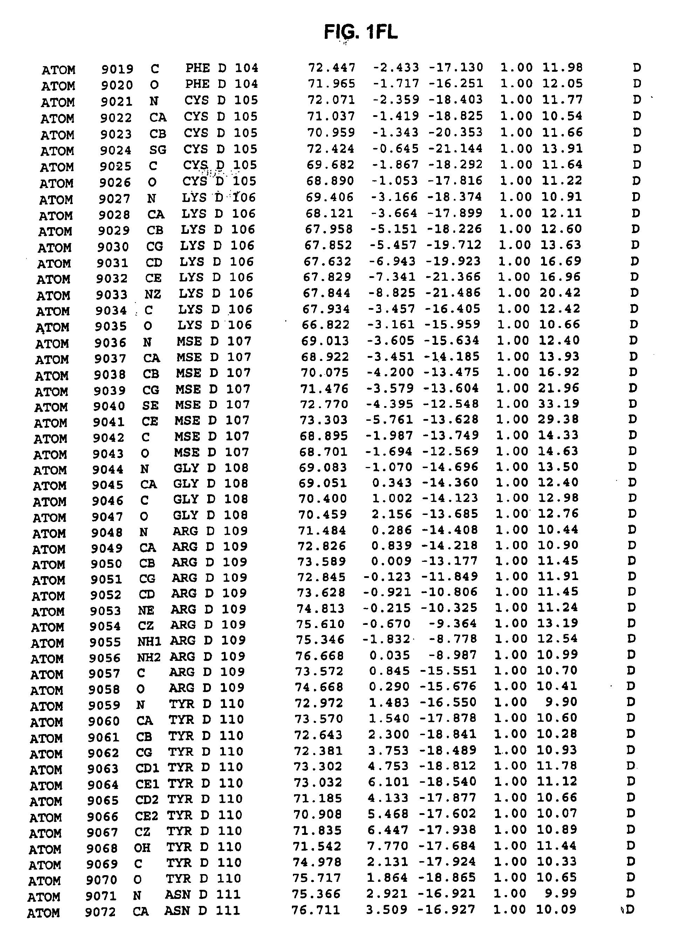

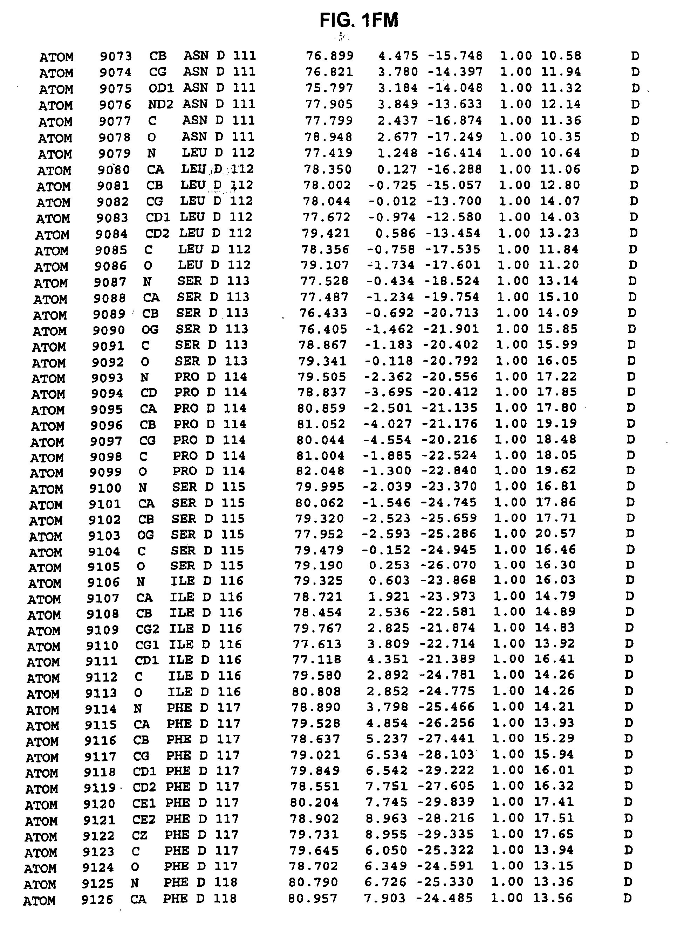

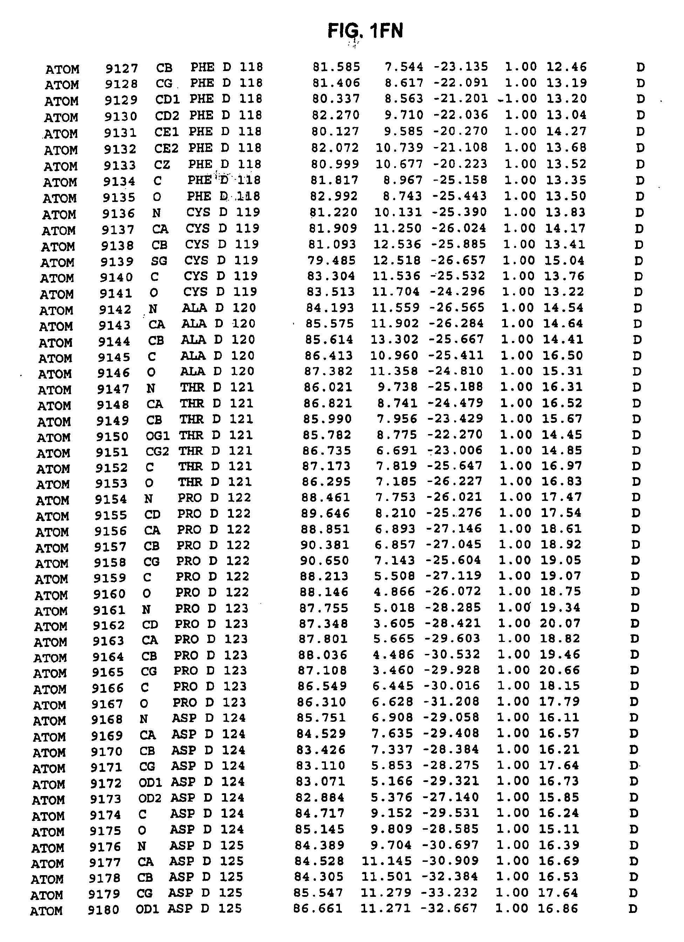

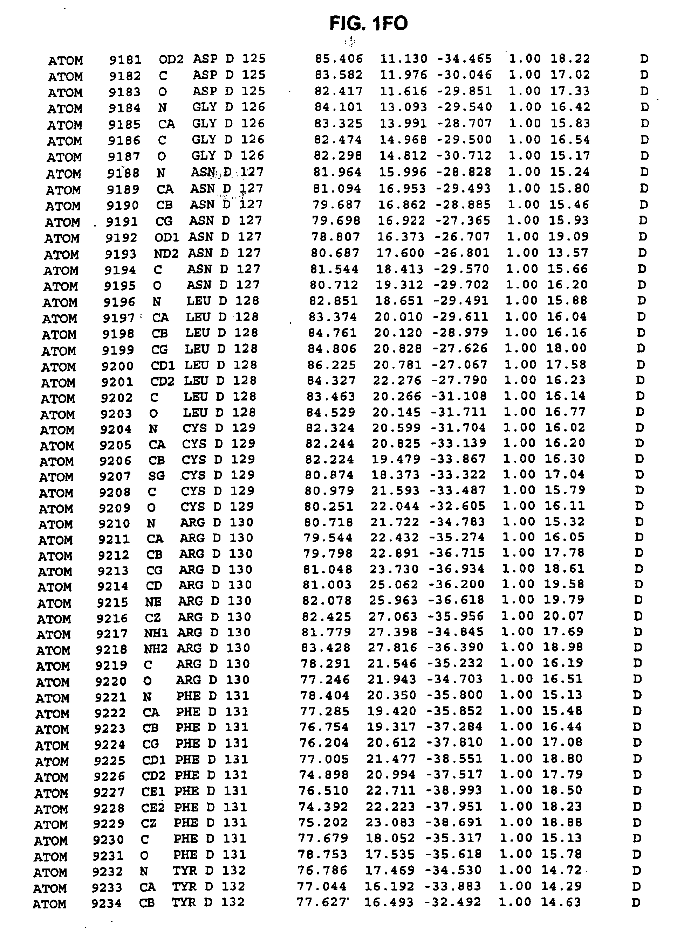

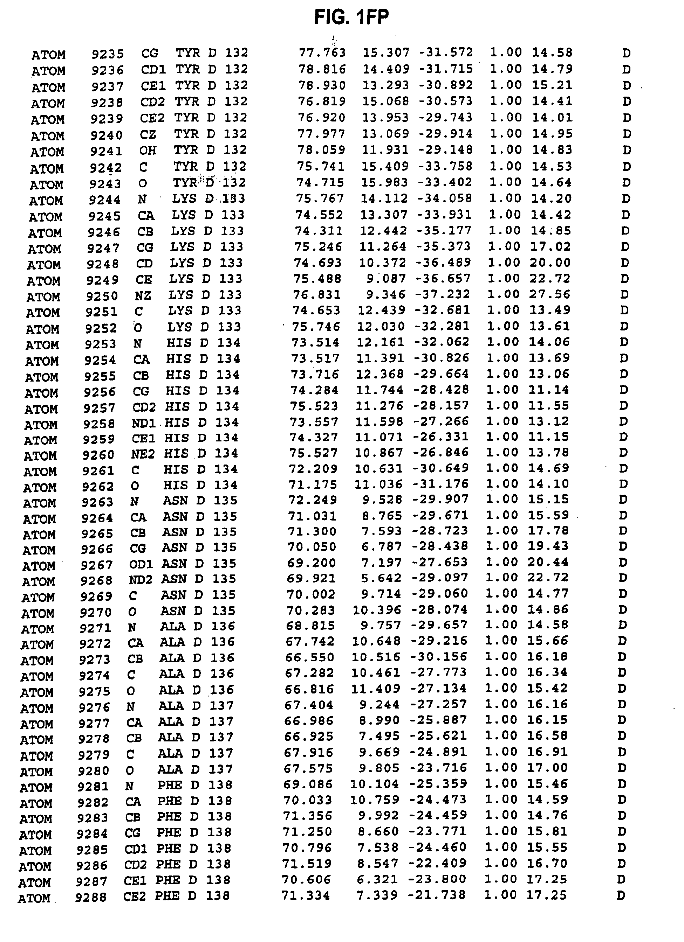

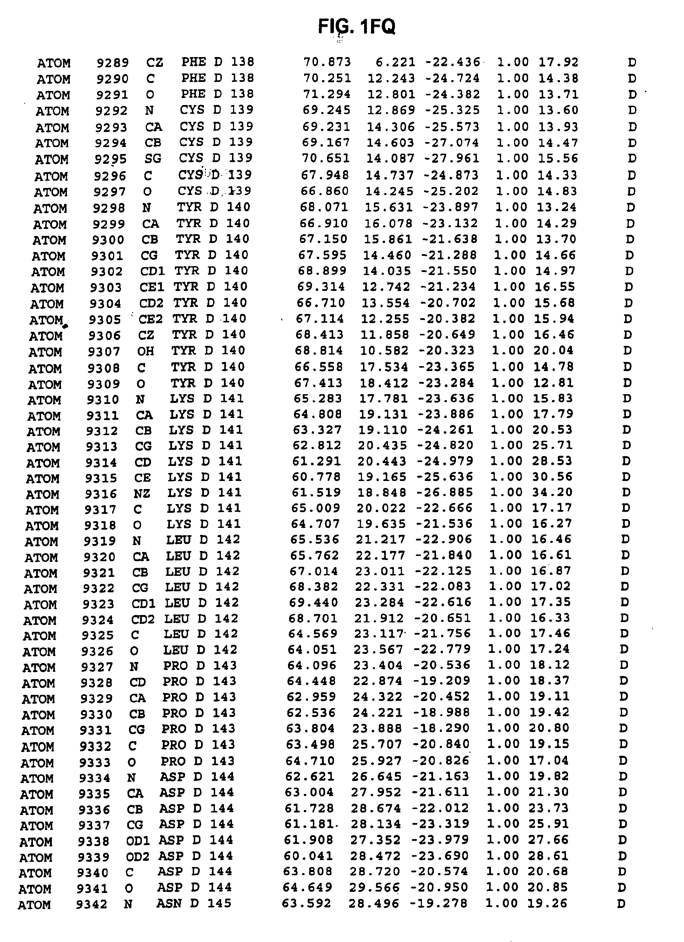

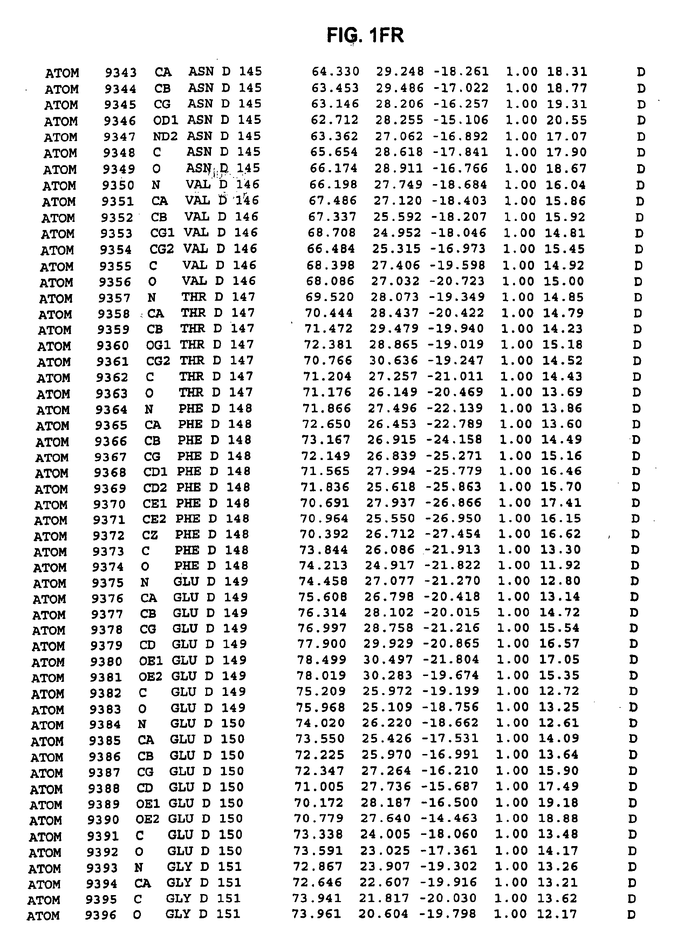

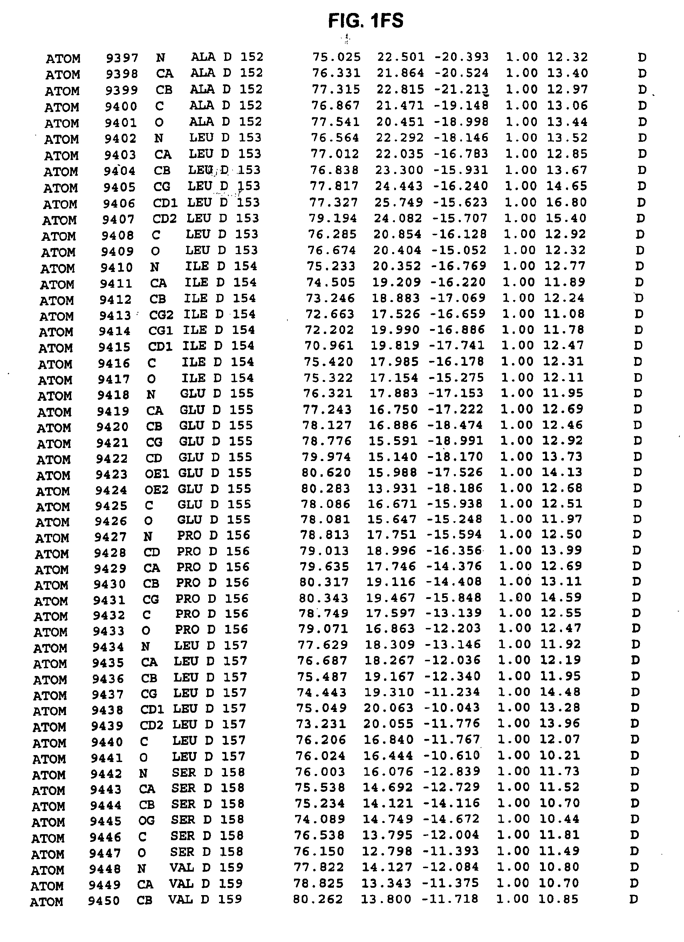

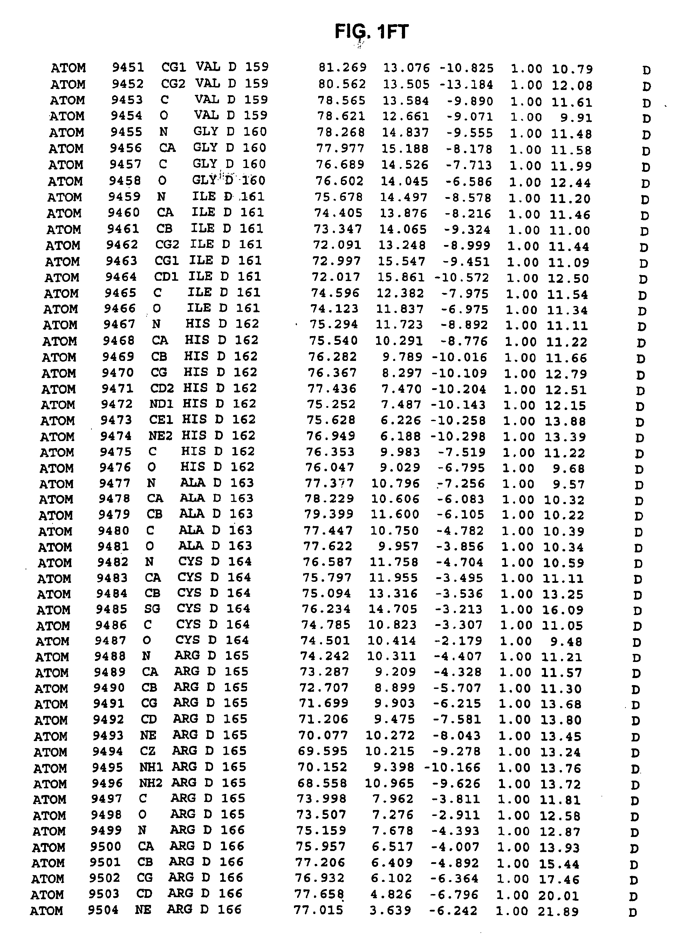

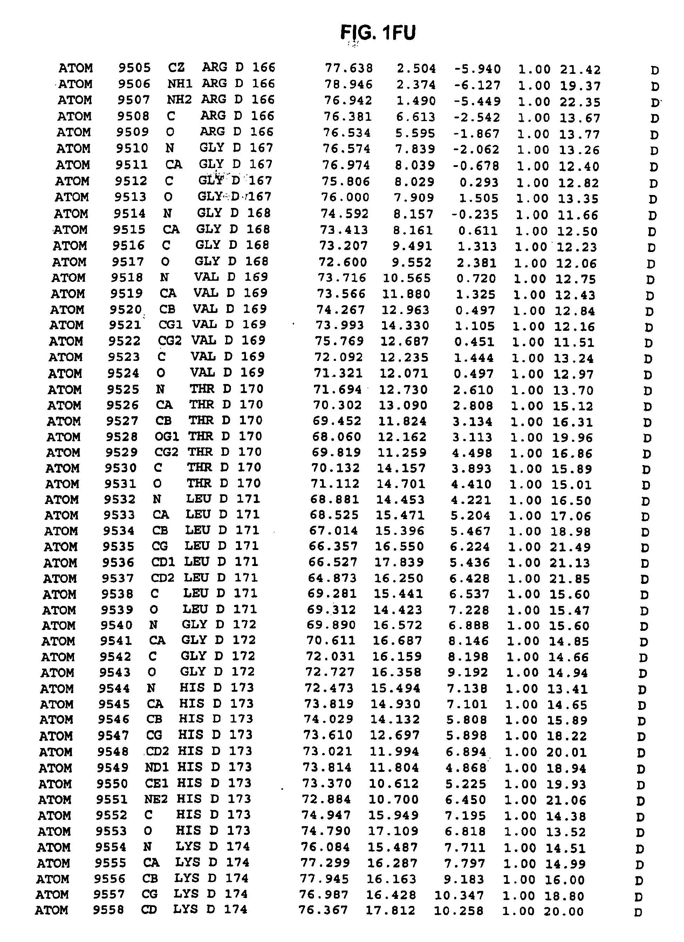

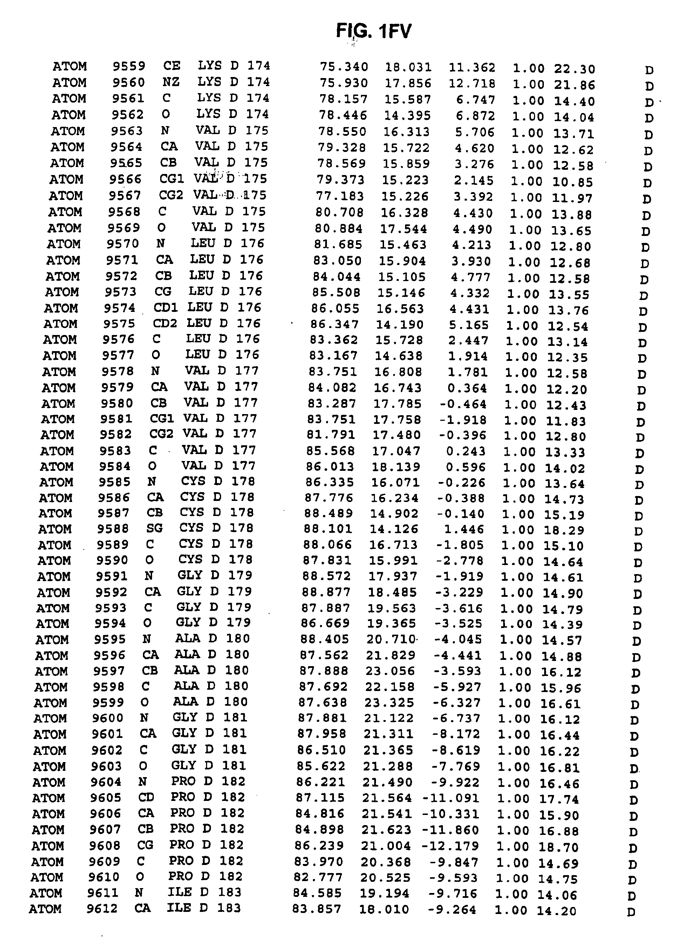

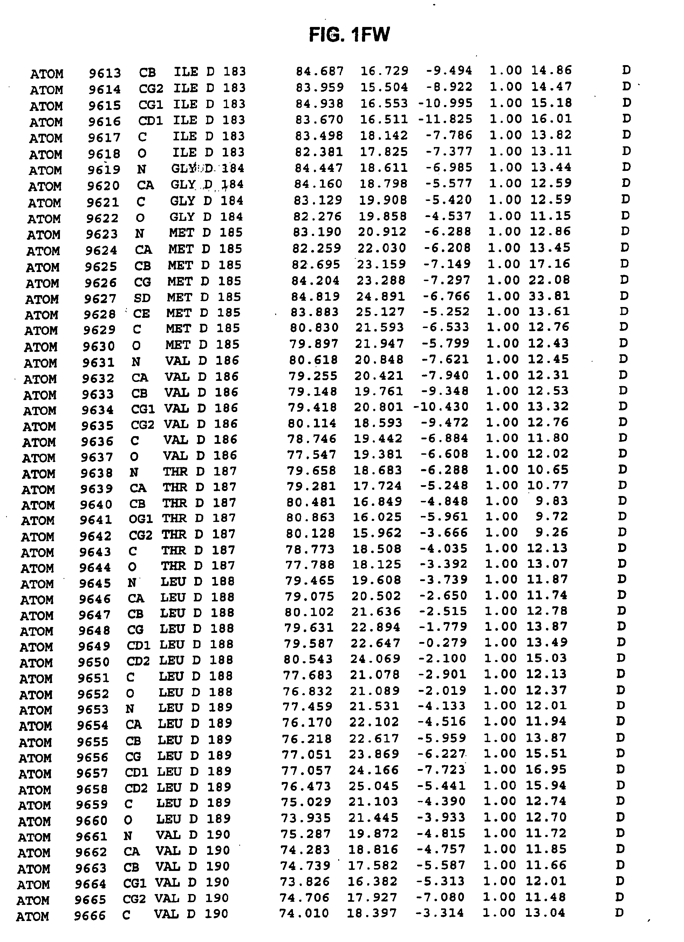

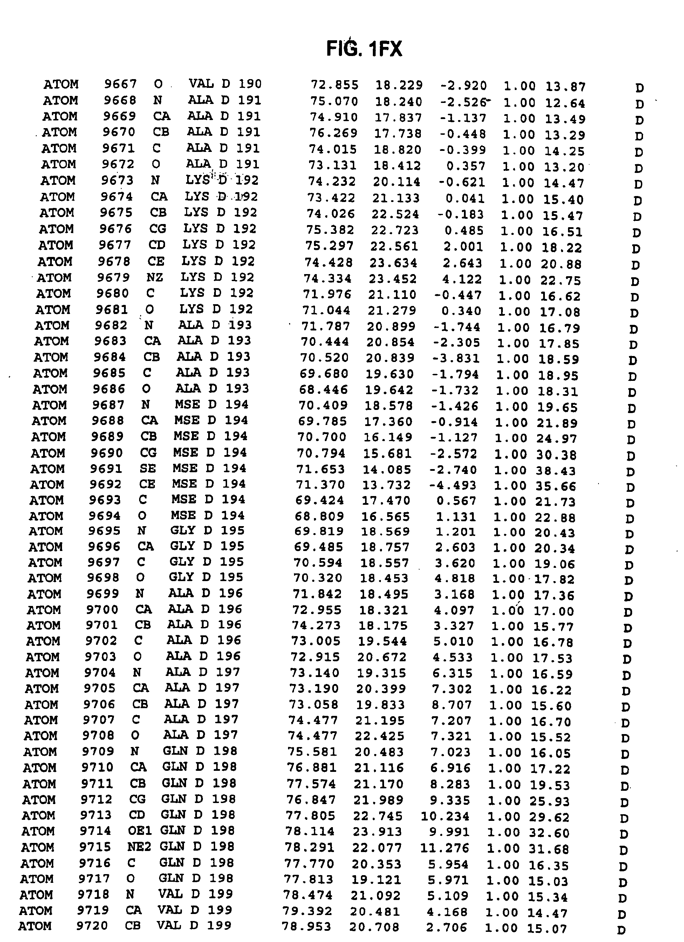

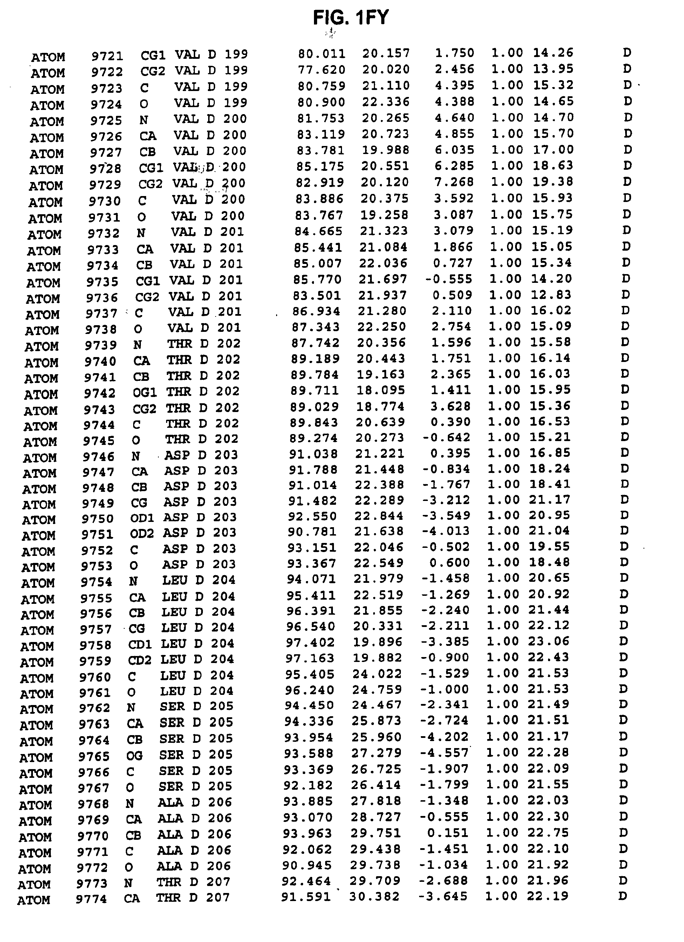

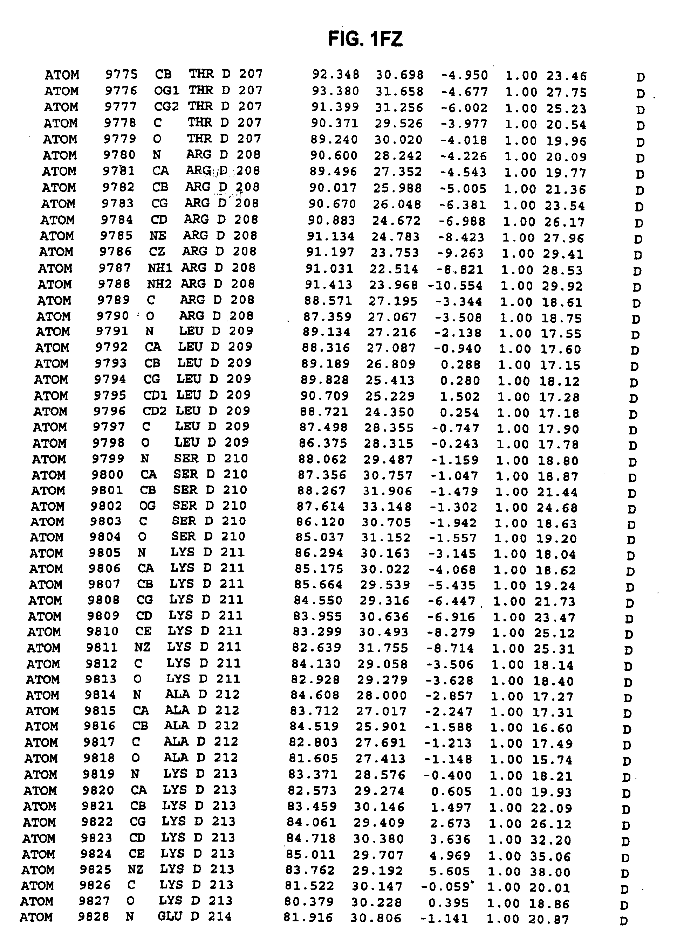

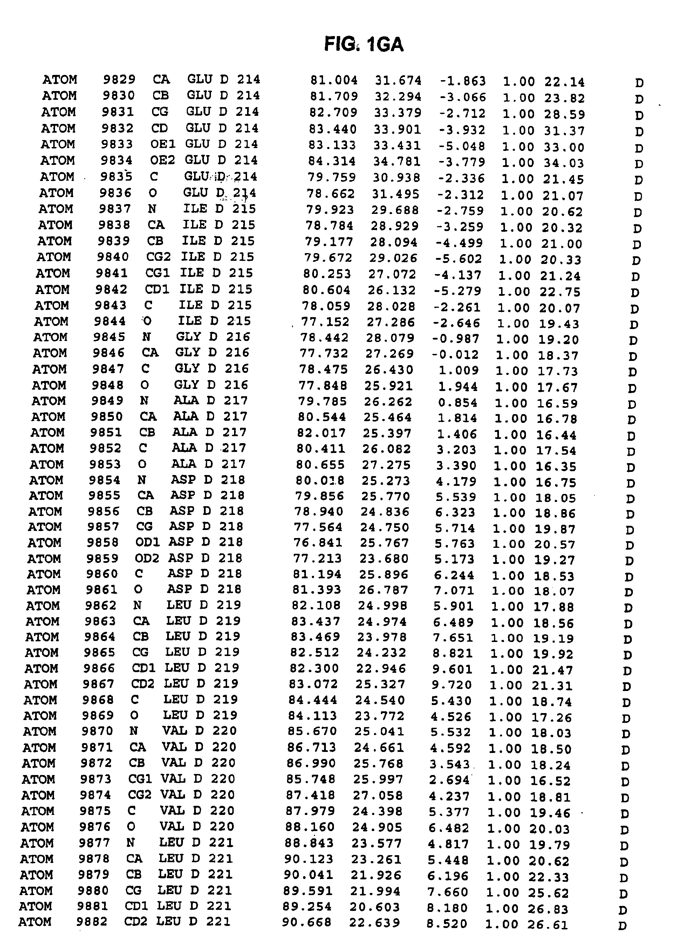

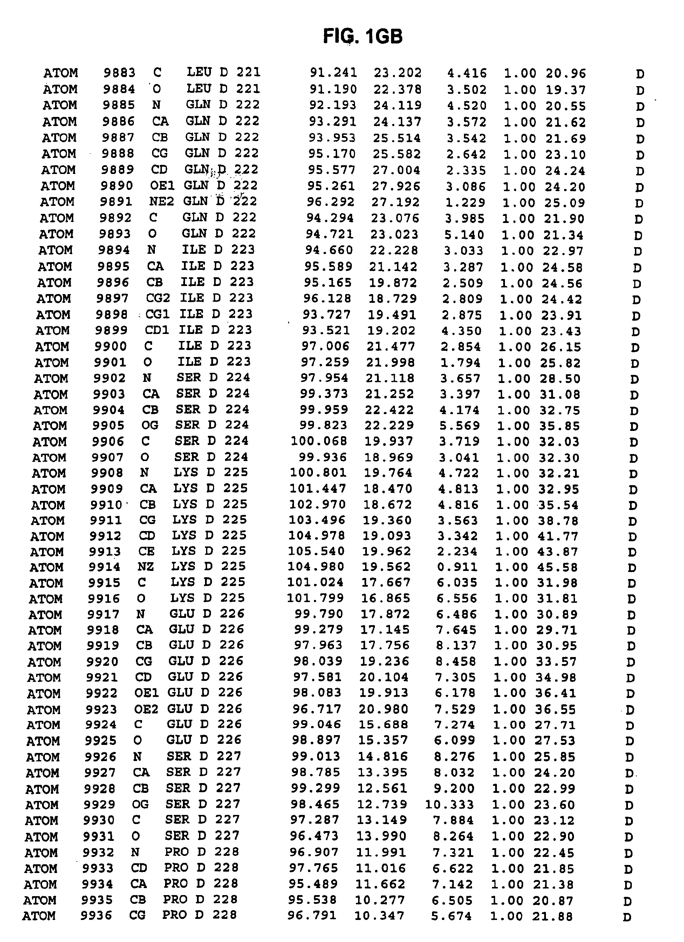

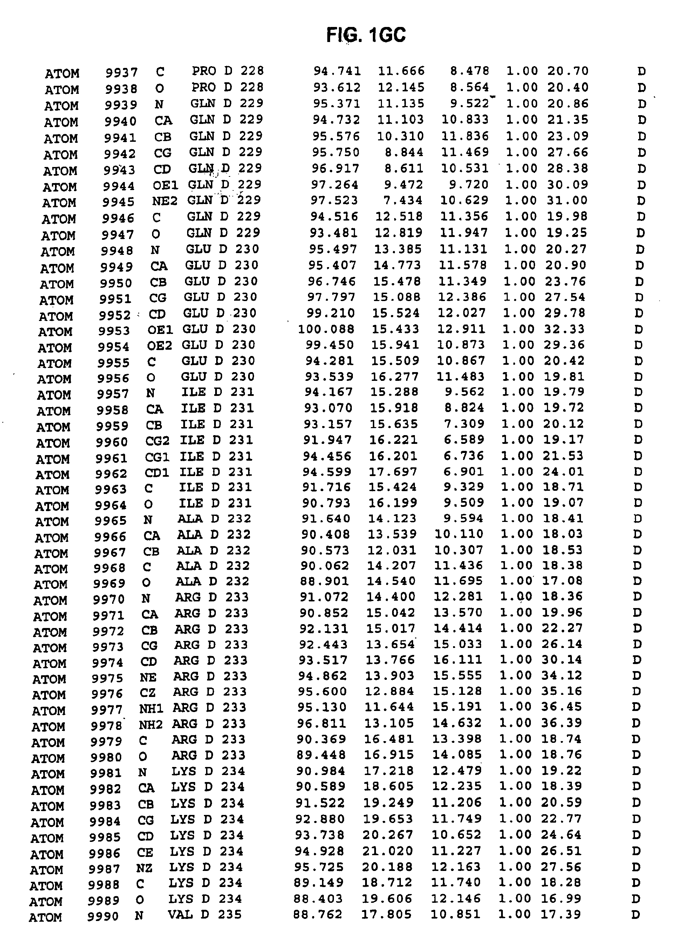

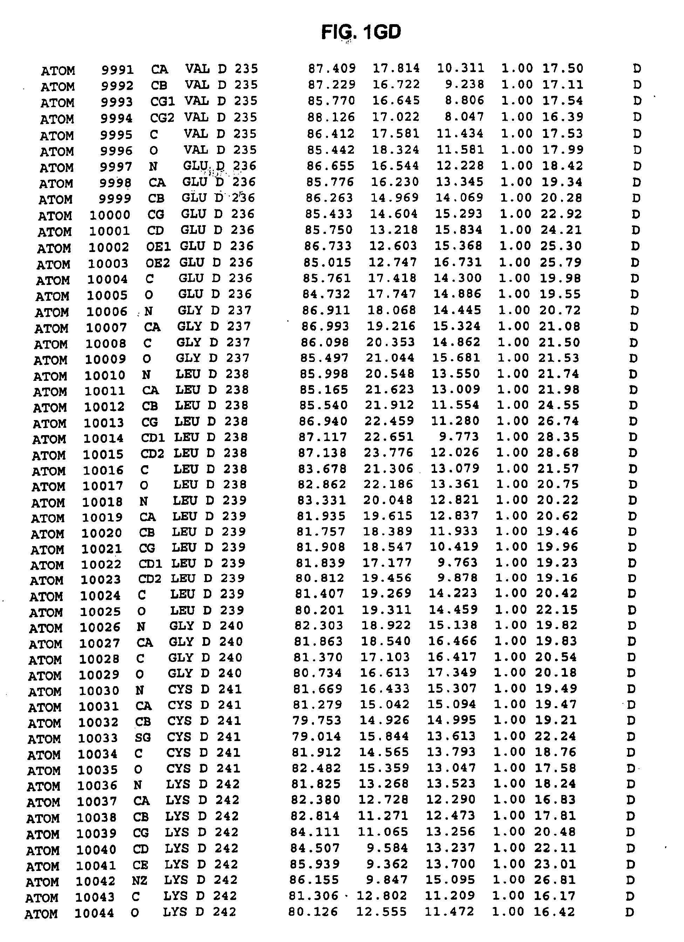

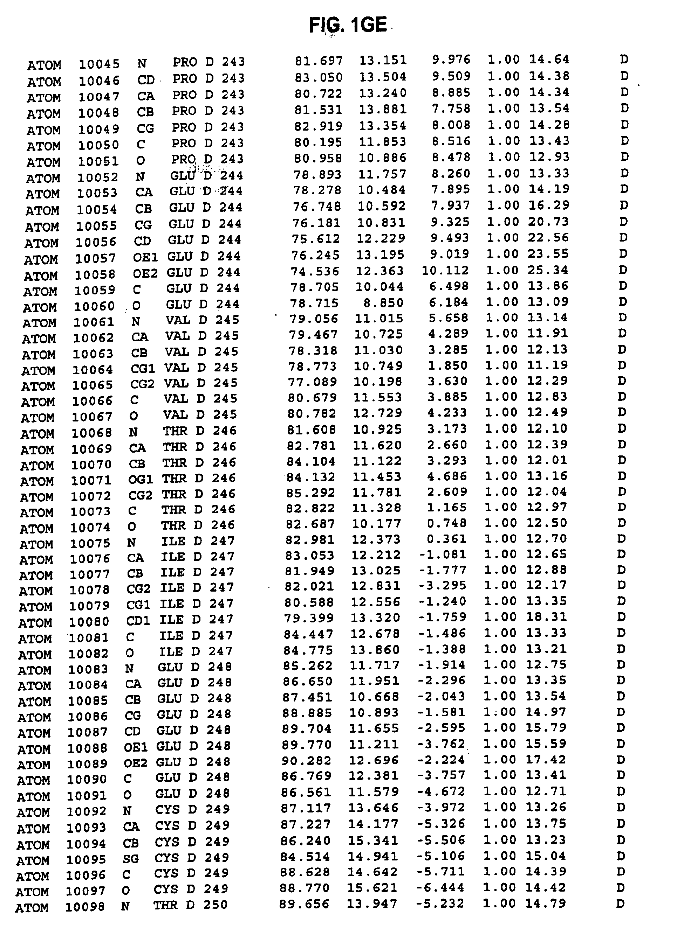

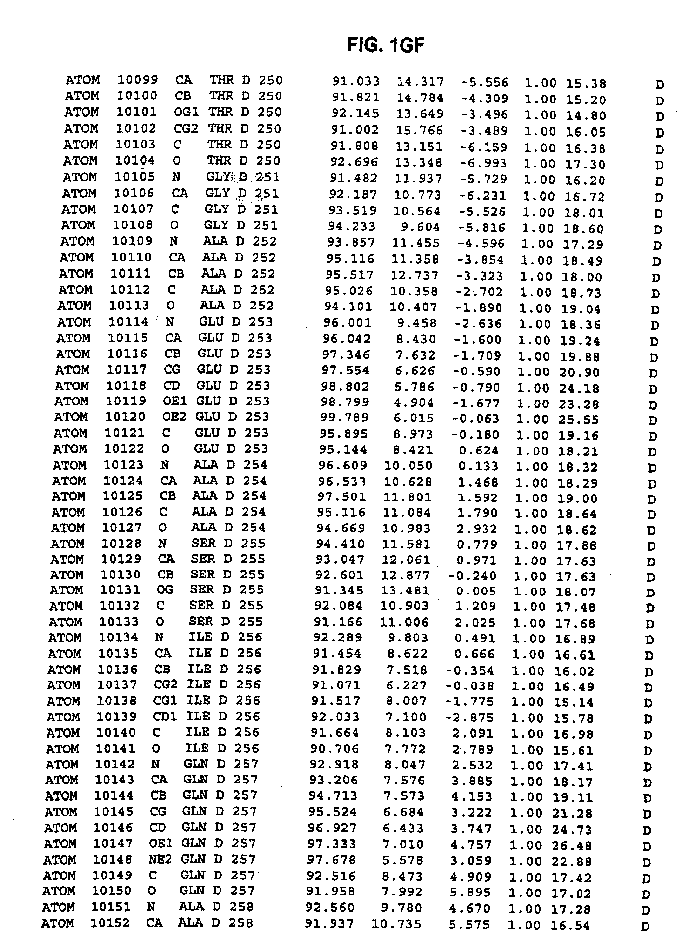

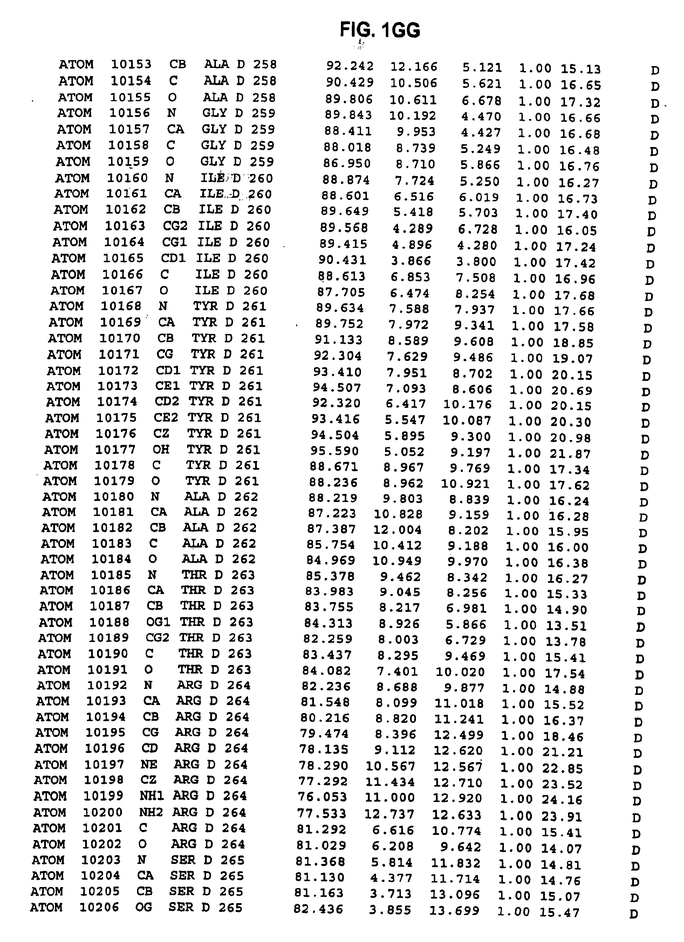

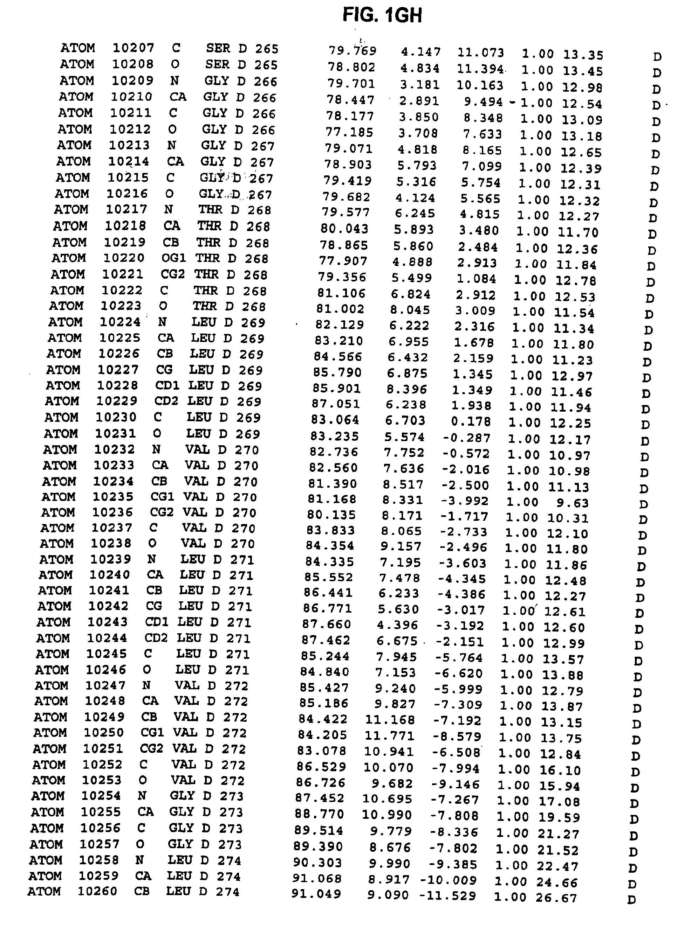

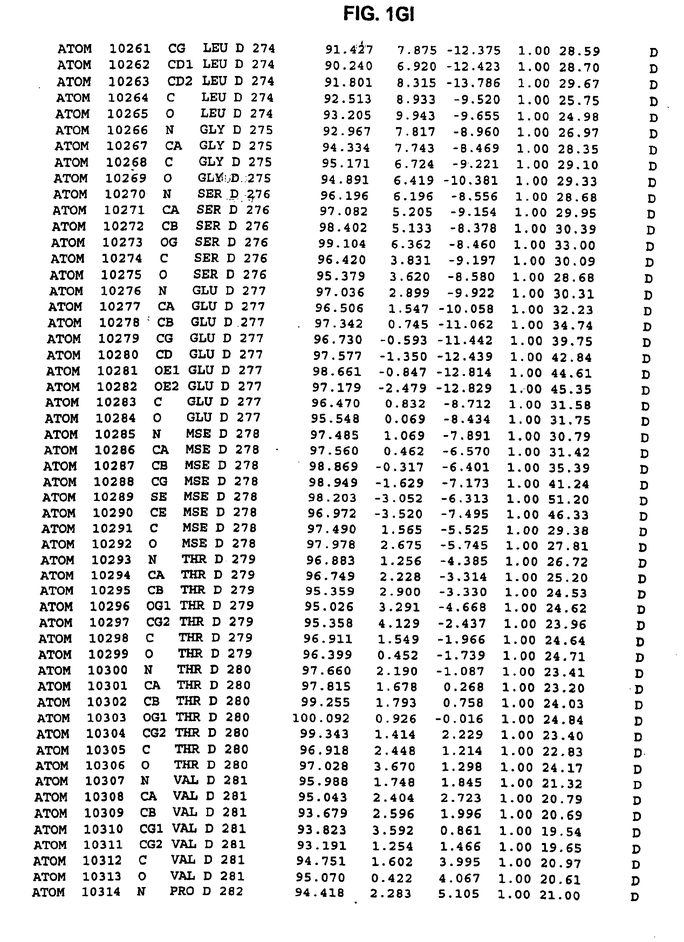

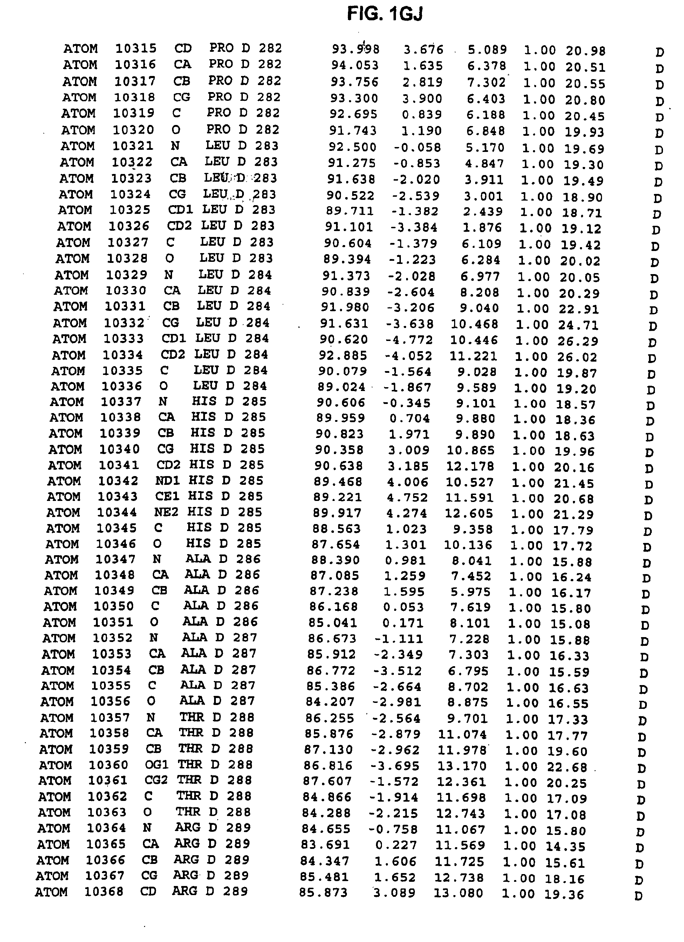

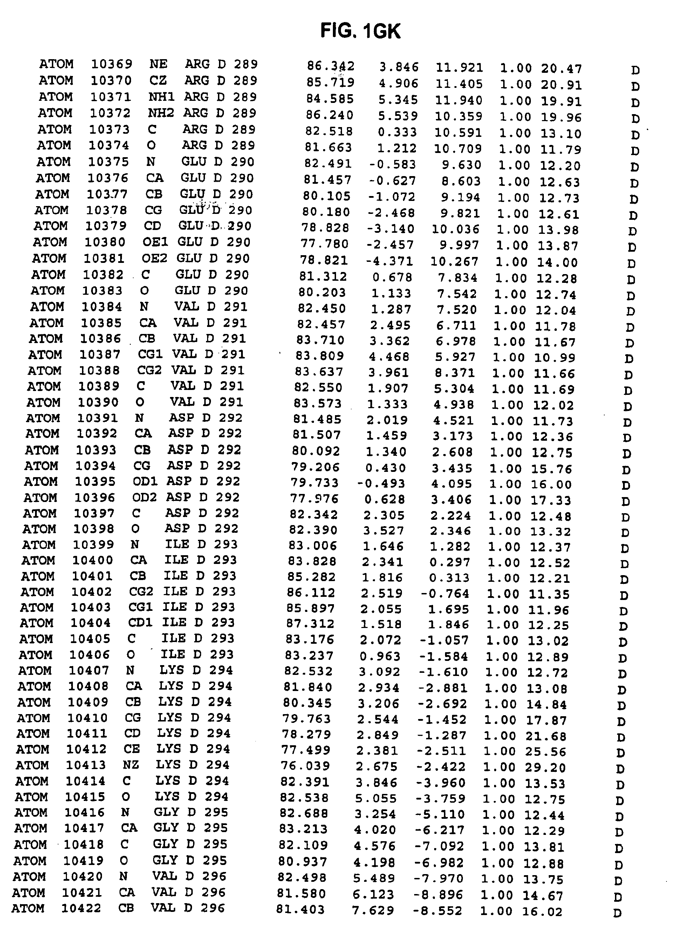

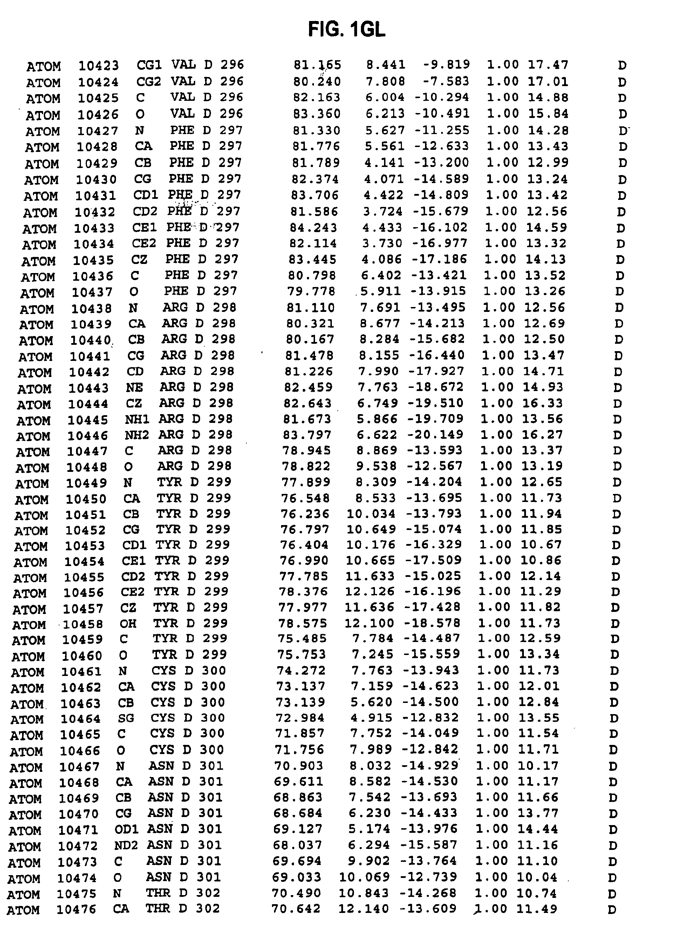

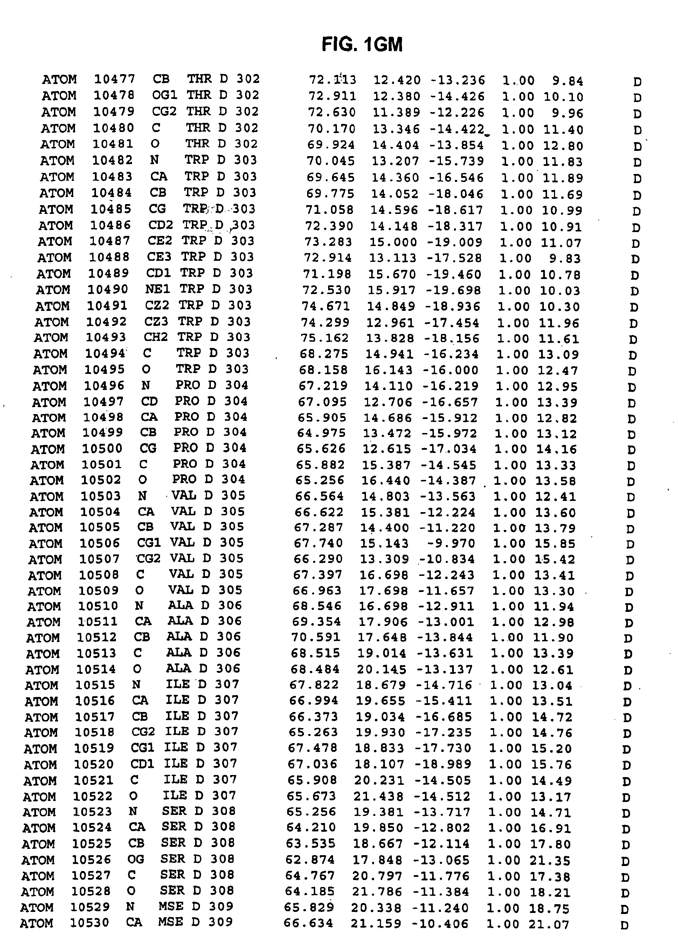

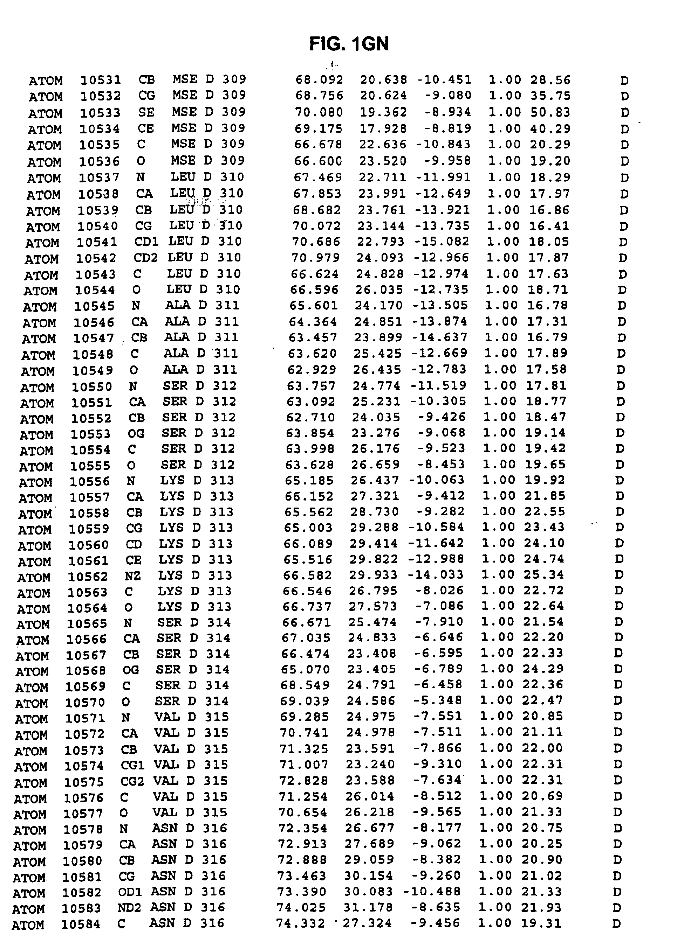

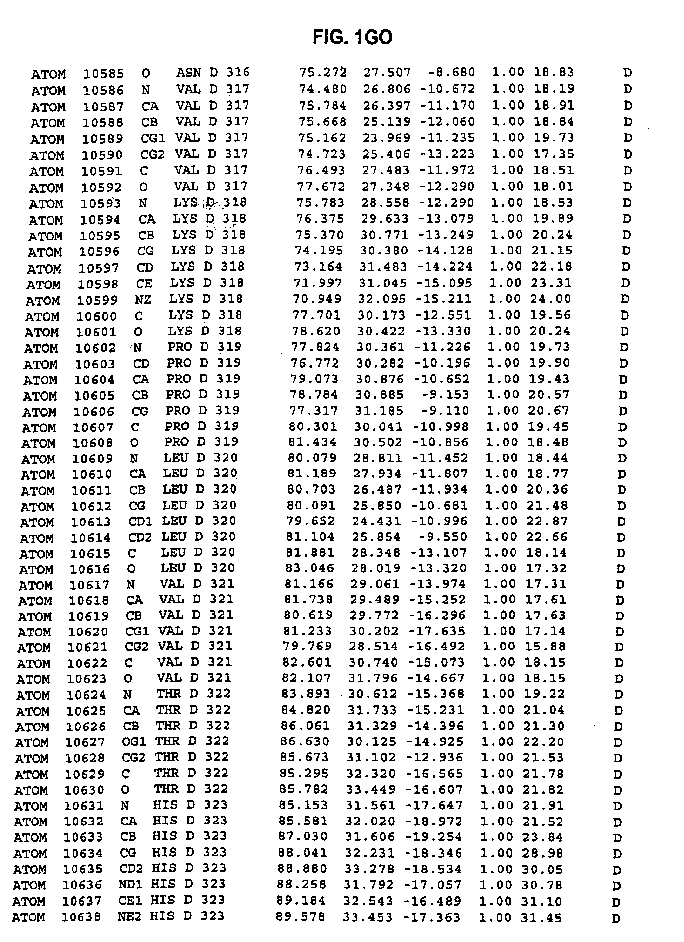

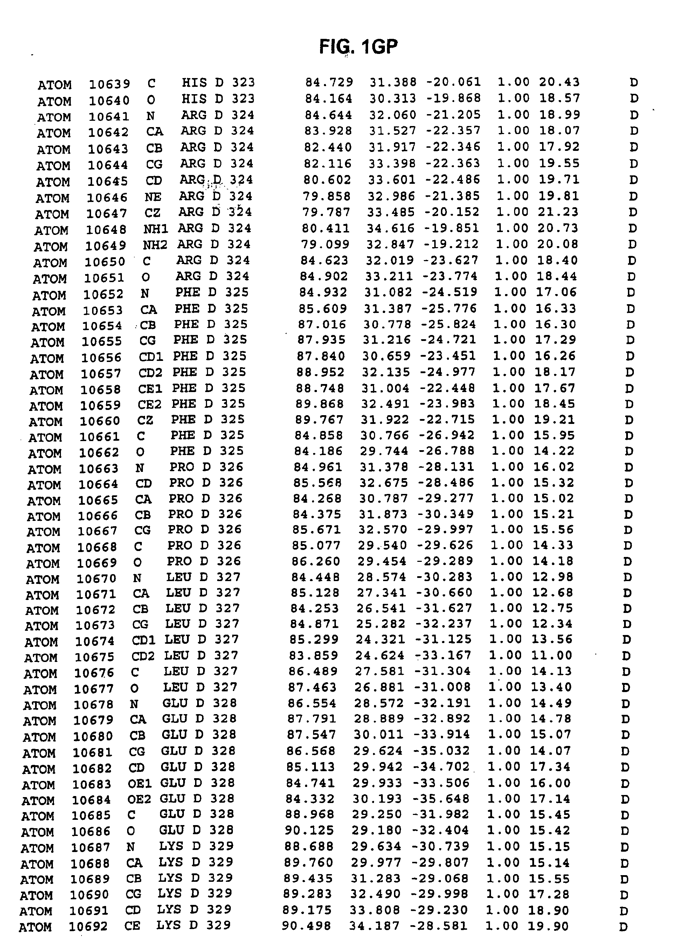

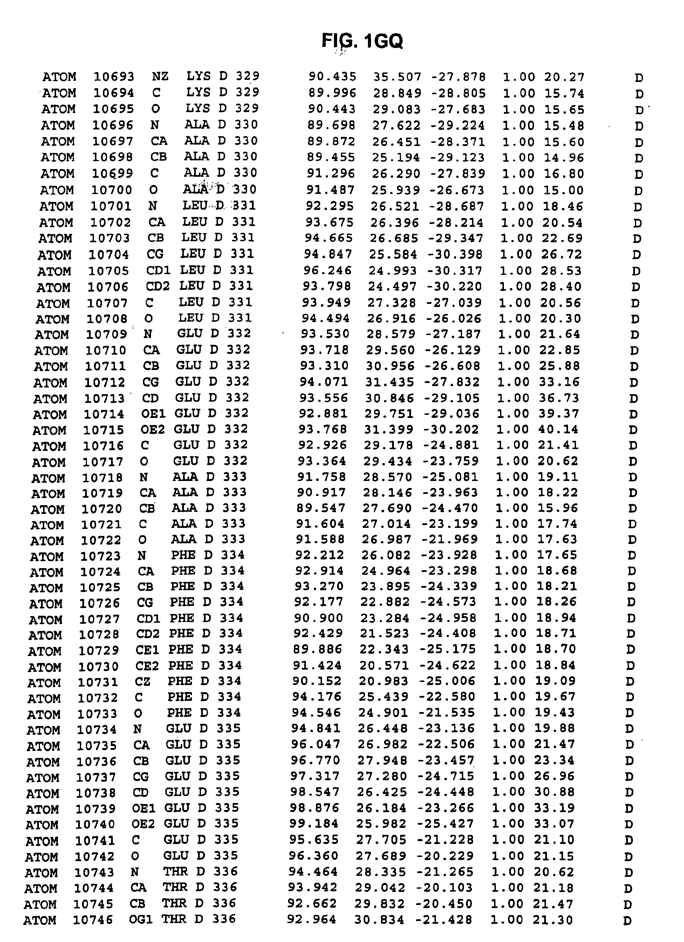

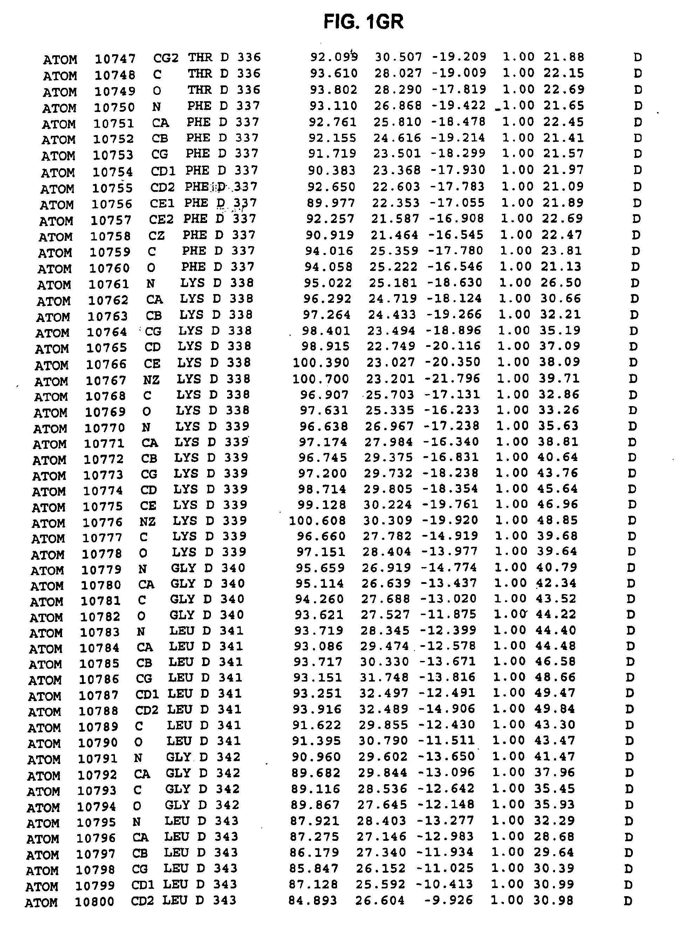

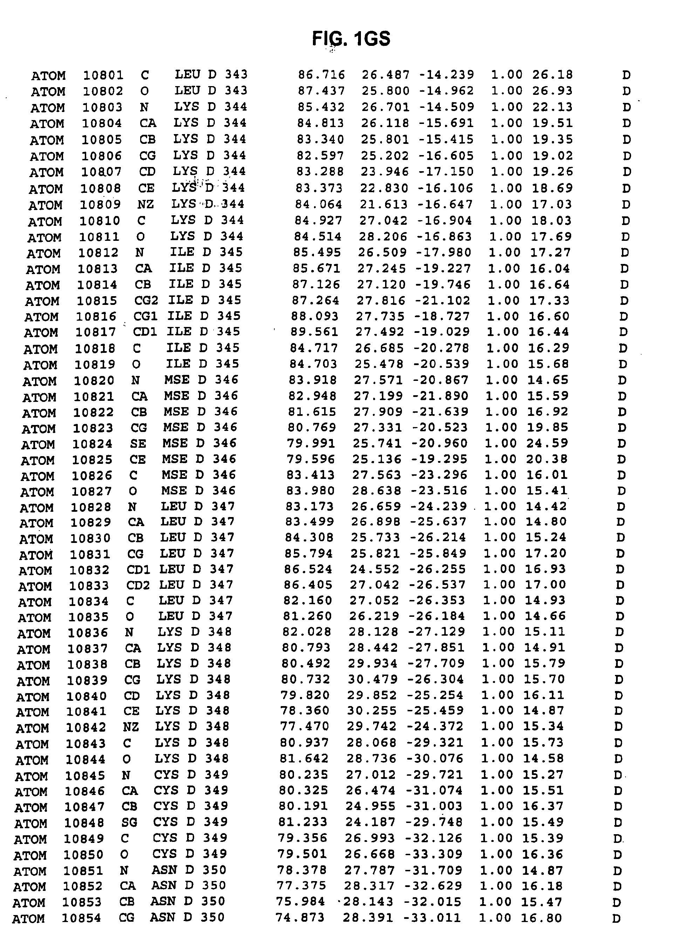

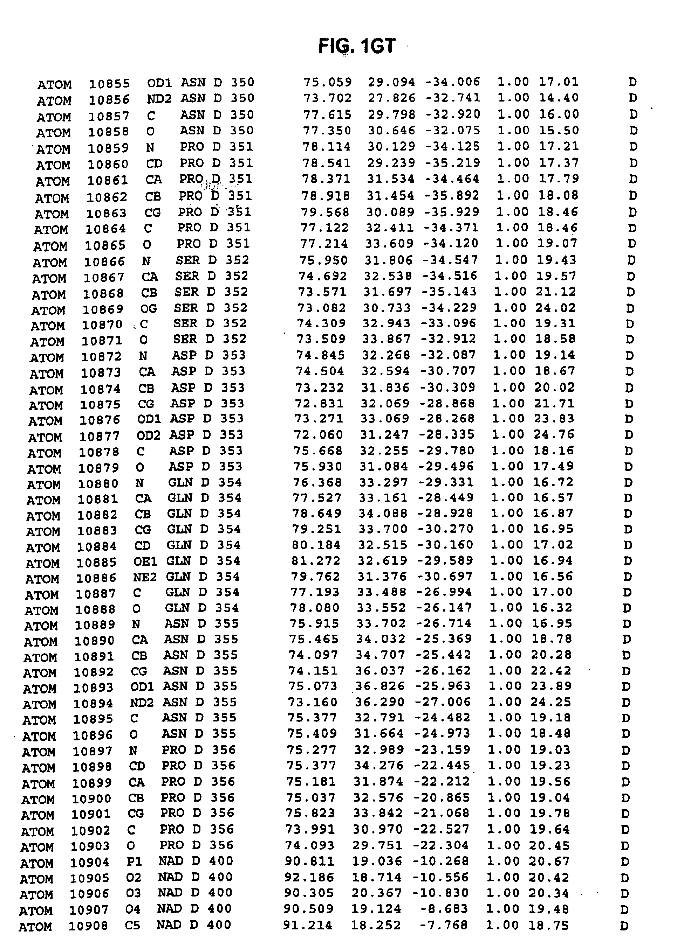

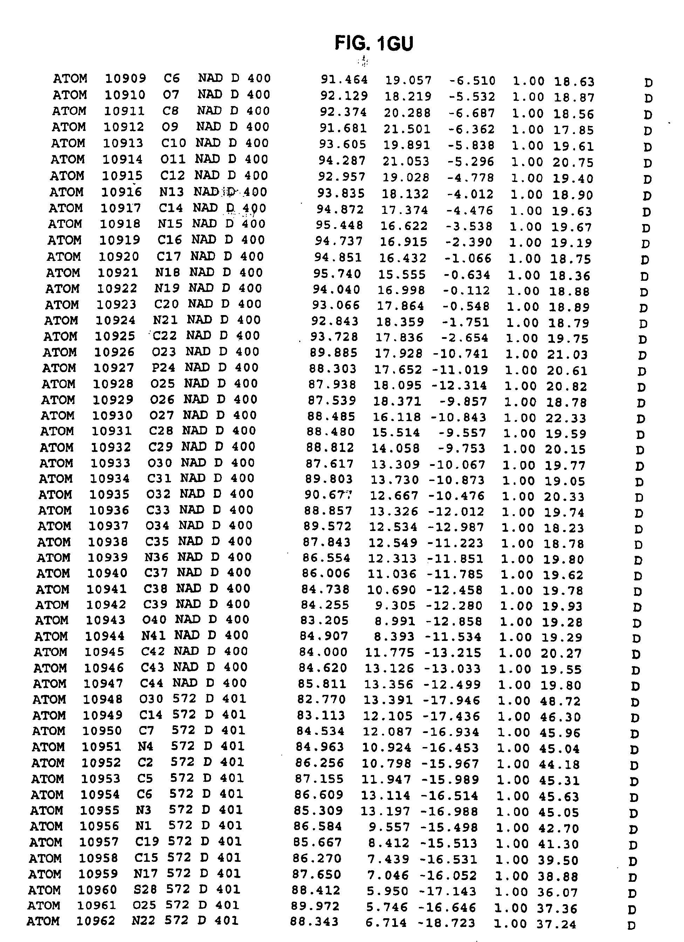

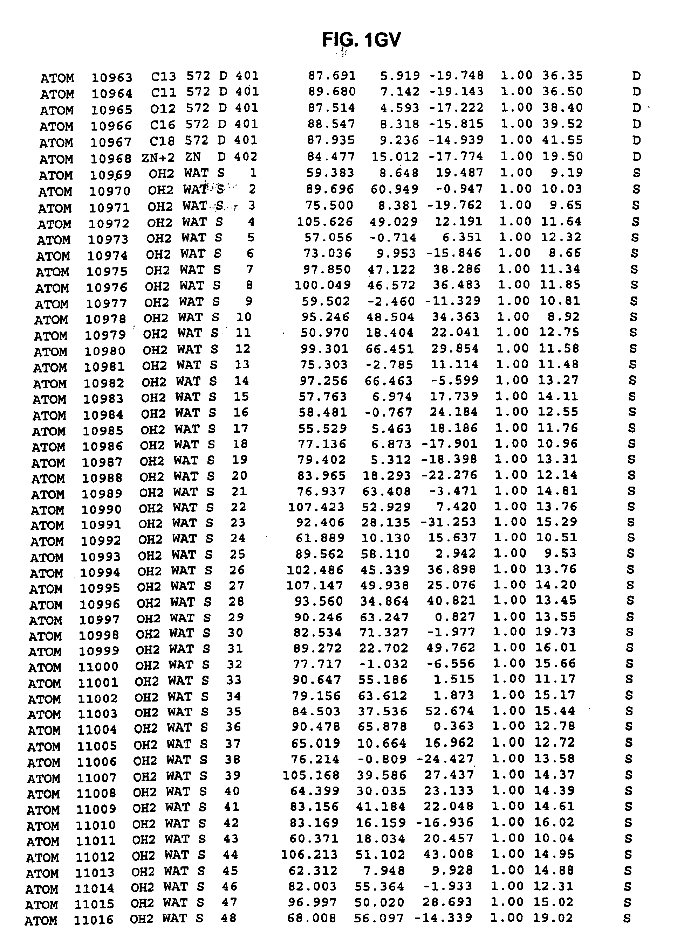

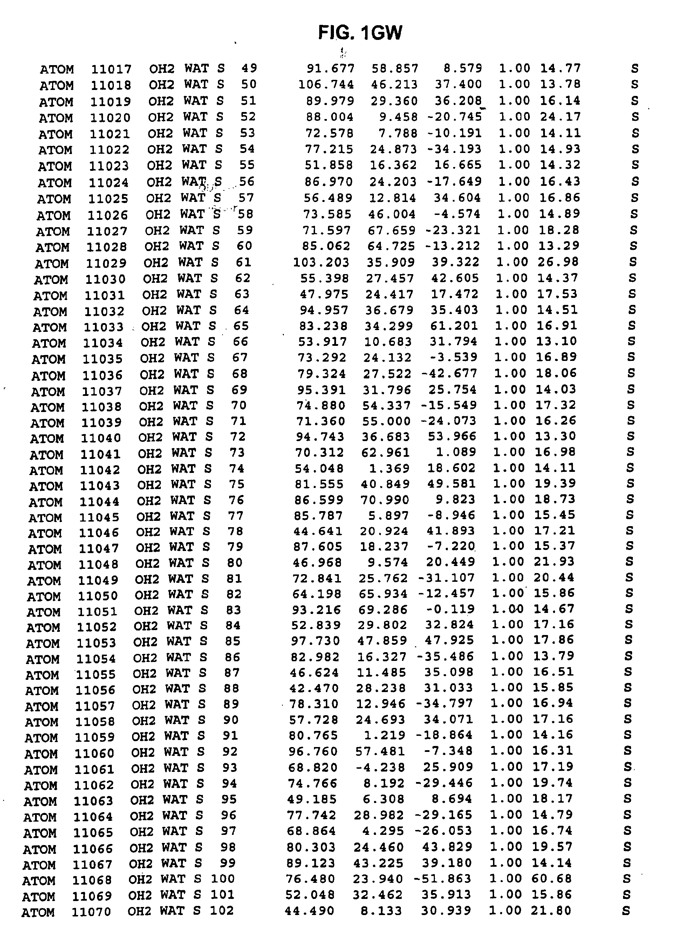

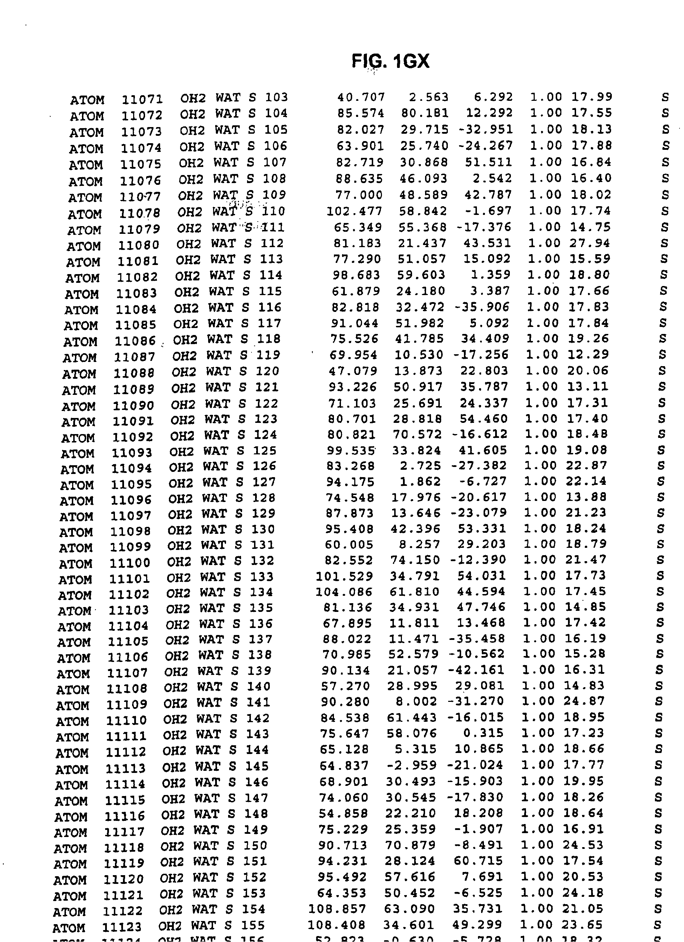

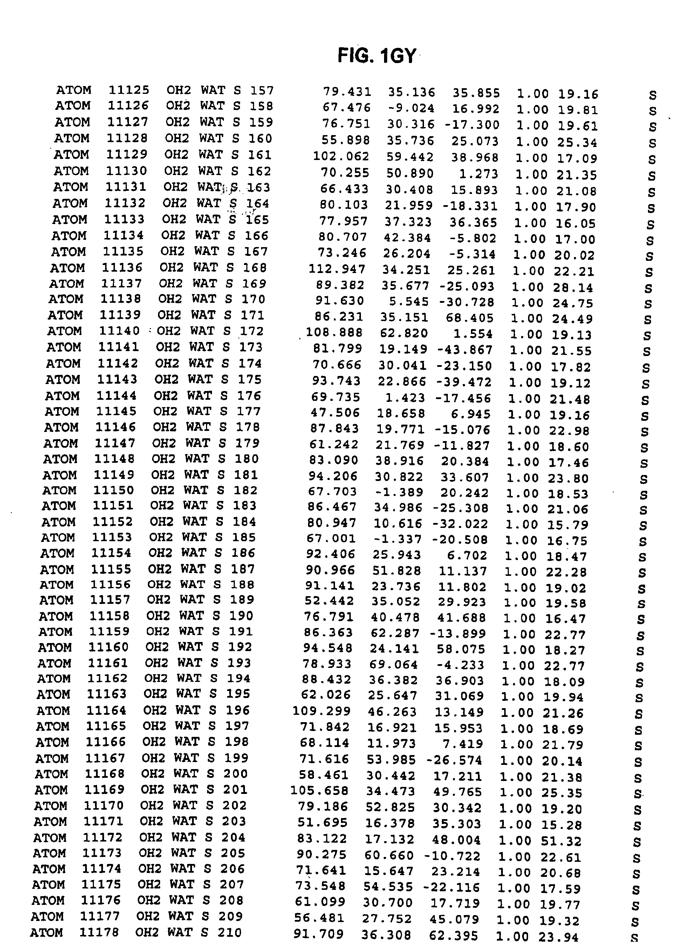

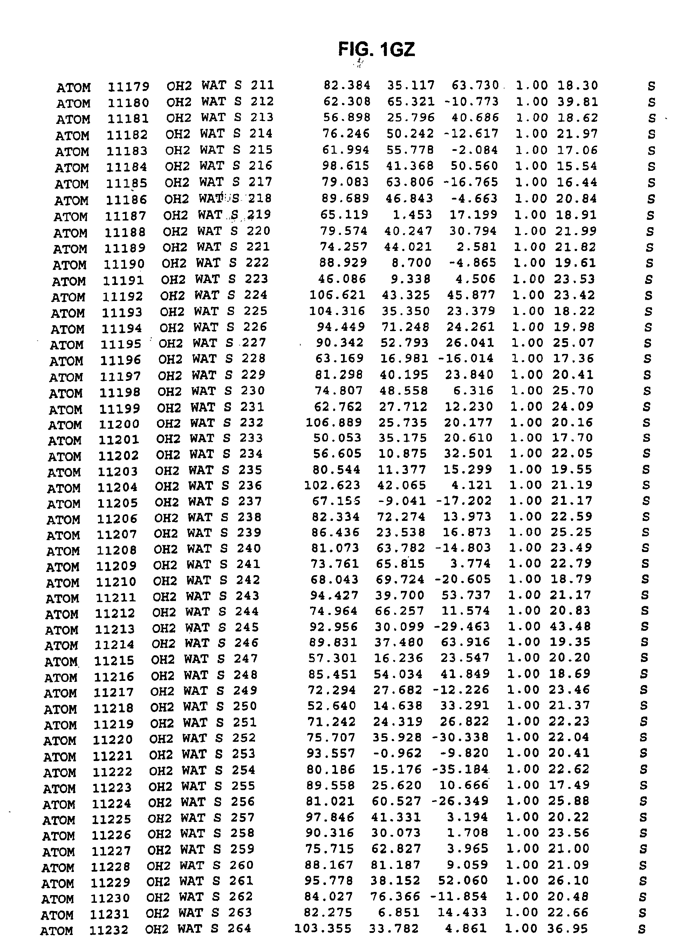

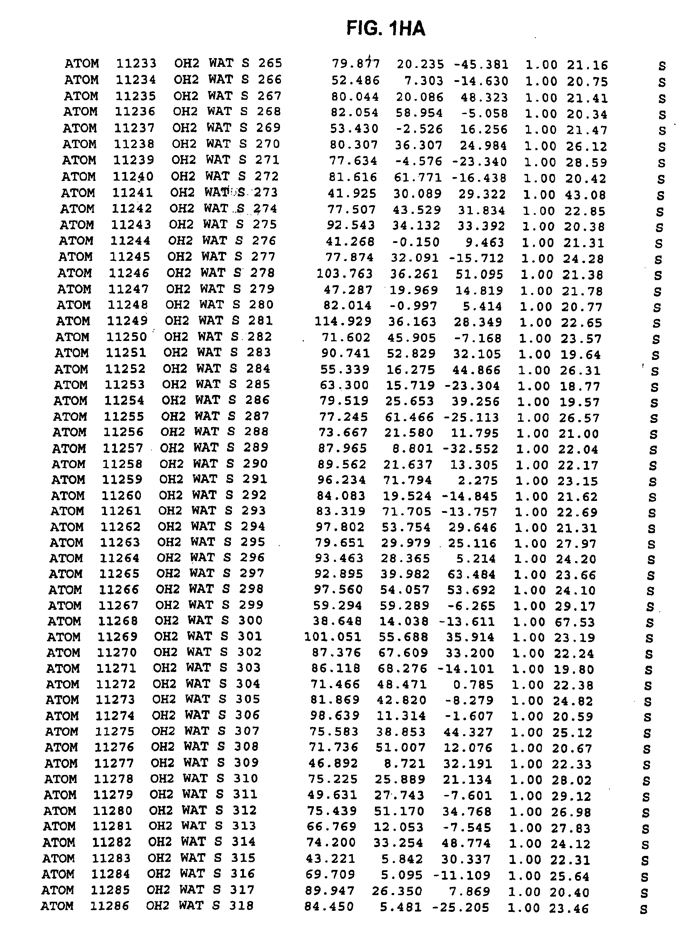

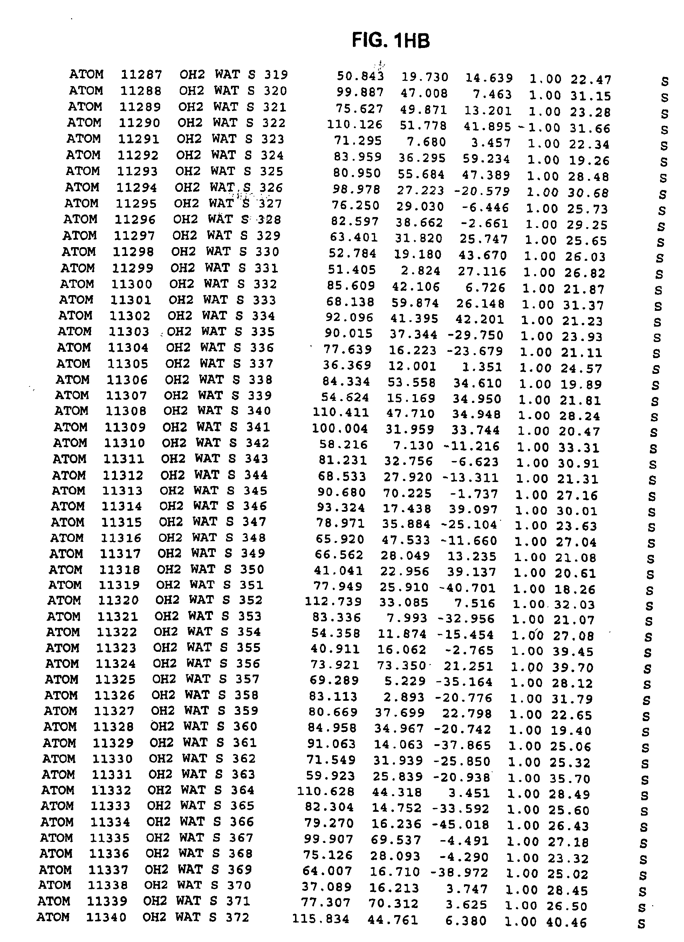

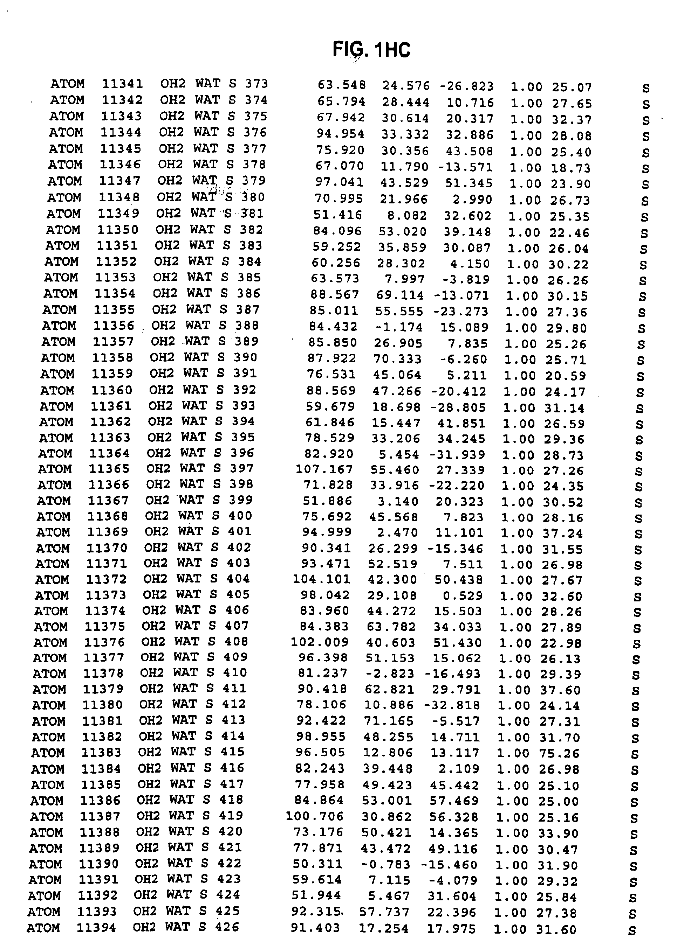

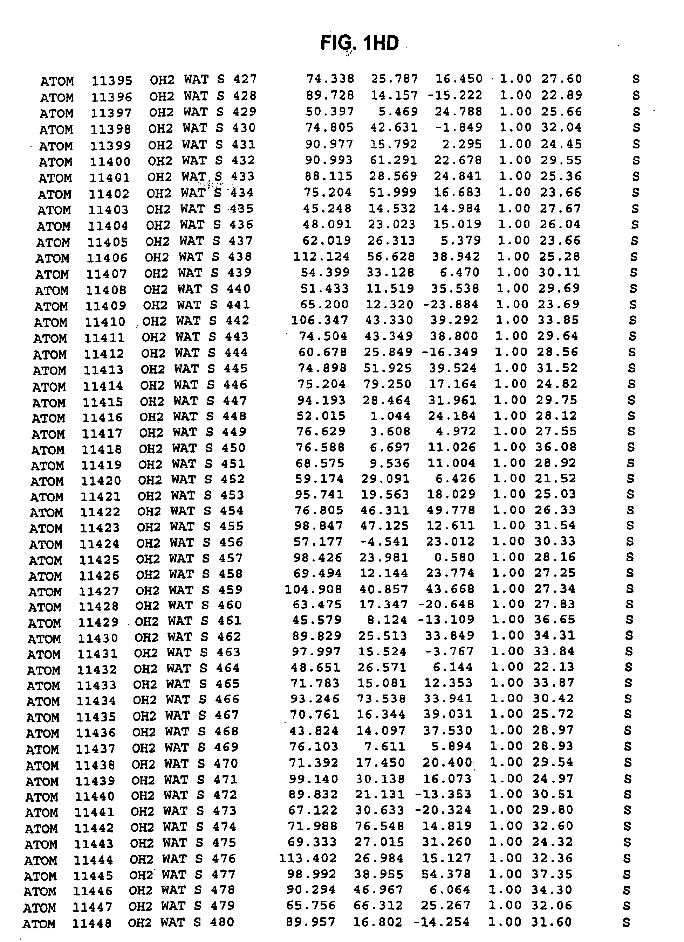

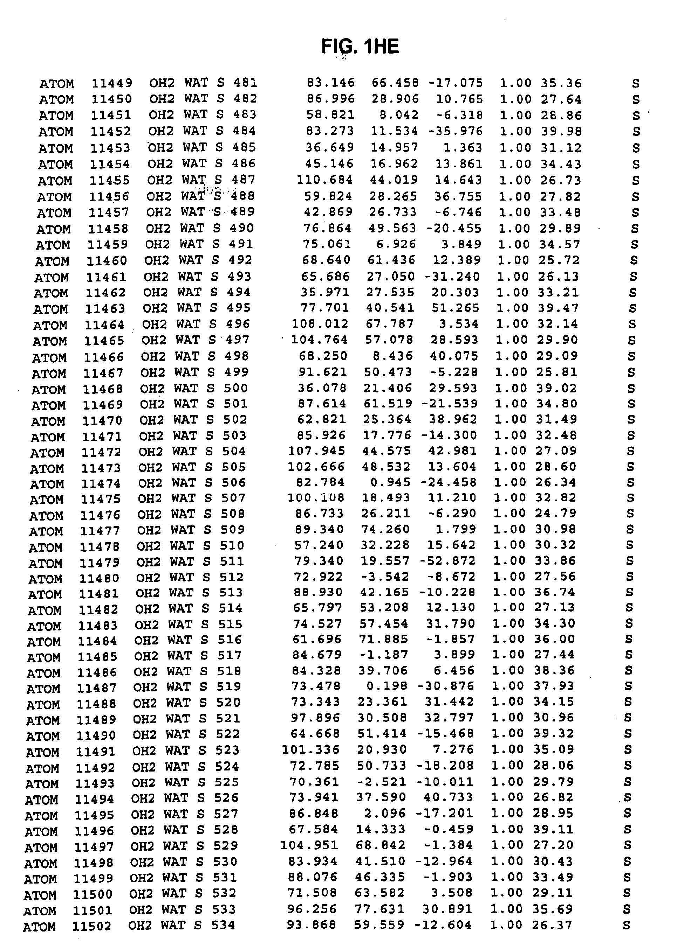

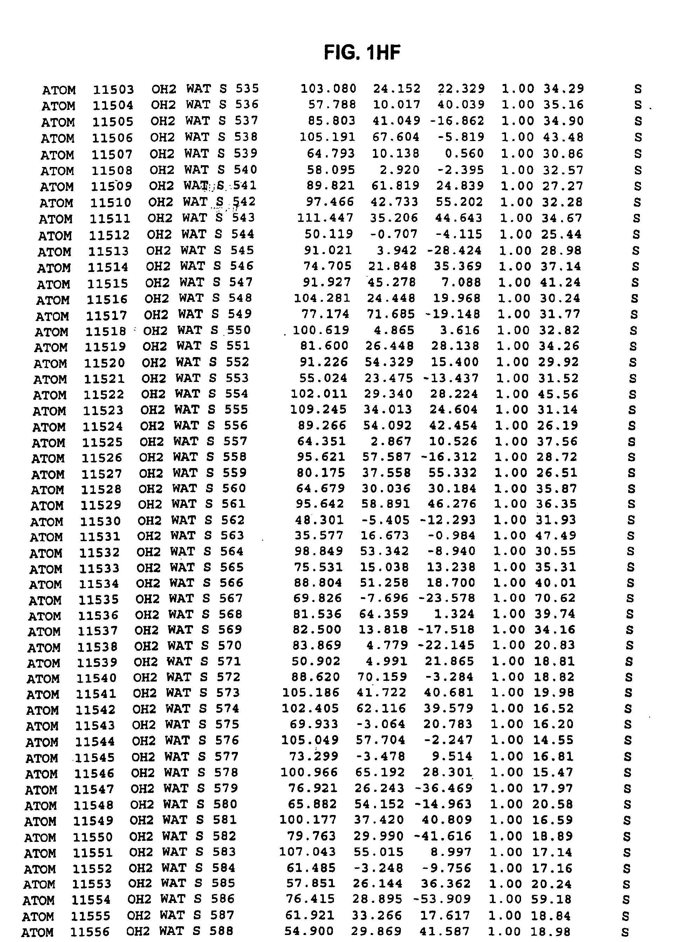

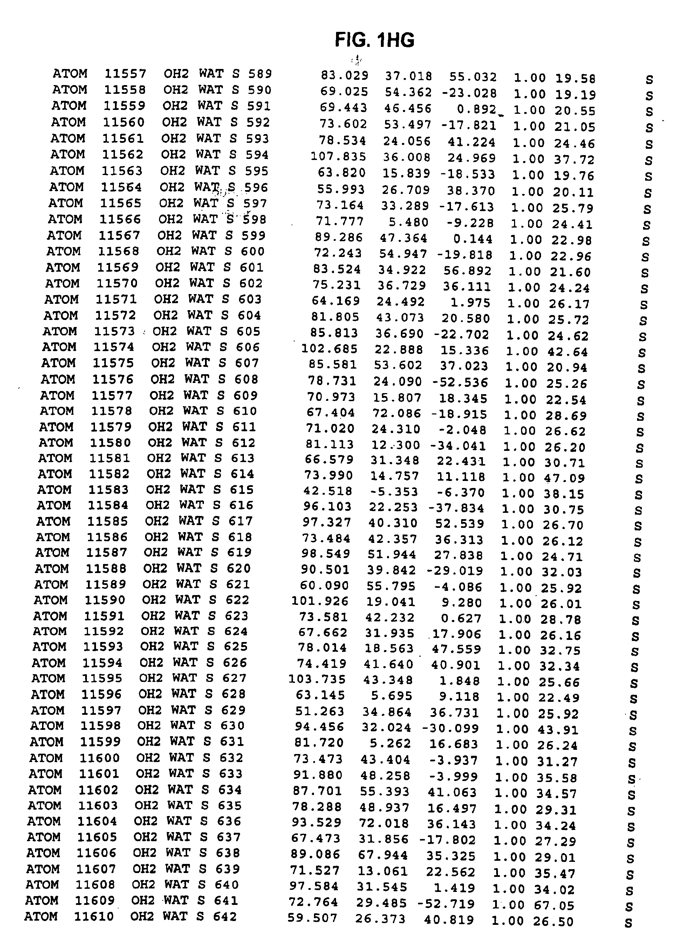

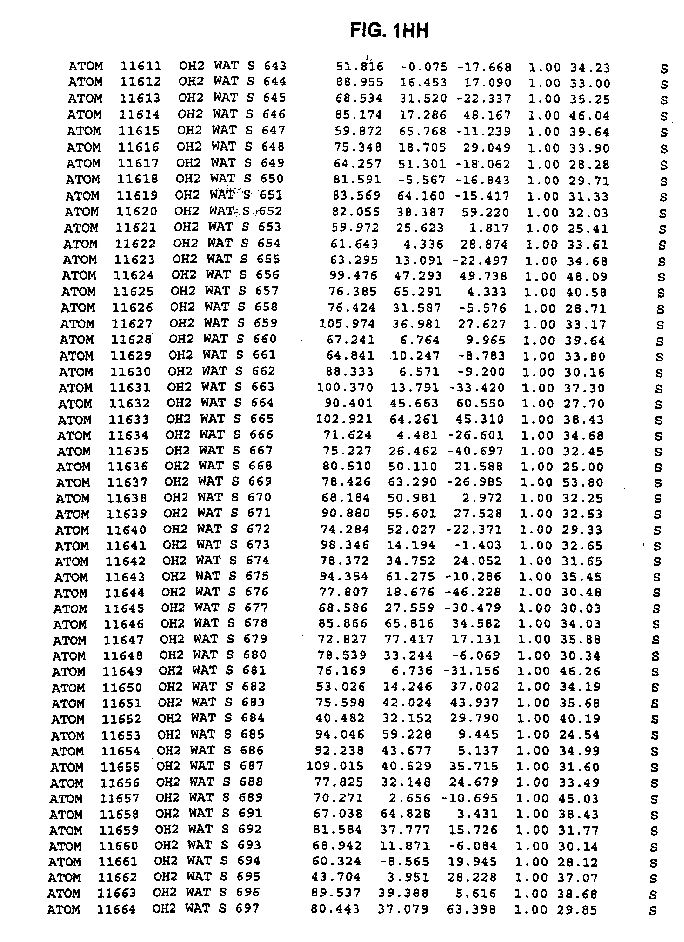

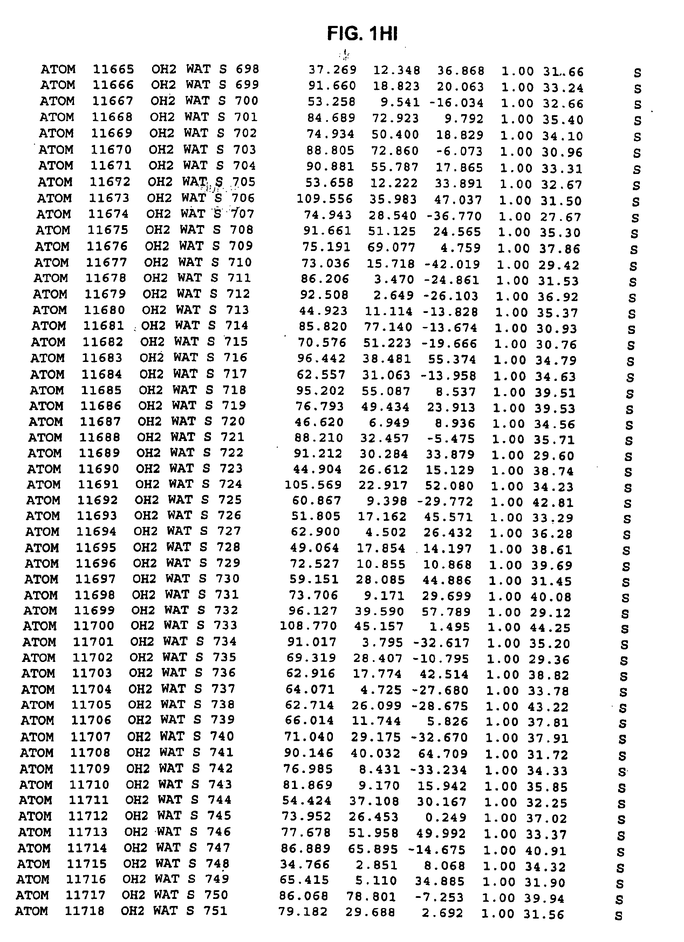

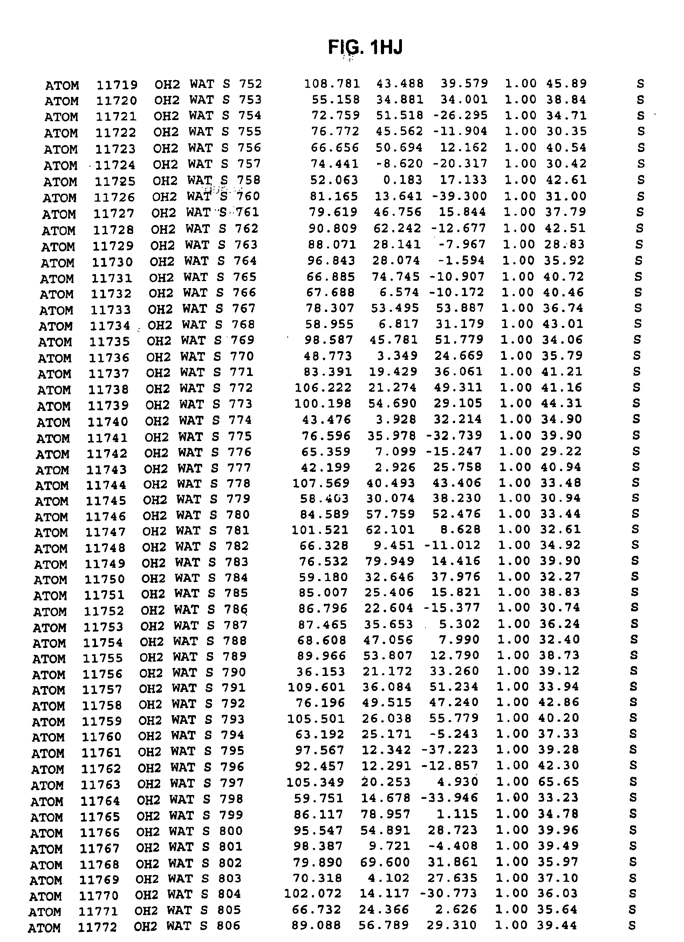

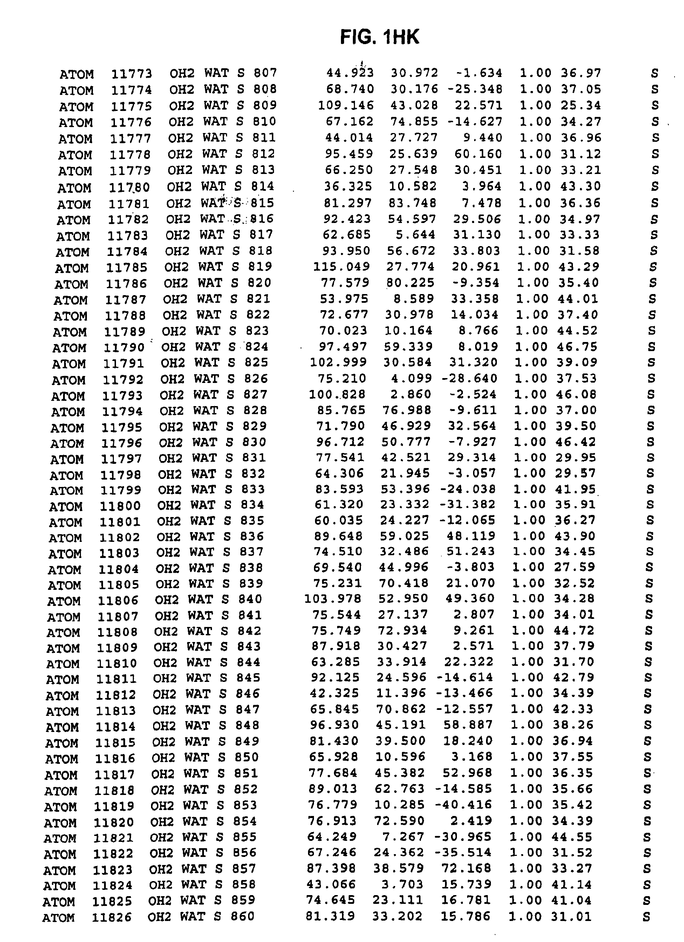

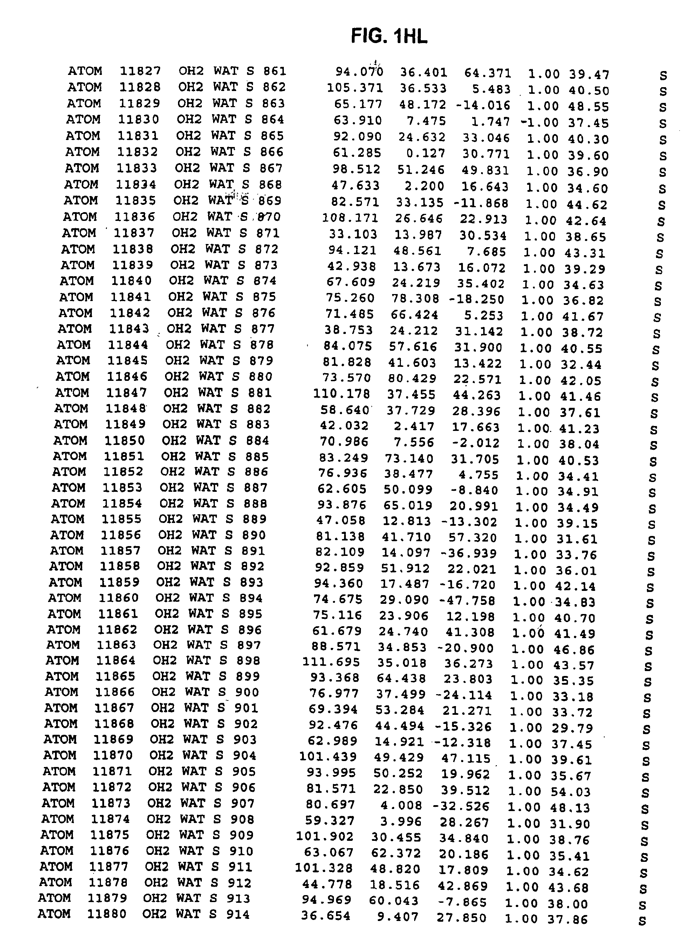

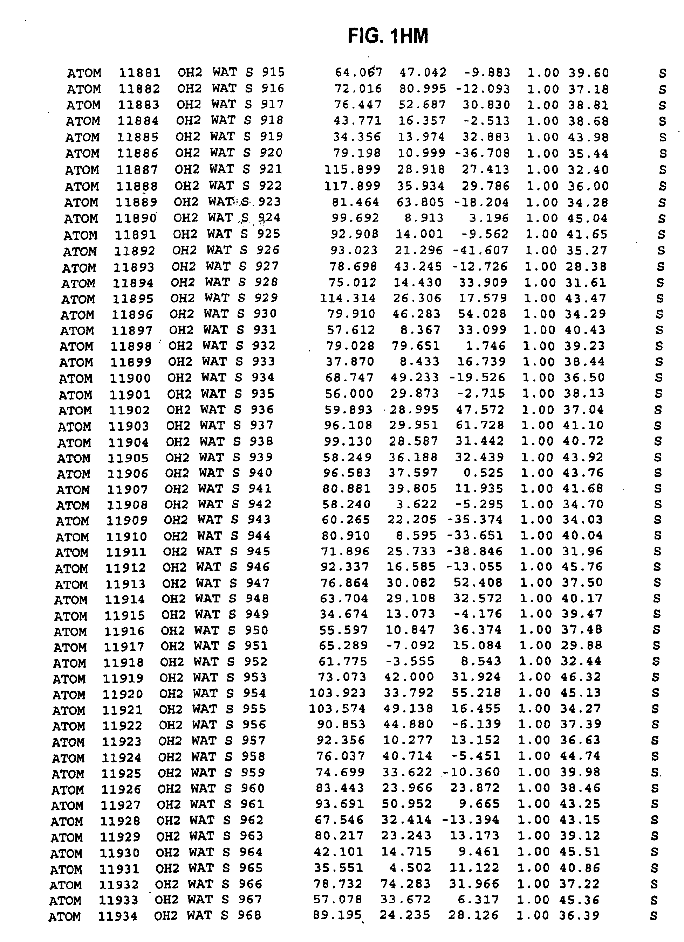

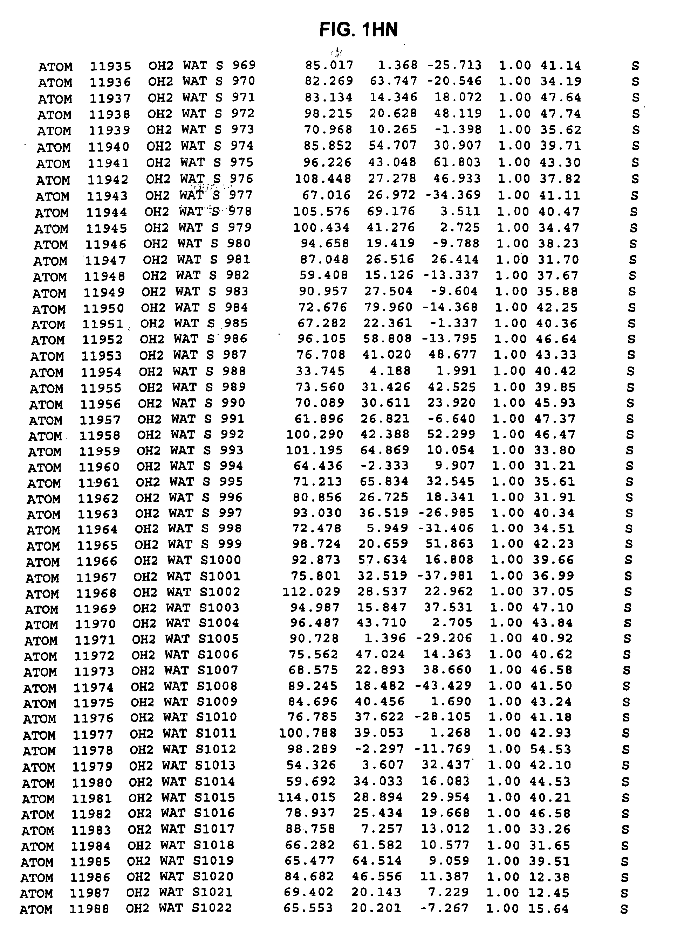

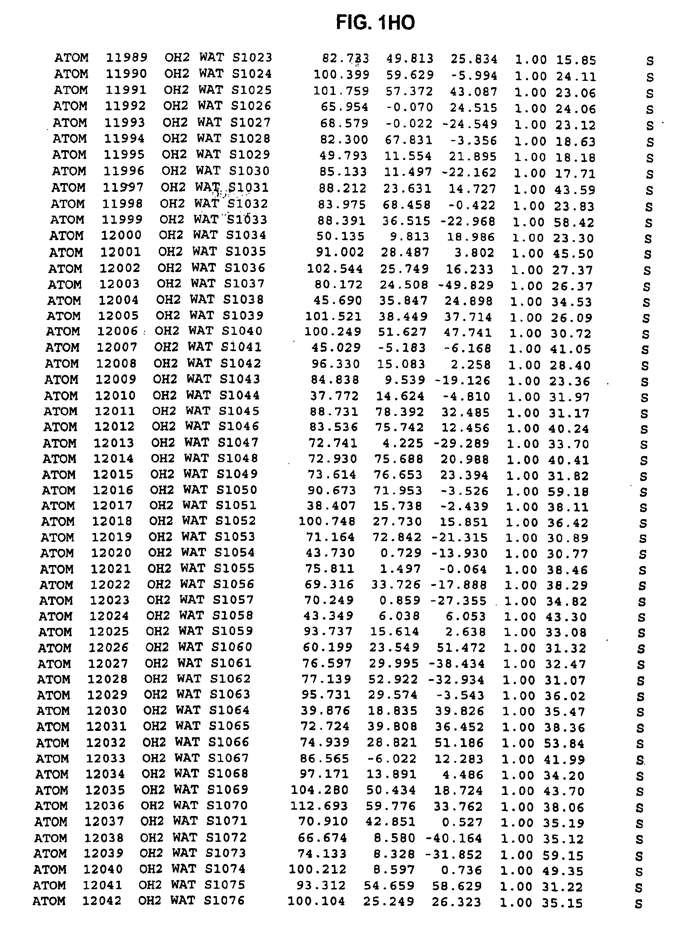

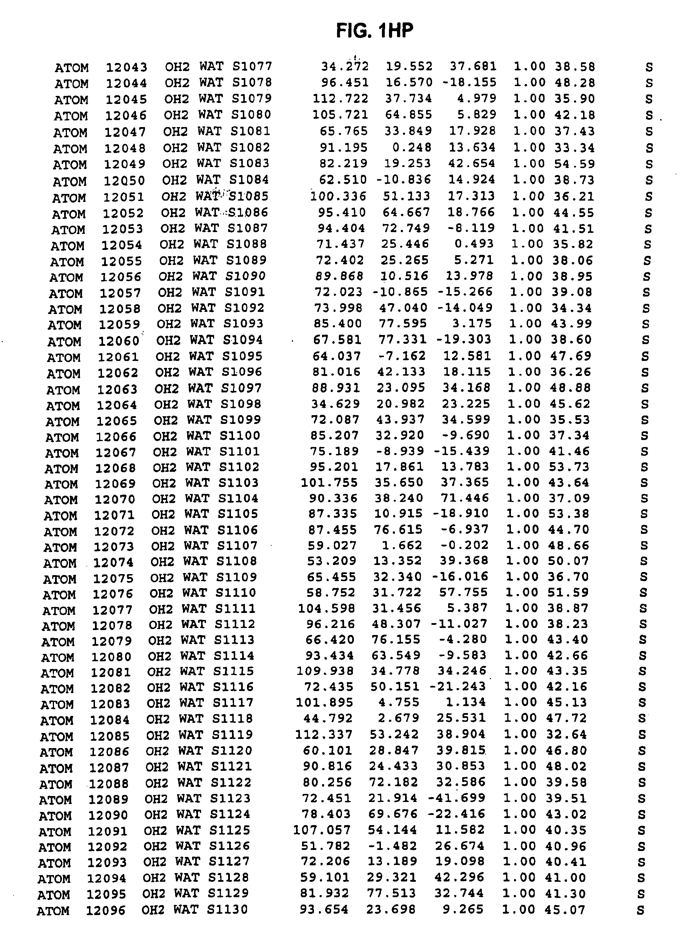

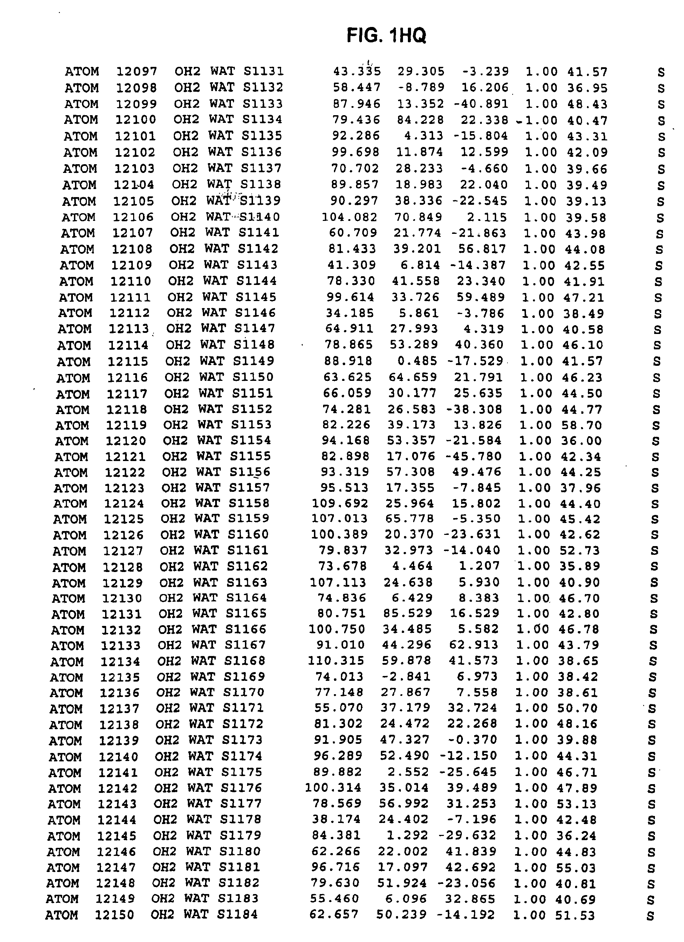

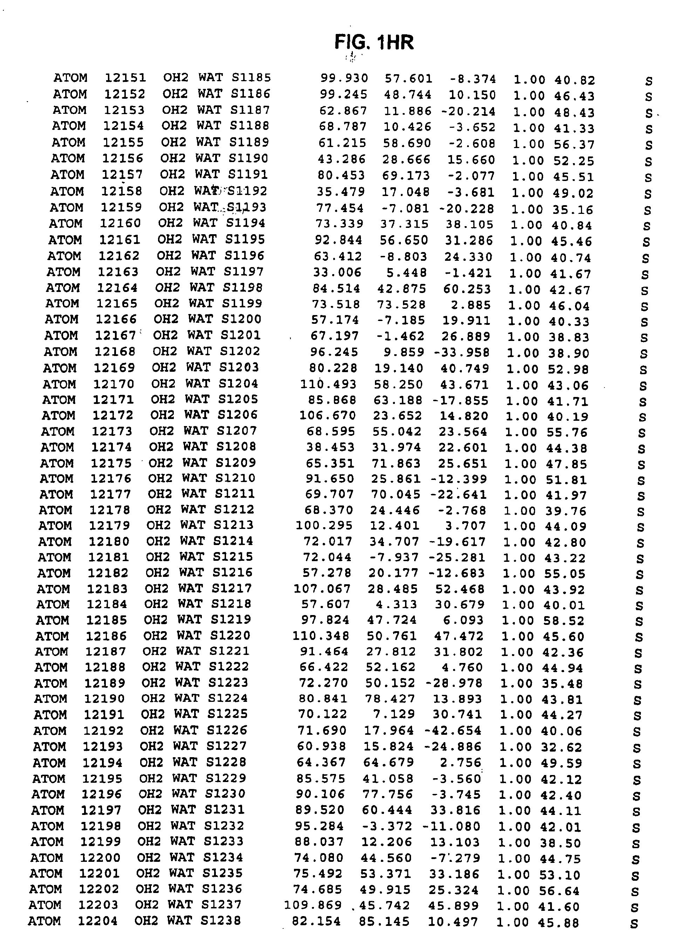

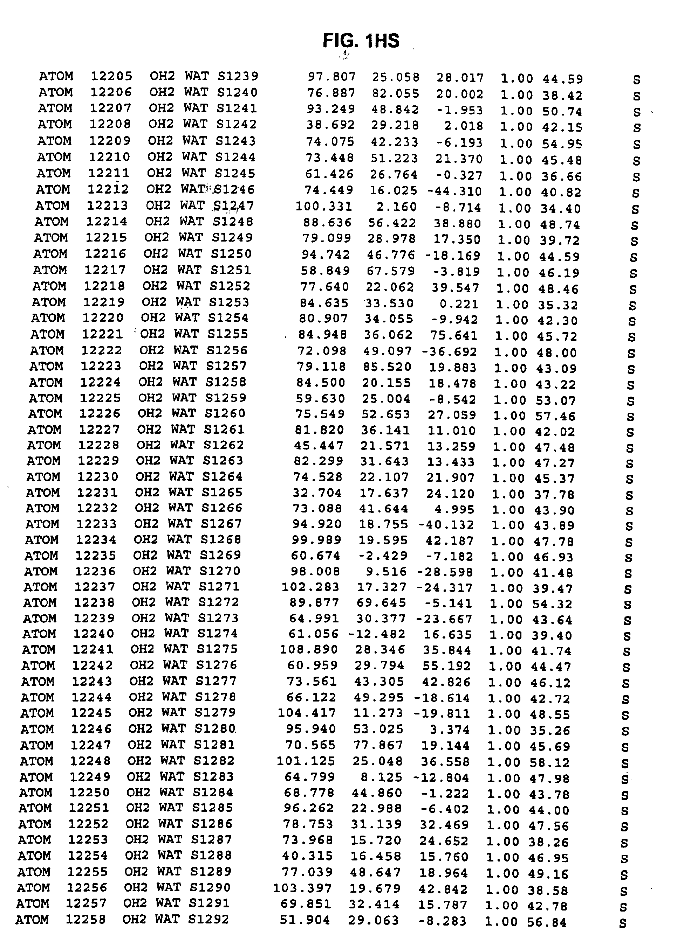

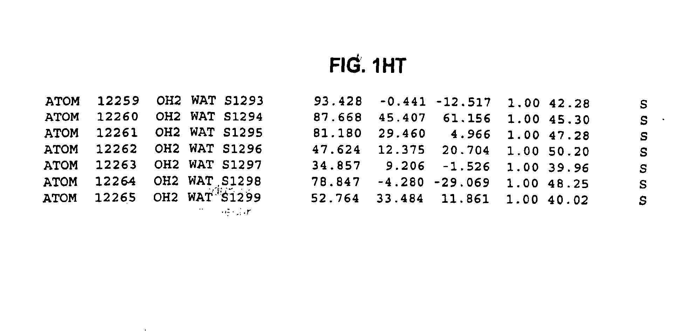

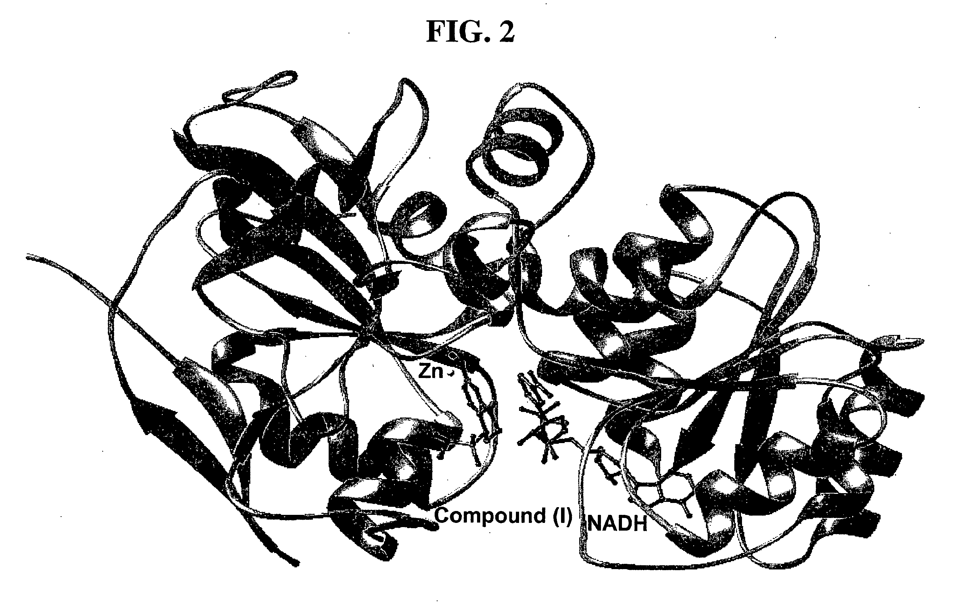

In another embodiment of the present invention, a three dimensional structure of hSDH is provided that comprises atomic coordinates as given in Figure 1 and any three-dimensional structure thereof having an overall fold as illustrated in Figure 2 and is of a protein containing at least about 80% of the amino acids in SEQ. ID. NO. 1. The atomic coordinates of hSDH, or a fragment, analog, or variant thereof may be used to model a hSDH protein, or a hSDH-related protein. A model may also be developed based on a binding site identified herein.

In yet another embodiment of the present invention, a catalytic site of hSDH is provided comprising the cofactor NADH, a catalytic zinc atom, and hSDH amino acid residues or water molecules located within 10 angstroms of a hSDH ligand as given in Table 4.

In still another embodiment of the present invention, a binding site for a hSDH ligand is provided comprising hSDH amino acid residues or water molecules which form either hydrogen bonds or van der Waals contacts to said ligand as defined in Table 2 or Table 3. The binding site may further comprise the cofactor NADH and a catalytic zinc atom.

Another aspect of the present invention is a computer readable medium having stored thereon atomic coordinate/X-ray diffraction data defining the three dimensional structure of a temary complex of hSDH, NADH, and a ligand that binds to hSDH, or a fragment, analog, or variant thereof.

In another aspect of the present invention, a computer medium is provided having stored thereon the computer model output data defining the three dimensional structure of a ternary complex of hSDH, NADH, and a ligand that binds to hSDH or a subunit thereof. The computer model is derived from analysis of atomic coordinate/X-ray diffraction data defining the three dimensional structure of a ternary complex of hSDH, NADH, and a ligand that binds to hSDH or a subunit thereof.

In yet another embodiment of the present invention, a method is provided for identifying a ligand of hSDH or a subunit thereof comprising the steps of: (I) providing a computer readable medium having stored thereon computer model output data defining the three dimensional structure of a ternary complex of hSDH, NADH, and a ligand that binds to hSDH or a subunit thereof; (2) providing a computer readable medium having stored thereon computer model output data defining the three dimensional structure of a potential ligand that binds to hSDH or a subunit thereof; (3) providing a computer system comprising a computer and a computer algorithm where the computer system is capable of processing the computer model and the output data of steps (1) and (2); and (4) processing the computer model output data of steps (1) and (2) using the computer system of step (3) wherein the processing calculates the ability of the potential ligand to bind to hSDH or a subunit thereof. The method may further comprise the steps of (5) incorporating a test compound of the potential ligand in a biological hSDH activity assay; and (6) determining whether the test compound inhibits enzymatic activity in the assay.

### Definitions

As used herein, the term "Compound (I)" refers to *N,N*-dimethyl-4-(2-hydroxymethyl-pyrimidin-4-yl)-piperazine-1-sulfonamide having the following structural formula:

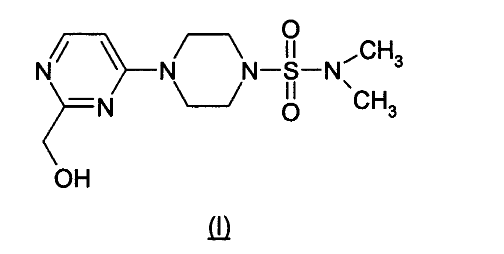

The term "computer-based system" or "computer system" refers to the hardware means, software means, and data storage means used to analyze the amino acid sequence and/or atomic coordinate/x-ray diffraction data of the present invention.

The term "computer readable medium" or "computer readable media" refers to any available media which can be accessed by a computer. For example, computer readable media can comprise RAM, ROM, EEPROM, CD-ROM or other optical disk storage (read only or rewritable), magnetic disk storage or other magnetic storage devices, or any other medium which can be used to store the amino acid sequence and/or atomic coordinate/x-ray diffraction data of the present invention and which can be accessed by a computer.

### BRIEF DESCRIPTION OF THE FIGURES

Figure 1 presents the atomic coordinates derived from X-ray diffraction data (atomic coordinate/X-ray diffraction data) defining the three dimensional structure of a ternary complex of hSDH complexed with NADH and a SDH ligand (i.e., Compound (I) as defined above).
Figure 2 presents the overall fold of one subunit with Compound (I) and NADH bound.
Figure 3 shows the residues forming either hydrogen bonds or van der Waals contacts to Compound (I).
Figure 4 shows the stacking interaction between Compound (I) and NADH.
Figure 5 shows the ligands of the protein and Compound (I) which coordinate the catalytic zinc ion.

### DETAILED DESCRIPTION

The present invention provides methods for expressing, purifying and crystallizing hSDH to form a crystal which diffracts x-rays with sufficiently high resolution to allow determination of the three-dimensional structure of the hSDH, or a portion or subdomain thereof. The three-dimensional structure is useful for rational design of ligands of hSDH. The crystals may be used for determining the binding mode of potential ligands (e.g., inhibitors) by soaking the compounds into the crystals and then determining the structure of the complex.

In one aspect of the present invention, a novel crystalline ternary complex of hSDH complexed with NADH and a SDH ligand (including a novel SDH active site) is provided as well as methods for using the crystalline form and active site to identify potential hSDH ligands.

### The human SDH Three-Dimensional Crystal Structure:

Atomic coordinates of a novel crystalline ternary complex of SDH complexed with NADH and Compound (I) generated from x-ray diffraction are provided in Figure 1. Human SDH functions biologically as a tetramer with 357 residues (38 kDa) per subunit. Crystals of hSDH were prepared by forming a complex of the protein with NADH and Compound (I) prior to setting up the crystallization trials. In the crystal structure, each subunit contains one catalytic zinc atom, one molecule of the required cofactor, NADH, and one molecule of Compound (I).

The structure reveals the binding mode of NADH and of the inhibitor (Compound (I)). Compound (I) is bound directly to the catalytic zinc ion, through a hydroxyl group and the nitrogen atom N3. The position of Compound (I) is also stabilized by a stacking interaction with the nicotinimide ring of NADH, bound to hSDH.

### Methods for identifying Potential SDH ligands:

Another aspect of the present invention is the use of the crystalline form and active site to identify potential SDH ligands. The atomic coordinate/x-ray diffraction data may be used to create a physical model which can then be used to design molecular models of compounds that may interact with the determined active sites, binding sites or other structural or functional domains or subdomains of the hSDH. Alternatively, the atomic coordinate/x-ray diffraction data of the ternary complex may be represented as atomic model output data on computer readable media which can be used in a computer modeling system to calculate different molecules expected to interact with the determined active sites, binding sites, or other structural or functional domains or subdomains of the hSDH. For example, computer analysis of the data allows one to calculate the three-dimensional interaction of the hSDH and the ligand to confirm that the ligand binds to, or changes the conformation of, particular domain(s) or subdomain(s) of the hSDH. The binding mode of the required cofactor NADH and the nature of its interactions with the ligand can also be evaluated. Ligands identified from the analysis of the physical or computer model can then be synthesized and tested for biological activity;with an appropriate screen.

The atomic coordinate/x-ray diffraction data of the present invention are generally provided on computer readable media. A skilled artisan can then access the data and analyze it for structure determination and/or rational ligand (e.g., inhibitor) design using a computer-based system. A typical computer system includes hardware means, software means, and data storage means. The hardware means typically includes a central processing unit (CPU), input means, output means and data storage means. One skilled in the art will readily appreciate which of the currently available computer-based systems are suitable for use in the practice of the present invention.

A variety of commercially available software programs are available for conducting the analysis and comparison of data in the computer-based system. One skilled in the art will readily recognize which of the available algorithms or implementing software packages for conducting computer analyses can be utilized or adapted for use in the computer-based system. As used herein, "a target structural motif' or "target motif' refers to any rationally selected sequence or combination of sequences in which the sequence(s) are chosen based on a three-dimensional configuration or electron density map which is formed upon the folding of the target motif. There are a variety of target motifs known in the art. Protein target motifs include, but are not limited to, enzymatic active sites, structural subdomains, epitopes, functional domains and signal sequences. A variety of structural formats for the input and output means can be used to input and output the information in the computer-based systems of the present invention.

A variety of comparing means can be used to compare a target sequence or target motif with the data storage means to identify structural motifs or interpret electron density maps derived in part from the atomic coordinate/x-ray diffraction data. One skilled in the art can readily recognize any one of the publicly available computer modeling programs that can be used.

Suitable software that can be used to view, analyze, design, and/or model a protein include Alchemy™, LabVision™, Sybyl™, Molcadd™, Leapfrog™, Matchmaker™, Genefold™ and Sitel™ (available from Tripos Inc., St. Louis, MO.); Quanta™, Cerius2™, X-Plor™, CNS™, Catalyst™, Modeller™, ChemX™, Ludi™, Insight™, Discover™, Cameleon™ and Iditis™ (available from Accelrys Inc., Princeton NJ); Rasmol™ (available from Glaxo Research and Development, Greenford, Middlesex, U.K.); MOE™ (available from Chemical Computing Group, Montreal, Quebec, Canada); Maestro™ (available from Shrödinger Inc.,); Midas/MidasPlus™ (available from UCSF, San Francisco, CA); VRML (webviewer- freeware on the internet); Chime (MDL - freeware on the intemet); MOIL (available from University of Illinois, Urbana-Champaign, IL); MacroModel™ and GRASP™ (available from Columbia University, New York, NY); Ribbon™ (available from University of Alabama, Tuscaloosa, Alabama); NAOMI™ (available from Oxford University, Oxford, UK); Explorer Eyechem™ (available from Silicon Graphics Inc., Mountain View, CA.); Univision™ (available from Cray Research Inc., Seattle WA); Molscript™ and O (available from Uppsala University, Uppsala, Sweden); Chem 3D™ and Protein Expert™ (available from Cambridge Scientific); Chain™ (available from Baylor College of Medicine, Houston, TX); Spartan™, MacSpartan™ and Titan™ (available from Wavefunction Inc., Irvine, CA); VMD™ (available from U. Illinois/Beckman Institute); Sculpt™ (available from Interactive Simulations, Inc., Portland, OR.); Procheck™ (available from Brookhaven National Laboratory, Upton, New York); DGEOM (available from QCPE - Quantum Chemistry Program Exchange, Indiana University Bloomington, IN); RE_VIEW (available from Brunel University, London, UK); Xmol (available from Minnesota Supercomputing Center, University of Minnesota, Minneapolis, MN); Hyperchem™ (available from Hypercube, Inc., Gainesville, FL); MD Display (available from University of Washington, Seattle, WA); PKB (available from National Center for Biotechnology Information, NIH, Bethesda, MD); Molecular Discovery Programmes (available from Molecular Discovery Limited, Mayfair, London); Growmol™ (available from Thistlesoft, Morris Township, N.J); MICE (available from the San Diego Supercomputer Center. La Jolla, CA); Yummie and MCPro (available from Yale University, New Haven CT); and upgraded versions thereof.

### Inhibitors of SDH Activity:

The present invention also provides inhibitors of SDH activity identified or designed by the methods of the present invention. Once a potential ligand is identified from the computer analysis described above, then the ligand can be synthesized and tested for biological activity using an appropriate screening method.

Compounds that act as SDH inhibitors lower fructose levels in the tissues of mammals affected by diabetes (e.g., nerve, kidney and retina tissue) and are therefore useful in the treatment and/or prevention of diabetic complications such as diabetic neuropathy, diabetic retinopathy, diabetic nephropathy, diabetic microangiopathy and diabetic macroangiopathy in mammals. One method for testing SDH inhibitor activity involves the use of male Sprague-Dawley rats (200-400 g). Diabetes is induced in some of the rats by a tail vein injection of streptozocin, 85 mg/kg. Twenty-four hours later, groups of diabetic rats are given a single dose of the test compound (0.01 - 300 mg/kg) by oral gavage. Animals are sacrificed 4-6 hours after dosing and blood and sciatic nerves are harvested. Tissues and cells are extracted with 6% perchloric acid.

Sorbitol in erythrocytes and nerves is measured by a modification of the method developed by R. S. Clements, et al. (Science, **166**, 1007-8, (1969)). Aliquots of tissue extracts are added to an assay system which has final concentrations of reagents of 0.033M glycine, pH 9.4, 800 µM β-nicotine adenine dinucleotide, and 4 units/mL of sorbitol dehydrogenase. After incubation for 30 minutes at room temperature, sample fluorescence is determined on a fluorescence spectrophotometer with excitation at 366 nm and emission at 452 nm. After subtracting appropriate blanks, the amount of sorbitol in each sample is determined from a linear regression of sorbitol standards processed in the same manner as the tissue extracts.

Fructose is determined by a modification of the method described by M. Ameyama, Methods in Enzymology, **89**, 20-25 (1982). Resazurin is substituted for ferricyanide. Aliquots of tissue extracts are added to the assay system, which has final concentrations of reagents of 1.2M citric acid, pH 4.5, 13 µM resazurin, 3.3 units/mL of fructose dehydrogenase and 0.068% Triton X-100. After incubation for 60 minutes at room temperature, sample fluorescence is determined on a fluorescence spectrophotometer with excitation at 560 nm and emission at 580 nm. After subtracting appropriate blanks, the amount of fructose in each sample is determined from a linear regression of fructose standards processed in the same manner as the tissue extracts.

SDH activity is measured by a modification of the method described by U. Gerlach, *Methodology of Enzymatic Analyses*, edited by H. U. Bergmeyer, **3**, 112-117 (1983). Aliquots of sera or urine are added to the assay system, which has final concentrations of reagents of 0.1M potassium phosphate buffer, pH 7.4, 5 mM NADH, 20 mM sorbitol, and 0.7 units/mL of sorbitol dehydrogenase. After incubation for 10 minutes at room temperature, the average change in sample absorbance is determined at 340 nm. SDH activity is generally presented as milliOD₃₄₀ units/minute (OD₃₄₀ = optical density at 340 nm).

Other useful methods for evaluating SDH inhibitor activity are described in Geisen, K., et al., Arzneim.-Forsch./Drug Res., **44**(11), 1032-1043 (1994). The methods described therein include *in vivo* studies (e.g., evaluations of blood and organ sampling, sorbitol excretion in the urine, motor nerve conduction velocity, glomerular filtration rate and cataract development); *in vitro* incubation and perfusion studies (e.g., evaluations of sorbitol accumulation in erythocytes and perfusion of the liver); histological techniques (e.g., evaluation of retinal capillaries); biochemical and analytical assays (e.g., enzymatic substrate assays, GLC assays, and HPLC assays). Those skilled in the art will know how to evaluate inhibitor activity using one or more of the several methods known in the art or a modification thereof.

Compounds identified by the methods of the present invention may be used to treat and/or prevent diabetic complications such as diabetic neuropathy, diabetic retinopathy, diabetic nephropathy, diabetic microangiopathy and diabetic macroangiopathy in mammals. The SDH inhibitor is preferably administered as a pharmaceutical composition.

The active compounds and compositions may be administered to a subject in need of treatment by a variety of conventional routes of administration, including orally, parenterally and topically. In general, SDH inhibitors and their pharmaceutically acceptable salts will be administered orally or parenterally at dosages between about 0.01 and about 50 mg/kg body weight of the subject to be treated per day, preferably from about 0.1 to 15 mg/kg, in single or divided doses. However, some variation in dosage will necessarily occur depending on the condition of the subject being treated. The person responsible for administration will, in any event, determine the appropriate dose for the individual subject.

The active compounds and compositions may be administered alone or in combination with pharmaceutically acceptable carriers, in either single or multiple doses. Suitable pharmaceutical carriers include inert solid diluents or fillers, sterile aqueous solutions and various organic solvents. The pharmaceutical compositions formed by combining the active compounds and the pharmaceutically acceptable carriers are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the active compounds and compositions in sesame or peanut oil, aqueous propylene glycol, or in sterile aqueous solutions may be employed. Such aqueous solutions should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, the sterile aqueous media employed are all readily available by standard techniques known to those skilled in the art.

The active compounds and compositions may be employed in the preparation of ophthalmic solutions. Such ophthalmic solutions are of principal interest for the treatment of diabetic cataracts by topical administration. For the treatment of diabetic cataracts, the active compounds and compositions are administered to the eye in the form of an ophthalmic preparation prepared in accordance with conventional pharmaceutical practice.

The following examples illustrate various aspects of the present invention and are not intended to limit the scope of the invention as defined by the claims.

### EXAMPLES

Compound I was prepared using the general procedures disclosed in U.S. Patent No. 5,215,990. Unless designated otherwise, reagents used in the following examples are generally available from a variety of vendors who supply chemical reagents such as Fisher Scientific Products (Hanover Park, IL).

Sequence data for the hSDH gene (SEQ. ID. NO. 1: Accession No. U07361) is available from Entrez Protein, National Center for Biotechnology Information, National Library of Medicine, Bethesda, MD. To generate the protein for hSDH, the gene was recloned from a human liver cDNA library and inserted into an expression vector. The expression vector was re-sequenced to confirm the correctness of the DNA insert. Some natural variation in the sequence of human SDH (on the order of 10-20% at the amino acid level) is expected. Such variants may or may not have functional significance and are expected to have the same or similar three-dimensional structures to the crystal structure described here.

### Cloning and expression of human SDH:

A pET vector system was used for expression of human SDH (hSDH). The hSDH gene previously identified in a human liver cDNA library was used as a template for polymerase chain reaction (PCR) amplification. (See, Iwata, T., et al., "Structural Organization of the Human Sorbitol Dehydrogenase Gene (SORD)", *Genomics,* **26**, 55-62 (1995).) Two primers were designed to amplify the DNA fragment coding for hSDH. The 5' primer (5'-GGAATTCCATATGGCGGCGGCGGCCAAGCCC-3') (SEQ. ID.NO.2) introduced a unique *Nd*eI site while the 3' primer (5'-GGCCGCTCGAGCTATTAGGGATTCTGGTCACTGGG-3') (SEQ.ID.NO.3) introduced a unique Xhol site preceded by TAA and TAG stop codons. PCR amplification was carried out under standard conditions using Vent polymerase (available from New England Biolabs Inc., Beverly, MA) and the human liver cDNA library as a template. The PCR-amplified product was digested with *Ndel* and *Xho*l, gel purified and ligated into pET23a (available from Novagen, Madison, WI). The ligation mixture was transformed into *E. coli* DH5α cells and plated on LB+agar plates containing ampicillin (100 µg/mL). A few colonies were picked, grown in LB+ ampicillin and used for plasmid preparation. These recombinant plasmids were subjected to restriction digestion and PCR analysis to confirm the presence of the insert. The DNA sequence of the 1071 base pair insert in pET23a was confirmed (identical to the human SDH sequence GenBank Accession No. U07361, SEQ. ID. NO. 1)) on both strands using a 373A Automated fluorescent sequencer (available from Applied Biosystems, Foster City, CA). The gene coded for a 357-amino acid protein. One of the positive clones, hSDH/pET23a 1/3, was transformed into *E.coli* BL21 (DE3) cells and used for expression experiments.

### Cell growth and initial expression:

*E. coli* BL21 (DE3) cells harboring hSDH/pET23a 1/3 were grown at 37°C in LB medium supplemented with ampicillin (100 µg/mL) to an A₆₀₀ = 0.7. The cells were induced with 0.4 mM IPTG for 4 hours at 26°C and harvested by centrifugation. The cell paste was either used for experiments or stored frozen at -20°C until needed. When expressed in LB+ ampicillin medium, the protein was expressed minimally even under non-inducing conditions. Although the protein was expressed in higher quantities when induced at 37°C, incubation at 26°C gave higher soluble expression and overall yield.

### Growth of selenomethionine substituted hSDH:

A glycerol stock of *E. coli* BL21 (DE3) cells harboring hSDH/pET23a 1/3 were cultured overnight at 37°C with shaking at 300 rpm in 2x YT media (16 g bacto-tryptone, 10 g bacto-yeast extract, 5 g NaCI and 900 mL distilled water) containing 100 µg/mL carbenicillin. Plasmid DNA was prepared from these cells using the Qiagen Prep Spin procedure (see, Qiagen product literature). The purified plasmid was transformed into B834(DE3) cells from Novagen Inc. (Madison, WI) and used for expression in selenomethionine media.

The selenomethionine (SeMet) media contained 10 g/L (NH₄)₂SO₄, 2.16 g/L Na₂HPO₄, 1.28 g/L KH₂PO₄, 5 g/L NaCI, 0.4 g/L trisodium citrate (Sigma), 0.2 g/L MgSO₄-7H₂O, 2 mg/L thiamine, 10 g/L glycerol, 0.1 g/L carbenicillin, 40 mg/L of all L-amino acids except methionine, 100 mg/L D, L-selenomethionine, 10 mg/L CaCl₂, 4 mg/L H₃BO₃, 1.71 mg/L MnSO₄-H₂O, 2 mg/L ZnSO₄-7H₂O, 0.373 mg/L CuSO₄-5H₂O, 0.4 mg/L CoCl₂-6H₂O, 0.2 mg/L Na₂MoO₄-2H₂O. The pH of the media was adjusted to 7.0. Fernbach tribaffled flasks (2.8 liter) were used for growth of a 500 mL culture. A 500 mL culture of hSDH clone in B834(DE3) in SeMet Media was inoculated from an overnight culture grown at 30°C in the same media. The culture was grown at 30°C and 300 rpm until the O.D.₆₀₀ reached 0.5 to 1.0. At this point, the cells were induced with IPTG to a final concentration of 1.0 mM and growth was continued for another 24 hours at room temperature. The cells were harvested by centrifugation and stored at -80°C until use.

### Purification of recombinant human sorbitol dehydrogenase from E.coli cells:

The method was modified from a published procedure (Maret and Auld, *Biochemistry*, **27**, 1622-1628 (1988)). *E. coli* BL21(DE3) pET 23a/hSDH cells (40-50 grams) were resuspended with two volumes of lysis buffer (20 mM 4-(2-hydroxyethyl)-1-piperazineethanesulfonic acid (HEPES) /NaOH pH 8, 5 µM ZnCl₂, 2 mM dithiothreitol (DTT), 1 mM phenylmethyl sulfonyl fluoride (PMSF), and 20 µg/mL aprotinin). Two volumes of lysis buffer containing 2 mg/mL lysozyme were added and the mixture stirred on ice for 60 minutes. The cells were sonicated for two periods of 3 minutes in 30 mL aliquots, while in an ice bath, using a Branson™ 450 sonifier (available from Branson Ultrasonics Corp., Danbury, CT) set to a 50% duty cycle, power setting #6. The sonicated cells were centrifuged at 12,000 rpm for 10 minutes at 4°C in a Sorvall SS-34 rotor. The supernatant was decanted and the pellet discarded.

To fractionate the supernatant, ammonium sulfate was added to a final concentration of 40% (equivalent to 226 mg NH₄⁺SO₄⁻/mL supernatant). The solution was incubated on ice for 30 minutes, and then centrifuged at 12,000 rpm for 10 minutes in GSA rotor at 4°C. The supernatant was removed and ammonium sulfate was added to a final saturation of 60% (equal to 135 mg/mL of the original supernatant). The mixture was incubated on ice for 30 minutes, and then centrifuged as before.

The 40-60% ammonium sulfate pellet was dissolved with 100 mL 20 mM Hepes/NaOH, pH 8.0, 5 µM ZnCl₂, 2 mM DTT, 1 mM PMSF and 1 µg/mL each of aprotinin, pepstatin and leupeptin. The sample was dialyzed overnight at 4°C against four liters of the same buffer.

### Green A chromatography:

A Green A dye column (Amicon™ type XK-50, 100 ml bed volume; available from Millipore Corporation, Bedford, MA) was equilibrated with 800 ml Buffer A (20 mM Hepes/NaOH pH 8, 5 µM ZnCl₂, and 2 mM DTT). The 40-60% ammonium sulfate cut was loaded onto the column at a rate of 5 mL/minute. This load contained about 2000 mg protein, of which approximately 50 - 200 mg was hSDH protein. The column was eluted with Buffer B (Buffer A plus 1.0 M NaCI) at 5 mL/minute as follows: 0% B for 100 min (5 column volumes), 0-100% B for 200 min (10 column volumes), 100% B for 40 min (2 column volumes). Four minute fractions were collected and assayed for activity. Two peaks of activity were identified; a major peak which eluted between 15-30% B and a minor peak which eluted between 30-40% B. Each peak was pooled separately. The major peak was concentrated to about 50 mL in an Amicon™ cell using a YM-30 membrane (available from Millipore Corporation, Bedford, MA) and dialyzed overnight at 4°C against 2 liters of Buffer A.

### Mono S chromatography:

Half of the dialyzed material was loaded at a rate of 2 mL/minute onto a Mono S column (10/10; available from Pharmacia) equilibrated in Buffer A at 4°C. The column was eluted as follows: 0% B for 20 minutes, a gradient of 0-15% B for 120 minutes, a gradient of 15-100% B for 20 minutes followed by 100% B for 10 minutes. Two minute fractions were collected. The second half of the dialyzed green A column peak was then chromatographed in the same way. Material prepared in this way was at least 95% pure by silver-stained SDS-PAGE and had a specific activity between 8-10 U in the assay described below. Electrospray mass spectrometry indicated a major peak centered at 38178 and a lesser peak centered at 38112 mass units (expected mass 38202). N-terminal sequence analysis indicated AAAAKPNNLSLV with approximately 29% of the protein missing the first alanine.

### Activity Assay:

An aliquot of each fraction (with an average concentration of 0.1-0.2 mg/mL) was diluted 20-fold in a solution containing 20 mM Hepes/NaOH, pH 8 and 200 mM NaCI. The reaction was initiated by adding 5-10 microliters of this diluted sample to a reaction mixture consisting of 1 mM NADH, 50 mM sorbitol, 0.1 M glycine/NaOH, pH 10, at 25°C. The absorbance of the sample was read at 340 nm. The expected absorbance of hSDH at 280 nM (OD₂₈₀ 0.1%) is 0.600. The specific activity of the enzyme is defined as micromoles of NADH produced per minute per milligram at 25°C and were calculated as follows: µM/min/mg = [(ΔOD340)(1000 µM/mM)(0.001 L)]/[(6.22 )(mM/L)⁻¹(min)(mg SDH in assay)].

### Purification of selenomethionine substituted hSDH:

Selenomethionine substituted hSDH was purified in the same way as the wild type protein. Electrospray mass spectrometry indicated a major peak centered at 38565 and a lesser peak centered at 38492 mass units (expected mass for 7 selenomethionines and 1 Zn²⁺ is 38534). Two purified lots of selenomethionine substituted SDH had two masses; 38565 and 38492. These are +11 and +9 of the predicted masses of 38554 (des Met) and 38483 (des Met Ala) forms with all 8 methionines substituted with selenomethionine. The selenomethionine substituted protein was assayed using the same method as wild type protein and the activity was determined to be 11.6 +/-0.9 Units/mg.

### Crystallization of hSDH protein:

Both the wild type and selenomethionine substituted proteins were concentrated to 2.0 mg/mL in a 20 mM Hepes/NaOH, pH 7.8 containing 100 mM NaCI, 2 mM DTT, 0.1 mM NADH and 0.2 mM of Compound (I). Crystals were grown in hanging drops by vapor diffusion at 22 °C. The well solution consisted of 150 mM NH₄OAc, 100 mM sodium citrate, pH 6.15, 30% PEG 4000 and 2.5 mM DTT. Six microliters of protein solution were mixed with 3 microliters of well solution. Under these conditions, large (0.3 x 1.0 mm) crystals appeared in 4 to 8 days. The crystals belong to space group P6₂ with unit cell constants a = b = 134.42 angstroms and c = 224.52 angstroms. The crystal can be described by the parameters given in Table 1, Part (a) below.

### Stabilization of the crystals prior to freezing:

Prior to data measurement, the crystals were stabilized by stepwise transfer into a cryostabilization solution (CS) consisting of 30% PEG 4000 (polyethylene glycol), 37.5 mM NH₄OAc, 50 mM sodium citrate, pH 6.15, 8.75% glycerol, 0.07 mM NADH and 0.14 mM Compound (I). The crystals were first transferred to 10 microliters of well solution (described above) and 5 microliters of CS were added. Additional volumes of CS were added at 10 minute intervals as follows; 3 steps of 5 microliters, followed by 2 steps of 10 microliters. Finally, the crystal was transferred to 10 microliters of undiluted CS for 10 minutes, after which it was frozen either directly in a liquid nitrogen gas stream or in liquid propane.

### Data Measurement and Reduction:

The data were measured from a single crystal at 100 °K at beamline X12C at the National Synchrotron Light Source at the Brookhaven National Laboratory in Upton, New York. The X-ray absorption spectrum around the K edge of the anomalously scattering selenomethionine atoms was measured using a simple X-ray detector mounted at right angles to the X-ray beam and in the horizontal plane to capture fluorescence as an indirect measure of absorption. The inflection point (L1), peak (L2) and remote wavelength (L3, expected to be the Zn peak) were identified as 0.979148, 0.978712, and 1.249998 from the fluorescence scan. A complete three wavelength MAD dataset to a maximum resolution of 1.9 Å using inverse beam geometry was measured using the Brandeis™ B4 detector. See, Phillips, W.C., et al., "Multiple CCD detector for macromolecular X-ray crystallography", Journal of Applied Crystallography, **33**, 243-251 (2000). For each wavelength, a ten degree wedge of data was measured, after which phi was rotated by 180 degrees and the same wedge of data recollected to measure the Bijvoet pairs. Keeping the Bijvoet pairs and each wavelength separate, the data were reduced and scaled using Denzo and Scalepack. See, e.g., Otwinowski, Z. & Minor, W., "Processing of X-ray diffraction data collected in oscillation mode," Methods in Enzymology, **276,** 307-326 (1997). The data collection statistics for each wavelength are given in Table 1, Part b.

### Structure solution:

The structure was solved using the multiple anomalous diffraction method (MAD) as implemented in Crystallography and NMR System (CNS) beta release version. See, Brunger, A.T., et al., "Crystallography and NMR System; A New Software Suite for Macromolecular Structure Determination," Acta Crystallographica, **D54,** 905-921 (1998). There are 8 methionines in hSDH. Assuming that the N-terminal methionine is disordered, this leaves 7 selenomethionine sites per monomer. Assuming space group P6₂, four molecules of hSDH (the biologically relevant tetramer) in the asymmetric unit and a high Matthews coefficient of 3.8, we expected to find at least 28 selenomethionine sites.

The intensities derived from Scalepack© (available from HKL Research, Inc., Charlottesville, VA) for each wavelength were converted to separate CNS reflection files which were then merged together, and scaled using L1 (to which Wilson scaling had been applied) as the reference dataset.

Initial phasing was done in a higher symmetry space group P6_{2 or 4}22, which was chosen based on scaling statistics in Scalepack© and a systematic search for the *0,0,l* reflections in the native dataset. Based on SAD phasing statistics and map quality, space group P6₂22 was subsequently chosen. The structure was re-solved in the correct space group P6₂ as a result of the analysis described below. Because the initial coordinates were built into electron density maps phased in space group P6₂22, those phases were calculated as follows.

In space group P6₂22, 18 potential zinc or selenomethionine sites were identified using heavy atom search/Patterson correlation refinement procedure in CNS applied to L2 (peak) anomalous difference Patterson maps. The positions of these sites and the values for f and f" were further refined using maximum likelihood methods, sites with high B factors were rejected and Hendrickson-Lattman phase probability distributions calculated. The correctness of the sites were confirmed by calculating anomalous difference maps using the Hendrickson-Lattman coefficients and anomalous difference structure factors and comparing these to log-likelihood gradient maps. The MAD phases were improved by solvent flattening, histogram matching and density truncation. A new map was calculated using these improved Hendrickson-Lattman coefficients and structure factors from L1. The resultant map was of excellent quality and the protein sequence could be deduced directly from the map. As an initial model, the Cα trace of a theoretical model of sheep liver sorbitol dehydrogenase (Protein Data Bank entry 1SDG) was used, based on horse liver alcohol dehydrogenase (see, Eklund, H., et al., "Molecular Aspects of Functional Differences between Alcohol and Sorbitol Dehydrogenases," Biochemistry, **24**, 8005-8012 (1985)), NADH, Compound (I), the catalytic zinc and residues 4-356 of the protein (molecule 1) were positioned in the map unambiguously using the program *O*. For a description of program O, see, Jones, T.A., et al., "Improved methods for building protein models in electron density maps and the location of errors in these models," Acta Crystallographica, **A 47**, 110-119(1991).

The map revealed electron density of sufficient size to accommodate one additional molecule. However, this quality of the density was too poor to identify side chains unambiguously and efforts to place a second molecule in the asymmetric unit using molecular replacement methods did not yield a solution which packed properly. We therefore reconsidered whether the space group was correct.

The native Patterson map showed a large peak at 0, 0, 0.4 indicating that the asymmetric unit exhibited translational symmetry. Using the vector from this peak, we used a real space transform to translate molecule 1 by 0, 0, 93 angstroms, creating molecule 2. Molecules 1 and 2 were combined and examination of the unit cell in the program *O* in both space groups P6₂22 and P6₂ suggested that the correct space group was P6₂ and that the asymmetric unit contained two dimers belonging to two different tetramers.

Coordinate files corresponding to the two dimers were made and used to create a new file of the 28 selenomethionine and 4 zinc positions. A heavy atom search was recalculated in space group P6₂ using L2 (peak) anomalous differences and confirmed a subset of these solutions. Single anomalous difference phases to 3 angstroms using L2 and the 32 sites derived from the coordinates were calculated. A log-likelihood gradient map revealed 3 other peaks greater than 5 sigma which corresponded to partially occupied alternate conformations of selenomethionines 107 and 309 and revealed that residue 185 was a selenomethionine, rather than glutamic acid. Four sites corresponding to methionine 185 for each monomer were added to the site database for a total of 36 selenomethionine sites and 4 zinc sites. The phases were recalculated to 1.9 angstroms and subjected to density modification using a solvent content of 35%.

### Refinement of the Structure:

The structure was refined using 5% of the reflections as a test set. One molecule was manually refit in O and the other 3 generated by application of non-crystallographic symmetry. Standard protocols of rigid body refinement, overall B factor refinement of each monomer, grouped B factor refinement of main chain and side chain atoms, minimization, torsional dynamics, individual B factor refinement and automatic water picking, were used. Prior to torsional dynamics, f and f" were refined for selenium ("SE") and zinc ion ("ZN+2") and included in an anomalous library for all subsequent steps. The structure was refined using all data between 99.0 and 1.9 angstroms. The final refinement statistics are good: R_{free} = 23.0%, R factor = 21.5%, with root mean square deviations in bond lengths of 0.009 and bond angles of 1.51 degrees for four molecules of human SDH consisting of residues 1-356, 4 molecules of Compound (I), 4 molecules of NADH and 1299 water molecules. All residues lie within the allowed regions of the Ramachandran plot. Table 1, Part c shows the statistics of the refined structure of the SDH/NADH/Compound (I) complex. These statistics give the contents of the asymmetric unit of the crystal and provide the statistics required to evaluate the quality of the refined structure.

Table 1, Part (a) below provides the parameters of the crystals that were used to solve the structure. Table 1, Part (b) below provides the details and statistical analysis of the X-ray diffraction data measured. Table 1, Part (c) below provides the details of the final, refined structure and a statistical analysis of the correctness of the refined structure.

### Results:

The X-ray coordinates of the refined structure are listed in Figure 1, along with the B factors for each atom. The occupancy of each atom is fixed at 1:0. This coordinate set is generated by the last refinement cycle of the structure using the program CNS (Brunger, A. T. et al., (1998) Acta Crystallographica D54, 905-921). Figure 2 is a ribbon diagram showing the overall fold of one subunit of the biological tetramer of the enzyme human sorbitol dehydrogenase (SEQ. ID. NO.1) (made using Molscript; P. Kraulis (1991) J. Appl. Cryst., 24, 946-950) and the location of Compound (I), NADH, and the catalytic zinc atom. The protein is colored gray, the zinc atom is colored gray, Compound (I) and NADH are shown as ball and stick representations with carbon atoms in green, oxygen atoms in red, nitrogen atoms in blue, sulfur atom in yellow and phosphorus atom in purple.

The overall fold of one subunit of hSDH is similar to that of the alcohol dehydrogenases, consisting of two beta barrel domains with the active site located in between the two.

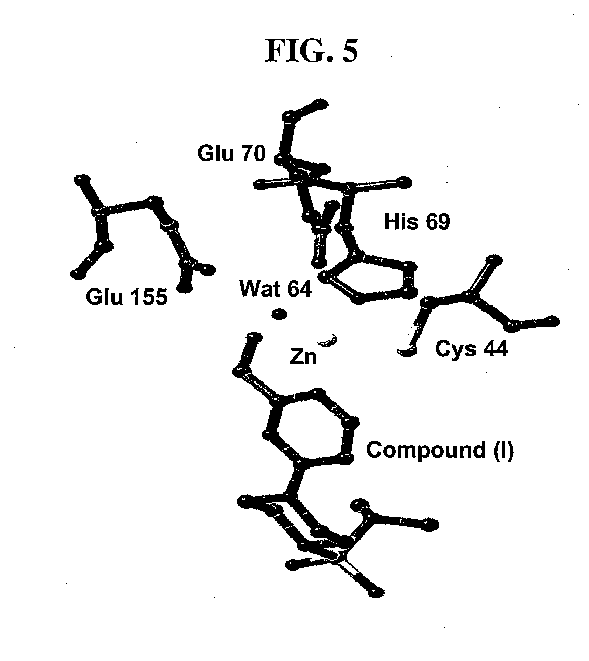

The catalytic site is marked by the presence of the zinc atom. The zinc atom is chelated by interactions with cysteine 44, histidine 69, a water molecule (Wat 64) and by two interactions with Compound (I). The specific contacts and the distances between the zinc atom and each chelating atom are given in Table 5 below. Figure 5 (made using Molscript; P. Kraulis (1991) J. Appl. Cryst., 24, 946-950; carbon atoms in green, nitrogen atoms in blue, oxygen atoms in red, sulfur atoms in yellow) shows the vicinity of the zinc atom and the relative positions of cysteine 44, histidine 69 and Wat 64. Water molecule 64 also forms hydrogen bonds to two other side chains of hSDH, glutamic acid 155 and glutamic acid 70. The hydrogen bonds formed between Wat 64, the zinc atom, and the two glutamic acid side chains serve to stabilize the position of this water molecule.

The structure also shows how Compound (I) chelates the catalytic zinc atom through interactions between nitrogen 3 of the pyrimidine ring and oxygen 30 of the hydroxymethyl group. Compound (I) sterically inhibits substrate binding and blocks access of the substrate to the catalytic zinc atom.

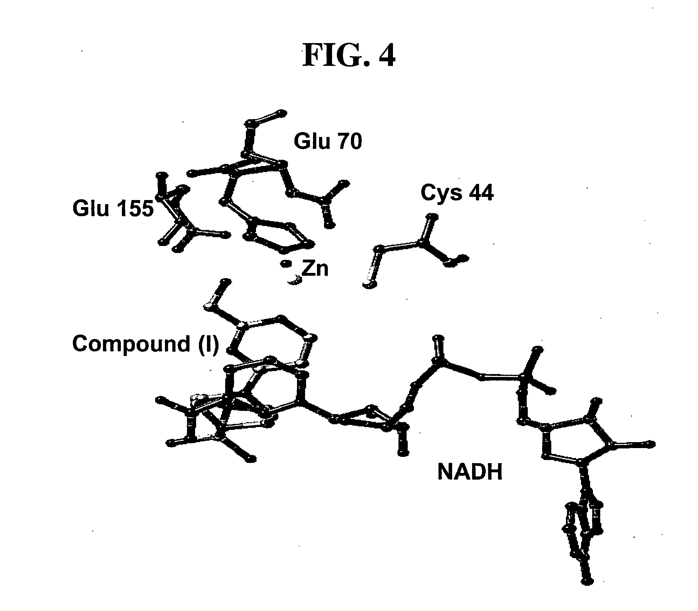

Compound (I) also interacts with the required cofactor, NADH as shown in Figure 4 (made using Molscript; P. Kraulis (1991) J. Appl. Cryst., 24, 946-950; carbon atoms in green, nitrogen atoms in blue, oxygen atoms in red, sulfur atoms in yellow, except for Compound (I) where the carbons are colored gold). The pyrimidine ring of Compound (I) stacks over the nicotinimide ring of NADH forming a tight hydrophobic contact.

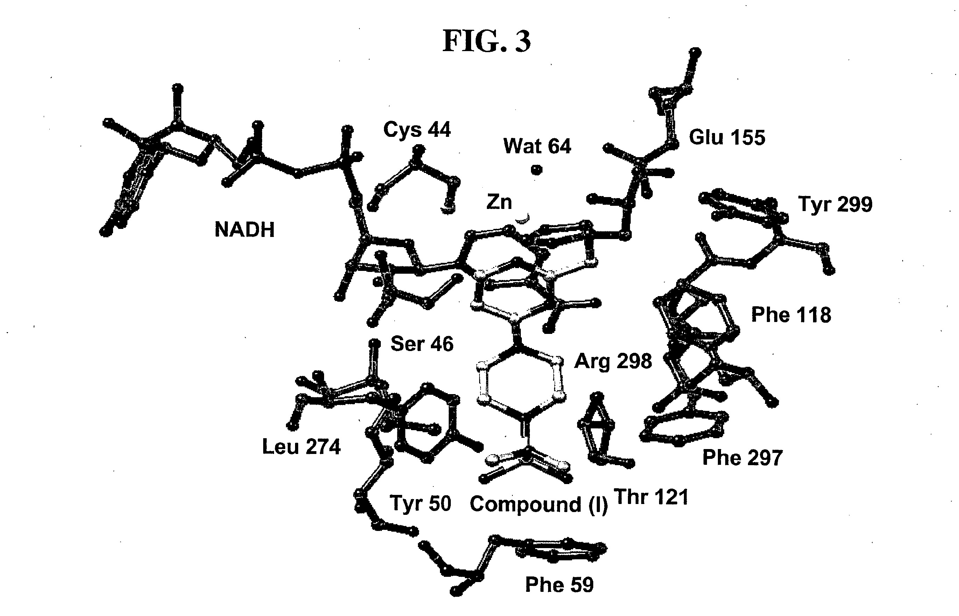

An overall view of the binding site of Compound (I) is shown in Figure 3. All residues of hSDH which form either hydrogen bonds or van der Waals contacts to Compound (I) are shown, in addition to NADH, Wat 64 and the catalytic zinc atom (made using Molscript; P. Kraulis (1991) J. Appl. Cryst., 24, 946-950; carbon atoms in green, nitrogen atoms in blue, oxygen atoms in red, sulfur atoms in yellow, except for Compound (I) where the carbons are colored gold). A list of the residues shown in Figure 3 is given in Table 3 below.

The binding site of the inhibitor (e.g., Compound (I)) may also be described as those residues of the protein within 10 angstroms of any atom of the compound. Table 4 below lists all residues of SDH which are within 10 angstroms of any atom of Compound (I).

**Table 1**

| **Crystallographic data and Refinement Statistics for SDH/NADH/Compound (I) Complex** | | | |
|---|---|---|---|
| **(a) Crystal Parameters** | | | |
| Space group | P6₂ | | |
| Cell constants*: | a = b = 134.814 Å, c = 225.184 Å | | |
| | α=β=90.0°,γ= 120.0° | | |
| Asymmetric unit: | biological tetramer | | |

| **(b) Data Collection Statistics** | | | |
|---|---|---|---|
| Wavelength (Å) | 0.97949 | 0.97977 | 0.96859 |
| No. of Unique Reflections | 84,713 | 85,068 | 86,799 |
| Fold Redundancy | 13 | 13 | 13 |
| Resolution (Å) | 30.0 - 1.9 | 30.0 - 1.9 | 30.0 - 1.9 |
| Last Shell (Å) | 1.93 - 1.9 | 1.93 - 1.9 | 1.93 - 1.9 |
| *Chi² | 1.55 (1.35) | 1.14 (1.01) | 1.30 (0.107) |
| *R_{merge} | 0.123 (0.691) | 0.101 (0.821) | 1.07 (0.0) |
| *I/error | 25.9 (2.3) | 24.6 (1.5) | 25.9 (1.4) |
| *%Completeness | 88.6 (46.0) | 88.6 (46.2) | 90.2 (52.6) |

| **(c) Refinement Statistics** | | | |
|---|---|---|---|
| Protein | 1 - 356 (4 molecules) | | |
| ligands | Compound (I) (4 molecules) | | |
| | NADH (4 molecules) | | |
| water molecules | 1299 | | |
| Resolution | 99.0 - 1.9 Å | | |
| Last Shell (Å) | 1.93 - 1.9 | | |
| R | 0.216 (0.300) | | |
| R_{free} | 0.228 (0.306) | | |
| rms bond | 0.013 Å | | |
| rms angles | 2.17° | | |

| | | | |
|---|---|---|---|
| * The cell constants a, b and c are within ± 15%. *last shell in () | | | |

Table 2 lists the hydrogen bonds formed between Compound (I) and the hSDH protein and the distances between the non-hydrogen atoms of the hydrogen bonds in the crystal structure.

**Table 2**

| **Hydrogen Bonds between SDH and Compound (I).** | | | |
|---|---|---|---|
| **Compound (I)** | **Residue** | **Atom** | **Distance (Å)** |
| O30 | Wat 64 | O | 2.98 |
| | Glu 155 | OE1 | 3.37 |
| | Glu 155 | OE2 | 2.54 |
| | His 69 | NE2 | 3.05 |
| N3 | Ser 46 | OG | 3.25 |

Table 3 lists those residues of hSDH forming van der Waals contacts to Compound (I) in the crystal structure. 'Van der Waals contacts" is defined herein as distances between non-hydrogen atoms from about 3.4 to about 4.5 angstroms.

**Table 3**

| **Van der Waals contacts between Compound (I) and SDH.** | |
|---|---|
| Cys | 44 |
| His | 46 |
| Tyr | 50 |
| lie | 56 |
| Phe | 59 |
| His | 69 |
| Phe | 118 |
| Thr | 121 |
| Glu | 155 |
| Leu | 274 |
| Phe | 297 |
| Arg | 298 |
| Tyr | 299 |

Table 4 lists those residues of hSDH within 10 angstroms of any atom of Compound (I) in the crystal structure. The binding site of Compound (I) may also be described as those residues of the protein within 10 angstroms of any atom of the inhibitor.

**Table 4**

| **Residues of hSDH within 10 Å of Compound (I)** |
|---|
| 42-50 |
| 55-60 |
| 64 |
| 68-70 |
| 93 |
| 95 |
| 111 |
| 114 |
| 118-122 |
| 154-160 |
| 183 |
| 272 |
| 274-275 |
| 296-299 |

Table 5 lists the ligands which coordinate the catalytic zinc atom of hSDH and the distances between such atoms and the zinc.

**Table 5**

| **Zn ligands** | | |
|---|---|---|
| **Residue** | **Atom** | **Distance (Å) to Zn** |
| Cys 44 | SG | 2.56 |
| His 69 | NE2 | 2.36 |
| Compound (I) | N3 | 2.15 |
| Compound (I) | 030 | 2.35 |
| Wat 64 | O | 2.0 |

SEQUENCE LISTING

<110> Pfizer Products Inc.
Mylari, Banavara L
Pauly, Thomas A
Rath, Virginia L

<120> Three dimensional Crystal Structure of Human Sorbitol Dehydrogenase and Uses Thereof

<130> PC11039AKM

<160> 1

<170> PatentIn version 3.1

<210> 1
<211> 357
<212> PRT
<213> Homo sapiens

<300>
<301> Lee, F.K., Cheung, M. C. and Chung, S.
<302> The human sorbitol dehydrogenase gene: cDNA cloning, sequence determination, and mapping by fluroescence in situ hybridization
<303> Genomics
<304> 21
<305> 2
<306> 354-358
<307> 1994-03-04
<308> GenBank/U07361
<309> 1995-05-11
<313> (1)..(357)

<400> 1

PC11039AKM-Sequence 1.ST25.txt

Met Ala Ala Ala Ala Lys Pro Asn Asn Leu Ser Leu Val Val His Gly
1               5                   10                  15

Pro Gly Asp Leu Arg Leu Glu Asn Tyr Pro Ile Pro Glu Pro Gly Pro
            20                  25                  30

Asn Glu Val Leu Leu Arg Met His Ser Val Gly Ile Cys Gly Ser Asp
        35                  40                  45

Val His Tyr Trp Glu Tyr Gly Arg Ile Gly Asn Phe Ile Val Lys Lys
    50                  55                  60

Pro Met Val Leu Gly His Glu Ala Ser Gly Thr Val Glu Lys Val Gly
65                  70                  75                  80

Ser Ser Val Lys His Leu Lys Pro Gly Asp Arg Val Ala Ile Glu Pro
                85                  90                  95

Gly Ala Pro Arg Glu Asn Asp Glu Phe Cys Lys Met Gly Arg Tyr Asn
            100                 105                 110

Leu Ser Pro Ser Ile Phe Phe Cys Ala Thr Pro Pro Asp Asp Gly Asn
        115                 120                 125

Leu Cys Arg Phe Tyr Lys His Asn Ala Ala Phe Cys Tyr Lys Leu Pro
    130                 135                 140

Asp Asn Val Thr Phe Glu Glu Gly Ala Leu Ile Glu Pro Leu Ser Val
145                 150                 155                 160

Gly Ile His Ala Cys Arg Arg Gly Gly Val Thr Leu Gly His Lys Val
                165                 170                 175

Leu Val Cys Gly Ala Gly Pro Ile Gly Met Val Thr Leu Leu Val Ala
            180                 185                 190

Lys Ala Met Gly Ala Ala Gln Val Val Val Thr Asp Leu Ser Ala Thr
        195                 200                 205

Arg Leu Ser Lys Ala Lys Glu Ile Gly Ala Asp Leu Val Leu Gln Ile
    210                 215                 220

Ser Lys Glu Ser Pro Gln Glu Ile Ala Arg Lys Val Glu Gly Gln Leu
225                 230                 235                 240

Gly Cys Lys Pro Glu Val Thr Ile Glu Cys Thr Gly Ala Glu Ala Ser
                245                 250                 255

Ile Gln Ala Gly Ile Tyr Ala Thr Arg Ser Gly Gly Thr Leu Val Leu
            260                 265                 270

PC11039AKM-Sequence 1.ST25.txt

Val Gly Leu Gly Ser Glu Met Thr Thr Val Pro Leu Leu His Ala Ala
        275                 280                 285

Ile Arg Glu Val Asp Ile Lys Gly Val Phe Arg Tyr Cys Asn Thr Trp
    290                 295                 300

Pro Val Ala Ile Ser Met Leu Ala Ser Lys Ser Val Asn Val Lys Pro
305                 310                 315                 320

Leu Val Thr His Arg Phe Pro Leu Glu Lys Ala Leu Glu Ala Phe Glu
                325                 330                 335

Thr Phe Lys Lys Gly Leu Gly Leu Lys Ile Met Leu Lys Cys Asp Pro
            340                 345                 350

Ser Asp Gln Asn Pro
        355

SEQUENCE LISTING

<110> Pfizer Products Inc.

Mylari, Banavara L.

Pauly, Thomas A

Rath, Virginia L.

<120> Three Dimensional Crystal Structure of Human Sorbitol Dehydrogenase and
Uses Thereof

<130> PC11039AKM

<160> 1

<170> PatentIn version 3.1

<210> 1

<211> 31

<212> DNA

<213> Homo Sapiens

<400> 1
ggaattccat atggcggcgg cggccaagcc c 31

SEQUENCE LISTING

<110> Pfizer Products Inc.

Mylari, Banavara L

Pauly, Thomas A

Rath, Virginia L

<120> Three Dimensional Crystal Structure of Human Sorbitol Dehydrogenase and
Uses Thereof

<130> PC11039AKM

<160> 1

<170> PatentIn version 3.1

<210> 1

<211> 35

<212> DNA

<213> Homo sapiens

<400> 1
ggccgctcga gctattaggg attctggtca ctggg 35

## Claims

**1.** A crystal form of human sorbitol dehydrogenase, or a subunit thereof, wherein said crystal form is defined as a crystal of space group P6₂ and cell constants having the values a equals 134.814 Å ± 15%, b equals 134.814 Å ± 15%, c equals 225.184 Å ± 15%, α equals 90.0°, β equals 90.0°, and γ equals 120.0°.

**2.** The crystal form of Claim 1 further comprising NADH, a zinc ion, a ligand, or a combination thereof that binds to said hSDH.

**3.** A three dimensional structure of hSDH comprising atomic coordinates as given in Figure 1 and any three-dimensional structure thereof having an overall fold as illustrated in Figure 2 and is of a protein containing at least about 80% of the amino acids in SEQ. ID. NO. 1.

**5.** A catalytic site of hSDH comprising cofactor NADH, a catalytic zinc atom, and hSDH amino acid residues or water molecules located within 10 angstroms of a hSDH ligand as given in Table 4.

**6.** A binding site for a hSDH ligand comprising hSDH amino acid residues or water molecules which form either hydrogen bonds or van der Waals contacts to said ligand as defined in Table 2 or Table 3.

**7.** The binding site of Claim 6 wherein said binding site further comprises cofactor NADH and a catalytic zinc atom.

**8.** A method for identifying a ligand of hSDH or a subunit thereof comprising the steps of:
(1) providing a first computer readable medium having stored thereon computer model output data defining the three dimensional structure of a crystalline ternary complex of hSDH, NADH, and a ligand that binds to said hSDH or a subunit thereof;
(2) providing a second computer readable medium having stored thereon computer model output data defining the three dimensional structure of a potential ligand that binds to said hSDH or a subunit thereof;
(3) providing a computer system comprising a computer and a computer algorithm where the computer system is capable of processing the computer model and the output data of steps (1) and (2); and
(4) processing the computer model output data of steps (1) and (2) using the computer system of step (3) wherein the processing calculates the ability of said potential ligand to bind to hSDH or a subunit thereof.

**9.** The method of Claim 8 further comprising the steps of
(5) incorporating a test compound of said potential ligand in a biological hSDH activity assay; and
(6) determining whether the test compound inhibits enzymatic activity in said assay.
